# EUROPEAN PATENT SPECIFICATION

(11) **EP 0 994 949 B1**
(45) Date of publication and mention of the grant of the patent: **09.03.2011**
(21) Application number: 98926055.9
(22) Date of filing: 22.05.1998
(51) Int. Cl.: C12N 15/45, C07K 14/115, C12N 5/10, C12N 7/01, A61K 39/155

(54) **PRODUCTION OF ATTENUATED PARAINFLUENZA VIRUS VACCINES FROM CLONED NUCLEOTIDE SEQUENCES**
HERSTELLUNG ATTENUIERTER PARAINFLUENZA-VIRUS IMPFSTOFFE AUS KLONIERTEN NUKLEOTIDSEQUENZEN
PRODUCTION DE VACCINS A VIRUS PARA-INFLUENZA ATTENUE A PARTIR DE SEQUENCES NUCLEOTIDIQUES CLONEES.

(30) Priority: 23.05.1997 US 47575 P; 19.09.1997 US 59385 P
(43) Date of publication of application: 26.04.2000
(73) Proprietor: THE UNITED STATES OF AMERICA, as represented by the Secretary of the Department of Health and Human Services, Bethesda, Maryland 20892 (US)
(72) Inventor: MURPHY, Brian, R., Bethesda, MD 20816 (US); COLLINS, Peter, L., Rockville, MD 20852 (US); DURBIN, Anna, P., Germantown, MD 20874 (US); SKIADOPOULOS, Mario, H., Potomac, MD 20854 (US); TAO, Tao, Bethesda, MD 20814 (US)
(74) Representative: Walton, Seán Malcolm
(86) International application number: PCT/US1998/010551
(87) International publication number: WO 1998/053078

(56) References cited:
- EP-A- 0 440 219
- EP-A- 0 702 085
- WO-A-97/11093
- WO-A-97/20468
- DIMOCK K. & COLLINS P.L.: "Rescue of synthetic analogs of genomic RNA and replicative intermediate RNA of human parainfluenza virus type 3" J. VIROL., vol. 67, no. 5, May 1993, pages 2772-2778, XP002078295 cited in the application
- STOKES A ET AL: "THE COMPLETE NUCLEOTIDE SEQUENCE OF TWO COLD-ADAPTED, TEMPERATURE-SENSITIVE ATTENUATED MUTANT VACCINE CIRUSES (CP12 AND CP45) DERIVED FROM THE JS STRAIN OF HUMAN PARAINFLUENZA VIRUS TYPE 3 (PIV3)" VIRUS RESEARCH, vol. 30, 1993, pages 43-52, XP002051711 cited in the application & DATABASE GENBANK Accession No. U51116, 6 March 1997 STOKES A.: "Human parainfluenza virus 3"
- STOKES A. ET AL.: "The complete nucleotide sequence of the JS strain of human parainfluenza virus type 3: comparison with the Wash/47885/57 prototype strain" VIRUS RESEARCH, vol. 25, 1992, pages 91-103, XP002078296 & DATABASE GENBANK Accession No. Z11575, 23 August 1993 STOKES A.: "Human parainfluenza virus type 3 (wt)"
- COLLINS P L ET AL: "PRODUCTION OF INFECTIOUS HUMAN RESPIRATORY SYNCYTIAL VIRUS FROM CLONED CDNA CONFIRMS AN ESSENTIAL ROLE FOR THE TRANSCRIPTION ELONGATION FACTOR FROM THE 5' PROXIMAL OPEN READING FRAME OF THE M2 MRNA IN GENE EXPRESSION AND PROVIDES A CAPABILITY FOR VACCINE DEVELOPMENT" PROCEEDINGS OF THE NATIONAL ACADEMY OF SCIENCES OF USA, vol. 92, December 1995, pages 11563-11567, XP002066592
- TAO T. ET AL.: "Recovery of a fully viable chimeric human parainfluenza virus (PIV) type 3 in which the hemagglutinin-Neuraminidase and Fusion glycoproteins have been replaced by those of PIV type 1" J. VIROL., vol. 72, no. 4, April 1998, pages 2955-2961, XP002078294
- SKIADOPOULOS M.H. ET AL.: "Three amino acid substitutions in the L protein of the human parainfluenzavirus type 3 cp45 live attenuated vaccine candidate contribute to its temperature-sensitive and attenuation phenotypes." J. VIROLOGY, vol. 72, no. 3, March 1998, pages 1762-1768, XP002078298
- DURBIN A.P. ET AL.: "Recovery of infectious human parainfluenza virus type 3 from cDNA" VIROLOGY, vol. 235, 1 September 1997, pages 323-332, XP002078299

### SEQUENCE LISTING

(1) GENERAL INFORMATION:
   (i) APPLICANT:
      (A) NAME: The Government of the United States
      (B) STREET: Box OTT
      (C) CITY: Bethesda
      (D) STATE: Maryland
      (E) COUNTRY: United States of America
      (F) POSTAL CODE (ZIP): 20892
      (G) TELEPHONE:
      (H) TELEFAX:
      (I) TELEX:
   (ii) TITLE OF INVENTION: PRODUCTION OF ATTENUATED PARAINFLUENZA VIRUS VACCINES FROM CLONED NUCLEOTIDE SEQUENCE
   (iii) NUMBER OF SEQUENCES: 74
   (iv) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (v) CURRENT APPLICATION DATA:
      (A) APPLICATION NUMBER: WO
      (B) FILING DATE: 22-MAY-1998
      (C) CLASSIFICATION:
   (vi) PRIOR APPLICATION DATA:
      (A) APPLICATION NUMBER: US 60/059,385
      (B) FILING DATE: 23-MAY-1997
   (vii) ATTORNEY/AGENT INFORMATION:
      (A) NAME: King, Jeffrey J.
      (B) REGISTRATION NUMBER: 38,515
      (C) REFERENCE/DOCKET NUMBER: 17634-000320PC
   (viii) TELECOMMUNICATION INFORMATION:
      (A) TELEPHONE: 206-467-9600
      (B) TELEFAX: 415-576-0300
(2) INFORMATION FOR SEQ ID NO:1:
   (i) SEQUENCE CHARACTERISTICS:
      (A) LENGTH: 15669 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:1:
   GCTGGGTACC GGGCCCGTCG ACGCGTATAT AGTTCCTCCT TTCAGCAAAA AACCCCTCAA      60
   GACCCGTTTA GAGGCCCCAA GGGGTTATGC TACTGCAGGC TCTCCCTTAG CCATCCGAGT     120
   GGACGTGCGT CCTCCTTCGG ATGCCCAGGT CGGACCGCGA GGAGGTGGAG ATGCCATGCC 180
   GACCCACCAA ACAAGAGAAG AAACTTGTCT GGGAATATAA ATTTAACTTT AAATTAACTT 240
   AGGATTAAAG ACATTGACTA GAAGGTCAAG AAAAGGGAAC TCTATAATTT CAAAAATGTT 300
   GAGCCTATTT GATACATTTA ATGCACGTAG GCAAGAAAAC ATAACAAAAT CAGCCGGTGG 360
   AGCTATCATT CCTGGACAGA AAAATACTGT CTCTATATTC GCCCTTGGAC CGACAATAAC 420
   TGATGATAAT GAGAAAATGA CATTAGCTCT TCTATTTCTA TCTCATTCAC TAGATAATGA 480
   GAAACAACAT GCACAAAGGG CAGGGTTCTT GGTGTCTTTA TTGTCAATGG CTTATGCCAA 540
   TCCAGAGCTC TACCTAACAA CAAATGGAAG TAATGCAGAT GTCAAGTATG TCATATACAT 600
   GATTGAGAAA GATCTAAAAC GGCAAAAGTA TGGAGGATTT GTGGTTAAGA CGAGAGAGAT 660
   GATATATGAA AAGACAACTG ATTGGATATT TGGAAGTGAC CTGGATTATG ATCAGGAAAC 720
   TATGTTGCAG AACGGCAGGA ACAATTCAAC AATTGAAGAC CTTGTCCACA CATTTGGGTA 780
   TCCATCATGT TTAGGAGCTC TTATAATACA GATCTGGATA GTTCTGGTCA AAGCTATCAC 840
   TAGTATCTCA GGGTTAAGAA AAGGCTTTTT CACCCGATTG GAAGCTTTCA GACAAGATGG 900
   AACAGTGCAG GCAGGGCTGG TATTGAGCGG TGACACAGTG GATCAGATTG GGTCAATCAT 960
   GCGGTCTCAA CAGAGCTTGG TAACTCTTAT GGTTGAAACA TTAATAACAA TGAATACCAG 1020
   CAGAAATGAC CTCACAACCA TAGAAAAGAA TATACAAATT GTTGGCAACT ACATAAGAGA 1080
   TGCAGGTCTC GCTTCATTCT TCAATACAAT CAGATATGGA ATTGAGACCA GAATGGCAGC 1140
   TTTGACTCTA TCCACTCTCA GACCAGATAT CAATAGATTA AAAGCTTTGA TGGAACTGTA 1200
   TTTATCAAAG GGACCACGCG CTCCTTTCAT CTGTATCCTC AGAGATCCTA TACATGGTGA 1260
   GTTCGCACCA GGCAACTATC CTGCCATATG GAGCTATGCA ATGGGGGTGG CAGTTGTACA 1320
   AAATAGAGCC ATGCAACAGT ATGTGACGGG AAGATCATAT CTAGACATTG ATATGTTCCA 1380
   GCTAGGACAA GCAGTAGCAC GTGATGCCGA AGCTCAAATG AGCTCAACAC TGGAAGATGA 1440
   ACTTGGAGTG ACACACGAAT CTAAAGAAAG CTTGAAGAGA CATATAAGGA ACATAAACAG 1500
   TTCAGAGACA TCTTTCCACA AACCGACAGG TGGATCAGCC ATAGAGATGG CAATAGATGA 1560
   AGAGCCAGAA CAATTCGAAC ATAGAGCAGA TCAAGAACAA AATGGAGAAC CTCAATCATC 1620
   CATAATTCAA TATGCCTGGG CAGAAGGAAA TAGAAGCGAT GATCAGACTG AGCAAGCTAC 1680
   AGAATCTGAC AATATCAAGA CCGAACAACA AAACATCAGA GACAGACTAA ACAAGAGACT 1740
   CAACGACAAG AAGAAACAAA GCAGTCAACC ACCCACTAAT CCCACAAACA GAACAAACCA 1800
   GGACGAAATA GATGATCTGT TTAACGCATT TGGAAGCAAC TAATCGAATC AACATTTTAA 1860
   TCTAAATCAA TAATAAATAA GAAAAACTTA GGATTAAAGA ATCCTATCAT ACCGGAATAT 1920
   AGGGTGGTAA ATTTAGAGTC TGCTTGAAAC TCAATCAATA GAGAGTTGAT GGAAAGCGAT 1980
   GCTAAAAACT ATCAAATCAT GGATTCTTGG GAAGAGGAAT CAAGAGATAA ATCAACTAAT 2040
   ATCTCCTCGG CCCTCAACAT CATTGAATTC ATACTCAGCA CCGACCCCCA AGAAGACTTA 2100
   TCGGAAAACG ACACAATCAA CACAAGAACC CAGCAACTCA GTGCCACCAT CTGTCAACCA 2160
   GAAATCAAAC CAACAGAAAC AAGTGAGAAA GATAGTGGAT CAACTGACAA AAATAGACAG 2220
   TCTGGGTCAT CACACGAATG TACAACAGAA GCAAAAGATA GAAATATTGA TCAGGAAACT 2280
   GTACAGAGAG GACCTGGGAG AAGAAGCAGC TCAGATAGTA GAGCTGAGAC TGTGGTCTCT 2340
   GGAGGAATCC CCAGAAGCAT CACAGATTCT AAAAATGGAA CCCAAAACAC GGAGGATATT 2400
   GATCTCAATG AAATTAGAAA GATGGATAAG GACTCTATTG AGGGGAAAAT GCGACAATCT 2460
   GCAAATGTTC CAAGCGAGAT ATCAGGAAGT GATGACATAT TTACAACAGA ACAAAGTAGA 2520
   AACAGTGATC ATGGAAGAAG CCTGGAATCT ATCAGTACAC CTGATACAAG ATCAATAAGT 2580
   GTTGTTACTG CTGCAACACC AGATGATGAA GAAGAAATAC TAATGAAAAA TAGTAGGACA 2640
   AAGAAAAGTT CTTCAACACA TCAAGAAGAT GACAAAAGAA TTAAAAAAGG GGGAAAAGGG 2700
   AAAGACTGGT TTAAGAAATC AAAAGATACC GACAACCAGA TACCAACATC AGACTACAGA 2760
   TCCACATCAA AAGGGCAGAA GAAAATCTCA AAGACAACAA CCACCAACAC CGACACAAAG 2820
   GGGCAAACAG AAATACAGAC AGAATCATCA GAAACACAAT CCTCATCATG GAATCTCATC 2880
   ATCGACAACA ACACCGACCG GAACGAACAG ACAAGCACAA CTCCTCCAAC AACAACTTCC 2940
   AGATCAACTT ATACAAAAGA ATCGATCCGA ACAAACTCTG AATCCAAACC CAAGACACAA 3000
   AAGACAAATG GAAAGGAAAG GAAGGATACA GAAGAGAGCA ATCGATTTAC AGAGAGGGCA 3060
   ATTACTCTAT TGCAGAATCT TGGTGTAATT CAATCCACAT CAAAACTAGA TTTATATCAA 3120
   GACAAACGAG TTGTATGTGT AGCAAATGTA CTAAACAATG TAGATACTGC ATCAAAGATA 3180
   GATTTCCTGG CAGGATTAGT CATAGGGGTT TCAATGGACA ACGACACAAA ATTAACACAG 3240
   ATACAAAATG AAATGCTAAA CCTCAAAGCA GATCTAAAGA AAATGGACGA ATCACATAGA 3300
   AGATTGATAG AAAATCAAAG AGAACAACTG TCATTGATCA CGTCACTAAT TTCAAATCTC 3360
   AAAATTATGA CTGAGAGAGG AGGAAAGAAA GACCAAAATG AATCCAATGA GAGAGTATCC 3420
   ATGATCAAAA CAAAATTGAA AGAAGAAAAG ATCAAGAAGA CCAGGTTTGA CCCACTTATG 3480
   GAGGCACAAG GCATTGACAA GAATATACCC GATCTATATC GACATGCAGG AGATACACTA 3540
   GAGAACGATG TACAAGTTAA ATCAGAGATA TTAAGTTCAT ACAATGAGTC AAATGCAACA 3600
   AGACTAATAC CCAAAAAAGT GAGCAGTACA ATGAGATCAC TAGTTGCAGT CATCAACAAC 3660
   AGCAATCTCT CACAAAGCAC AAAACAATCA TACATAAACG AACTCAAACG TTGCAAAAAT 3720
   GATGAAGAAG TATCTGAATT AATGGACATG TTCAATGAAG ATGTCAACAA TTGCCAATGA 3780
   TCCAACAAAG AAACGACACC GAACAAACAG ACAAGAAACA ACAGTAGATC AAAACCTGTC 3840
   AACACACACA AAATCAAGCA GAATGAAACA ACAGATATCA ATCAATATAC AAATAAGAAA 3900
   AACTTAGGAT TAAAGAATAA ATTAATCCTT GTCCAAAATG AGTATAACTA ACTCTGCAAT 3960
   ATACACATTC CCAGAATCAT CATTCTCTGA AAATGGTCAT ATAGAACCAT TACCACTCAA 4020
   AGTCAATGAA CAGAGGAAAG CAGTACCCCA CATTAGAGTT GCCAAGATCG GAAATCCACC 4080
   AAAACACGGA TCCCGGTATT TAGATGTCTT CTTACTCGGC TTCTTCGAGA TGGAACGAAT 4140
   CAAAGACAAA TACGGGAGTG TGAATGATCT CGACAGTGAC CCGAGTTACA AAGTTTGTGG 4200
   CTCTGGATCA TTACCAATCG GATTGGCTAA GTACACTGGG AATGACCAGG AATTGTTACA 4260
   AGCCGCAACC AAACTGGATA TAGAAGTGAG AAGAACAGTC AAAGCGAAAG AGATGGTTGT 4320
   TTACACGGTA CAAAATATAA AACCAGAACT GTACCCATGG TCCAATAGAC TAAGAAAAGG 4380
   AATGCTGTTC GATGCCAACA AAGTTGCTCT TGCTCCTCAA TGTCTTCCAC TAGATAGGAG 4440
   CATAAAATTT AGAGTAATCT TCGTGAATTG TACGGCAATT GGATCAATAA CCTTGTTCAA 4500
   AATTCCTAAG TCAATGGCAT CACTATCTCT ACCCAACACA ATATCAATCA ATCTGCAGGT 4560
   ACACATAAAA ACAGGGGTTC AGACTGATTC TAAAGGGATA GTTCAAATTT TGGATGAGAA 4620
   AGGCGAAAAA TCACTGAATT TCATGGTCCA TCTCGGATTG ATCAAAAGAA AAGTAGGCAG 4680
   AATGTACTCT GTTGAATACT GTAAACAGAA AATCGAGAAA ATGAGATTGA TATTTTCTTT 4740
   AGGACTAGTT GGAGGAATCA GTCTTCATGT CAATGCAACT GGGTCCATAT CAAAAACACT 4800
   AGCAAGTCAG CTGGTATTCA AAAGAGAGAT TTGTTATCCT TTAATGGATC TAAATCCGCA 4860
   TCTCAATCTA GTTATCTGGG CTTCATCAGT AGAGATTACA AGAGTGGATG CAATTTTCCA 4920
   ACCTTCTTTA CCTGGCGAGT TCAGATACTA TCCTAATATT ATTGCAAAAG GAGTTGGGAA 4980
   AATCAAACAA TGGAACTAGT AATCTCTATT TTAGTCCGGA CGTATCTATT AAGCCGAAGC 5040
   AAATAAAGGA TAATCAAAAA CTTAGGACAA AAGAGGTCAA TACCAACAAC TATTAGCAGT 5100
   CACACTCGCA AGAATAAGAG AGAAGGGACC AAAAAAGTCA AATAGGAGAA ATCAAAACAA 5160
   AAGGTACAGA ACACCAGAAC AACAAAATCA AAACATCCAA CTCACTCAAA ACAAAAATTC 5220
   CAAAAGAGAC CGGCAACACA ACAAGCACTG AACACAATGC CAACTTCAAT ACTGCTAATT 5280
   ATTACAACCA TGATCATGGC ATCTTTCTGC CAAATAGATA TCACAAAACT ACAGCACGTA 5340
   GGTGTATTGG TCAACAGTCC CAAAGGGATG AAGATATCAC AAAACTTTGA AACAAGATAT 5400
   CTAATTTTGA GCCTCATACC AAAAATAGAA GACTCTAACT CTTGTGGTGA CCAACAGATC 5460
   AAGCAATACA AGAAGTTATT GGATAGACTG ATCATCCCTT TATATGATGG ATTAAGATTA 5520
   CAGAAAGATG TGATAGTAAC CAATCAAGAA TCCAATGAAA ACACTGATCC CAGAACAAAA 5580
   CGATTCTTTG GAGGGGTAAT TGGAACCATT GCTCTGGGAG TAGCAACCTC AGCACAAATT 5640
   ACAGCGGCAG TTGCTCTGGT TGAAGCCAAG CAGGCAAGAT CAGACATCGA AAAACTCAAA 5700
   GAAGCAATTA GGGACACAAA CAAAGCAGTG CAGTCAGTTC AGAGCTCCAT AGGAAATTTA 5760
   ATAGTAGCAA TTAAATCAGT CCAGGATTAT GTTAACAAAG AAATCGTGCC ATCGATTGCG 5820
   AGGCTAGGTT GTGAAGCAGC AGGACTTCAA TTAGGAATTG CATTAACACA GCATTACTCA 5880
   GAATTAACAA ACATATTTGG TGATAACATA GGATCGTTAC AAGAAAAAGG AATAAAATTA 5940
   CAAGGTATAG CATCATTATA CCGCACAAAT ATCACAGAAA TATTCACAAC ATCAACAGTT 6000
   GATAAATATG ATATCTATGA TCTGTTATTT ACAGAATCAA TAAAGGTGAG AGTTATAGAT 6060
   GTTGACTTGA ATGATTACTC AATCACCCTC CAAGTCAGAC TCCCTTTATT AACTAGGCTG 6120
   CTGAACACTC AGATCTACAA AGTAGATTCC ATATCATATA ACATCCAAAA CAGAGAATGG 6180
   TATATCCCTC TTCCCAGCCA TATCATGACG AAAGGGGCAT TTCTAGGTGG AGCAGACGTC 6240
   AAAGAATGTA TAGAAGCATT CAGCAGCTAT ATATGCCCTT CTGATCCAGG ATTTGTATTA 6300
   AACCATGAAA TAGAGAGCTG CTTATCAGGA AACATATCCC AATGTCCAAG AACAACGGTC 6360
   ACATCAGACA TTGTTCCAAG ATATGCATTT GTCAATGGAG GAGTGGTTGC AAACTGTATA 6420
   ACAACCACCT GTACATGCAA CGGAATTGGT AATAGAATCA ATCAACCACC TGATCAAGGA 6480
   GTAAAAATTA TAACACATAA AGAATGTAGT ACAATAGGTA TCAACGGAAT GCTGTTCAAT 6540
   ACAAATAAAG AAGGAACTCT TGCATTCTAT ACACCAAATG ATATAACACT AAACAATTCT 6600
   GTTGCACTTG ATCCAATTGA CATATCAATC GAGCTCAACA AGGCCAAATC AGATCTAGAA 6660
   GAATCAAAAG AATGGATAAG AAGGTCAAAT CAAAAACTAG ATTCTATTGG AAATTGGCAT 6720
   CAATCTAGCA CTACAATCAT AATTATTTTG ATAATGATCA TTATATTGTT TATAATTAAT 6780
   ATAACGATAA TTACAATTGC AATTAAGTAT TACAGAATTC AAAAGAGAAA TCGAGTGGAT 6840
   CAAAATGACA AGCCATATGT ACTAACAAAC AAATAACATA TCTACAGATC ATTAGATATT 6900
   AAAATTATAA AAAACTTAGG AGTAAAGTTA CGCAATCCAA CTCTACTCAT ATAATTGAGG 6960
   AAGGACCCAA TAGACAAATC CAAATTCGAG ATGGAATACT GGAAGCATAC CAATCACGGA 7020
   AAGGATGCTG GTAATGAGCT GGAGACGTCT ATGGCTACTC ATGGCAACAA GCTCACTAAT 7080
   AAGATAATAT ACATATTATG GACAATAATC CTGGTGTTAT TATCAATAGT CTTCATCATA 7140
   GTGCTAATTA ATTCCATCAA AAGTGAAAAG GCCCACGAAT CATTGCTGCA AGACATAAAT 7200
   AATGAGTTTA TGGAAATTAC AGAAAAGATC CAAATGGCAT CGGATAATAC CAATGATCTA 7260
   ATACAGTCAG GAGTGAATAC AAGGCTTCTT ACAATTCAGA GTCATGTCCA GAATTACATA 7320
   CCAATATCAT TGACACAACA GATGTCAGAT CTTAGGAAAT TCATTAGTGA AATTACAATT 7380
   AGAAATGATA ATCAAGAAGT GCTGCCACAA AGAATAACAC ATGATGTAGG TATAAAACCT 7440
   TTAAATCCAG ATGATTTTTG GAGATGCACG TCTGGTCTTC CATCTTTAAT GAAAACTCCA 7500
   AAAATAAGGT TAATGCCAGG GCCGGGATTA TTAGCTATGC CAACGACTGT TGATGGCTGT 7560
   GTTAGAACTC CGTCTTTAGT TATAAATGAT CTGATTTATG CTTATACCTC AAATCTAATT 7620
   ACTCGAGGTT GTCAGGATAT AGGAAAATCA TATCAAGTCT TACAGATAGG GATAATAACT 7680
   GTAAACTCAG ACTTGGTACC TGACTTAAAT CCTAGGATCT CTCATACCTT TAACATAAAT 7740
   GACAATAGGA AGTCATGTTC TCTAGCACTC CTAAATATAG ATGTATATCA ACTGTGTTCA 7800
   ACTCCCAAAG TTGATGAAAG ATCAGATTAT GCATCATCAG GCATAGAAGA TATTGTACTT 7860
   GATATTGTCA ATTATGATGG TTCAATCTCA ACAACAAGAT TTAAGAATAA TAACATAAGC 7920
   TTTGATCAAC CATATGCTGC ACTATACCCA TCTGTTGGAC CAGGGATATA CTACAAAGGC 7980
   AAAATAATAT TTCTCGGGTA TGGAGGTCTT GAACATCCAA TAAATGAGAA TGTAATCTGC 8040
   AACACAACTG GGTGCCCCGG GAAAACACAG AGAGACTGTA ATCAAGCATC TCATAGTACT 8100
   TGGTTTTCAG ATAGGAGGAT GGTCAACTCC ATCATTGTTG TTGACAAAGG CTTAAACTCA 8160
   ATTCCAAAAT TGAAAGTATG GACGATATCT ATGCGACAAA ATTACTGGGG GTCAGAAGGA 8220
   AGGTTACTTC TACTAGGTAA CAAGATCTAT ATATATACAA GATCTACAAG TTGGCATAGC 8280
   AAGTTACAAT TAGGAATAAT TGATATTACT GATTACAGTG ATATAAGGAT AAAATGGACA 8340
   TGGCATAATG TGCTATCAAG ACCAGGAAAC AATGAATGTC CATGGGGACA TTCATGTCCA 8400
   GATGGATGTA TAACAGGAGT ATATACTGAT GCATATCCAC TCAATCCCAC AGGGAGCATT 8460
   GTGTCATCTG TCATATTAGA CTCACAAAAA TCGAGAGTGA ACCCAGTCAT AACTTACTCA 8520
   ACAGCAACCG AAAGAGTAAA CGAGCTGGCC ATCCTAAACA GAACACTCTC AGCTGGATAT 8580
   ACAACAACAA GCTGCATTAC ACACTATAAC AAAGGATATT GTTTTCATAT AGTAGAAATA 8640
   AATCATAAAA GCTTAAACAC ATTTCAACCC ATGTTGTTCA AAACAGAGAT TCCAAAAAGC 8700
   TGCAGTTAAT CATAATTAAC CATAATATGC ATCAATCTAT CTATAATACA AGTATATGAT 8760
   AAGTAATCAG CAATCAGACA ATAGACAAAA GGGAAATATA AAAAACTTAG GAGCAAAGCG 8820
   TGCTCGGGAA ATGGACACTG AATCTAACAA TGGCACTGTA TCTGACATAC TCTATCCTGA 8880
   GTGTCACCTT AACTCTCCTA TCGTTAAAGG TAAAATAGCA CAATTACACA CTATTATGAG 8940
   TCTACCTCAG CCTTATGATA TGGATGACGA CTCAATACTA GTTATCACTA GACAGAAAAT 9000
   AAAACTTAAT AAATTGGATA AAAGACAACG ATCTATTAGA AGATTAAAAT TAATATTAAC 9060
   TGAAAAAGTG AATGACTTAG GAAAATACAC ATTTATCAGA TATCCAGAAA TGTCAAAAGA 9120
   AATGTTCAAA TTATATATAC CTGGTATTAA CAGTAAAGTG ACTGAATTAT TACTTAAAGC 9180
   AGATAGAACA TATAGTCAAA TGACTGATGG ATTAAGAGAT CTATGGATTA ATGTGCTATC 9240
   AAAATTAGCC TCAAAAAATG ATGGAAGCAA TTATGATCTT AATGAAGAAA TTAATAATAT 9300
   ATCGAAAGTT CACACAACCT ATAAATCAGA TAAATGGTAT AATCCATTCA AAACATGGTT 9360
   TACTATCAAG TATGATATGA GAAGATTACA AAAAGCTCGA AATGAGATCA CTTTTAATGT 9420
   TGGGAAGGAT TATAACTTGT TAGAAGACCA GAAGAATTTC TTATTGATAC ATCCAGAATT 9480
   GGTTTTGATA TTAGATAAAC AAAACTATAA TGGTTATCTA ATTACTCCTG AATTAGTATT 9540
   GATGTATTGT GACGTAGTCG AAGGCCGATG GAATATAAGT GCATGTGCTA AGTTAGATCC 9600
   AAAATTACAA TCTATGTATC AGAAAGGTAA TAACCTGTGG GAAGTGATAG ATAAATTGTT 9660
   TCCAATTATG GGAGAAAAGA CATTTGATGT GATATCGTTA TTAGAACCAC TTGCATTATC 9720
   CTTAATTCAA ACTCATGATC CTGTTAAACA ACTAAGAGGA GCTTTTTTAA ATCATGTGTT 9780
   ATCCGAGATG GAATTAATAT TTGAATCTAG AGAATCGATT AAGGAATTTC TGAGTGTAGA 9840
   TTACATTGAT AAAATTTTAG ATATATTTAA TAAGTCTACA ATAGATGAAA TAGCAGAGAT 9900
   TTTCTCTTTT TTTAGAACAT TTGGGCATCC TCCATTAGAA GCTAGTATTG CAGCAGAAAA 9960
   GGTTAGAAAA TATATGTATA TTGGAAAACA ATTAAAATTT GACACTATTA ATAAATGTCA 10020
   TGCTATCTTC TGTACAATAA TAATTAACGG ATATAGAGAG AGGCATGGTG GACAGTGGCC 10080
   TCCTGTGACA TTACCTGATC ATGCACACGA ATTCATCATA AATGCTTACG GTTCAAACTC 10140
   TGCGATATCA TATGAAAATG CTGTTGATTA TTACCAGAGC TTTATAGGAA TAAAATTCAA 10200
   TAAATTCATA GAGCCTCAGT TAGATGAGGA TTTGACAATT TATATGAAAG ATAAAGCATT 10260
   ATCTCCAAAA AAATCAAATT GGGACACAGT TTATCCTGCA TCTAATTTAC TGTACCGTAC 10320
   TAACGCATCC AACGAATCAC GAAGATTAGT TGAAGTATTT ATAGCAGATA GTAAATTTGA 10380
   TCCTCATCAG ATATTGGATT ATGTAGAATC TGGGGACTGG TTAGATGATC CAGAATTTAA 10440
   TATTTCTTAT AGTCTTAAAG AAAAAGAGAT CAAACAGGAA GGTAGACTCT TTGCAAAAAT 10500
   GACATACAAA ATGAGAGCTA CACAAGTTTT ATCAGAGACC CTACTTGCAA ATAACATAGG 10560
   AAAATTCTTT CAAGAAAATG GGATGGTGAA GGGAGAGATT GAATTACTTA AGAGATTAAC 10620
   AACCATATCA ATATCAGGAG TTCCACGGTA TAATGAAGTG TACAATAATT CTAAAAGCCA 10680
   TACAGATGAC CTTAAAACCT ACAATAAAAT AAGTAATCTT AATTTGTCTT CTAATCAGAA 10740
   ATCAAAGAAA TTTGAATTCA AGTCAACGGA TATCTACAAT GATGGATACG AGACTGTGAG 10800
   CTGTTTCCTA ACAACAGATC TCAAAAAATA CTGTCTTAAT TGGAGATATG AATCAACAGC 10860
   TCTATTTGGA GAAACTTGCA ACCAAATATT TGGATTAAAT AAATTGTTTA ATTGGTTACA 10920
   CCCTCGTCTT GAAGGAAGTA CAATCTATGT AGGTGATCCT TACTGTCCTC CATCAGATAA 10980
   AGAACATATA TCATTAGAGG ATCACCCTGA TTCTGGTTTT TACGTTCATA ACCCAAGAGG 11040
   GGGTATAGAA GGATTTTGTC AAAAATTATG GACACTCATA TCTATAAGTG CAATACATCT 11100
   AGCAGCTGTT AGAATAGGCG TGAGGGTGAC TGCAATGGTT CAAGGAGACA ATCAAGCTAT 11160
   AGCTGTAACC ACAAGAGTAC CCAACAATTA TGACTACAGA GTTAAGAAGG AGATAGTTTA 11220
   TAAAGATGTA GTGAGATTTT TTGATTCATT AAGAGAAGTG ATGGATGATC TAGGTCATGA 11280
   ACTTAAATTA AATGAAACGA TTATAAGTAG CAAGATGTTC ATATATAGCA AAAGAATCTA 11340
   TTATGATGGG AGAATTCTTC CTCAAGCTCT AAAAGCATTA TCTAGATGTG TCTTCTGGTC 11400
   AGAGACAGTA ATAGACGAAA CAAGATCAGC ATCTTCAAAT TTGGCAACAT CATTTGCAAA 11460
   AGCAATTGAG AATGGTTATT CACCTGTTCT AGGATATGCA TGCTCAATTT TTAAGAATAT 11520
   TCAACAACTA TATATTGCCC TTGGGATGAA TATCAATCCA ACTATAACAC AGAATATCAG 11580
   AGATCAGTAT TTTAGGAATC CAAATTGGAT GCAATATGCC TCTTTAATAC CTGCTAGTGT 11640
   TGGGGGATTC AATTACATGG CCATGTCAAG ATGTTTTGTA AGGAATATTG GTGATCCATC 11700
   AGTTGCCGCA TTGGCTGATA TTAAAAGATT TATTAAGGCG AATCTATTAG ACCGAAGTGT 11760
   TCTTTATAGG ATTATGAATC AAGAACCAGG TGAGTCATCT TTTTTGGACT GGGCTTCAGA 11820
   TCCATATTCA TGCAATTTAC CACAATCTCA AAATATAACC ACCATGATAA AAAATATAAC 11880
   AGCAAGGAAT GTATTACAAG ATTCACCAAA TCCATTATTA TCTGGATTAT TCACAAATAC 11940
   AATGATAGAA GAAGATGAAG AATTAGCTGA GTTCCTGATG GACAGGAAGG TAATTCTCCC 12000
   TAGAGTTGCA CATGATATTC TAGATAATTC TCTCACAGGA ATTAGAAATG CCATAGCTGG 12060
   AATGTTAGAT ACGACAAAAT CACTAATTCG GGTTGGCATA AATAGAGGAG GACTGACATA 12120
   TAGTTTGTTG AGGAAAATCA GTAATTACGA TCTAGTACAA TATGAAACAC TAAGTAGGAC 12180
   TTTGCGACTA ATTGTAAGTG ATAAAATCAA GTATGAAGAT ATGTGTTCGG TAGACCTTGC 12240
   CATAGCATTG CGACAAAAGA TGTGGATTCA TTTATCAGGA GGAAGGATGA TAAGTGGACT 12300
   TGAAACGCCT GACCCATTAG AATTACTATC TGGGGTAGTA ATAACAGGAT CAGAACATTG 12360
   TAAAATATGT TATTCTTCAG ATGGCACAAA CCCATATACT TGGATGTATT TACCCGGTAA 12420
   TATCAAAATA GGATCAGCAG AAACAGGTAT ATCGTCATTA AGAGTTCCTT ATTTTGGATC 12480
   AGTCACTGAT GAAAGATCTG AAGCACAATT AGGATATATC AAGAATCTTA GTAAACCTGC 12540
   AAAAGCCGCA ATAAGAATAG CAATGATATA TACATGGGCA TTTGGTAATG ATGAGATATC 12600
   TTGGATGGAA GCCTCACAGA TAGCACAAAC ACGTGCAAAT TTTACACTAG ATAGTCTCAA 12660
   AATTTTAACA CCGGTAGCTA CATCAACAAA TTTATCACAC AGATTAAAGG ATACTGCAAC 12720
   TCAGATGAAA TTCTCCAGTA CATCATTGAT CAGAGTCAGC AGATTCATAA CAATGTCCAA 12780
   TGATAACATG TCTATCAAAG AAGCTAATGA AACCAAAGAT ACTAATCTTA TTTATCAACA 12840
   AATAATGTTA ACAGGATTAA GTGTTTTCGA ATATTTATTT AGATTAAAAG AAACCACAGG 12900
   ACACAACCCT ATAGTTATGC ATCTGCACAT AGAAGATGAG TGTTGTATTA AAGAAAGTTT 12960
   TAATGATGAA CATATTAATC CAGAGTCTAC ATTAGAATTA ATTCGATATC CTGAAAGTAA 13020
   TGAATTTATT TATGATAAAG ACCCACTCAA AGATGTGGAC TTATCAAAAC TTATGGTTAT 13080
   TAAAGACCAT TCTTACACAA TTGATATGAA TTATTGGGAT GATACTGACA TCATACATGC 13140
   AATTTCAATA TGTACTGCAA TTACAATAGC AGATACTATG TCACAATTAG ATCGAGATAA 13200
   TTTAAAAGAG ATAATAGTTA TTGCAAATGA TGATGATATT AATAGCTTAA TCACTGAATT 13260
   TTTGACTCTT GACATACTTG TATTTCTCAA GACATTTGGT GGATTATTAG TAAATCAATT 13320
   TGCATACACT CTTTATAGTC TAAAAATAGA AGGTAGGGAT CTCATTTGGG ATTATATAAT 13380
   GAGAACACTG AGAGATACTT CCCATTCAAT ATTAAAAGTA TTATCTAATG CATTATCTCA 13440
   TCCTAAAGTA TTCAAGAGGT TCTGGGATTG TGGAGTTTTA AACCCTATTT ATGGTCCTAA 13500
   TACTGCTAGT CAAGACCAGA TAAAACTTGC CCTATCTATA TGTGAATATT CACTAGATCT 13560
   ATTTATGAGA GAATGGTTGA ATGGTGTATC ACTTGAAATA TACATTTGTG ACAGCGATAT 13620
   GGAAGTTGCA AATGATAGGA AACAAGCCTT TATTTCTAGA CACCTTTCAT TTGTTTGTTG 13680
   TTTAGCAGAA ATTGCATCTT TCGGACCTAA CCTGTTAAAC TTAACATACT TGGAGAGACT 13740
   TGATCTATTG AAACAATATC TTGAATTAAA TATTAAAGAA GACCCTACTC TTAAATATGT 13800
   ACAAATATCT GGATTATTAA TTAAATCGTT CCCATCAACT GTAACATACG TAAGAAAGAC 13860
   TGCAATCAAA TATCTAAGGA TTCGCGGTAT TAGTCCACCT GAGGTAATTG ATGATTGGGA 13920
   TCCGGTAGAA GATGAAAATA TGCTGGATAA CATTGTCAAA ACTATAAATG ATAACTGTAA 13980
   TAAAGATAAT AAAGGGAATA AAATTAACAA TTTCTGGGGA CTAGCACTTA AGAACTATCA 14040
   AGTCCTTAAA ATCAGATCTA TAACAAGTGA TTCTGATGAT AATGATAGAC TAGATGCTAA 14100
   TACAAGTGGT TTGACACTTC CTCAAGGAGG GAATTATCTA TCGCATCAAT TGAGATTATT 14160
   CGGAATCAAC AGCACTAGTT GTCTGAAAGC TCTTGAGTTA TCACAAATTT TAATGAAGGA 14220
   AGTCAATAAA GACAAGGACA GGCTCTTCCT GGGAGAAGGA GCAGGAGCTA TGCTAGCATG 14280
   TTATGATGCC ACATTAGGAC CTGCAGTTAA TTATTATAAT TCAGGTTTGA ATATAACAGA 14340
   TGTAATTGGT CAACGAGAAT TGAAAATATT TCCTTCAGAG GTATCATTAG TAGGTAAAAA 14400
   ATTAGGAAAT GTGACACAGA TTCTTAACAG GGTAAAAGTA CTGTTCAATG GGAATCCTAA 14460
   TTCAACATGG ATAGGAAATA TGGAATGTGA GAGCTTAATA TGGAGTGAAT TAAATGATAA 14520
   GTCCATTGGA TTAGTACATT GTGATATGGA AGGAGCTATC GGTAAATCAG AAGAAACTGT 14580
   TCTACATGAA CATTATAGTG TTATAAGAAT TACATACTTG ATTGGGGATG ATGATGTTGT 14640
   TTTAGTTTCC AAAATTATAC CTACAATCAC TCCGAATTGG TCTAGAATAC TTTATCTATA 14700
   TAAATTATAT TGGAAAGATG TAAGTATAAT ATCACTCAAA ACTTCTAATC CTGCATCAAC 14760
   AGAATTATAT CTAATTTCGA AAGATGCATA TTGTACTATA ATGGAACCTA GTGAAATTGT 14820
   TTTATCAAAA CTTAAAAGAT TGTCACTCTT GGAAGAAAAT AATCTATTAA AATGGATCAT 14880
   TTTATCAAAG AAGAGGAATA ATGAATGGTT ACATCATGAA ATCAAAGAAG GAGAAAGAGA 14940
   TTATGGAATC ATGAGACCAT ATCATATGGC ACTACAAATC TTTGGATTTC AAATCAATTT 15000
   AAATCATCTG GCGAAAGAAT TTTTATCAAC CCCAGATCTG ACTAATATCA ACAATATAAT 15060
   CCAAAGTTTT CAGCGAACAA TAAAGGATGT TTTATTTGAA TGGATTAATA TAACTCATGA 15120
   TGATAAGAGA CATAAATTAG GCGGAAGATA TAACATATTC CCACTGAAAA ATAAGGGAAA 15180
   GTTAAGACTG CTATCGAGAA GACTAGTATT AAGTTGGATT TCATTATCAT TATCGACTCG 15240
   ATTACTTACA GGTCGCTTTC CTGATGAAAA ATTTGAACAT AGAGCACAGA CTGGATATGT 15300
   ATCATTAGCT GATACTGATT TAGAATCATT AAAGTTATTG TCGAAAAACA TCATTAAGAA 15360
   TTACAGAGAG TGTATAGGAT CAATATCATA TTGGTTTCTA ACCAAAGAAG TTAAAATACT 15420
   TATGAAATTG ATCGGTGGTG CTAAATTATT AGGAATTCCC AGACAATATA AAGAACCCGA 15480
   AGACCAGTTA TTAGAAAACT ACAATCAACA TGATGAATTT GATATCGATT AAAACATAAA 15540
   TACAATGAAG ATATATCCTA ACCTTTATCT TTAAGCCTAG GAATAGACAA AAAGTAAGAA 15600
   AAACATGTAA TATATATATA CCAAACAGAG TTCTTCTCTT GTTTGGTTAT AGTGAGTCGT 15660
   ATTACAATC 15669
(2) INFORMATION FOR SEQ ID NO:2:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:2:
      AATACGACTC ACTATAACCA AACAAGAGAA C 31
(2) INFORMATION FOR SEQ ID NO:3:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:3:
      CCAAGTACTA TGAGATGCTT CATT 24
(2) INFORMATION FOR SEQ ID NO:4:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:4:
      CCCTATAATT TCAACATGTT GAGCCTATTT G 31
(2) INFORMATION FOR SEQ ID NO:5:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:5:
      GATTAAAATG TTGGTCGACT TAGTTGCTTC C 31
(2) INFORMATION FOR SEQ ID NO:6:
   (i) SEQUENCE CHARACTERISTICS:
      (A) LENGTH: 38 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:6:
      CCATAGAGAG TCCATGGAAA GCGACGCTAA AAACTATC 38
(2) INFORMATION FOR SEQ ID NO:7:
   (i) SEQUENCE CHARACTERISTICS:
      (A) LENGTH: 37 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:7: CGGTGTCGTT TCTTTGTCGA CTCATTGGCA ATTGTTG 37
(2) INFORMATION FOR SEQ ID NO:8:
   (i) SEQUENCE CHARACTERISTICS:
      (A) LENGTH: 42 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO: 8:
      GCAAAGCGTG CCCGGGCCAT GGACACTGAA TCTAACAATG GC 42
(2) INFORMATION FOR SEQ ID NO:9:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:9:
      GAAATTCCTT AATCGATTCT CTAGATTC 28
(2) INFORMATION FOR SEQ ID NO:10:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:10:
      CCCATCAACT GTAACATACG TAAGAAAGAC 30
(2) INFORMATION FOR SEQ ID NO:11:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:11:
      GGTTAGGATA TGTCGACATT GTATTTATG 29
(2) INFORMATION FOR SEQ ID NO:12:
   (i) SEQUENCE CHARACTERISTICS:
      (A) LENGTH: 43 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:12:
      GGGGTTATGC TACTGCAGGC TTTTTTTCTC CTTAGCCAT CGG 43
(2) INFORMATION FOR SEQ ID NO:13:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:13:
      CTCCATTCTA GANTTATAAA AATTATAGAG TTCCC 35
(2) INFORMATION FOR SEQ ID NO:14:
   (i) SEQUENCE CHARACTERISTICS:
      (A) LENGTH: 15660 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:14:
   TAATACGACT CACTATAACC AAACAAGAGA AGAAACTTGT CTGGGAATAT AAATTTAACT 60
   TTAAATTAAC TTAGGATTAA AGACATTGAC TAGAAGGTCA AGAAAAGGGA ACTCTATAAT 120
   TTCAAAAATG TTGAGCCTAT TTGATACATT TAATGCACGT AGGCAAGAAA ACATAACAAA 180
   ATCAGCCGGT GGAGCTATCA TTCCTGGACA GAAAAATACT GTCTCTATAT TCGCCCTTGG 240
   ACCGACAATA ACTGATGATA ATGAGAAAAT GACATTAGCT CTTCTATTTC TATCTCATTC 300
   ACTAGATAAT GAGAAACAAC ATGCACAAAG GGCAGGGTTC TTGGTGTCTT TATTGTCAAT 360
   GGCTTATGCC AATCCAGAGC TCTACCTAAC AACAAATGGA AGTAATGCAG ATGTCAAGTA 420
   TGTCATATAC ATGATTGAGA AAGATCTAAA ACGGCAAAAG TATGGAGGAT TTGTGGTTAA 480
   GACGAGAGAG ATGATATATG AAAAGACAAC TGATTGGATA TTTGGAAGTG ACCTGGATTA 540
   TGATCAGGAA ACTATGTTGC AGAACGGCAG GAACAATTCA ACAATTGAAG ACCTTGTCCA 600
   CACATTTGGG TATCCATCAT GTTTAGGAGC TCTTATAATA CAGATCTGGA TAGTTCTGGT 660
   CAAAGCTATC ACTAGTATCT CAGGGTTAAG AAAAGGCTTT TTCACCCGAT TGGAAGCTTT 720
   CAGACAAGAT GGAACAGTGC AGGCAGGGCT GGTATTGAGC GGTGACACAG TGGATCAGAT 780
   TGGGTCAATC ATGCGGTCTC AACAGAGCTT GGTAACTCTT ATGGTTGAAA CATTAATAAC 840
   AATGAATACC AGCAGAAATG ACCTCACAAC CATAGAAAAG AATATACAAA TTGTTGGCAA 900
   CTACATAAGA GATGCAGGTC TCGCTTCATT CTTCAATACA ATCAGATATG GAATTGAGAC 960
   CAGAATGGCA GCTTTGACTC TATCCACTCT CAGACCAGAT ATCAATAGAT TAAAAGCTTT 1020
   GATGGAACTG TATTTATCAA AGGGACCACG CGCTCCTTTC ATCTGTATCC TCAGAGATCC 1080
   TATACATGGT GAGTTCGCAC CAGGCAACTA TCCTGCCATA TGGAGCTATG CAATGGGGGT 1140
   GGCAGTTGTA CAAAATAGAG CCATGCAACA GTATGTGACG GGAAGATCAT ATCTAGACAT 1200
   TGATATGTTC CAGCTAGGAC AAGCAGTAGC ACGTGATGCC GAAGCTCAAA TGAGCTCAAC 1260
   ACTGGAAGAT GAACTTGGAG TGACACACGA ATCTAAAGAA AGCTTGAAGA GACATATAAG 1320
   GAACATAAAC AGTTCAGAGA CATCTTTCCA CAAACCGACA GGTGGATCAG CCATAGAGAT 1380
   GGCAATAGAT GAAGAGCCAG AACAATTCGA ACATAGAGCA GATCAAGAAC AAAATGGAGA 1440
   ACCTCAATCA TCCATAATTC AATATGCCTG GGCAGAAGGA AATAGAAGCG ATGATCAGAC 1500
   TGAGCAAGCT ACAGAATCTG ACAATATCAA GACCGAACAA CAAAACATCA GAGACAGACT 1560
   AAACAAGAGA CTCAACGACA AGAAGAAACA AAGCAGTCAA CCACCCACTA ATCCCACAAA 1620
   CAGAACAAAC CAGGACGAAA TAGATGATCT GTTTAACGCA TTTGGAAGCA ACTAATCGAA 1680
   TCAACATTTT AATCTAAATC AATAATAAAT AAGAAAAACT TAGGATTAAA GAATCCTATC 1740
   ATACCGGAAT ATAGGGTGGT AAATTTAGAG TCTGCTTGAA ACTCAATCAA TAGAGAGTTG 1800
   ATGGAAAGCG ATGCTAAAAA CTATCAAATC ATGGATTCTT GGGAAGAGGA ATCAAGAGAT 1860
   AAATCAACTA ATATCTCCTC GGCCCTCAAC ATCATTGAAT TCATACTCAG CACCGACCCC 1920
   CAAGAAGACT TATCGGAAAA CGACACAATC AACACAAGAA CCCAGCAACT CAGTGCCACC 1980
   ATCTGTCAAC CAGAAATCAA ACCAACAGAA ACAAGTGAGA AAGATAGTGG ATCAACTGAC 2040
   AAAAATAGAC AGTCTGGGTC ATCACACGAA TGTACAACAG AAGCAAAAGA TAGAAATATT 2100
   GATCAGGAAA CTGTACAGAG AGGACCTGGG AGAAGAAGCA GCTCAGATAG TAGAGCTGAG 2160
   ACTGTGGTCT CTGGAGGAAT CCCCAGAAGC ATCACAGATT CTAAAAATGG AACCCAAAAC 2220
   ACGGAGGATA TTGATCTCAA TGAAATTAGA AAGATGGATA AGGACTCTAT TGAGGGGAAA 2280
   ATGCGACAAT CTGCAAATGT TCCAAGCGAG ATATCAGGAA GTGATGACAT ATTTACAACA 2340
   GAACAAAGTA GAAACAGTGA TCATGGAAGA AGCCTGGAAT CTATCAGTAC ACCTGATACA 2400
   AGATCAATAA GTGTTGTTAC TGCTGCAACA CCAGATGATG AAGAAGAAAT ACTAATGAAA 2460
   AATAGTAGGA CAAAGAAAAG TTCTTCAACA CATCAAGAAG ATGACAAAAG AATTAAAAAA 2520
   GGGGGAAAAG GGAAAGACTG GTTTAAGAAA TCAAAAGATA CCGACAACCA GATACCAACA 2580
   TCAGACTACA GATCCACATC AAAAGGGCAG AAGAAAATCT CAAAGACAAC AACCACCAAC 2640
   ACCGACACAA AGGGGCAAAC AGAAATACAG ACAGAATCAT CAGAAACACA ATCCTCATCA 2700
   TGGAATCTCA TCATCGACAA CAACACCGAC CGGAACGAAC AGACAAGCAC AACTCCTCCA 2760
   ACAACAACTT CCAGATCAAC TTATACAAAA GAATCGATCC GAACAAACTC TGAATCCAAA 2820
   CCCAAGACAC AAAAGACAAA TGGAAAGGAA AGGAAGGATA CAGAAGAGAG CAATCGATTT 2880
   ACAGAGAGGG CAATTACTCT ATTGCAGAAT CTTGGTGTAA TTCAATCCAC ATCAAAACTA 2940
   GATTTATATC AAGACAAACG AGTTGTATGT GTAGCAAATG TACTAAACAA TGTAGATACT 3000
   GCATCAAAGA TAGATTTCCT GGCAGGATTA GTCATAGGGG TTTCAATGGA CAACGACACA 3060
   AAATTAACAC AGATACAAAA TGAAATGCTA AACCTCAAAG CAGATCTAAA GAAAATGGAC 3120
   GAATCACATA GAAGATTGAT AGAAAATCAA AGAGAACAAC TGTCATTGAT CACGTCACTA 3180
   ATTTCAAATC TCAAAATTAT GACTGAGAGA GGAGGAAAGA AAGACCAAAA TGAATCCAAT 3240
   GAGAGAGTAT CCATGATCAA AACAAAATTG AAAGAAGAAA AGATCAAGAA GACCAGGTTT 3300
   GACCCACTTA TGGAGGCACA AGGCATTGAC AAGAATATAC CCGATCTATA TCGACATGCA 3360
   GGAGATACAC TAGAGAACGA TGTACAAGTT AAATCAGAGA TATTAAGTTC ATACAATGAG 3420
   TCAAATGCAA CAAGACTAAT ACCCAAAAAA GTGAGCAGTA CAATGAGATC ACTAGTTGCA 3480
   GTCATCAACA ACAGCAATCT CTCACAAAGC ACAAAACAAT CATACATAAA CGAACTCAAA 3540
   CGTTGCAAAA ATGATGAAGA AGTATCTGAA TTAATGGACA TGTTCAATGA AGATGTCAAC 3600
   AATTGCCAAT GATCCAACAA AGAAACGACA CCGAACAAAC AGACAAGAAA CAACAGTAGA 3660
   TCAAAACCTG TCAACACACA CAAAATCAAG CAGAATGAAA CAACAGATAT CAATCAATAT 3720
   ACAAATAAGA AAAACTTAGG ATTAAAGAAT AAATTAATCC TTGTCCAAAA TGAGTATAAC 3780
   TAACTCTGCA ATATACACAT TCCCAGAATC ATCATTCTCT GAAAATGGTC ATATAGAACC 3840
   ATTACCACTC AAAGTCAATG AACAGAGGAA AGCAGTACCC CACATTAGAG TTGCCAAGAT 3900
   CGGAAATCCA CCAAAACACG GATCCCGGTA TTTAGATGTC TTCTTACTCG GCTTCTTCGA 3960
   GATGGAACGA ATCAAAGACA AATACGGGAG TGTGAATGAT CTCGACAGTG ACCCGAGTTA 4020
   CAAAGTTTGT GGCTCTGGAT CATTACCAAT CGGATTGGCT AAGTACACTG GGAATGACCA 4080
   GGAATTGTTA CAAGCCGCAA CCAAACTGGA TATAGAAGTG AGAAGAACAG TCAAAGCGAA 4140
   AGAGATGGTT GTTTACACGG TACAAAATAT AAAACCAGAA CTGTACCCAT GGTCCAATAG 4200
   ACTAAGAAAA GGAATGCTGT TCGATGCCAA CAAAGTTGCT CTTGCTCCTC AATGTCTTCC 4260
   ACTAGATAGG AGCATAAAAT TTAGAGTAAT CTTCGTGAAT TGTACGGCAA TTGGATCAAT 4320
   AACCTTGTTC AAAATTCCTA AGTCAATGGC ATCACTATCT CTACCCAACA CAATATCAAT 4380
   CAATCTGCAG GTACACATAA AAACAGGGGT TCAGACTGAT TCTAAAGGGA TAGTTCAAAT 4440
   TTTGGATGAG AAAGGCGAAA AATCACTGAA TTTCATGGTC CATCTCGGAT TGATCAAAAG 4500
   AAAAGTAGGC AGAATGTACT CTGTTGAATA CTGTAAACAG AAAATCGAGA AAATGAGATT 4560
   GATATTTTCT TTAGGACTAG TTGGAGGAAT CAGTCTTCAT GTCAATGCAA CTGGGTCCAT 4620
   ATCAAAAACA CTAGCAAGTC AGCTGGTATT CAAAAGAGAG ATTTGTTATC CTTTAATGGA 4680
   TCTAAATCCG CATCTCAATC TAGTTATCTG GGCTTCATCA GTAGAGATTA CAAGAGTGGA 4740
   TGCAATTTTC CAACCTTCTT TACCTGGCGA GTTCAGATAC TATCCTAATA TTATTGCAAA 4800
   AGGAGTTGGG AAAATCAAAC AATGGAACTA GTAATCTCTA TTTTAGTCCG GACGTATCTA 4860
   TTAAGCCGAA GCAAATAAAG GATAATCAAA AACTTAGGAC AAAAGAGGTC AATACCAACA 4920
   ACTATTAGCA GTCACACTCG CAAGAATAAG AGAGAAGGGA CCAAAAAAGT CAAATAGGAG 4980
   AAATCAAAAC AAAAGGTACA GAACACCAGA ACAACAAAAT CAAAACATCC AACTCACTCA 5040
   AAACAAAAAT TCCAAAAGAG ACCGGCAACA CAACAAGCAC TGAACACAAT GCCAACTTCA 5100
   ATACTGCTAA TTATTACAAC CATGATCATG GCATCTTTCT GCCAAATAGA TATCACAAAA 5160
   CTACAGCACG TAGGTGTATT GGTCAACAGT CCCAAAGGGA TGAAGATATC ACAAAACTTT 5220
   GAAACAAGAT ATCTAATTTT GAGCCTCATA CCAAAAATAG AAGACTCTAA CTCTTGTGGT 5280
   GACCAACAGA TCAAGCAATA CAAGAAGTTA TTGGATAGAC TGATCATCCC TTTATATGAT 5340
   GGATTAAGAT TACAGAAAGA TGTGATAGTA ACCAATCAAG AATCCAATGA AAACACTGAT 5400
   CCCAGAACAA AACGATTCTT TGGAGGGGTA ATTGGAACCA TTGCTCTGGG AGTAGCAACC 5460
   TCAGCACAAA TTACAGCGGC AGTTGCTCTG GTTGAAGCCA AGCAGGCAAG ATCAGACATC 5520
   GAAAAACTCA AAGAAGCAAT TAGGGACACA AACAAAGCAG TGCAGTCAGT TCAGAGCTCC 5580
   ATAGGAAATT TAATAGTAGC AATTAAATCA GTCCAGGATT ATGTTAACAA AGAAATCGTG 5640
   CCATCGATTG CGAGGCTAGG TTGTGAAGCA GCAGGACTTC AATTAGGAAT TGCATTAACA 5700
   CAGCATTACT CAGAATTAAC AAACATATTT GGTGATAACA TAGGATCGTT ACAAGAAAAA 5760
   GGAATAAAAT TACAAGGTAT AGCATCATTA TACCGCACAA ATATCACAGA AATATTCACA 5820
   ACATCAACAG TTGATAAATA TGATATCTAT GATCTGTTAT TTACAGAATC AATAAAGGTG 5880
   AGAGTTATAG ATGTTGACTT GAATGATTAC TCAATCACCC TCCAAGTCAG ACTCCCTTTA 5940
   TTAACTAGGC TGCTGAACAC TCAGATCTAC AAAGTAGATT CCATATCATA TAACATCCAA 6000
   AACAGAGAAT GGTATATCCC TCTTCCCAGC CATATCATGA CGAAAGGGGC ATTTCTAGGT 6060
   GGAGCAGACG TCAAAGAATG TATAGAAGCA TTCAGCAGCT ATATATGCCC TTCTGATCCA 6120
   GGATTTGTAT TAAACCATGA AATAGAGAGC TGCTTATCAG GAAACATATC CCAATGTCCA 6180
   AGAACAACGG TCACATCAGA CATTGTTCCA AGATATGCAT TTGTCAATGG AGGAGTGGTT 6240
   GCAAACTGTA TAACAACCAC CTGTACATGC AACGGAATTG GTAATAGAAT CAATCAACCA 6300
   CCTGATCAAG GAGTAAAAAT TATAACACAT AAAGAATGTA GTACAATAGG TATCAACGGA 6360
   ATGCTGTTCA ATACAAATAA AGAAGGAACT CTTGCATTCT ATACACCAAA TGATATAACA 6420
   CTAAACAATT CTGTTGCACT TGATCCAATT GACATATCAA TCGAGCTCAA CAAGGCCAAA 6480
   TCAGATCTAG AAGAATCAAA AGAATGGATA AGAAGGTCAA ATCAAAAACT AGATTCTATT 6540
   GGAAATTGGC ATCAATCTAG CACTACAATC ATAATTATTT TGATAATGAT CATTATATTG 6600
   TTTATAATTA ATATAACGAT AATTACAATT GCAATTAAGT ATTACAGAAT TCAAAAGAGA 6660
   AATCGAGTGG ATCAAAATGA CAAGCCATAT GTACTAACAA ACAAATAACA TATCTACAGA 6720
   TCATTAGATA TTAAAATTAT AAAAAACTTA GGAGTAAAGT TACGCAATCC AACTCTACTC 6780
   ATATAATTGA GGAAGGACCC AATAGACAAA TCCAAATTCG AGATGGAATA CTGGAAGCAT 6840
   ACCAATCACG GAAAGGATGC TGGTAATGAG CTGGAGACGT CTATGGCTAC TCATGGCAAC 6900
   AAGCTCACTA ATAAGATAAT ATACATATTA TGGACAATAA TCCTGGTGTT ATTATCAATA 6960
   GTCTTCATCA TAGTGCTAAT TAATTCCATC AAAAGTGAAA AGGCCCACGA ATCATTGCTG 7020
   CAAGACATAA ATAATGAGTT TATGGAAATT ACAGAAAAGA TCCAAATGGC ATCGGATAAT 7080
   ACCAATGATC TAATACAGTC AGGAGTGAAT ACAAGGCTTC TTACAATTCA GAGTCATGTC 7140
   CAGAATTACA TACCAATATC ATTGACACAA CAGATGTCAG ATCTTAGGAA ATTCATTAGT 7200
   GAAATTACAA TTAGAAATGA TAATCAAGAA GTGCTGCCAC AAAGAATAAC ACATGATGTA 7260
   GGTATAAAAC CTTTAAATCC AGATGATTTT TGGAGATGCA CGTCTGGTCT TCCATCTTTA 7320
   ATGAAAACTC CAAAAATAAG GTTAATGCCA GGGCCGGGAT TATTAGCTAT GCCAACGACT 7380
   GTTGATGGCT GTGTTAGAAC TCCGTCTTTA GTTATAAATG ATCTGATTTA TGCTTATACC 7440
   TCAAATCTAA TTACTCGAGG TTGTCAGGAT ATAGGAAAAT CATATCAAGT CTTACAGATA 7500
   GGGATAATAA CTGTAAACTC AGACTTGGTA CCTGACTTAA ATCCTAGGAT CTCTCATACC 7560
   TTTAACATAA ATGACAATAG GAAGTCATGT TCTCTAGCAC TCCTAAATAT AGATGTATAT 7620
   CAACTGTGTT CAACTCCCAA AGTTGATGAA AGATCAGATT ATGCATCATC AGGCATAGAA 7680
   GATATTGTAC TTGATATTGT CAATTATGAT GGTTCAATCT CAACAACAAG ATTTAAGAAT 7740
   AATAACATAA GCTTTGATCA ACCATATGCT GCACTATACC CATCTGTTGG ACCAGGGATA 7800
   TACTACAAAG GCAAAATAAT ATTTCTCGGG TATGGAGGTC TTGAACATCC AATAAATGAG 7860
   AATGTAATCT GCAACACAAC TGGGTGCCCC GGGAAAACAC AGAGAGACTG TAATCAAGCA 7920
   TCTCATAGTA CTTGGTTTTC AGATAGGAGG ATGGTCAACT CCATCATTGT TGTTGACAAA 7980
   GGCTTAAACT CAATTCCAAA ATTGAAAGTA TGGACGATAT CTATGCGACA AAATTACTGG 8040
   GGGTCAGAAG GAAGGTTACT TCTACTAGGT AACAAGATCT ATATATATAC AAGATCTACA 8100
   AGTTGGCATA GCAAGTTACA ATTAGGAATA ATTGATATTA CTGATTACAG TGATATAAGG 8160
   ATAAAATGGA CATGGCATAA TGTGCTATCA AGACCAGGAA ACAATGAATG TCCATGGGGA 8220
   CATTCATGTC CAGATGGATG TATAACAGGA GTATATACTG ATGCATATCC ACTCAATCCC 8280
   ACAGGGAGCA TTGTGTCATC TGTCATATTA GACTCACAAA AATCGAGAGT GAACCCAGTC 8340
   ATAACTTACT CAACAGCAAC CGAAAGAGTA AACGAGCTGG CCATCCTAAA CAGAACACTC 8400
   TCAGCTGGAT ATACAACAAC AAGCTGCATT ACACACTATA ACAAAGGATA TTGTTTTCAT 8460
   ATAGTAGAAA TAAATCATAA AAGCTTAAAC ACATTTCAAC CCATGTTGTT CAAAACAGAG 8520
   ATTCCAAAAA GCTGCAGTTA ATCATAATTA ACCATAATAT GCATCAATCT ATCTATAATA 8580
   CAAGTATATG ATAAGTAATC AGCAATCAGA CAATAGACAA AAGGGAAATA TAAAAAACTT 8640
   AGGAGCAAAG CGTGCTCGGG AAATGGACAC TGAATCTAAC AATGGCACTG TATCTGACAT 8700
   ACTCTATCCT GAGTGTCACC TTAACTCTCC TATCGTTAAA GGTAAAATAG CACAATTACA 8760
   CACTATTATG AGTCTACCTC AGCCTTATGA TATGGATGAC GACTCAATAC TAGTTATCAC 8820
   TAGACAGAAA ATAAAACTTA ATAAATTGGA TAAAAGACAA CGATCTATTA GAAGATTAAA 8880
   ATTAATATTA ACTGAAAAAG TGAATGACTT AGGAAAATAC ACATTTATCA GATATCCAGA 8940
   AATGTCAAAA GAAATGTTCA AATTATATAT ACCTGGTATT AACAGTAAAG TGACTGAATT 9000
   ATTACTTAAA GCAGATAGAA CATATAGTCA AATGACTGAT GGATTAAGAG ATCTATGGAT 9060
   TAATGTGCTA TCAAAATTAG CCTCAAAAAA TGATGGAAGC AATTATGATC TTAATGAAGA 9120
   AATTAATAAT ATATCGAAAG TTCACACAAC CTATAAATCA GATAAATGGT ATAATCCATT 9180
   CAAAACATGG TTTACTATCA AGTATGATAT GAGAAGATTA CAAAAAGCTC GAAATGAGAT 9240
   CACTTTTAAT GTTGGGAAGG ATTATAACTT GTTAGAAGAC CAGAAGAATT TCTTATTGAT 9300
   ACATCCAGAA TTGGTTTTGA TATTAGATAA ACAAAACTAT AATGGTTATC TAATTACTCC 9360
   TGAATTAGTA TTGATGTATT GTGACGTAGT CGAAGGCCGA TGGAATATAA GTGCATGTGC 9420
   TAAGTTAGAT CCAAAATTAC AATCTATGTA TCAGAAAGGT AATAACCTGT GGGAAGTGAT 9480
   AGATAAATTG TTTCCAATTA TGGGAGAAAA GACATTTGAT GTGATATCGT TATTAGAACC 9540
   ACTTGCATTA TCCTTAATTC AAACTCATGA TCCTGTTAAA CAACTAAGAG GAGCTTTTTT 9600
   AAATCATGTG TTATCGGAGA TGGAATTAAT ATTTGAATCT AGAGAATCGA TTAAGGAATT 9660
   TCTGAGTGTA GATTACATTG ATAAAATTTT AGATATATTT AATAAGTCTA CAATAGATGA 9720
   AATAGCAGAG ATTTTCTCTT TTTTTAGAAC ATTTGGGCAT CCTCCATTAG AAGCTAGTAT 9780
   TGCAGCAGAA AAGGTTAGAA AATATATGTA TATTGGAAAA CAATTAAAAT TTGACACTAT 9840
   TAATAAATGT CATGCTATCT TCTGTACAAT AATAATTAAC GGATATAGAG AGAGGCATGG 9900
   TGGACAGTGG CCTCCTGTGA CATTACCTGA TCATGCACAC GAATTCATCA TAAATGCTTA 9960
   CGGTTCAAAC TCTGCGATAT CATATGAAAA TGCTGTTGAT TATTACCAGA GCTTTATAGG 10020
   AATAAAATTC AATAAATTCA TAGAGCCTCA GTTAGATGAG GATTTGACAA TTTATATGAA 10080
   AGATAAAGCA TTATCTCCAA AAAAATCAAA TTGGGACACA GTTTATCCTG CATCTAATTT 10140
   ACTGTACCGT ACTAACGCAT CCAACGAATC ACGAAGATTA GTTGAAGTAT TTATAGCAGA 10200
   TAGTAAATTT GATCCTCATC AGATATTGGA TTATGTAGAA TCTGGGGACT GGTTAGATGA 10260
   TCCAGAATTT AATATTTCTT ATAGTCTTAA AGAAAAAGAG ATCAAACAGG AAGGTAGACT 10320
   CTTTGCAAAA ATGACATACA AAATGAGAGC TACACAAGTT TTATCAGAGA CCCTACTTGC 10380
   AAATAACATA GGAAAATTCT TTCAAGAAAA TGGGATGGTG AAGGGAGAGA TTGAATTACT 10440
   TAAGAGATTA ACAACCATAT CAATATCAGG AGTTCCACGG TATAATGAAG TGTACAATAA 10500
   TTCTAAAAGC CATACAGATG ACCTTAAAAC CTACAATAAA ATAAGTAATC TTAATTTGTC 10560
   TTCTAATCAG AAATCAAAGA AATTTGAATT CAAGTCAACG GATATCTACA ATGATGGATA 10620
   CGAGACTGTG AGCTGTTTCC TAACAACAGA TCTCAAAAAA TACTGTCTTA ATTGGAGATA 10680
   TGAATCAACA GCTCTATTTG GAGAAACTTG CAACCAAATA TTTGGATTAA ATAAATTGTT 10740
   TAATTGGTTA CACCCTCGTC TTGAAGGAAG TACAATCTAT GTAGGTGATC CTTACTGTCC 10800
   TCCATCAGAT AAAGAACATA TATCATTAGA GGATCACCCT GATTCTGGTT TTTACGTTCA 10860
   TAACCCAAGA GGGGGTATAG AAGGATTTTG TCAAAAATTA TGGACACTCA TATCTATAAG 10920
   TGCAATACAT CTAGCAGCTG TTAGAATAGG CGTGAGGGTG ACTGCAATGG TTCAAGGAGA 10980
   CAATCAAGCT ATAGCTGTAA CCACAAGAGT ACCCAACAAT TATGACTACA GAGTTAAGAA 11040
   GGAGATAGTT TATAAAGATG TAGTGAGATT TTTTGATTCA TTAAGAGAAG TGATGGATGA 11100
   TCTAGGTCAT GAACTTAAAT TAAATGAAAC GATTATAAGT AGCAAGATGT TCATATATAG 11160
   CAAAAGAATC TATTATGATG GGAGAATTCT TCCTCAAGCT CTAAAAGCAT TATCTAGATG 11220
   TGTCTTCTGG TCAGAGACAG TAATAGACGA AACAAGATCA GCATCTTCAA ATTTGGCAAC 11280
   ATCATTTGCA AAAGCAATTG AGAATGGTTA TTCACCTGTT CTAGGATATG CATGCTCAAT 11340
   TTTTAAGAAT ATTCAACAAC TATATATTGC CCTTGGGATG AATATCAATC CAACTATAAC 11400
   ACAGAATATC AGAGATCAGT ATTTTAGGAA TCCAAATTGG ATGCAATATG CCTCTTTAAT 11460
   ACCTGCTAGT GTTGGGGGAT TCAATTACAT GGCCATGTCA AGATGTTTTG TAAGGAATAT 11520
   TGGTGATCCA TCAGTTGCCG CATTGGCTGA TATTAAAAGA TTTATTAAGG CGAATCTATT 11580
   AGACCGAAGT GTTCTTTATA GGATTATGAA TCAAGAACCA GGTGAGTCAT CTTTTTTGGA 11640
   CTGGGCTTCA GATCCATATT CATGCAATTT ACCACAATCT CAAAATATAA CCACCATGAT 11700
   AAAAAATATA ACAGCAAGGA ATGTATTACA AGATTCACCA AATCCATTAT TATCTGGATT 11760
   ATTCACAAAT ACAATGATAG AAGAAGATGA AGAATTAGCT GAGTTCCTGA TGGACAGGAA 11820
   GGTAATTCTC CCTAGAGTTG CACATGATAT TCTAGATAAT TCTCTCACAG GAATTAGAAA 11880
   TGCCATAGCT GGAATGTTAG ATACGACAAA ATCACTAATT CGGGTTGGCA TAAATAGAGG 11940
   AGGACTGACA TATAGTTTGT TGAGGAAAAT CAGTAATTAC GATCTAGTAC AATATGAAAC 12000
   ACTAAGTAGG ACTTTGCGAC TAATTGTAAG TGATAAAATC AAGTATGAAG ATATGTGTTC 12060
   GGTAGACCTT GCCATAGCAT TGCGACAAAA GATGTGGATT CATTTATCAG GAGGAAGGAT 12120
   GATAAGTGGA CTTGAAACGC CTGACCCATT AGAATTACTA TCTGGGGTAG TAATAACAGG 12180
   ATCAGAACAT TGTAAAATAT GTTATTCTTC AGATGGCACA AACCCATATA CTTGGATGTA 12240
   TTTACCCGGT AATATCAAAA TAGGATCAGC AGAAACAGGT ATATCGTCAT TAAGAGTTCC 12300
   TTATTTTGGA TCAGTCACTG ATGAAAGATC TGAAGCACAA TTAGGATATA TCAAGAATCT 12360
   TAGTAAACCT GCAAAAGCCG CAATAAGAAT AGCAATGATA TATACATGGG CATTTGGTAA 12420
   TGATGAGATA TCTTGGATGG AAGCCTCACA GATAGCACAA ACACGTGCAA ATTTTACACT 12480
   AGATAGTCTC AAAATTTTAA CACCGGTAGC TACATCAACA AATTTATCAC ACAGATTAAA 12540
   GGATACTGCA ACTCAGATGA AATTCTCCAG TACATCATTG ATCAGAGTCA GCAGATTCAT 12600
   AACAATGTCC AATGATAACA TGTCTATCAA AGAAGCTAAT GAAACCAAAG ATACTAATCT 12660
   TATTTATCAA CAAATAATGT TAACAGGATT AAGTGTTTTC GAATATTTAT TTAGATTAAA 12720
   AGAAACCACA GGACACAACC CTATAGTTAT GCATCTGCAC ATAGAAGATG AGTGTTGTAT 12780
   TAAAGAAAGT TTTAATGATG AACATATTAA TCCAGAGTCT ACATTAGAAT TAATTCGATA 12840
   TCCTGAAAGT AATGAATTTA TTTATGATAA AGACCCACTC AAAGATGTGG ACTTATCAAA 12900
   ACTTATGGTT ATTAAAGACC ATTCTTACAC AATTGATATG AATTATTGGG ATGATACTGA 12960
   CATCATACAT GCAATTTCAA TATGTACTGC AATTACAATA GCAGATACTA TGTCACAATT 13020
   AGATCGAGAT AATTTAAAAG AGATAATAGT TATTGCAAAT GATGATGATA TTAATAGCTT 13080
   AATCACTGAA TTTTTGACTC TTGACATACT TGTATTTCTC AAGACATTTG GTGGATTATT 13140
   AGTAAATCAA TTTGCATACA CTCTTTATAG TCTAAAAATA GAAGGTAGGG ATCTCATTTG 13200
   GGATTATATA ATGAGAACAC TGAGAGATAC TTCCCATTCA ATATTAAAAG TATTATCTAA 13260
   TGCATTATCT CATCCTAAAG TATTCAAGAG GTTCTGGGAT TGTGGAGTTT TAAACCCTAT 13320
   TTATGGTCCT AATACTGCTA GTCAAGACCA GATAAAACTT GCCCTATCTA TATGTGAATA 13380
   TTCACTAGAT CTATTTATGA GAGAATGGTT GAATGGTGTA TCACTTGAAA TATACATTTG 13440
   TGACAGCGAT ATGGAAGTTG CAAATGATAG GAAACAAGCC TTTATTTCTA GACACCTTTC 13500
   ATTTGTTTGT TGTTTAGCAG AAATTGCATC TTTCGGACCT AACCTGTTAA ACTTAACATA 13560
   CTTGGAGAGA CTTGATCTAT TGAAACAATA TCTTGAATTA AATATTAAAG AAGACCCTAC 13620
   TCTTAAATAT GTACAAATAT CTGGATTATT AATTAAATCG TTCCCATCAA CTGTAACATA 13680
   CGTAAGAAAG ACTGCAATCA AATATCTAAG GATTCGCGGT ATTAGTCCAC CTGAGGTAAT 13740
   TGATGATTGG GATCCGGTAG AAGATGAAAA TATGCTGGAT AACATTGTCA AAACTATAAA 13800
   TGATAACTGT AATAAAGATA ATAAAGGGAA TAAAATTAAC AATTTCTGGG GACTAGCACT 13860
   TAAGAACTAT CAAGTCCTTA AAATCAGATC TATAACAAGT GATTCTGATG ATAATGATAG   13920
   ACTAGATGCT AATACAAGTG GTTTGACACT TCCTCAAGGA GGGAATTATC TATCGCATCA   13980
   ATTGAGATTA TTCGGAATCA ACAGCACTAG TTGTCTGAAA GCTCTTGAGT TATCACAAAT   14040
   TTTAATGAAG GAAGTCAATA AAGACAAGGA CAGGCTCTTC CTGGGAGAAG GAGCAGGAGC   14100
   TATGCTAGCA TGTTATGATG CCACATTAGG ACCTGCAGTT AATTATTATA ATTCAGGTTT   14160
   GAATATAACA GATGTAATTG GTCAACGAGA ATTGAAAATA TTTCCTTCAG AGGTATCATT   14220
   AGTAGGTAAA AAATTAGGAA ATGTGACACA GATTCTTAAC AGGGTAAAAG TACTGTTCAA   14280
   TGGGAATCCT AATTCAACAT GGATAGGAAA TATGGAATGT GAGAGCTTAA TATGGAGTGA   14340
   ATTAAATGAT AAGTCCATTG GATTAGTACA TTGTGATATG GAAGGAGCTA TCGGTAAATC   14400
   AGAAGAAACT GTTCTACATG AACATTATAG TGTTATAAGA ATTACATACT TGATTGGGGA   14460
   TGATGATGTT GTTTTAGTTT CCAAAATTAT ACCTACAATC ACTCCGAATT GGTCTAGAAT   14520
   ACTTTATCTA TATAAATTAT ATTGGAAAGA TGTAAGTATA ATATCACTCA AAACTTCTAA   14580
   TCCTGCATCA ACAGAATTAT ATCTAATTTC GAAAGATGCA TATTGTACTA TAATGGAACC   14640
   TAGTGAAATT GTTTTATCAA AACTTAAAAG ATTGTCACTC TTGGAAGAAA ATAATCTATT   14700
   AAAATGGATC ATTTTATCAA AGAAGAGGAA TAATGAATGG TTACATCATG AAATCAAAGA   14760
   AGGAGAAAGA GATTATGGAA TCATGAGACC ATATCATATG GCACTACAAA TCTTTGGATT   14820
   TCAAATCAAT TTAAATCATC TGGCGAAAGA ATTTTTATCA ACCCCAGATC TGACTAATAT   14880
   CAACAATATA ATCCAAAGTT TTCAGCGAAC AATAAAGGAT GTTTTATTTG AATGGATTAA   14940
   TATAACTCAT GATGATAAGA GACATAAATT AGGCGGAAGA TATAACATAT TCCCACTGAA   15000
   AAATAAGGGA AAGTTAAGAC TGCTATCGAG AAGACTAGTA TTAAGTTGGA TTTCATTATC   15060
   ATTATCGACT CGATTACTTA CAGGTCGCTT TCCTGATGAA AAATTTGAAC ATAGAGCACA   15120
   GACTGGATAT GTATCATTAG CTGATACTGA TTTAGAATCA TTAAAGTTAT TGTCGAAAAA   15180
   CATCATTAAG AATTACAGAG AGTGTATAGG ATCAATATCA TATTGGTTTC TAACCAAAGA   15240
   AGTTAAAATA CTTATGAAAT TGATCGGTGG TGCTAAATTA TTAGGAATTC CCAGACAATA   15300
   TAAAGAACCC GAAGACCAGT TATTAGAAAA CTACAATCAA CATGATGAAT TTGATATCGA   15360
   TTAAAACATA AATACAATGA AGATATATCC TAACCTTTAT CTTTAAGCCT AGGAATAGAC   15420
   AAAAAGTAAG AAAAACATGT AATATATATA TACCAAACAG AGTTCTTCTC TTGTTTGGTG   15480
   GGTCGGCATG GCATCTCCAC CTCCTCGCGG TCCGGACCTG GGCATCCGAA GGAGGACGCA   15540
   CGTCCACTCG GATGGCTAAG GGAGAGCCTG CAGTAGCATA ACCCCTTGGG GCCTCTAAAC   15600
   GGGTCTTGAG GGGTTTTTTG CTGAAAGGAG GAACTATATA CGCGTCGACG GGCCCCGCGC   15660
(2) INFORMATION FOR SEQ ID NO:15:
   (i) SEQUENCE CHARACTERISTICS:
      (A) LENGTH: 15666 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:15:
   TAATACGACT CACTATAGGA CCAAACAAGA GAAGAAACTT GTCTGGGAAT ATAAATTTAA    60
   CTTTAAATTA ACTTAGGATT AAAGACATTG ACTAGAAGGT CAAGAAAAGG GAACTCTATA   120
   ATTTCAAAAA TGTTGAGCCT ATTTGATACA TTTAATGCAC GTAGGCAAGA AAACATAACA   180
   AAATCAGCCG GTGGAGCTAT CATTCCTGGA CAGAAAAATA CTGTCTCTAT ATTCGCCCTT   240
   GGACCGACAA TAACTGATGA TAATGAGAAA ATGACATTAG CTCTTCTATT TCTATCTCAT   300
   TCACTAGATA ATGAGAAACA ACATGCACAA AGGGCAGGGT TCTTGGTGTC TTTATTGTCA   360
   ATGGCTTATG CCAATCCAGA GCTCTACCTA ACAACAAATG GAAGTAATGC AGATGTCAAG   420
   TATGTCATAT ACATGATTGA GAAAGATCTA AAACGGCAAA AGTATGGAGG ATTTGTGGTT   480
   AAGACGAGAG AGATGATATA TGAAAAGACA ACTGATTGGA TATTTGGAAG TGACCTGGAT   540
   TATGATCAGG AAACTATGTT GCAGAACGGC AGGAACAATT CAACAATTGA AGACCTTGTC   600
   CACACATTTG GGTATCCATC ATGTTTAGGA GCTCTTATAA TACAGATCTG GATAGTTCTG   660
   GTCAAAGCTA TCACTAGTAT CTCAGGGTTA AGAAAAGGCT TTTTCACCCG ATTGGAAGCT   720
   TTCAGACAAG ATGGAACAGT GCAGGCAGGG CTGGTATTGA GCGGTGACAC AGTGGATCAG   780
   ATTGGGTCAA TCATGCGGTC TCAACAGAGC TTGGTAACTC TTATGGTTGA AACATTAATA   840
   ACAATGAATA CCAGCAGAAA TGACCTCACA ACCATAGAAA AGAATATACA AATTGTTGGC   900
   AACTACATAA GAGATGCAGG TCTCGCTTCA TTCTTCAATA CAATCAGATA TGGAATTGAG   960
   ACCAGAATGG CAGCTTTGAC TCTATCCACT CTCAGACCAG ATATCAATAG ATTAAAAGCT  1020
   TTGATGGAAC TGTATTTATC AAAGGGACCA CGCGCTCCTT TCATCTGTAT CCTCAGAGAT  1080
   CCTATACATG GTGAGTTCGC ACCAGGCAAC TATCCTGCCA TATGGAGCTA TGCAATGGGG  1140
   GTGGCAGTTG TACAAAATAG AGCCATGCAA CAGTATGTGA CGGGAAGATC ATATCTAGAC 1200
   ATTGATATGT TCCAGCTAGG ACAAGCAGTA GCACGTGATG CCGAAGCTCA AATGAGCTCA  1260
   ACACTGGAAG ATGAACTTGG AGTGACACAC GAATCTAAAG AAAGCTTGAA GAGACATATA  1320
   AGGAACATAA ACAGTTCAGA GACATCTTTC CACAAACCGA CAGGTGGATC AGCCATAGAG  1380
   ATGGCAATAG ATGAAGAGCC AGAACAATTC GAACATAGAG CAGATCAAGA ACAAAATGGA  1440
   GAACCTCAAT CATCCATAAT TCAATATGCC TGGGCAGAAG GAAATAGAAG CGATGATCAG  1500
   ACTGAGCAAG CTACAGAATC TGACAATATC AAGACCGAAC AACAAAACAT CAGAGACAGA  1560
   CTAAACAAGA GACTCAACGA CAAGAAGAAA CAAAGCAGTC AACCACCCAC TAATCCCACA  1620
   AACAGAACAA ACCAGGACGA AATAGATGAT CTGTTTAACG CATTTGGAAG CAACTAATCG  1680
   AATCAACATT TTAATCTAAA TCAATAATAA ATAAGAAAAA CTTAGGATTA AAGAATCCTA  1740
   TCATACCGGA ATATAGGGTG GTAAATTTAG AGTCTGCTTG AAACTCAATC AATAGAGAGT  1800
   TGATGGAAAG CGATGCTAAA AACTATCAAA TCATGGATTC TTGGGAAGAG GAATCAAGAG  1860
   ATAAATCAAC TAATATCTCC TCGGCCCTCA ACATCATTGA ATTCATACTC AGCACCGACC  1920
   CCCAAGAAGA CTTATCGGAA AACGACACAA TCAACACAAG AACCCAGCAA CTCAGTGCCA 1980
   CCATCTGTCA ACCAGAAATC AAACCAACAG AAACAAGTGA GAAAGATAGT GGATCAACTG 2040
   ACAAAAATAG ACAGTCTGGG TCATCACACG AATGTACAAC AGAAGCAAAA GATAGAAATA 2100
   TTGATCAGGA AACTGTACAG AGAGGACCTG GGAGAAGAAG CAGCTCAGAT AGTAGAGCTG 2160
   AGACTGTGGT CTCTGGAGGA ATCCCCAGAA GCATCACAGA TTCTAAAAAT GGAACCCAAA 2220
   ACACGGAGGA TATTGATCTC AATGAAATTA GAAAGATGGA TAAGGACTCT ATTGAGGGGA 2280
   AAATGCGACA ATCTGCAAAT GTTCCAAGCG AGATATCAGG AAGTGATGAC ATATTTACAA 2340
   CAGAACAAAG TAGAAACAGT GATCATGGAA GAAGCCTGGA ATCTATCAGT ACACCTGATA 2400
   CAAGATCAAT AAGTGTTGTT ACTGCTGCAA CACCAGATGA TGAAGAAGAA ATACTAATGA 2460
   AAAATAGTAG GACAAAGAAA AGTTCTTCAA CACATCAAGA AGATGACAAA AGAATTAAAA 2520
   AAGGGGGAAA AGGGAAAGAC TGGTTTAAGA AATCAAAAGA TACCGACAAC CAGATACCAA 2580
   CATCAGACTA CAGATCCACA TCAAAAGGGC AGAAGAAAAT CTCAAAGACA ACAACCACCA 2640
   ACACCGACAC AAAGGGGCAA ACAGAAATAC AGACAGAATC ATCAGAAACA CAATCCTCAT 2700
   CATGGAATCT CATCATCGAC AACAACACCG ACCGGAACGA ACAGACAAGC ACAACTCCTC 2760
   CAACAACAAC TTCCAGATCA ACTTATACAA AAGAATCGAT CCGAACAAAC TCTGAATCCA 2820
   AACCCAAGAC ACAAAAGACA AATGGAAAGG AAAGGAAGGA TACAGAAGAG AGCAATCGAT 2880
   TTACAGAGAG GGCAATTACT CTATTGCAGA ATCTTGGTGT AATTCAATCC ACATCAAAAC 2940
   TAGATTTATA TCAAGACAAA CGAGTTGTAT GTGTAGCAAA TGTACTAAAC AATGTAGATA 3000
   CTGCATCAAA GATAGATTTC CTGGCAGGAT TAGTCATAGG GGTTTCAATG GACAACGACA 3060
   CAAAATTAAC ACAGATACAA AATGAAATGC TAAACCTCAA AGCAGATCTA AAGAAAATGG 3120
   ACGAATCACA TAGAAGATTG ATAGAAAATC AAAGAGAACA ACTGTCATTG ATCACGTCAC 3180
   TAATTTCAAA TCTCAAAATT ATGACTGAGA GAGGAGGAAA GAAAGACCAA AATGAATCCA 3240
   ATGAGAGAGT ATCCATGATC AAAACAAAAT TGAAAGAAGA AAAGATCAAG AAGACCAGGT 3300
   TTGACCCACT TATGGAGGCA CAAGGCATTG ACAAGAATAT ACCCGATCTA TATCGACATG 3360
   CAGGAGATAC ACTAGAGAAC GATGTACAAG TTAAATCAGA GATATTAAGT TCATACAATG 3420
   AGTCAAATGC AACAAGACTA ATACCCAAAA AAGTGAGCAG TACAATGAGA TCACTAGTTG 3480
   CAGTCATCAA CAACAGCAAT CTCTCACAAA GCACAAAACA ATCATACATA AACGAACTCA 3540
   AACGTTGCAA AAATGATGAA GAAGTATCTG AATTAATGGA CATGTTCAAT GAAGATGTCA 3600
   ACAATTGCCA ATGATCCAAC AAAGAAACGA CACCGAACAA ACAGACAAGA AACAACAGTA 3660
   GATCAAAACC TGTCAACACA CACAAAATCA AGCAGAATGA AACAACAGAT ATCAATCAAT 3720
   ATACAAATAA GAAAAACTTA GGATTAAAGA ATAAATTAAT CCTTGTCCAA AATGAGTATA 3780
   ACTAACTCTG CAATATACAC ATTCCCAGAA TCATCATTCT CTGAAAATGG TCATATAGAA 3840
   CCATTACCAC TCAAAGTCAA TGAACAGAGG AAAGCAGTAC CCCACATTAG AGTTGCCAAG 3900
   ATCGGAAATC CACCAAAACA CGGATCCCGG TATTTAGATG TCTTCTTACT CGGCTTCTTC 3960
   GAGATGGAAC GAATCAAAGA CAAATACGGG AGTGTGAATG ATCTCGACAG TGACCCGAGT 4020
   TACAAAGTTT GTGGCTCTGG ATCATTACCA ATCGGATTGG CTAAGTACAC TGGGAATGAC 4080
   CAGGAATTGT TACAAGCCGC AACCAAACTG GATATAGAAG TGAGAAGAAC AGTCAAAGCG 4140
   AAAGAGATGG TTGTTTACAC GGTACAAAAT ATAAAACCAG AACTGTACCC ATGGTCCAAT 4200
   AGACTAAGAA AAGGAATGCT GTTCGATGCC AACAAAGTTG CTCTTGCTCC TCAATGTCTT 4260
   CCACTAGATA GGAGCATAAA ATTTAGAGTA ATCTTCGTGA ATTGTACGGC AATTGGATCA 4320
   ATAACCTTGT TCAAAATTCC TAAGTCAATG GCATCACTAT CTCTACCCAA CACAATATCA 4380
   ATCAATCTGC AGGTACACAT AAAAACAGGG GTTCAGACTG ATTCTAAAGG GATAGTTCAA 4440
   ATTTTGGATG AGAAAGGCGA AAAATCACTG AATTTCATGG TCCATCTCGG ATTGATCAAA 4500
   AGAAAAGTAG GCAGAATGTA CTCTGTTGAA TACTGTAAAC AGAAAATCGA GAAAATGAGA 4560
   TTGATATTTT CTTTAGGACT AGTTGGAGGA ATCAGTCTTC ATGTCAATGC AACTGGGTCC 4620
   ATATCAAAAA CACTAGCAAG TCAGCTGGTA TTCAAAAGAG AGATTTGTTA TCCTTTAATG 4680
   GATCTAAATC CGCATCTCAA TCTAGTTATC TGGGCTTCAT CAGTAGAGAT TACAAGAGTG 4740
   GATGCAATTT TCCAACCTTC TTTACCTGGC GAGTTCAGAT ACTATCCTAA TATTATTGCA 4800
   AAAGGAGTTG GGAAAATCAA ACAATGGAAC TAGTAATCTC TATTTTAGTC CGGACGTATC 4860
   TATTAAGCCG AAGCAAATAA AGGATAATCA AAAACTTAGG ACAAAAGAGG TCAATACCAA 4920
   CAACTATTAG CAGTCACACT CGCAAGAATA AGAGAGAAGG GACCAAAAAA GTCAAATAGG 4980
   AGAAATCAAA ACAAAAGGTA CAGAACACCA GAACAACAAA ATCAAAACAT CCAACTCACT 5040
   CAAAACAAAA ATTCCAAAAG AGACCGGCAA CACAACAAGC ACTGAACACA ATGCCAACTT 5100
   CAATACTGCT AATTATTACA ACCATGATCA TGGCATCTTT CTGCCAAATA GATATCACAA 5160
   AACTACAGCA CGTAGGTGTA TTGGTCAACA GTCCCAAAGG GATGAAGATA TCACAAAACT 5220
   TTGAAACAAG ATATCTAATT TTGAGCCTCA TACCAAAAAT AGAAGACTCT AACTCTTGTG 5280
   GTGACCAACA GATCAAGCAA TACAAGAAGT TATTGGATAG ACTGATCATC CCTTTATATG 5340
   ATGGATTAAG ATTACAGAAA GATGTGATAG TAACCAATCA AGAATCCAAT GAAAACACTG 5400
   ATCCCAGAAC AAAACGATTC TTTGGAGGGG TAATTGGAAC CATTGCTCTG GGAGTAGCAA 5460
   CCTCAGCACA AATTACAGCG GCAGTTGCTC TGGTTGAAGC CAAGCAGGCA AGATCAGACA 5520
   TCGAAAAACT CAAAGAAGCA ATTAGGGACA CAAACAAAGC AGTGCAGTCA GTTCAGAGCT 5580
   CCATAGGAAA TTTAATAGTA GCAATTAAAT CAGTCCAGGA TTATGTTAAC AAAGAAATCG 5640
   TGCCATCGAT TGCGAGGCTA GGTTGTGAAG CAGCAGGACT TCAATTAGGA ATTGCATTAA 5700
   CACAGCATTA CTCAGAATTA ACAAACATAT TTGGTGATAA CATAGGATCG TTACAAGAAA 5760
   AAGGAATAAA ATTACAAGGT ATAGCATCAT TATACCGCAC AAATATCACA GAAATATTCA 5820
   CAACATCAAC AGTTGATAAA TATGATATCT ATGATCTGTT ATTTACAGAA TCAATAAAGG 5880
   TGAGAGTTAT AGATGTTGAC TTGAATGATT ACTCAATCAC CCTCCAAGTC AGACTCCCTT 5940
   TATTAACTAG GCTGCTGAAC ACTCAGATCT ACAAAGTAGA TTCCATATCA TATAACATCC 6000
   AAAACAGAGA ATGGTATATC CCTCTTCCCA GCCATATCAT GACGAAAGGG GCATTTCTAG 6060
   GTGGAGCAGA CGTCAAAGAA TGTATAGAAG CATTCAGCAG CTATATATGC CCTTCTGATC 6120
   CAGGATTTGT ATTAAACCAT GAAATAGAGA GCTGCTTATC AGGAAACATA TCCCAATGTC 6180
   CAAGAACAAC GGTCACATCA GACATTGTTC CAAGATATGC ATTTGTCAAT GGAGGAGTGG 6240
   TTGCAAACTG TATAACAACC ACCTGTACAT GCAACGGAAT TGGTAATAGA ATCAATCAAC 6300
   CACCTGATCA AGGAGTAAAA ATTATAACAC ATAAAGAATG TAGTACAATA GGTATCAACG 6360
   GAATGCTGTT CAATACAAAT AAAGAAGGAA CTCTTGCATT CTATACACCA AATGATATAA 6420
   CACTAAACAA TTCTGTTGCA CTTGATCCAA TTGACATATC AATCGAGCTC AACAAGGCCA 6480
   AATCAGATCT AGAAGAATCA AAAGAATGGA TAAGAAGGTC AAATCAAAAA CTAGATTCTA 6540
   TTGGAAATTG GCATCAATCT AGCACTACAA TCATAATTAT TTTGATAATG ATCATTATAT 6600
   TGTTTATAAT TAATATAACG ATAATTACAA TTGCAATTAA GTATTACAGA ATTCAAAAGA 6660
   GAAATCGAGT GGATCAAAAT GACAAGCCAT ATGTACTAAC AAACAAATAA CATATCTACA 6720
   GATCATTAGA TATTAAAATT ATAAAAAACT TAGGAGTAAA GTTACGCAAT CCAACTCTAC 6780
   TCATATAATT GAGGAAGGAC CCAATAGACA AATCCAAATT CGAGATGGAA TACTGGAAGC 6840
   ATACCAATCA CGGAAAGGAT GCTGGTAATG AGCTGGAGAC GTCTATGGCT ACTCATGGCA 6900
   ACAAGCTCAC TAATAAGATA ATATACATAT TATGGACAAT AATCCTGGTG TTATTATCAA 6960
   TAGTCTTCAT CATAGTGCTA ATTAATTCCA TCAAAAGTGA AAAGGCCCAC GAATCATTGC 7020
   TGCAAGACAT AAATAATGAG TTTATGGAAA TTACAGAAAA GATCCAAATG GCATCGGATA 7080
   ATACCAATGA TCTAATACAG TCAGGAGTGA ATACAAGGCT TCTTACAATT CAGAGTCATG 7140
   TCCAGAATTA CATACCAATA TCATTGACAC AACAGATGTC AGATCTTAGG AAATTCATTA 7200
   GTGAAATTAC AATTAGAAAT GATAATCAAG AAGTGCTGCC ACAAAGAATA ACACATGATG 7260
   TAGGTATAAA ACCTTTAAAT CCAGATGATT TTTGGAGATG CACGTCTGGT CTTCCATCTT 7320
   TAATGAAAAC TCCAAAAATA AGGTTAATGC CAGGGCCGGG ATTATTAGCT ATGCCAACGA 7380
   CTGTTGATGG CTGTGTTAGA ACTCCGTCTT TAGTTATAAA TGATCTGATT TATGCTTATA 7440
   CCTCAAATCT AATTACTCGA GGTTGTCAGG ATATAGGAAA ATCATATCAA GTCTTACAGA 7500
   TAGGGATAAT AACTGTAAAC TCAGACTTGG TACCTGACTT AAATCCTAGG ATCTCTCATA 7560
   CCTTTAACAT AAATGACAAT AGGAAGTCAT GTTCTCTAGC ACTCCTAAAT ATAGATGTAT 7620
   ATCAACTGTG TTCAACTCCC AAAGTTGATG AAAGATCAGA TTATGCATCA TCAGGCATAG 7680
   AAGATATTGT ACTTGATATT GTCAATTATG ATGGTTCAAT CTCAACAACA AGATTTAAGA 7740
   ATAATAACAT AAGCTTTGAT CAACCATATG CTGCACTATA CCCATCTGTT GGACCAGGGA 7800
   TATACTACAA AGGCAAAATA ATATTTCTCG GGTATGGAGG TCTTGAACAT CCAATAAATG 7860
   AGAATGTAAT CTGCAACACA ACTGGGTGCC CCGGGAAAAC ACAGAGAGAC TGTAATCAAG 7920
   CATCTCATAG TACTTGGTTT TCAGATAGGA GGATGGTCAA CTCCATCATT GTTGTTGACA 7980
   AAGGCTTAAA CTCAATTCCA AAATTGAAAG TATGGACGAT ATCTATGCGA CAAAATTACT 8040
   GGGGGTCAGA AGGAAGGTTA CTTCTACTAG GTAACAAGAT CTATATATAT ACAAGATCTA 8100
   CAAGTTGGCA TAGCAAGTTA CAATTAGGAA TAATTGATAT TACTGATTAC AGTGATATAA 8160
   GGATAAAATG GACATGGCAT AATGTGCTAT CAAGACCAGG AAACAATGAA TGTCCATGGG 8220
   GACATTCATG TCCAGATGGA TGTATAACAG GAGTATATAC TGATGCATAT CCACTCAATC 8280
   CCACAGGGAG CATTGTGTCA TCTGTCATAT TAGACTCACA AAAATCGAGA GTGAACCCAG 8340
   TCATAACTTA CTCAACAGCA ACCGAAAGAG TAAACGAGCT GGCCATCCTA AACAGAACAC 8400
   TCTCAGCTGG ATATACAACA ACAAGCTGCA TTACACACTA TAACAAAGGA TATTGTTTTC 8460
   ATATAGTAGA AATAAATCAT AAAAGCTTAA ACACATTTCA ACCCATGTTG TTCAAAACAG 8520
   AGATTCCAAA AAGCTGCAGT TAATCATAAT TAACCATAAT ATGCATCAAT CTATCTATAA 8580
   TACAAGTATA TGATAAGTAA TCAGCAATCA GACAATAGAC AAAAGGGAAA TATAAAAAAC 8640
   TTAGGAGCAA AGCGTGCTCG GGAAATGGAC ACTGAATCTA ACAATGGCAC TGTATCTGAC 8700
   ATACTCTATC CTGAGTGTCA CCTTAACTCT CCTATCGTTA AAGGTAAAAT AGCACAATTA 8760
   CACACTATTA TGAGTCTACC TCAGCCTTAT GATATGGATG ACGACTCAAT ACTAGTTATC 8820
   ACTAGACAGA AAATAAAACT TAATAAATTG GATAAAAGAC AACGATCTAT TAGAAGATTA 8880
   AAATTAATAT TAACTGAAAA AGTGAATGAC TTAGGAAAAT ACACATTTAT CAGATATCCA 8940
   GAAATGTCAA AAGAAATGTT CAAATTATAT ATACCTGGTA TTAACAGTAA AGTGACTGAA 9000
   TTATTACTTA AAGCAGATAG AACATATAGT CAAATGACTG ATGGATTAAG AGATCTATGG 9060
   ATTAATGTGC TATCAAAATT AGCCTCAAAA AATGATGGAA GCAATTATGA TCTTAATGAA 9120
   GAAATTAATA ATATATCGAA AGTTCACACA ACCTATAAAT CAGATAAATG GTATAATCCA 9180
   TTCAAAACAT GGTTTACTAT CAAGTATGAT ATGAGAAGAT TACAAAAAGC TCGAAATGAG 9240
   ATCACTTTTA ATGTTGGGAA GGATTATAAC TTGTTAGAAG ACCAGAAGAA TTTCTTATTG 9300
   ATACATCCAG AATTGGTTTT GATATTAGAT AAACAAAACT ATAATGGTTA TCTAATTACT 9360
   CCTGAATTAG TATTGATGTA TTGTGACGTA GTCGAAGGCC GATGGAATAT AAGTGCATGT 9420
   GCTAAGTTAG ATCCAAAATT ACAATCTATG TATCAGAAAG GTAATAACCT GTGGGAAGTG 9480
   ATAGATAAAT TGTTTCCAAT TATGGGAGAA AAGACATTTG ATGTGATATC GTTATTAGAA 9540
   CCACTTGCAT TATCCTTAAT TCAAACTCAT GATCCTGTTA AACAACTAAG AGGAGCTTTT 9600
   TTAAATCATG TGTTATCCGA GATGGAATTA ATATTTGAAT CTAGAGAATC GATTAAGGAA 9660
   TTTCTGAGTG TAGATTACAT TGATAAAATT TTAGATATAT TTAATAAGTC TACAATAGAT 9720
   GAAATAGCAG AGATTTTCTC TTTTTTTAGA ACATTTGGGC ATCCTCCATT AGAAGCTAGT 9780
   ATTGCAGCAG AAAAGGTTAG AAAATATATG TATATTGGAA AACAATTAAA ATTTGACACT 9840
   ATTAATAAAT GTCATGCTAT CTTCTGTACA ATAATAATTA ACGGATATAG AGAGAGGCAT 9900
   GGTGGACAGT GGCCTCCTGT GACATTACCT GATCATGCAC ACGAATTCAT CATAAATGCT 9960
   TACGGTTCAA ACTCTGCGAT ATCATATGAA AATGCTGTTG ATTATTACCA GAGCTTTATA 10020
   GGAATAAAAT TCAATAAATT CATAGAGCCT CAGTTAGATG AGGATTTGAC AATTTATATG 10080
   AAAGATAAAG CATTATCTCC AAAAAAATCA AATTGGGACA CAGTTTATCC TGCATCTAAT 10140
   TTACTGTACC GTACTAACGC ATCCAACGAA TCACGAAGAT TAGTTGAAGT ATTTATAGCA 10200
   GATAGTAAAT TTGATCCTCA TCAGATATTG GATTATGTAG AATCTGGGGA CTGGTTAGAT 10260
   GATCCAGAAT TTAATATTTC TTATAGTCTT AAAGAAAAAG AGATCAAACA GGAAGGTAGA 10320
   CTCTTTGCAA AAATGACATA CAAAATGAGA GCTACACAAG TTTTATCAGA GACCCTACTT 10380
   GCAAATAACA TAGGAAAATT CTTTCAAGAA AATGGGATGG TGAAGGGAGA GATTGAATTA 10440
   CTTAAGAGAT TAACAACCAT ATCAATATCA GGAGTTCCAC GGTATAATGA AGTGTACAAT 10500
   AATTCTAAAA GCCATACAGA TGACCTTAAA ACCTACAATA AAATAAGTAA TCTTAATTTG 10560
   TCTTCTAATC AGAAATCAAA GAAATTTGAA TTCAAGTCAA CGGATATCTA CAATGATGGA 10620
   TACGAGACTG TGAGCTGTTT CCTAACAACA GATCTCAAAA AATACTGTCT TAATTGGAGA 10680
   TATGAATCAA CAGCTCTATT TGGAGAAACT TGCAACCAAA TATTTGGATT AAATAAATTG 10740
   TTTAATTGGT TACACCCTCG TCTTGAAGGA AGTACAATCT ATGTAGGTGA TCCTTACTGT 10800
   CCTCCATCAG ATAAAGAACA TATATCATTA GAGGATCACC CTGATTCTGG TTTTTACGTT 10860
   CATAACCCAA GAGGGGGTAT AGAAGGATTT TGTCAAAAAT TATGGACACT CATATCTATA 10920
   AGTGCAATAC ATCTAGCAGC TGTTAGAATA GGCGTGAGGG TGACTGCAAT GGTTCAAGGA 10980
   GACAATCAAG CTATAGCTGT AACCACAAGA GTACCCAACA ATTATGACTA CAGAGTTAAG 11040
   AAGGAGATAG TTTATAAAGA TGTAGTGAGA TTTTTTGATT CATTAAGAGA AGTGATGGAT 11100
   GATCTAGGTC ATGAACTTAA ATTAAATGAA ACGATTATAA GTAGCAAGAT GTTCATATAT 11160
   AGCAAAAGAA TCTATTATGA TGGGAGAATT CTTCCTCAAG CTCTAAAAGC ATTATCTAGA 11220
   TGTGTCTTCT GGTCAGAGAC AGTAATAGAC GAAACAAGAT CAGCATCTTC AAATTTGGCA 11280
   ACATCATTTG CAAAAGCAAT TGAGAATGGT TATTCACCTG TTCTAGGATA TGCATGCTCA 11340
   ATTTTTAAGA ATATTCAACA ACTATATATT GCCCTTGGGA TGAATATCAA TCCAACTATA 11400
   ACACAGAATA TCAGAGATCA GTATTTTAGG AATCCAAATT GGATGCAATA TGCCTCTTTA 11460
   ATACCTGCTA GTGTTGGGGG ATTCAATTAC ATGGCCATGT CAAGATGTTT TGTAAGGAAT 11520
   ATTGGTGATC CATCAGTTGC CGCATTGGCT GATATTAAAA GATTTATTAA GGCGAATCTA 11580
   TTAGACCGAA GTGTTCTTTA TAGGATTATG AATCAAGAAC CAGGTGAGTC ATCTTTTTTG 11640
   GACTGGGCTT CAGATCCATA TTCATGCAAT TTACCACAAT CTCAAAATAT AACCACCATG 11700
   ATAAAAAATA TAACAGCAAG GAATGTATTA CAAGATTCAC CAAATCCATT ATTATCTGGA 11760
   TTATTCACAA ATACAATGAT AGAAGAAGAT GAAGAATTAG CTGAGTTCCT GATGGACAGG 11820
   AAGGTAATTC TCCCTAGAGT TGCACATGAT ATTCTAGATA ATTCTCTCAC AGGAATTAGA 11880
   AATGCCATAG CTGGAATGTT AGATACGACA AAATCACTAA TTCGGGTTGG CATAAATAGA 11940
   GGAGGACTGA CATATAGTTT GTTGAGGAAA ATCAGTAATT ACGATCTAGT ACAATATGAA 12000
   ACACTAAGTA GGACTTTGCG ACTAATTGTA AGTGATAAAA TCAAGTATGA AGATATGTGT 12060
   TCGGTAGACC TTGCCATAGC ATTGCGACAA AAGATGTGGA TTCATTTATC AGGAGGAAGG 12120
   ATGATAAGTG GACTTGAAAC GCCTGACCCA TTAGAATTAC TATCTGGGGT AGTAATAACA 12180
   GGATCAGAAC ATTGTAAAAT ATGTTATTCT TCAGATGGCA CAAACCCATA TACTTGGATG 12240
   TATTTACCCG GTAATATCAA AATAGGATCA GCAGAAACAG GTATATCGTC ATTAAGAGTT 12300
   CCTTATTTTG GATCAGTCAC TGATGAAAGA TCTGAAGCAC AATTAGGATA TATCAAGAAT 12360
   CTTAGTAAAC CTGCAAAAGC CGCAATAAGA ATAGCAATGA TATATACATG GGCATTTGGT 12420
   AATGATGAGA TATCTTGGAT GGAAGCCTCA CAGATAGCAC AAACACGTGC AAATTTTACA 12480
   CTAGATAGTC TCAAAATTTT AACACCGGTA GCTACATCAA CAAATTTATC ACACAGATTA 12540
   AAGGATACTG CAACTCAGAT GAAATTCTCC AGTACATCAT TGATCAGAGT CAGCAGATTC 12600
   ATAACAATGT CCAATGATAA CATGTCTATC AAAGAAGCTA ATGAAACCAA AGATACTAAT 12660
   CTTATTTATC AACAAATAAT GTTAACAGGA TTAAGTGTTT TCGAATATTT ATTTAGATTA 12720
   AAAGAAACCA CAGGACACAA CCCTATAGTT ATGCATCTGC ACATAGAAGA TGAGTGTTGT 12780
   ATTAAAGAAA GTTTTAATGA TGAACATATT AATCCAGAGT CTACATTAGA ATTAATTCGA 12840
   TATCCTGAAA GTAATGAATT TATTTATGAT AAAGACCCAC TCAAAGATGT GGACTTATCA 12900
   AAACTTATGG TTATTAAAGA CCATTCTTAC ACAATTGATA TGAATTATTG GGATGATACT 12960
   GACATCATAC ATGCAATTTC AATATGTACT GCAATTACAA TAGCAGATAC TATGTCACAA 13020
   TTAGATCGAG ATAATTTAAA AGAGATAATA GTTATTGCAA ATGATGATGA TATTAATAGC 13080
   TTAATCACTG AATTTTTGAC TCTTGACATA CTTGTATTTC TCAAGACATT TGGTGGATTA 13140
   TTAGTAAATC AATTTGCATA CACTCTTTAT AGTCTAAAAA TAGAAGGTAG GGATCTCATT 13200
   TGGGATTATA TAATGAGAAC ACTGAGAGAT ACTTCCCATT CAATATTAAA AGTATTATCT 13260
   AATGCATTAT CTCATCCTAA AGTATTCAAG AGGTTCTGGG ATTGTGGAGT TTTAAACCCT 13320
   ATTTATGGTC CTAATACTGC TAGTCAAGAC CAGATAAAAC TTGCCCTATC TATATGTGAA 13380
   TATTCACTAG ATCTATTTAT GAGAGAATGG TTGAATGGTG TATCACTTGA AATATACATT 13440
   TGTGACAGCG ATATGGAAGT TGCAAATGAT AGGAAACAAG CCTTTATTTC TAGACACCTT 13500
   TCATTTGTTT GTTGTTTAGC AGAAATTGCA TCTTTCGGAC CTAACCTGTT AAACTTAACA 13560
   TACTTGGAGA GACTTGATCT ATTGAAACAA TATCTTGAAT TAAATATTAA AGAAGACCCT 13620
   ACTCTTAAAT ATGTACAAAT ATCTGGATTA TTAATTAAAT CGTTCCCATC AACTGTAACA 13680
   TACGTAAGAA AGACTGCAAT CAAATATCTA AGGATTCGCG GTATTAGTCC ACCTGAGGTA 13740
   ATTGATGATT GGGATCCGGT AGAAGATGAA AATATGCTGG ATAACATTGT CAAAACTATA 13800
   AATGATAACT GTAATAAAGA TAATAAAGGG AATAAAATTA ACAATTTCTG GGGACTAGCA 13860
   CTTAAGAACT ATCAAGTCCT TAAAATCAGA TCTATAACAA GTGATTCTGA TGATAATGAT 13920
   AGACTAGATG CTAATACAAG TGGTTTGACA CTTCCTCAAG GAGGGAATTA TCTATCGCAT 13980
   CAATTGAGAT TATTCGGAAT CAACAGCACT AGTTGTCTGA AAGCTCTTGA GTTATCACAA 14040
   ATTTTAATGA AGGAAGTCAA TAAAGACAAG GACAGGCTCT TCCTGGGAGA AGGAGCAGGA 14100
   GCTATGCTAG CATGTTATGA TGCCACATTA GGACCTGCAG TTAATTATTA TAATTCAGGT 14160
   TTGAATATAA CAGATGTAAT TGGTCAACGA GAATTGAAAA TATTTCCTTC AGAGGTATCA 14220
   TTAGTAGGTA AAAAATTAGG AAATGTGACA CAGATTCTTA ACAGGGTAAA AGTACTGTTC 14280
   AATGGGAATC CTAATTCAAC ATGGATAGGA AATATGGAAT GTGAGAGCTT AATATGGAGT 14340
   GAATTAAATG ATAAGTCCAT TGGATTAGTA CATTGTGATA TGGAAGGAGC TATCGGTAAA 14400
   TCAGAAGAAA CTGTTCTACA TGAACATTAT AGTGTTATAA GAATTACATA CTTGATTGGG 14460
   GATGATGATG TTGTTTTAGT TTCCAAAATT ATACCTACAA TCACTCCGAA TTGGTCTAGA 14520
   ATACTTTATC TATATAAATT ATATTGGAAA GATGTAAGTA TAATATCACT CAAAACTTCT 14580
   AATCCTGCAT CAACAGAATT ATATCTAATT TCGAAAGATG CATATTGTAC TATAATGGAA 14640
   CCTAGTGAAA TTGTTTTATC AAAACTTAAA AGATTGTCAC TCTTGGAAGA AAATAATCTA 14700
   TTAAAATGGA TCATTTTATC AAAGAAGAGG AATAATGAAT GGTTACATCA TGAAATCAAA 14760
   GAAGGAGAAA GAGATTATGG AATCATGAGA CCATATCATA TGGCACTACA AATCTTTGGA 14820
   TTTCAAATCA ATTTAAATCA TCTGGCGAAA GAATTTTTAT CAACCCCAGA TCTGACTAAT 14880
   ATCAACAATA TAATCCAAAG TTTTCAGCGA ACAATAAAGG ATGTTTTATT TGAATGGATT 14940
   AATATAACTC ATGATGATAA GAGACATAAA TTAGGCGGAA GATATAACAT ATTCCCACTG 15000
   AAAAATAAGG GAAAGTTAAG ACTGCTATCG AGAAGACTAG TATTAAGTTG GATTTCATTA 15060
   TCATTATCGA CTCGATTACT TACAGGTCGC TTTCCTGATG AAAAATTTGA ACATAGAGCA 15120
   CAGACTGGAT ATGTATCATT AGCTGATACT GATTTAGAAT CATTAAAGTT ATTGTCGAAA 15180
   AACATCATTA AGAATTACAG AGAGTGTATA GGATCAATAT CATATTGGTT TCTAACCAAA 15240
   GAAGTTAAAA TACTTATGAA ATTGATCGGT GGTGCTAAAT TATTAGGAAT TCCCAGACAA 15300
   TATAAAGAAC CCGAAGACCA GTTATTAGAA AACTACAATC AACATGATGA ATTTGATATC 15360
   GATTAAAACA TAAATACAAT GAAGATATAT CCTAACCTTT ATCTTTAAGC CTAGGAATAG 15420
   ACAAAAAGTA AGAAAAACAT GTAATATATA TATACCAAAC AGAGTTCTTC TCTTGTTTGG 15480
   TGGGTCGGCA TGGCATCTCC ACCTCCTCGC GGTCCGGACC TGGGCATCCG AAGGAGGACG 15540
   CACGTCCACT CGGATGGCTA AGGGAGAGCC TGCAGTAGCA TAACCCCTTG GGCCTCTAAA 15600
   ACGGGTCTTG AGGGGTTTTT TGCTGAAAGG AGGAACTATA TACGCGTCGA CGGGCCCCGC 15660
   GCTCAC 15666
(2) INFORMATION FOR SEQ ID NO:16:
   (i) SEQUENCE CHARACTERISTICS:
      (A) LENGTH: 28 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:16:
      GGATTTGCGC GCAATTTAAA TCATCTGG 28
(2) INFORMATION FOR SEQ ID NO:17:
   (i) SEQUENCE CHARACTERISTICS:
      (A) LENGTH: 70 base pairs
      (B) TYPE: nucleic acid
      (C) STRANDEDNESS: single
      (D) TOPOLOGY: linear
   (ii) MOLECULE TYPE: cDNA (xi) SEQUENCE DESCRIPTION: SEQ ID NO:17:
(2) INFORMATION FOR SEQ ID NO:18:
   (i) SEQUENCE CHARACTERISTICS:
      (A) LENGTH: 45 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:18:
      GGCCCGTCGA CGCGTAATAC GACTCACTAT AGGACCAAAC AAGAG 45
(2) INFORMATION FOR SEQ ID NO:19:
   (i) SEQUENCE CHARACTERISTICS:
      (A) LENGTH: 16 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:19:
      CGGCATCACG TGCTAC 16
(2) INFORMATION FOR SEQ ID NO:20:
   (i) SEQUENCE CHARACTERISTICS:
      (A) LENGTH: 6843 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:20:
   ATGTTGAGCC TATTTGATAC ATTTAATGCA CGTAGGCAAG AAAACATAAC AAAATCAGCC    60
   GGTGGAGCTA TCATTCCTGG ACAGAAAAAT ACTGTCTCTA TATTCGCCCT TGGACCGACA   120
   ATAACTGATG ATAATGAGAA AATGACATTA GCTCTTCTAT TTCTATCTCA TTCACTAGAT   180
   AATGAGAAAC AACATGCACA AAGGGCAGGG TTCTTGGTGT CTTTATTGTC AATGGCTTAT   240
   GCCAATCCAG AGCTCTACCT AACAACAAAT GGAAGTAATG CAGATGTCAA GTATGTCATA   300
   TACATGATTG AGAAAGATCT AAAACGGCAA AAGTATGGAG GATTTGTGGT TAAGACGAGA   360
   GAGATGATAT ATGAAAAGAC AACTGATTGG ATATTTGGAA GTGACCTGGA TTATGATCAG   420
   GAAACTATGT TGCAGAACGG CAGGAACAAT TCAACAATTG AAGACCTTGT CCACACATTT   480
   GGGTATCCAT CATGTTTAGG AGCTCTTATA ATACAGATCT GGATAGTTCT GGTCAAAGCT   540
   ATCACTAGTA TCTCAGGGTT AAGAAAAGGC TTTTTCACCC GATTGGAAGC TTTCAGACAA 600
   GATGGAACAG TGCAGGCAGG GCTGGTATTG AGCGGTGACA CAGTGGATCA GATTGGGTCA 660
   ATCATGCGGT CTCAACAGAG CTTGGTAACT CTTATGGTTG AAACATTAAT AACAATGAAT 720
   ACCAGCAGAA ATGACCTCAC AACCATAGAA AAGAATATAC AAATTGTTGG CAACTACATA 780
   AGAGATGCAG GTCTCGCTTC ATTCTTCAAT ACAATCAGAT ATGGAATTGA GACCAGAATG 840
   GCAGCTTTGA CTCTATCCAC TCTCAGACCA GATATCAATA GATTAAAAGC TTTGATGGAA 900
   CTGTATTTAT CAAAGGGACC ACGCGCTCCT TTCATCTGTA TCCTCAGAGA TCCTATACAT 960
   GGTGAGTTCG CACCAGGCAA CTATCCTGCC ATATGGAGCT ATGCAATGGG GGTGGCAGTT 1020
   GTACAAAATA GAGCCATGCA ACAGTATGTG ACGGGAAGAT CATATCTAGA CATTGATATG 1080
   TTCCAGCTAG GACAAGCAGT AGCACGTGAT GCCGAAGCTC AAATGAGCTC AACACTGGAA 1140
   GATGAACTTG GAGTGACACA CGAATCTAAA GAAAGCTTGA AGAGACATAT AAGGAACATA 1200
   AACAGTTCAG AGACATCTTT CCACAAACCG ACAGGTGGAT CAGCCATAGA GATGGCAATA 1260
   GATGAAGAGC CAGAACAATT CGAACATAGA GCAGATCAAG AACAAAATGG AGAACCTCAA 1320
   TCATCCATAA TTCAATATGC CTGGGCAGAA GGAAATAGAA GCGATGATCA GACTGAGCAA 1380
   GCTACAGAAT CTGACAATAT CAAGACCGAA CAACAAAACA TCAGAGACAG ACTAAACAAG 1440
   AGACTCAACG ACAAGAAGAA ACAAAGCAGT CAACCACCCA CTAATCCCAC AAACAGAACA 1500
   AACCAGGACG AAATAGATGA TCTGTTTAAC GCATTTGGAA GCAACTAAGT CGACGATCCG 1560
   GCTGCTAACA AAGCCCGAAA GGAAGCTGAG TTGGCTGCTG CCACCGCTGA GCAATAACTA 1620
   GCATAACCCC TTGGGGCCTC TAAACGGGTC TTGAGGGGTT TTTTGCTGAA AGGAGGAACT 1680
   ATATCCGGAT CGAGATCAAT TCTGTGAGCG TATGGCAAAC GAAGGAAAAA TAGTTATAGT 1740
   AGCCGCACTC GATGGGACAT TTCAACGTAA ACCGTTTAAT AATATTTTGA ATCTTATTCC 1800
   ATTATCTGAA ATGGTGGTAA AACTAACTGC TGTGTGTATG AAATGCTTTA AGGAGGCTTC 1860
   CTTTTCTAAA CGATTGGGTG AGGAAACCGA GATAGAAATA ATAGGAGGTA ATGATATGTA 1920
   TCAATCGGTG TGTAGAAAGT GTTACATCGA CTCATAATAT TATATTTTTT ATCTAAAAAA 1980
   CTAAAAATAA ACATTGATTA AATTTTAATA TAATACTTAA AAATGGATGT TGTGTCGTTA 2040
   GATAAACCGT TTATGTATTT TGAGGAAATT GATAATGAGT TAGATTACGA ACCAGAAAGT 2100
   GCAAATGAGG TCGCAAAAAA ACTGCCGTAT CAAGGACAGT TAAAACTATT ACTAGGAGAA 2160
   TTATTTTTTC TTAGTAAGTT ACAGCGACAC GGTATATTAG ATGGTGCCAC CGTAGTGTAT 2220
   ATAGGATCTG CTCCCGGTAC ACATATACGT TATTTGAGAG ATCATTTCTA TAATTTAGGA 2280
   GTGATCATCA AATGGATGCT AATTGACGGC CGCCATCATG ATCCTATTTT AAATGGATTG 2340
   CGTGATGTGA CTCTAGTGAC TCGGTTCGTT GATGAGGAAT ATCTACGATC CATCAAAAAA 2400
   CAACTGCATC CTTCTAAGAT TATTTTAATT TCTGATGTGA GATCCAAACG AGGAGGAAAT 2460
   GAACCTAGTA CGGCGGATTT ACTAAGTAAT TACGCTCTAC AAAATGTCAT GATTAGTATT 2520
   TTAAACCCGG TGGCGTCTAG TCTTAAATGG AGATGCCCGT TTCCAGATCA ATGGATCAAG 2580
   GACTTTTATA TCCCACACGG TAATAAAATG TTACAACCTT TTGCTCCTTC ATATTCAGGG 2640
   CCGTCGTTTT ACAACGTCGT GACTGGGAAA ACCCTGGCGT TACCCAACTT AATCGCCTTG 2700
   CAGCACATCC CCCTTTCGCC AGCTGGCGTA ATAGCGAAGA GGCCCGCACC GATCGCCCTT 2760
   CCCAACAGTT GCGCAGCCTG AATGGCGAAT GGCGCGACGC GCCCTGTAGC GGCGCATTAA 2820
   GCGCGGCGGG TGTGGTGGTT ACGCGCAGCG TGACCGCTAC ACTTGCCAGC GCCCTAGCGC 2880
   CCGCTCCTTT CGCTTTCTTC CCTTCCTTTC TCGCCACGTT CGCCGGCTTT CCCCGTCAAG 2940
   CTCTAAATCG GGGGCTCCCT TTAGGGTTCC GATTTAGTGC TTTACGGCAC CTCGACCCCA 3000
   AAAAACTTGA TTAGGGTGAT GGTTCACGTA GTGGGCCATC GCCCTGATAG ACGGTTTTTC 3060
   GCCCTTTGAC GTTGGAGTCC ACGTTCTTTA ATAGTGGACT CTTGTTCCAA ACTGGAACAA 3120
   CACTCAACCC TATCTCGGTC TATTCTTTTG ATTTATAAGG GATTTTGCCG ATTTCGGCCT 3180
   ATTGGTTAAA AAATGAGCTG ATTTAACAAA AATTTAACGC GAATTTTAAC AAAATATTAA 3240
   CGTTTACAAT TTCCCAGGTG GCACTTTTCG GGGAAATGTG CGCGGAACCC CTATTTGTTT 3300
   ATTTTTCTAA ATACATTCAA ATATGTATCC GCTCATGAGA CAATAACCCT GATAAATGCT 3360
   TCAATAATAT TGAAAAAGGA AGAGTATGAG TATTCAACAT TTCCGTGTCG CCCTTATTCC 3420
   CTTTTTTGCG GCATTTTGCC TTCCTGTTTT TGCTCACCCA GAAACGCTGG TGAAAGTAAA 3480
   AGATGCTGAA GATCAGTTGG GTGCACGAGT GGGTTACATC GAACTGGATC TCAACAGCGG 3540
   TAAGATCCTT GAGAGTTTTC GCCCCGAAGA ACGTTTTCCA ATGATGAGCA CTTTTAAAGT 3600
   TCTGCTATGT GGCGCGGTAT TATCCCGTAT TGACGCCGGG CAAGAGCAAC TCGGTCGCCG 3660
   CATACACTAT TCTCAGAATG ACTTGGTTGA GTACTCACCA GTCACAGAAA AGCATCTTAC 3720
   GGATGGCATG ACAGTAAGAG AATTATGCAG TGCTGCCATA ACCATGAGTG ATAACACTGC 3780
   GGCCAACTTA CTTCTGACAA CGATCGGAGG ACCGAAGGAG CTAACCGCTT TTTTGCACAA 3840
   CATGGGGGAT CATGTAACTC GCCTTGATCG TTGGGAACCG GAGCTGAATG AAGCCATACC 3900
   AAACGACGAG CGTGACACCA CGATGCCTGT AGCAATGGCA ACAACGTTGC GCAAACTATT 3960
   AACTGGCGAA CTACTTACTC TAGCTTCCCG GCAACAATTA ATAGACTGGA TGGAGGCGGA 4020
   TAAAGTTGCA GGACCACTTC TGCGCTCGGC CCTTCCGGCT GGCTGGTTTA TTGCTGATAA 4080
   ATCTGGAGCC GGTGAGCGTG GGTCTCGCGG TATCATTGCA GCACTGGGGC CAGATGGTAA 4140
   GCCCTCCCGT ATCGTAGTTA TCTACACGAC GGGGAGTCAG GCAACTATGG ATGAACGAAA 4200
   TAGACAGATC GCTGAGATAG GTGCCTCACT GATTAAGCAT TGGTAACTGT CAGACCAAGT 4260
   TTACTCATAT ATACTTTAGA TTGATTTAAA ACTTCATTTT TAATTTAAAA GGATCTAGGT 4320
   GAAGATCCTT TTTGATAATC TCATGACCAA AATCCCTTAA CGTGAGTTTT CGTTCCACTG 4380
   AGCGTCAGAC CCCGTAGAAA AGATCAAAGG ATCTTCTTGA GATCCTTTTT TTCTGCGCGT 4440
   AATCTGCTGC TTGCAAACAA AAAAACCACC GCTACCAGCG GTGGTTTGTT TGCCGGATCA 4500
   AGAGCTACCA ACTCTTTTTC CGAAGGTAAC TGGCTTCAGC AGAGCGCAGA TACCAAATAC 4560
   TGTCCTTCTA GTGTAGCCGT AGTTAGGCCA CCACTTCAAG AACTCTGTAG CACCGCCTAC 4620
   ATACCTCGCT CTGCTAATCC TGTTACCAGT GGCTGCTGCC AGTGGCGATA AGTCGTGTCT 4680
   TACCGGGTTG GACTCAAGAC GATAGTTACC GGATAAGGCG CAGCGGTCGG GCTGAACGGG 4740
   GGGTTCGTGC ACACAGCCCA GCTTGGAGCG AACGACCTAC ACCGAACTGA GATACCTACA 4800
   GCGTGAGCTA TGAGAAAGCG CCACGCTTCC CGAAGGGAGA AAGGCGGACA GGTATCCGGT 4860
   AAGCGGCAGG GTCGGAACAG GAGAGCGCAC GAGGGAGCTT CCAGGGGGAA ACGCCTGGTA 4920
   TCTTTATAGT CCTGTCGGGT TTCGCCACCT CTGACTTGAG CGTCGATTTT TGTGATGCTC 4980
   GTCAGGGGGG CGGAGCCTAT GGAAAAACGC CAGCAACGCG GCCTTTTTAC GGTTCCTGGC 5040
   CTTTTGCTGG CCTTTTGCTC ACATGTTCTT TCCTGCGTTA TCCCCTGATT CTGTGGATAA 5100
   CCGTATTACC GCCTTTGAGT GAGCTGATAC CGCTCGCCGC AGCCGAACGA CCGAGCGCAG 5160
   CGAGTCAGTG AGCGAGGAAG CGGAAGAGCG CCCAATACGC AAACCGCCTC TCCCCGCGCG 5220
   TTGGCCGATT CATTAATGCA GCTGGCACGA CAGGTTTCCC GACTGGAAAG CGGGCAGTGA 5280
   GCGCAACGCA ATTAATGTGA GTTAGCTCAC TCATTAGGCA CCCCAGGCTT TACACTTTAT 5340
   GCTTCCGGCT CGTATGTTGT GTGGAATTGT GAGCGGATAA CAATTTCACA CAGGAAACAG 5400
   CTATGACCAT GATTACGCCA AGCTTTTGCG ATCAATAAAT GGATCACAAC CAGTATCTCT 5460
   TAACGATGTT CTTCGCAGAT GATGATTCAT TTTTTAAGTA TTTGGCTAGT CAAGATGATG 5520
   AATCTTCATT ATCTGATATA TTGCAAATCA CTCAATATCT AGACTTTCTG TTATTATTAT 5580
   TGATCCAATC AAAAAATAAA TTAGAAGCCG TGGGTCATTG TTATGAATCT CTTTCAGAGG 5640
   AATACAGACA ATTGACAAAA TTCACAGACT TTCAAGATTT TAAAAAACTG TTTAACAAGG 5700
   TCCCTATTGT TACAGATGGA AGGGTCAAAC TTAATAAAGG ATATTTGTTC GACTTTGTGA 5760
   TTAGTTTGAT GCGATTCAAA AAAGAATCCT CTCTAGCTAC CACCGCAATA GATCCTGTTA 5820
   GATACATAGA TCCTCGTCGC AATATCGCAT TTTCTAACGT GATGGATATA TTAAAGTCGA 5880
   ATAAAGTGAA CAATAATTAA TTCTTTATTG TCATCATGAA CGGCGGACAT ATTCAGTTGA 5940
   TAATCGGCCC CATGTTTTCA GGTAAAAGTA CAGAATTAAT TAGACGAGTT AGACGTTATC 6000
   AAATAGCTCA ATATAAATGC GTGACTATAA AATATTCTAA CGATAATAGA TACGGAACGG 6060
   GACTATGGAC GCATGATAAG AATAATTTTG AAGCATTGGA AGCAACTAAA CTATGTGATG 6120
   TCTTGGAATC AATTACAGAT TTCTCCGTGA TAGGTATCGA TGAAGGACAG TTCTTTCCAG 6180
   ACATTGTTGA ATTGATCTCG ATCCCGCGAA ATTAATACGA CTCACTATAG GGAGACCACA 6240
   ACGGTTTCCC TCTAGCGGGA TCAATTCCGC CCCTCTCCCT CCCCCCCCCC TAACGTTACT 6300
   GGCCGAAGCC GCTTGGAATA AGGCCGGTGT GCGTTTGTCT ATATGTTATT TTCCACCATA 6360
   TTGCCGTCTT TTGGCAATGT GAGGGCCCGG AAACCTGGCC CTGTCTTCTT GACGAGCATT 6420
   CCTAGGGGTC TTTCCCCTCT CGCCAAAGGA ATGCAAGGTC TGTTGAATGT CGTGAAGGAA 6480
   GCAGTTCCTC TGGAAGCTTC TTGAAGACAA ACAACGTCTG TAGCGACCCT TTGCAGGCAG 6540
   CGGAACCCCC CACCTGGCGA CAGGTGCCTC TGCGGCCAAA AGCCACGTGT ATAAGATACA 6600
   CCTGCAAAGG CGGCACAACC CCAGTGCCAC GTTGTGAGTT GGATAGTTGT GGAAAGAGTC 6660
   AAATGGCTCT CCTCAAGCGT ATTCAACAAG GGGCTGAAGG ATGCCCAGAA GGTACCCCAT 6720
   TGTATGGGAT CTGATCTGGG GCCTCGGTGC ACATGCTTTA CATGTGTTTA GTCGAGGTTA 6780
   AAAAACGTCT AGGCCCCCCG AACCACGGGG ACGTGGTTTT CCTTTGAAAA ACACGATAAT 6840
   ACC 6843
(2) INFORMATION FOR SEQ ID NO:21:
   (i) SEQUENCE CHARACTERISTICS:
      (A) LENGTH: 7107 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:21:
   ATGGAAAGCG ACGCTAAAAA CTATCAAATC ATGGATTCTT GGGAAGAGGA ATCAAGAGAT     60
   AAATCAACTA ATATCTCCTC GGCCCTCAAC ATCATTGAAT TCATACTCAG CACCGACCCC    120
   CAAGAAGACT TATCGGAAAA CGACACAATC AACACAAGAA CCCAGCAACT CAGTGCCACC    180
   ATCTGTCAAC CAGAAATCAA ACCAACAGAA ACAAGTGAGA AAGATAGTGG ATCAACTGAC    240
   AAAAATAGAC AGTCCGGGTC ATCACACGAA TGTACAACAG AAGCAAAAGA TAGAAATATT    300
   GATCAGGAAA CTGTACAGAG AGGACCTGGG AGAAGAAGCA GCTCAGATAG TAGAGCTGAG    360
   ACTGTGGTCT CTGGAGGAAT CCCCAGAAGC ATCACAGATT CTAAAAATGG AACCCAAAAC    420
   ACGGAGGATA TTGATCTCAA TGAAATTAGA AAGATGGATA AGGACTCTAT TGAGGGGAAA    480
   ATGCGACAAT CTGCAAATGT TCCAAGCGAG ATATCAGGAA GTGATGACAT ATTTACAACA    540
   GAACAAAGTA GAAACAGTGA TCATGGAAGA AGCCTGGAAT CTATCAGTAC ACCTGATACA    600
   AGATCAATAA GTGTTGTTAC TGCTGCAACA CCAGATGATG AAGAAGAAAT ACTAATGAAA    660
   AATAGTAGGA CAAAGAAAAG TTCTTCAACA CATCAAGAAG ATGACAAAAG AATTAAAAAA    720
   GGGGGAAAAG GGAAAGACTG GTTTAAGAAA TCAAAAGATA CCGACAACCA GATACCAACA    780
   TCAGACTACA GATCCACATC AAAAGGGCAG AAGAAAATCT CAAAGACAAC AACCACCAAC    840
   ACCGACACAA AGGGGCAAAC AGAAATACAG ACAGAATCAT CAGAAACACA ATCCTCATCA    900
   TGGAATCTCA TCATCGACAA CAACACCGAC CGGAACGAAC AGACAAGCAC AACTCCTCCA    960
   ACAACAACTT CCAGATCAAC TTATACAAAA GAATCGATCC GAACAAACTC TGAATCCAAA   1020
   CCCAAGACAC AAAAGACAAA TGGAAAGGAA AGGAAGGATA CAGAAGAGAG CAATCGATTT   1080
   ACAGAGAGGG CAATTACTCT ATTGCAGAAT CTTGGTGTAA TTCAATCCAC ATCAAAACTA   1140
   GATTTATATC AAGACAAACG AGTTGTATGT GTAGCAAATG TACTAAACAA TGTAGATACT   1200
   GCATCAAAGA TAGATTTCCT GGCAGGATTA GTCATAGGGG TTTCAATGGA CAACGACACA   1260
   AAATTAACAC AGATACAAAA TGAAATGCTA AACCTCAAAG CAGATCTAAA GAAAATGGAC   1320
   GAATCACATA GAAGATTGAT AGAAAATCAA AGAGAACAAC TGTCATTGAT CACGTCACTA   1380
   ATTTCAAATC TCAAAATTAT GACTGAGAGA GGAGGAAAGA AAGACCAAAA TGAATCCAAT   1440
   GAGAGAGTAT CCATGATCAA AACAAAATTG AAAGAAGAAA AGATCAAGAA GACCAGGTTT   1500
   GACCCACTTA TGGAGGCACA AGGCATTGAC AAGAATATAC CCGATCTATA TCGACATGCA   1560
   GGAGATACAC TAGAGAACGA TGTACAAGTT AAATCAGAGA TATTAAGTTC ATACAATGAG   1620
   TCAAATGCAA CAAGACTAAT ACCCAAAAAA GTGAGCAGTA CAATGAGATC ACTAGTTGCA 1680
   GTCATCAACA ACAGCAATCT CTCACAAAGC ACAAAACAAT CATACATAAA CGAACTCAAA 1740
   CGTTGCAAAA ATGATGAAGA AGTATCTGAA TTAATGGACA TGTTCAATGA AGATGTCAAC 1800
   AATTGCCAAT GAGTCGACGA TCCGGCTGCT AACAAAGCCC GAAAGGAAGC TGAGTTGGCT 1860
   GCTGCCACCG CTGAGCAATA ACTAGCATAA CCCCTTGGGG CCTCTAAACG GGTCTTGAGG 1920
   GGTTTTTTGC TGAAAGGAGG AACTATATCC GGATCGAGAT CAATTCTGTG AGCGTATGGC 1980
   AAACGAAGGA AAAATAGTTA TAGTAGCCGC ACTCGATGGG ACATTTCAAC GTAAACCGTT 2040
   TAATAATATT TTGAATCTTA TTCCATTATC TGAAATGGTG GTAAAACTAA CTGCTGTGTG 2100
   TATGAAATGC TTTAAGGAGG CTTCCTTTTC TAAACGATTG GGTGAGGAAA CCGAGATAGA 2160
   AATAATAGGA GGTAATGATA TGTATCAATC GGTGTGTAGA AAGTGTTACA TCGACTCATA 2220
   ATATTATATT TTTTATCTAA AAAACTAAAA ATAAACATTG ATTAAATTTT AATATAATAC 2280
   TTAAAAATGG ATGTTGTGTC GTTAGATAAA CCGTTTATGT ATTTTGAGGA AATTGATAAT 2340
   GAGTTAGATT ACGAACCAGA AAGTGCAAAT GAGGTCGCAA AAAAACTGCC GTATCAAGGA 2400
   CAGTTAAAAC TATTACTAGG AGAATTATTT TTTCTTAGTA AGTTACAGCG ACACGGTATA 2460
   TTAGATGGTG CCACCGTAGT GTATATAGGA TCTGCTCCCG GTACACATAT ACGTTATTTG 2520
   AGAGATCATT TCTATAATTT AGGAGTGATC ATCAAATGGA TGCTAATTGA CGGCCGCCAT 2580
   CATGATCCTA TTTTAAATGG ATTGCGTGAT GTGACTCTAG TGACTCGGTT CGTTGATGAG 2640
   GAATATCTAC GATCCATCAA AAAACAACTG CATCCTTCTA AGATTATTTT AATTTCTGAT 2700
   GTGAGATCCA AACGAGGAGG AAATGAACCT AGTACGGCGG ATTTACTAAG TAATTACGCT 2760
   CTACAAAATG TCATGATTAG TATTTTAAAC CCCGTGGCGT CTAGTCTTAA ATGGAGATGC 2820
   CCGTTTCCAG ATCAATGGAT CAAGGACTTT TATATCCCAC ACGGTAATAA AATGTTACAA 2880
   CCTTTTGCTC CTTCATATTC AGGGCCGTCG TTTTACAACG TCGTGACTGG GAAAACCCTG 2940
   GCGTTACCCA ACTTAATCGC CTTGCAGCAC ATCCCCCTTT CGCCAGCTGG CGTAATAGCG 3000
   AAGAGGCCCG CACCGATCGC CCTTCCCAAC AGTTGCGCAG CCTGAATGGC GAATGGCGCG 3060
   ACGCGCCCTG TAGCGGCGCA TTAAGCGCGG CGGGTGTGGT GGTTACGCGC AGCGTGACCG 3120
   CTACACTTGC CAGCGCCCTA GCGCCCGCTC CTTTCGCTTT CTTCCCTTCC TTTCTCGCCA 3180
   CGTTCGCCGG CTTTCCCCGT CAAGCTCTAA ATCGGGGGCT CCCTTTAGGG TTCCGATTTA 3240
   GTGCTTTACG GCACCTCGAC CCCAAAAAAC TTGATTAGGG TGATGGTTCA CGTAGTGGGC 3300
   CATCGCCCTG ATAGACGGTT TTTCGCCCTT TGACGTTGGA GTCCACGTTC TTTAATAGTG 3360
   GACTCTTGTT CCAAACTGGA ACAACACTCA ACCCTATCTC GGTCTATTCT TTTGATTTAT 3420
   AAGGGATTTT GCCGATTTCG GCCTATTGGT TAAAAAATGA GCTGATTTAA CAAAAATTTA 3480
   ACGCGAATTT TAACAAAATA TTAACGTTTA CAATTTCCCA GGTGGCACTT TTCGGGGAAA 3540
   TGTGCGCGGA ACCCCTATTT GTTTATTTTT CTAAATACAT TCAAATATGT ATCCGCTCAT 3600
   GAGACAATAA CCCTGATAAA TGCTTCAATA ATATTGAAAA AGGAAGAGTA TGAGTATTCA 3660
   ACATTTCCGT GTCGCCCTTA TTCCCTTTTT TGCGGCATTT TGCCTTCCTG TTTTTGCTCA 3720
   CCCAGAAACG CTGGTGAAAG TAAAAGATGC TGAAGATCAG TTGGGTGCAC GAGTGGGTTA 3780
   CATCGAACTG GATCTCAACA GCGGTAAGAT CCTTGAGAGT TTTCGCCCCG AAGAACGTTT 3840
   TCCAATGATG AGCACTTTTA AAGTTCTGCT ATGTGGCGCG GTATTATCCC GTATTGACGC 3900
   CGGGCAAGAG CAACTCGGTC GCCGCATACA CTATTCTCAG AATGACTTGG TTGAGTACTC 3960
   ACCAGTCACA GAAAAGCATC TTACGGATGG CATGACAGTA AGAGAATTAT GCAGTGCTGC 4020
   CATAACCATG AGTGATAACA CTGCGGCCAA CTTACTTCTG ACAACGATCG GAGGACCGAA 4080
   GGAGCTAACC GCTTTTTTGC ACAACATGGG GGATCATGTA ACTCGCCTTG ATCGTTGGGA 4140
   ACCGGAGCTG AATGAAGCCA TACCAAACGA CGAGCGTGAC ACCACGATGC CTGTAGCAAT 4200
   GGCAACAACG TTGCGCAAAC TATTAACTGG CGAACTACTT ACTCTAGCTT CCCGGCAACA 4260
   ATTAATAGAC TGGATGGAGG CGGATAAAGT TGCAGGACCA CTTCTGCGCT CGGCCCTTCC 4320
   GGCTGGCTGG TTTATTGCTG ATAAATCTGG AGCCGGTGAG CGTGGGTCTC GCGGTATCAT 4380
   TGCAGCACTG GGGCCAGATG GTAAGCCCTC CCGTATCGTA GTTATCTACA CGACGGGGAG 4440
   TCAGGCAACT ATGGATGAAC GAAATAGACA GATCGCTGAG ATAGGTGCCT CACTGATTAA 4500
   GCATTGGTAA CTGTCAGACC AAGTTTACTC ATATATACTT TAGATTGATT TAAAACTTCA 4560
   TTTTTAATTT AAAAGGATCT AGGTGAAGAT CCTTTTTGAT AATCTCATGA CCAAAATCCC 4620
   TTAACGTGAG TTTTCGTTCC ACTGAGCGTC AGACCCCGTA GAAAAGATCA AAGGATCTTC 4680
   TTGAGATCCT TTTTTTCTGC GCGTAATCTG CTGCTTGCAA ACAAAAAAAC CACCGCTACC 4740
   AGCGGTGGTT TGTTTGCCGG ATCAAGAGCT ACCAACTCTT TTTCCGAAGG TAACTGGCTT 4800
   CAGCAGAGCG CAGATACCAA ATACTGTCCT TCTAGTGTAG CCGTAGTTAG GCCACCACTT 4860
   CAAGAACTCT GTAGCACCGC CTACATACCT CGCTCTGCTA ATCCTGTTAC CAGTGGCTGC 4920
   TGCCAGTGGC GATAAGTCGT GTCTTACCGG GTTGGACTCA AGACGATAGT TACCGGATAA 4980
   GGCGCAGCGG TCGGGCTGAA CGGGGGGTTC GTGCACACAG CCCAGCTTGG AGCGAACGAC 5040
   CTACACCGAA CTGAGATACC TACAGCGTGA GCTATGAGAA AGCGCCACGC TTCCCGAAGG 5100
   GAGAAAGGCG GACAGGTATC CGGTAAGCGG CAGGGTCGGA ACAGGAGAGC GCACGAGGGA 5160
   GCTTCCAGGG GGAAACGCCT GGTATCTTTA TAGTCCTGTC GGGTTTCGCC ACCTCTGACT 5220
   TGAGCGTCGA TTTTTGTGAT GCTCGTCAGG GGGGCGGAGC CTATGGAAAA ACGCCAGCAA 5280
   CGCGGCCTTT TTACGGTTCC TGGCCTTTTG CTGGCCTTTT GCTCACATGT TCTTTCCTGC 5340
   GTTATCCCCT GATTCTGTGG ATAACCGTAT TACCGCCTTT GAGTGAGCTG ATACCGCTCG 5400
   CCGCAGCCGA ACGACCGAGC GCAGCGAGTC AGTGAGCGAG GAAGCGGAAG AGCGCCCAAT 5460
   ACGCAAACCG CCTCTCCCCG CGCGTTGGCC GATTCATTAA TGCAGCTGGC ACGACAGGTT 5520
   TCCCGACTGG AAAGCGGGCA GTGAGCGCAA CGCAATTAAT GTGAGTTAGC TCACTCATTA 5580
   GGCACCCCAG GCTTTACACT TTATGCTTCC GGCTCGTATG TTGTGTGGAA TTGTGAGCGG 5640
   ATAACAATTT CACACAGGAA ACAGCTATGA CCATGATTAC GCCAAGCTTT TGCGATCAAT 5700
   AAATGGATCA CAACCAGTAT CTCTTAACGA TGTTCTTCGC AGATGATGAT TCATTTTTTA 5760
   AGTATTTGGC TAGTCAAGAT GATGAATCTT CATTATCTGA TATATTGCAA ATCACTCAAT 5820
   ATCTAGACTT TCTGTTATTA TTATTGATCC AATCAAAAAA TAAATTAGAA GCCGTGGGTC 5880
   ATTGTTATGA ATCTCTTTCA GAGGAATACA GACAATTGAC AAAATTCACA GACTTTCAAG 5940
   ATTTTAAAAA ACTGTTTAAC AAGGTCCCTA TTGTTACAGA TGGAAGGGTC AAACTTAATA 6000
   AAGGATATTT GTTCGACTTT GTGATTAGTT TGATGCGATT CAAAAAAGAA TCCTCTCTAG 6060
   CTACCACCGC AATAGATCCT GTTAGATACA TAGATCCTCG TCGCAATATC GCATTTTCTA 6120
   ACGTGATGGA TATATTAAAG TCGAATAAAG TGAACAATAA TTAATTCTTT ATTGTCATCA 6180
   TGAACGGCGG ACATATTCAG TTGATAATCG GCCCCATGTT TTCAGGTAAA AGTACAGAAT 6240
   TAATTAGACG AGTTAGACGT TATCAAATAG CTCAATATAA ATGCGTGACT ATAAAATATT 6300
   CTAACGATAA TAGATACGGA ACGGGACTAT GGACGCATGA TAAGAATAAT TTTGAAGCAT 6360
   TGGAAGCAAC TAAACTATGT GATGTCTTGG AATCAATTAC AGATTTCTCC GTGATAGGTA 6420
   TCGATGAAGG ACAGTTCTTT CCAGACATTG TTGAATTGAT CTCGATCCCG CGAAATTAAT 6480
   ACGACTCACT ATAGGGAGAC CACAACGGTT TCCCTCTAGC GGGATCAATT CCGCCCCTCT 6540
   CCCTCCCCCC CCCCTAACGT TACTGGCCGA AGCCGCTTGG AATAAGGCCG GTGTGCGTTT 6600
   GTCTATATGT TATTTTCCAC CATATTGCCG TCTTTTGGCA ATGTGAGGGC CCGGAAACCT 6660
   GGCCCTGTCT TCTTGACGAG CATTCCTAGG GGTCTTTCCC CTCTCGCCAA AGGAATGCAA 6720
   GGTCTGTTGA ATGTCGTGAA GGAAGCAGTT CCTCTGGAAG CTTCTTGAAG ACAAACAACG 6780
   TCTGTAGCGA CCCTTTGCAG GCAGCGGAAC CCCCCACCTG GCGACAGGTG CCTCTGCGGC 6840
   CAAAAGCCAC GTGTATAAGA TACACCTGCA AAGGCGGCAC AACCCCAGTG CCACGTTGTG 6900
   AGTTGGATAG TTGTGGAAAG AGTCAAATGG CTCTCCTCAA GCGTATTCAA CAAGGGGCTG 6960
   AAGGATGCCC AGAAGGTACC CCATTGTATG GGATCTGATC TGGGGCCTCG GTGCACATGC 7020
   TTTACATGTG TTTAGTCGAG GTTAAAAAAC GTCTAGGCCC CCCGAACCAC GGGGACGTGG 7080
   TTTTCCTTTG AAAAACACGA TAATACC 7107
(2) INFORMATION FOR SEQ ID NO:22:
   (i) SEQUENCE CHARACTERISTICS:
      (A) LENGTH: 12011 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:22:
   ATGGACACTG AATCTAACAA TGGCACTGTA TCTGACATAC TCTATCCTGA GTGTCACCTT   60
   AACTCTCCTA TCGTTAAAGG TAAAATAGCA CAATTACACA CTATTATGAG TCTACCTCAG  120
   CCTTATGATA TGGATGACGA CTCAATACTA GTTATCACTA GACAGAAAAT AAAACTTAAT  180
   AAATTGGATA AAAGACAACG ATCTATTAGA AGATTAAAAT TAATATTAAC TGAAAAAGTG  240
   AATGACTTAG GAAAATACAC ATTTATCAGA TATCCAGAAA TGTCAAAAGA AATGTTCAAA  300
   TTATATATAC CTGGTATTAA CAGTAAAGTG ACTGAATTAT TACTTAAAGC AGATAGAACA  360
   TATAGTCAAA TGACTGATGG ATTAAGAGAT CTATGGATTA ATGTGCTATC AAAATTAGCC 420
   TCAAAAAATG ATGGAAGCAA TTATGATCTT AATGAAGAAA TTAATAATAT ATCGAAAGTT 480
   CACACAACCT ATAAATCAGA TAAATGGTAT AATCCATTCA AAACATGGTT TACTATCAAG 540
   TATGATATGA GAAGATTACA AAAAGCTCGA AATGAGATCA CTTTTAATGT TGGGAAGGAT 600
   TATAACTTGT TAGAAGACCA GAAGAATTTC TTATTGATAC ATCCAGAATT GGTTTTGATA 660
   TTAGATAAAC AAAACTATAA TGGTTATCTA ATTACTCCTG AATTAGTATT GATGTATTGT 720
   GACGTAGTCG AAGGCCGATG GAATATAAGT GCATGTGCTA AGTTAGATCC AAAATTACAA 780
   TCTATGTATC AGAAAGGTAA TAACCTGTGG GAAGTGATAG ATAAATTGTT TCCAATTATG 840
   GGAGAAAAGA CATTTGATGT GATATCGTTA TTAGAACCAC TTGCATTATC CTTAATTCAA 900
   ACTCATGATC CTGTTAAACA ACTAAGAGGA GCTTTTTTAA ATCATGTGTT ATCCGAGATG 960
   GAATTAATAT TTGAATCTAG AGAATCGATT AAGGAATTTC TGAGTGTAGA TTACATTGAT 1020
   AAAATTTTAG ATATATTTAA TAAGTCTACA ATAGATGAAA TAGCAGAGAT TTTCTCTTTT 1080
   TTTAGAACAT TTGGGCATCC TCCATTAGAA GCTAGTATTG CAGCAGAAAA GGTTAGAAAA 1140
   TATATGTATA TTGGAAAACA ATTAAAATTT GACACTATTA ATAAATGTCA TGCTATCTTC 1200
   TGTACAATAA TAATTAACGG ATATAGAGAG AGGCATGGTG GACAGTGGCC TCCTGTGACA 1260
   TTACCTGATC ATGCACACGA ATTCATCATA AATGCTTACG GTTCAAACTC TGCGATATCA 1320
   TATGAAAATG CTGTTGATTA TTACCAGAGC TTTATAGGAA TAAAATTCAA TAAATTCATA 1380
   GAGCCTCAGT TAGATGAGGA TTTGACAATT TATATGAAAG ATAAAGCATT ATCTCCAAAA 1440
   AAATCAAATT GGGACACAGT TTATCCTGCA TCTAATTTAC TGTACCGTAC TAACGCATCC 1500
   AACGAATCAC GAAGATTAGT TGAAGTATTT ATAGCAGATA GTAAATTTGA TCCTCATCAG 1560
   ATATTGGATT ATGTAGAATC TGGGGACTGG TTAGATGATC CAGAATTTAA TATTTCTTAT 1620
   AGTCTTAAAG AAAAAGAGAT CAAACAGGAA GGTAGACTCT TTGCAAAAAT GACATACAAA 1680
   ATGAGAGCTA CACAAGTTTT ATCAGAGACA CTACTTGCAA ATAACATAGG AAAATTCTTT 1740
   CAAGAAAATG GGATGGTGAA GGGAGAGATT GAATTACTTA AGAGATTAAC AACCATATCA 1800
   ATATCAGGAG TTCCACGGTA TAATGAAGTG TACAATAATT CTAAAAGCCA TACAGATGAC 1860
   CTTAAAACCT ACAATAAAAT AAGTAATCTT AATTTGTCTT CTAATCAGAA ATCAAAGAAA 1920
   TTTGAATTCA AGTCAACGGA TATCTACAAT GATGGATACG AGACTGTGAG CTGTTTCCTA 1980
   ACAACAGATC TCAAAAAATA CTGTCTTAAT TGGAGATATG AATCAACAGC TCTATTTGGA 2040
   GAAACTTGCA ACCAAATATT TGGATTAAAT AAATTGTTTA ATTGGTTACA CCCTCGTCTT 2100
   GAAGGAAGTA CAATCTATGT AGGTGATCCT TACTGTCCTC CATCAGATAA AGAACATATA 2160
   TCATTAGAGG ATCACCCTGA TTCTGGTTTT TACGTTCATA ACCCAAGAGG GGGTATAGAA 2220
   GGATTTTGTC AAAAATTATG GACACTCATA TCTATAAGTG CAATACATCT AGCAGCTGTT 2280
   AGAATAGGCG TGAGGGTGAC TGCAATGGTT CAAGGAGACA ATCAAGCTAT AGCTGTAACC 2340
   ACAAGAGTAC CCAACAATTA TGACTACAGA GTTAAGAAGG AGATAGTTTA TAAAGATGTA 2400
   GTGAGATTTT TTGATTCATT AAGAGAAGTG ATGGATGATC TAGGTCATGA ACTTAAATTA 2460
   AATGAAACGA TTATAAGTAG CAAGATGTTC ATATATAGCA AAAGAATCTA TTATGATGGG 2520
   AGAATTCTTC CTCAAGCTCT AAAAGCATTA TCTAGATGTG TCTTCTGGTC AGAGACAGTA 2580
   ATAGACGAAA CAAGATCAGC ATCTTCAAAT TTGGCAACAT CATTTGCAAA AGCAATTGAG 2640
   AATGGTTATT CACCTGTTCT AGGATATGCA TGCTCAATTT TTAAGAACAT TCAACAACTA 2700
   TATATTGCCC TTGGGATGAA TATCAATCCA ACTATAACAC AGAATATCAG AGATCAGTAT 2760
   TTTAGGAATC CAAATTGGAT GCAATATGCC TCTTTAATAC CTGCTAGTGT TGGGGGATTC 2820
   AATTACATGG CCATGTCAAG ATGTTTTGTA AGGAATATTG GTGATCCATC AGTTGCCGCA 2880
   TTGGCTGATA TTAAAAGATT TATTAAGGCG AATCTATTAG ACCGAAGTGT TCTTTATAGG 2940
   ATTATGAATC AAGAACCAGG TGAGTCATCT TTTTTGGACT GGGCTTCAGA TCCATATTCA 3000
   TGCAATTTAC CACAATCTCA AAATATAACC ACCATGATAA AAAATATAAC AGCAAGGAAT 3060
   GTATTACAAG ATTCACCAAA TCCATTATTA TCTGGATTAT TCACAAATAC AATGATAGAA 3120
   GAAGATGAAG AATTAGCTGA GTTCCTGATG GACAGGAAGG TAATTCTCCC TAGAGTTGCA 3180
   CATGATATTC TAGATAATTC TCTCACAGGA ATTAGAAATG CCATAGCTGG AATGTTAGAT 3240
   ACGACAAAAT CACTAATTCG GGTTGGCATA AATAGAGGAG GACTGACATA TAGTTTGTTG 3300
   AGGAAAATCA GTAATTACGA TCTAGTACAA TATGAAACAC TAAGTAGGAC TTTGCGACTA 3360
   ATTGTAAGTG ATAAAATCAA GTATGAAGAT ATGTGTTCGG TAGACCTTGC CATAGCATTG 3420
   CGACAAAAGA TGTGGATTCA TTTATCAGGA GGAAGGATGA TAAGTGGACT TGAAACGCCT 3480
   GACCCATTAG AATTACTATC TGGGGTAGTA ATAACAGGAT CAGAACATTG TAAAATATGT 3540
   TATTCTTCAG ATGGCACAAA CCCATATACT TGGATGTATT TACCCGGTAA TATCAAAATA 3600
   GGATCAGCAG AAACAGGTAT ATCGTCATTA AGAGTTCCTT ATTTTGGATC AGTCACTGAT 3660
   GAAAGATCTG AAGCACAATT AGGATATATC AAGAATCTTA GTAAACCTGC AAAAGCCGCA 3720
   ATAAGAATAG CAATGATATA TACATGGGCA TTTGGTAATG ATGAGATATC TTGGATGGAA 3780
   GCCTCACAGA TAGCACAAAC ACGTGCAAAT TTTACACTAG ATAGTCTCAA AATTTTAACA 3840
   CCGGTAGCTA CATCAACAAA TTTATCACAC AGATTAAAGG ATACTGCAAC TCAGATGAAA 3900
   TTCTCCAGTA CATCATTGAT CAGAGTCAGC AGATTCATAA CAATGTCCAA TGATAACATG 3960
   TCTATCAAAG AAGCTAATGA AACCAAAGAT ACTAATCTTA TTTATCAACA AATAATGTTA 4020
   ACAGGATTAA GTGTTTTCGA ATATTTATTT AGATTAAAAG AAACCACAGG ACACAACCCT 4080
   ATAGTTATGC ATCTGCACAT AGAAGATGAG TGTTGTATTA AAGAAAGTTT TAATGATGAA 4140
   CATATTAATC CAGAGTCTAC ATTAGAATTA ATTCGATATC CTGAAAGTAA TGAATTTATT 4200
   TATGATAAAG ACCCACTCAA AGATGTGGAC TTATCAAAAC TTATGGTTAT TAAAGACCAT 4260
   TCTTACACAA TTGATATGAA TTATTGGGAT GATACTGACA TCATACATGC AATTTCAATA 4320
   TGTACTGCAA TTACAATAGC AGATACTATG TCACAATTAG ATCGAGATAA TTTAAAAGAG 4380
   ATAATAGTTA TTGCAAATGA TGATGATATT AATAGCTTAA TCACTGAATT TTTGACTCTT 4440
   GACATACTTG TATTTCTCAA GACATTTGGT GGATTATTAG TAAATCAATT TGCATACACT 4500
   CTTTATAGTC TAAAAATAGA AGGTAGGGAT CTCATTTGGG ATTATATAAT GAGAACACTG 4560
   AGAGATACTT CCCATTCAAT ATTAAAAGTA TTATCTAATG CATTATCTCA TCCTAAAGTA 4620
   TTCAAGAGGT TCTGGGATTG TGGAGTTTTA AACCCTATTT ATGGTCCTAA TACTGCTAGT 4680
   CAAGACCAGA TAAAACTTGC CCTATCTATA TGTGAATATT CACTAGATCT ATTTATGAGA 4740
   GAATGGTTGA ATGGTGTATC ACTTGAAATA TACATTTGTG ACAGCGATAT GGAAGTTGCA 4800
   AATGATAGGA AACAAGCCTT TATTTCTAGA CACCTTTCAT TTGTTTGTTG TTTAGCAGAA 4860
   ATTGCATCTT TCGGACCTAA CCTGTTAAAC TTAACATACT TGGAGAGACT TGATCTATTG 4920
   AAACAATATC TTGAATTAAA TATTAAAGAA GACCCTACTC TTAAATATGT ACAAATATCT 4980
   GGATTATTAA TTAAATCGTT CCCATCAACT GTAACATACG TAAGAAAGAC TGCAATCAAA 5040
   TATCTAAGGA TTCGCGGTAT TAGTCCACCT GAGGTAATTG ATGATTGGGA TCCGGTAGAA 5100
   GATGAAAATA TGCTGGATAA CATTGTCAAA ACTATAAATG ATAACTGTAA TAAAGATAAT 5160
   AAAGGGAATA AAATTAACAA TTTCTGGGGA CTAGCACTTA AGAACTATCA AGTCCTTAAA 5220
   ATCAGATCTA TAACAAGTGA TTCTGATGAT AATGATAGAC TAGATGCTAA TACAAGTGGT 5280
   TTGACACTTC CTCAAGGAGG GAATTATCTA TCGCATCAAT TGAGATTATT CGGAATCAAC 5340
   AGCACTAGTT GTCTGAAAGC TCTTGAGTTA TCACAAATTT TAATGAAGGA AGTCAATAAA 5400
   GACAAGGACA GGCTCTTCCT GGGAGAAGGA GCAGGAGCTA TGCTAGCATG TTATGATGCC 5460
   ACATTAGGAC CTGCAGTTAA TTATTATAAT TCAGGTTTGA ATATAACAGA TGTAATTGGT 5520
   CAACGAGAAT TGAAAATATT TCCTTCAGAG GTATCATTAG TAGGTAAAAA ATTAGGAAAT 5580
   GTGACACAGA TTCTTAACAG GGTAAAAGTA CTGTTCAATG GGAATCCTAA TTCAACATGG 5640
   ATAGGAAATA TGGAATGTGA GAGCTTAATA TGGAGTGAAT TAAATGATAA GTCCATTGGA 5700
   TTAGTACATT GTGATATGGA AGGAGCTATC GGTAAATCAG AAGAAACTGT TCTACATGAA 5760
   CATTATAGTG TTATAAGAAT TACATACTTG ATTGGGGATG ATGATGTTGT TTTAGTTTCC 5820
   AAAATTATAC CTACAATCAC TCCGAATTGG TCTAGAATAC TTTATCTATA TAAATTATAT 5880
   TGGAAAGATG TAAGTATAAT ATCACTCAAA ACTTCTAATC CTGCATCAAC AGAATTATAT 5940
   CTAATTTCGA AAGATGCATA TTGTACTATA ATGGAACCTA GTGAAATTGT TTTATCAAAA 6000
   CTTAAAAGAT TGTCACTCTT GGAAGAAAAT AATCTATTAA AATGGATCAT TTTATCAAAG 6060
   AAGAGGAATA ATGAATGGTT ACATCATGAA ATCAAAGAAG GAGAAAGAGA TTATGGAATC 6120
   ATGAGACCAT ATCATATGGC ACTACAAATC TTTGGATTTC AAATCAATTT AAATCATCTG 6180
   GCGAAAGAAT TTTTATCAAC CCCAGATCTG ACTAATATCA ACAATATAAT CCAAAGTTTT 6240
   CAGCGAACAA TAAAGGATGT TTTATTTGAA TGGATTAATA TAACTCATGA TGATAAGAGA 6300
   CATAAATTAG GCGGAAGATA TAACATATTC CCACTGAAAA ATAAGGGAAA GTTAAGACTG 6360
   CTATCGAGAA GACTAGTATT AAGTTGGATT TCATTATCAT TATCGACTCG ATTACTTACA 6420
   GGTCGCTTTC CTGATGAAAA ATTTGAACAT AGAGCACAGA CTGGATATGT ATCATTAGCT 6480
   GATACTGATT TAGAATCATT AAAGTTATTG TCGAAAAACA TCATTAAGAA TTACAGAGAG 6540
   TGTATAGGAT CAATATCATA TTGGTTTCTA ACCAAAGAAG TTAAAATACT TATGAAATTG 6600
   ATTGGTGGTG CTAAATTATT AGGAATTCCC AGACAATATA AAGAACCGGA AGACCAGTTA 6660
   TTAGAAAACT ACAATCAACA TGATGAATTT GATATCGATT AAAACATAAA TACAATGTCG 6720
   ACGATCCGGC TGCTAACAAA GCCCGAAAGG AAGCTGAGTT GGCTGCTGCC ACCGCTGAGC 6780
   AATAACTAGC ATAACCCCTT GGGGCCTCTA AACGGGTCTT GAGGGGTTTT TTGCTGAAAG 6840
   GAGGAACTAT ATCCGGATCG AGATCAATTC TGTGAGCGTA TGGCAAACGA AGGAAAAATA 6900
   GTTATAGTAG CCGCACTCGA TGGGACATTT CAACGTAAAC CGTTTAATAA TATTTTGAAT 6960
   CTTATTCCAT TATCTGAAAT GGTGGTAAAA CTAACTGCTG TGTGTATGAA ATGCTTTAAG 7020
   GAGGCTTCCT TTTCTAAACG ATTGGGTGAG GAAACCGAGA TAGAAATAAT AGGAGGTAAT 7080
   GATATGTATC AATCGGTGTG TAGAAAGTGT TACATCGACT CATAATATTA TATTTTTTAT 7140
   CTAAAAAACT AAAAATAAAC ATTGATTAAA TTTTAATATA ATACTTAAAA ATGGATGTTG 7200
   TGTCGTTAGA TAAACCGTTT ATGTATTTTG AGGAAATTGA TAATGAGTTA GATTACGAAC 7260
   CAGAAAGTGC AAATGAGGTC GCAAAAAAAC TGCCGTATCA AGGACAGTTA AAACTATTAC 7320
   TAGGAGAATT ATTTTTTCTT AGTAAGTTAC AGCGACACGG TATATTAGAT GGTGCCACCG 7380
   TAGTGTATAT AGGATCTGCT CCCGGTACAC ATATACGTTA TTTGAGAGAT CATTTCTATA 7440
   ATTTAGGAGT GATCATCAAA TGGATGCTAA TTGACGGCCG CCATCATGAT CCTATTTTAA 7500
   ATGGATTGCG TGATGTGACT CTAGTGACTC GGTTCGTTGA TGAGGAATAT CTACGATCCA 7560
   TCAAAAAACA ACTGCATCCT TCTAAGATTA TTTTAATTTC TGATGTGAGA TCCAAACGAG 7620
   GAGGAAATGA ACCTAGTACG GCGGATTTAC TAAGTAATTA CGCTCTACAA AATGTCATGA 7680
   TTAGTATTTT AAACCCCGTG GCGTCTAGTC TTAAATGGAG ATGCCCGTTT CCAGATCAAT 7740
   GGATCAAGGA CTTTTATATC CCACACGGTA ATAAAATGTT ACAACCTTTT GCTCCTTCAT 7800
   ATTCAGGGCC GTCGTTTTAC AACGTCGTGA CTGGGAAAAC CCTGGCGTTA CCCAACTTAA 7860
   TCGCCTTGCA GCACATCCCC CTTTCGCCAG CTGGCGTAAT AGCGAAGAGG CCCGCACCGA 7920
   TCGCCCTTCC CAACAGTTGC GCAGCCTGAA TGGCGAATGG CGCGACGCGC CCTGTAGCGG 7980
   CGCATTAAGC GCGGCGGGTG TGGTGGTTAC GCGCAGCGTG ACCGCTACAC TTGCCAGCGC 8040
   CCTAGCGCCC GCTCCTTTCG CTTTCTTCCC TTCCTTTCTC GCCACGTTCG CCGGCTTTCC 8100
   CCGTCAAGCT CTAAATCGGG GGCTCCCTTT AGGGTTCCGA TTTAGTGCTT TACGGCACCT 8160
   CGACCCCAAA AAACTTGATT AGGGTGATGG TTCACGTAGT GGGCCATCGC CCTGATAGAC 8220
   GGTTTTTCGC CCTTTGACGT TGGAGTCCAC GTTCTTTAAT AGTGGACTCT TGTTCCAAAC 8280
   TGGAACAACA CTCAACCCTA TCTCGGTCTA TTCTTTTGAT TTATAAGGGA TTTTGCCGAT 8340
   TTCGGCCTAT TGGTTAAAAA ATGAGCTGAT TTAACAAAAA TTTAACGCGA ATTTTAACAA 8400
   AATATTAACG TTTACAATTT CCCAGGTGGC ACTTTTCGGG GAAATGTGCG CGGAACCCCT 8460
   ATTTGTTTAT TTTTCTAAAT ACATTCAAAT ATGTATCCGC TCATGAGACA ATAACCCTGA 8520
   TAAATGCTTC AATAATATTG AAAAAGGAAG AGTATGAGTA TTCAACATTT CCGTGTCGCC 8580
   CTTATTCCCT TTTTTGCGGC ATTTTGCCTT CCTGTTTTTG CTCACCCAGA AACGCTGGTG 8640
   AAAGTAAAAG ATGCTGAAGA TCAGTTGGGT GCACGAGTGG GTTACATCGA ACTGGATCTC 8700
   AACAGCGGTA AGATCCTTGA GAGTTTTCGC CCCGAAGAAC GTTTTCCAAT GATGAGCACT 8760
   TTTAAAGTTC TGCTATGTGG CGCGGTATTA TCCCGTATTG ACGCCGGGCA AGAGCAACTC 8820
   GGTCGCCGCA TACACTATTC TCAGAATGAC TTGGTTGAGT ACTCACCAGT CACAGAAAAG 8880
   CATCTTACGG ATGGCATGAC AGTAAGAGAA TTATGCAGTG CTGCCATAAC CATGAGTGAT 8940
   AACACTGCGG CCAACTTACT TCTGACAACG ATCGGAGGAC CGAAGGAGCT AACCGCTTTT 9000
   TTGCACAACA TGGGGGATCA TGTAACTCGC CTTGATCGTT GGGAACCGGA GCTGAATGAA 9060
   GCCATACCAA ACGACGAGCG TGACACCACG ATGCCTGTAG CAATGGCAAC AACGTTGCGC 9120
   AAACTATTAA CTGGCGAACT ACTTACTCTA GCTTCCCGGC AACAATTAAT AGACTGGATG 9180
   GAGGCGGATA AAGTTGCAGG ACCACTTCTG CGCTCGGCCC TTCCGGCTGG CTGGTTTATT 9240
   GCTGATAAAT CTGGAGCCGG TGAGCGTGGG TCTCGCGGTA TCATTGCAGC ACTGGGGCCA 9300
   GATGGTAAGC CCTCCCGTAT CGTAGTTATC TACACGACGG GGAGTCAGGC AACTATGGAT 9360
   GAACGAAATA GACAGATCGC TGAGATAGGT GCCTCACTGA TTAAGCATTG GTAACTGTCA 9420
   GACCAAGTTT ACTCATATAT ACTTTAGATT GATTTAAAAC TTCATTTTTA ATTTAAAAGG 9480
   ATCTAGGTGA AGATCCTTTT TGATAATCTC ATGACCAAAA TCCCTTAACG TGAGTTTTCG 9540
   TTCCACTGAG CGTCAGACCC CGTAGAAAAG ATCAAAGGAT CTTCTTGAGA TCCTTTTTTT 9600
   CTGCGCGTAA TCTGCTGCTT GCAAACAAAA AAACCACCGC TACCAGCGGT GGTTTGTTTG 9660
   CCGGATCAAG AGCTACCAAC TCTTTTTCCG AAGGTAACTG GCTTCAGCAG AGCGCAGATA 9720
   CCAAATACTG TCCTTCTAGT GTAGCCGTAG TTAGGCCACC ACTTCAAGAA CTCTGTAGCA 9780
   CCGCCTACAT ACCTCGCTCT GCTAATCCTG TTACCAGTGG CTGCTGCCAG TGGCGATAAG 9840
   TCGTGTCTTA CCGGGTTGGA CTCAAGACGA TAGTTACCGG ATAAGGCGCA GCGGTCGGGC 9900
   TGAACGGGGG GTTCGTGCAC ACAGCCCAGC TTGGAGCGAA CGACCTACAC CGAACTGAGA 9960
   TACCTACAGC GTGAGCTATG AGAAAGCGCC ACGCTTCCCG AAGGGAGAAA GGCGGACAGG 10020
   TATCCGGTAA GCGGCAGGGT CGGAACAGGA GAGCGCACGA GGGAGCTTCC AGGGGGAAAC 10080
   GCCTGGTATC TTTATAGTCC TGTCGGGTTT CGCCACCTCT GACTTGAGCG TCGATTTTTG 10140
   TGATGCTCGT CAGGGGGGCG GAGCCTATGG AAAAACGCCA GCAACGCGGC CTTTTTACGG 10200
   TTCCTGGCCT TTTGCTGGCC TTTTGCTCAC ATGTTCTTTC CTGCGTTATC CCCTGATTCT 10260
   GTGGATAACC GTATTACCGC CTTTGAGTGA GCTGATACCG CTCGCCGCAG CCGAACGACC 10320
   GAGCGCAGCG AGTCAGTGAG CGAGGAAGCG GAAGAGCGCC CAATACGCAA ACCGCCTCTC 10380
   CCCGCGCGTT GGCCGATTCA TTAATGCAGC TGGCACGACA GGTTTCCCGA CTGGAAAGCG 10440
   GGCAGTGAGC GCAACGCAAT TAATGTGAGT TAGCTCACTC ATTAGGCACC CCAGGCTTTA 10500
   CACTTTATGC TTCCGGCTCG TATGTTGTGT GGAATTGTGA GCGGATAACA ATTTCACACA 10560
   GGAAACAGCT ATGACCATGA TTACGCCAAG CTTTTGCGAT CAATAAATGG ATCACAACCA 10620
   GTATCTCTTA ACGATGTTCT TCGCAGATGA TGATTCATTT TTTAAGTATT TGGCTAGTCA 10680
   AGATGATGAA TCTTCATTAT CTGATATATT GCAAATCACT CAATATCTAG ACTTTCTGTT 10740
   ATTATTATTG ATCCAATCAA AAAATAAATT AGAAGCCGTG GGTCATTGTT ATGAATCTCT 10800
   TTCAGAGGAA TACAGACAAT TGACAAAATT CACAGACTTT CAAGATTTTA AAAAACTGTT 10860
   TAACAAGGTC CCTATTGTTA CAGATGGAAG GGTCAAACTT AATAAAGGAT ATTTGTTCGA 10920
   CTTTGTGATT AGTTTGATGC GATTCAAAAA AGAATCCTCT CTAGCTACCA CCGCAATAGA 10980
   TCCTGTTAGA TACATAGATC CTCGTCGCAA TATCGCATTT TCTAACGTGA TGGATATATT 11040
   AAAGTCGAAT AAAGTGAACA ATAATTAATT CTTTATTGTC ATCATGAACG GCGGACATAT 11100
   TCAGTTGATA ATCGGCCCCA TGTTTTCAGG TAAAAGTACA GAATTAATTA GACGAGTTAG 11160
   ACGTTATCAA ATAGCTCAAT ATAAATGCGT GACTATAAAA TATTCTAACG ATAATAGATA 11220
   CGGAACGGGA CTATGGACGC ATGATAAGAA TAATTTTGAA GCATTGGAAG CAACTAAACT 11280
   ATGTGATGTC TTGGAATCAA TTACAGATTT CTCCGTGATA GGTATCGATG AAGGACAGTT 11340
   CTTTCCAGAC ATTGTTGAAT TGATCTCGAT CCCGCGAAAT TAATACGACT CACTATAGGG 11400
   AGACCACAAC GGTTTCCCTC TAGCGGGATC AATTCCGCCC CTCTCCCTCC CCCCCCCCTA 11460
   ACGTTACTGG CCGAAGCCGC TTGGAATAAG GCCGGTGTGC GTTTGTCTAT ATGTTATTTT 11520
   CCACCATATT GCCGTCTTTT GGCAATGTGA GGGCCCGGAA ACCTGGCCCT GTCTTCTTGA 11580
   CGAGCATTCC TAGGGGTCTT TCCCCTCTCG CCAAAGGAAT GCAAGGTCTG TTGAATGTCG 11640
   TGAAGGAAGC AGTTCCTCTG GAAGCTTCTT GAAGACAAAC AACGTCTGTA GCGACCCTTT 11700
   GCAGGCAGCG GAACCCCCCA CCTGGCGACA GGTGCCTCTG CGGCCAAAAG CCACGTGTAT 11760
   AAGATACACC TGCAAAGGCG GCACAACCCC AGTGCCACGT TGTGAGTTGG ATAGTTGTGG 11820
   AAAGAGTCAA ATGGCTCTCC TCAAGCGTAT TCAACAAGGG GCTGAAGGAT GCCCAGAAGG 11880
   TACCCCATTG TATGGGATCT GATCTGGGGC CTCGGTGCAC ATGCTTTACA TGTGTTTAGT 11940
   CGAGGTTAAA AAACGTCTAG GCCCCCCGAA CCACGGGGAC GTGGTTTTCC TTTGAAAAAC 12000
   ACGATAATAC C 12011
(2) INFORMATION FOR SEQ ID NO:23:
   (i) SEQUENCE CHARACTERISTICS:
      (A) LENGTH: 15 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:23:
      GATCGATGCT AGCCC 15
(2) INFORMATION FOR SEQ ID NO:24:
   (i) SEQUENCE CHARACTERISTICS:
      (A) LENGTH: 15 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:24:
      GATCGGGCTA GCATC 15
(2) INFORMATION FOR SEQ ID NO:25:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:25:
      TTACATGGCC AT 12
(2) INFORMATION FOR SEQ ID NO:26:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:26:
      TCACATGGCG AT 12
(2) INFORMATION FOR SEQ ID NO:27:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:27:
      TTTGGACTGG GC 12
(2) INFORMATION FOR SEQ ID NO:28:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:28:
      TTTTGATTGG GC 12
(2) INFORMATION FOR SEQ ID NO:29:
   (i) SEQUENCE CHARACTERISTICS:
      (A) LENGTH: 14 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:29:
      TGGTCCTAAT ACTG 14
(2) INFORMATION FOR SEQ ID NO:30:
   (i) SEQUENCE CHARACTERISTICS:
      (A) LENGTH: 14 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:30:
      TGGGCCTAAT ATCG 14
(2) INFORMATION FOR SEQ ID NO:31:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:31:
      GCATTATCTA GATGTGTCTT CTGGTCAGAG 30
(2) INFORMATION FOR SEQ ID NO:32:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:32:
      CCTGAATTAT AATAATTAAC TGCAGGTCCT 30
(2) INFORMATION FOR SEQ ID NO:33:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:33:
      GGGAAAGAAT CCAGAGACAA GAACGG 26
(2) INFORMATION FOR SEQ ID NO:34:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:34:
      GGTGAAGTTG TGGATCCATT TGATTG 26
(2) INFORMATION FOR SEQ ID NO:35:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:35:
      CAACCTGTAA GGTACCAGCA TCCG 24
(2) INFORMATION FOR SEQ ID NO:36:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:36:
      GATATGGTGT TAGGCCTTGA TCTGTTC 27
(2) INFORMATION FOR SEQ ID NO:37:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:37:
      CGCCATGGAA AAATCAGAGA TCCTCTTCT 29
(2) INFORMATION FOR SEQ ID NO:38:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:38:
      CTGGATCCTA ATTGGAGTTG TTACCCATGT A 31
(2) INFORMATION FOR SEQ ID NO:39:
   (i) SEQUENCE CHARACTERISTICS:
      (A) LENGTH: 23 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:39:
      AACCATGGCT GAAAAAGGGA AAA 23
(2) INFORMATION FOR SEQ ID NO:40:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:40:
      GGTGAAGCTT AAGATGTGAT TTTACATATT TTA 33
(2) INFORMATION FOR SEQ ID NO:41:
   (i) SEQUENCE CHARACTERISTICS:
      (A) LENGTH: 35 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:41:
      AAATAGGATC CCTACAGATC ATTAGATATT AAAAT 35
(2) INFORMATION FOR SEQ ID NO:42:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:42:
      CGCCATGGTG TTCAGTGCTT GTTG 24
(2) INFORMATION FOR SEQ ID NO:43:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:43:
      CCACAAGCTT AATTAACCAT AATATGCATC A 31
(2) INFORMATION FOR SEQ ID NO:44:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:44:
      TTCCATGGAT TTGGATTTGT CTATTGGGT 29
(2) INFORMATION FOR SEQ ID NO:45:
   (i) SEQUENCE CHARACTERISTICS:
      (A) LENGTH: 15462 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:45:
   ACCAAACAAG AGAAGAAACT TGCTTGGTAA TATAAATTTA ACTTAAAATT AACTTAGGAT 60
   TTAAGACATT GACTAGAAGG TCAAGAAAAG GGAACTCTAT AATTTCAAAA ATGTTGAGCC 120
   TATTTGATAC ATTTAATGCA CGTAGGCAAG AAAACATAAC AAAATCAGCC GGTGGAGCTA 180
   TCATTCCTGG ACAGAAAAAT ACTGTCTCTA TATTCGCCCT TGGACCGACA ATAACTGATG 240
   ATAATGAGAA AATGACATTA GCTCTTCTAT TTCTATCTCA TTCACTAGAT AATGAGAAAC 300
   AACATGCACA AAGGGCAGGG TTCTTGGTGT CTTTATTGTC AATGGCTTAT GCCAATCCAG 360
   AGCTCTACCT AACAACAAAT GGAAGTAATG CAGATGCCAA GTATGTCATA TACATGATTG 420
   AGAAAGATCT AAAACGGCAA AAGTATGGAG GATTTGTGGT TAAGACGAGA GAGATGATAT 480
   ATGAAAAGAC AACTGATTGG ATATTTGGAA GTGACCTGGA TTATGATCAG GAAACTATGT 540
   TGCAGAACGG CAGGAACAAT TCAACAATTG AAGACCTTGT CCACACATTT GGGTATCCAT 600
   CATGTTTAGG AGCTCTTATA ATACAGATCT GGATAGTTCT GGTCAAAGCT ATCACTAGTA 660
   TCTCAGGGTT AAGAAAAGGC TTTTTCACCC GATTGGAAGC TTTCAGACAA GATGGAACAG 720
   TGCAGGCAGG GCTGGTATTG AGCGGTGACA CAGTGGATCA GATTGGGTCA ATCATGCGGT 780
   CTCAACAGAG CTTGGTAACT CTTATGGTTG AAACATTAAT AACAATGAAT ACCAGCAGAA 840
   ATGACCTCAC AACCATAGAA AAGAATATAC AAATTGTTGG CAACTACATA AGAGATGCAG 900
   GTCTCGCTTC ATTCTTCAAT ACAATCAGAT ATGGAATTGA GACCAGAATG GCAGCTTTGA 960
   CTCTATCCAC TCTCAGACCA GATATCAATA GATTAAAAGC TTTGATGGAA CTGTATTTAT 1020
   CAAAGGGACC ACGCGCTCCT TTCATCTGTA TCCTCAGAGA TCCTATACAT GGTGAGTTCG 1080
   CACCAGGCAA CTATCCTGCC ATATGGAGCT ATGCAATGGG GGTGGCAGTT GTACAAAATA 1140
   GAGCCATGCA ACAGTATGTG ACGGGAAGAT CATATCTAGA CATTGATATG TTCCAGCTAG 1200
   GACAAGCAGT AGCACGTGAT GCCGAAGCTC AAATGAGCTC AACACTGGAA GATGAACTTG 1260
   GAGTGACACA CGAAGCTAAA GAAAGCTTGA AGAGACATAT AAGGAACATA AACAGTTCAG 1320
   AGACATCTTT CCACAAACCG ACAGGTGGAT CAGCCATAGA GATGGCAATA GATGAAGAGC 1380
   CAGAACAATT CGAACATAGA GCAGATCAAG AACAAAATGG AGAACCTCAA TCATCCATAA 1440
   TTCAATATGC CTGGGCAGAA GGAAATAGAA GCGATGATCA GACTGAGCAA GCTACAGAAT 1500
   CTGACAATAT CAAGACCGAA CAACAAAACA TCAGAGACAG ACTAAACAAG AGACTCAACG 1560
   ACAAGAAGAA ACAAAGCAGT CAACCACCCA CTAATCCCAC AAACAGAACA AACCAGGACG 1620
   AAATAGATGA TCTGTTTAAC GCATTTGGAA GCAACTAATC GAATCAACAT TTTAATCTAA 1680
   ATCAATAATA AATAAGAAAA ACTTAGGATT AAAGAATCCT ATCATACGGG AATATAGGGT 1740
   GGTAAATTTA GAGTCTGCTT GAAACTCAAT CAATAGAGAG TTGATGGAAA GCGATGCTAA 1800
   AAACTATCAA ATCATGGATT CTTGGGAAGA GGAATCAAGA GATAAATCAA CTAATATCTC 1860
   CTCGGCCCTC AACATCATTG AATTCATACT CAGCACCGAC CCCCAAGAAG ACTTATCGGA 1920
   AAACGACACA ATCAACACAA GAACCCAGCA ACTCAGTGCC ACCATCTGTC AACCAGAAAT 1980
   CAAACCAACA GAAACAAGTG AGAAAGATAG TGGATCAACT GACAAAAATA GACAGTCTGG 2040
   GTCATCACAC GAATGTACAA CAGAAGCAAA AGATAGAAAC ATTGATCAGG AAACTGTACA 2100
   GAGAGGACCT GGGAGAAGAA GCAGCTCAGA TAGTAGAGCT GAGACTGTGG TCTCTGGAGG 2160
   AATCCCCAGA AGCATCACAG ATTCTAAAAA TGGAACCCAA AACACGGAGG ATATTGATCT 2220
   CAATGAAATT AGAAAGATGG ATAAGGACTC TATTGAGGGG AAAATGCGAC AATCTGCAAA 2280
   TGTTCCAAGC GAGATATCAG GAAGTGATGA CATATTTACA ACAGAACAAA GTAGAAACAG 2340
   TGATCATGGA AGAAGCCTGG AATCTATCAG TACACCTGAT ACAAGATCAA TAAGTGTTGT 2400
   TACTGCTGCA ACACCAGATG ATGAAGAAGA AATACTAATG AAAAATAGTA GGACAAAGAA 2460
   AAGTTCTTCA ACACATCAAG AAGATGACAA AAGAATTAAA AAAGGGGGAA AAGGGAAAGA 2520
   CTGGTTTAAG AAATCAAAAG ATACCGACAA CCAGATACCA ACATCAGACT ACAGATCCAC 2580
   ATCAAAAGGG CAGAAGAAAA TCTCAAAGAC AACAACCACC AACACCGACA CAAAGGGGCA 2640
   AACAGAAATA CAGACAGAAT CATCAGAAAC ACAATCCTCA TCATGGAATC TCATCATCGA 2700
   CAACAACACC GACCGGAACG AACAGACAAG CACAACTCCT CCAACAACAA CTTCCAGATC 2760
   AACTTATACA AAAGAATCGA TCCGAACAAA CTCTGAATCC AAACCCAAGA CACAAAAGAC 2820
   AAATGGAAAG GAAAGGAAGG ATACAGAAGA GAGCAATCGA TTTACAGAGA GGGCAATTAC 2880
   TCTATTGCAG AATCTTGGTG TAATTCAATC CACATCAAAA CTAGATTTAT ATCAAGACAA 2940
   ACGAGTTGTA TGTGTAGCAA ATGTACTAAA CAATGTAGAT ACTGCATCAA AGATAGATTT 3000
   CCTGGCAGGA TTAGTCATAG GGGTTTCAAT GGACAACGAC ACAAAATTAA CACAGATACA 3060
   AAATGAAATG CTAAACCTCA AAGCAGATCT AAAGAAAATG GACGAATCAC ATAGAAGATT 3120
   GATAGAAAAT CAAAGAGAAC AACTGTCATT GATCACGTCA CTAATTTCAA ATCTCAAAAT 3180
   TATGACTGAG AGAGGAGGAA AGAAAGACCA AAATGAATCC AATGAGAGAG TATCCATGAT 3240
   CAAAACAAAA TTGAAAGAAG AAAAGATCAA GAAGACCAGG TTTGACCCAC TTATGGAGGC 3300
   ACAAGGCATT GACAAGAATA TACCCGATCT ATATCGACAT GCAGGAGATA CACTAGAGAA 3360
   CGATGTACAA GTTAAATCAG AGATATTAAG TTCATACAAT GAGTCAAATG CAACAAGACT 3420
   AATACCCAAA AAAGTGAGCA GTACAATGAG ATCACTAGTT GCAGTCATCA ACAACAGCAA 3480
   TCTCTCACAA AGCACAAAAC AATCATACAT AAACGAACTC AAACGTTGCA AAAATGATGA 3540
   AGAAGTATCT GAATTAATGG ACATGTTCAA TGAAGATGTC AACAATTGCC AATGATCCAA 3600
   CAAAGAAACG ACACCGAACA AACAGACAAG AAACAACAGT AGATCAAAAC CTGTCAACAC 3660
   ACACAAAATC AAGCAGAATG AAACAACAGA TATCAATCAA TATACAAATA AGAAAAACTT 3720
   AGGATTAAAG AATAAATTAA TCCTTGTCCA AAATGAGTAT AACTAACTCT GCAATATACA 3780
   CATTCCCAGA ATCATCATTC TCTGAAAATG GTCATATAGA ACCATTACCA CTCAAAGTCA 3840
   ATGAACAGAG GAAAGCAGTA CCCCACATTA GAGTTGCCAA GATCGGAAAT CCACCAAAAC 3900
   ACGGATCCCG GTATTTAGAT GTCTTCTTAC TCGGCTTCTT CGAGATGGAA CGAATCAAAG 3960
   ACAAATACGG GAGTGTGAAT GATCTCGACA GTGACCCGAG TTACAAAGTT TGTGGCTCTG 4020
   GATCATTACC AATCGGATTG GCTAAGTACA CTGGGAATGA CCAGGAATTG TTACAAGCCG 4080
   CAACCAAACT GGATATAGAA GTGAGAAGAA CAGTCAAAGC GAAAGAGATG GTTGTTTACA 4140
   CGGTACAAAA TATAAAACCA GAACTGTACC CATGGTCCAA TAGACTAAGA AAAGGAATGC 4200
   TGTTCGATGC CAACAAAGTT GCTCTTGCTC CTCAATGTCT TCCACTAGAT AGGAGCATAA 4260
   AATTTAGAGT AATCTTCGTG AATTGTACGG CAATTGGATC AATAACCTTG TTCAAAATTC 4320
   CTAAGTCAAT GGCATCACTA TCGTTAACCA ACACAATATC AATCAATCTG CAGGTACACA 4380
   TAAAAACAGG GGTTCAGACT GATTCTAAAG GGATAGTTCA AATTTTGGAT GAGAAAGGCG 4440
   AAAAATCACT GAATTTCATG GTCCATCTCG GATTGATCAA AAGAAAAGTA GGCAGAATGT 4500
   ACTCTGTTGA ATACTGTAAA CAGAAAATCG AGAAAATGAG ATTGATATTT TCTTTAGGAC 4560
   TAGTTGGAGG AATCAGTCTT CATGTCAATG CAACTGGGTC CATATCAAAA ACACTAGCAA 4620
   GTCAGCTGGT ATTCAAAAGA GAGATTTGTT ATCCTTTAAT GGATCTAAAT CCGCATCTCA 4680
   ATCTAGTTAT CTGGGCTTCA TCAGTAGAGA TTACAAGAGT GGATGCAATT TTCCAACCTT 4740
   CTTTACCTGG CGAGTTCAGA TACTATCCTA ATATTATTGC AAAAGGAGTT GGGAAAATCA 4800
   AACAATGGAA CTAGTAATCT CTATTTTAGT CCGGACGTAT CTATTAAGCC GAAGCAAATA 4860
   AAGGATAATC AAAAACTTAG GACAAAAGAG GTCAATACCA ACAACTATTA GCAGTCACAC 4920
   TCGCAAGAAT AAGAGAGAAG GGACCAAAAA AGTCAAATAG GAGAAATCAA AACAAAAGGT 4980
   ACAGAACACC AGAACAACAA AATCAAAACA TCCAACTCAC TCAAAACAAA AATTCCAAAA 5040
   GAGACCGGCA ACACAACAAG CACTGAACAC AATGCCAACT TCAATACTGC TAATTATTAC 5100
   AACCATGATC ATGGCATCTT TCTGCCAAAT AGATATCACA AAACTACAGC ACGTAGGTGT 5160
   ATTGGTCAAC AGTCCCAAAG GGATGAAGAT ATCACAAAAC TTTGAAACAA GATATCTAAT 5220
   TTTGAGCCTC ATACCAAAAA TAGAAGACTC TAACTCTTGT GGTGACCAAC AGATCAAGCA 5280
   ATACAAGAAG TTATTGGATA GACTGATCAT CCCTTTATAT GATGGATTAA GATTACAGAA 5340
   AGATGTGATA GTAACCAATC AAGAATCCAA TGAAAACACT GATCCCAGAA CAAAACGATT 5400
   CTTTGGAGGG GTAATTGGAA CCATTGCTCT GGGAGTAGCA ACCTCAGCAC AAATTACAGC 5460
   GGCAGTTGCT CTGGTTGAAG CCAAGCAGGC AAGATCAGAC ATCGAAAAAC TCAAAGAAGC 5520
   AATTAGGGAC ACAAACAAAG CAGTGCAGTC AGTTCAGAGC TCCATAGGAA ATTTAATAGT 5580
   AGCAATTAAA TCAGTCCAGG ATTATGTTAA CAAAGAAATC GTGCCATCGA TTGCGAGGCT 5640
   AGGTTGTGAA GCAGCAGGAC TTCAATTAGG AATTGCATTA ACACAGCATT ACTCAGAATT 5700
   AACAAACATA TTTGGTGATA ACATAGGATC GTTACAAGAA AAAGGAATAA AATTACAAGG 5760
   TATAGCATCA TTATACCGCA CAAATATCAC AGAAATATTC ACAACATCAA CAGTTGATAA 5820
   ATATGATATC TATGATCTGT TATTTACAGA ATCAATAAAG GTGAGAGTTA TAGATGTTGA 5880
   CTTGAATGAT TACTCAATCA CCCTCCAAGT CAGACTCCCT TTATTAACTA GGCTGCTGAA 5940
   CACTCAGATC TACAAAGTAG ATTCCATATC ATATAACATC CAAAACAGAG AATGGTATAT 6000
   CCCTCTTCCC AGCCATATCA TGACGAAAGG GGCATTTCTA GGTGGAGCAG ACGTCAAAGA 6060
   ATGTATAGAA GCATTCAGCA GCTATATATG CCCTTCTGAT CCAGGATTTG TATTAAACCA 6120
   TGAAATAGAG AGCTGCTTAT CAGGAAACAT ATCCCAATGT CCAAGAACAA CGGTCACATC 6180
   AGACATTGTT CCAAGATATG CATTTGTCAA TGGAGGAGTG GTTGCAAACT GTATAACAAC 6240
   CACCTGTACA TGCAACGGAA TTGGTAATAG AATCAATCAA CCACCTGATC AAGGAGTAAA 6300
   AATTATAACA CATAAAGAAT GTAGTACTGT GGGTATCAAC GGAATGCTGT TCAATACAAA 6360
   TAAAGAAGGA ACTCTTGCAT TCTATACACC AAATGATATA ACACTAAACA ATTCTGTTAC 6420
   ACTGGATCCA ATTGACATAT CAATCGAGCT CAACAAGGCC AAATCAGATC TAGAAGAATC 6480
   AAAAGAATGG ATAAGAAGGT CAAATCAAAA ACTAGATTCT ATTGGAAATT GGCATCAATC 6540
   TAGCACTACA ATCATAATTA TTTTGATAAT GATCATTATA TTGTTTATAA TTAATATAAC 6600
   GATAATTACA ATTGCAATTA AGTATTACAG AATTCAAAAG AGAAATCGAG TGGATCAAAA 6660
   TGACAAGCCA TATGTACTAA CAAACAAATA ACATATCTAC AGATCATTAG ATATTAAAAT 6720
   TATAAAAAAC TTAGGAGTAA AGTTACGCAA TCCAACTCTA CTCATATAAT TGAGGAAGGA 6780
   CCCAATAGAC AAATCCAAAT TCGAGATGGA ATACTGGAAG CATACCAATC ACGGAAAGGA 6840
   TGCTGGTAAT GAGCTGGAGA CGTCTATGGC TACTCATGGC AACAAGCTCA CTAATAAGAT 6900
   AATATACATA TTATGGACAA TAATCCTGGT GTTATTATCA ATAGTCTTCA TCATAGTGCT 6960
   AATTAATTCC ATCAAAAGTG AAAAGGCCCA CGAATCATTG CTGCAAGACA TAAATAATGA 7020
   GTTTATGGAA ATTACAGAAA AGATCCAAAT GGCATCGGAT AATACCAATG ATCTAATACA 7080
   GTCAGGAGTG AATACAAGGC TTCTTACAAT TCAGAGTCAT GTCCAGAATT ACATACCAAT 7140
   ATCATTGACA CAACAGATGT CAGATCTTAG GAAATTCATT AGTGAAATTA CAATTAGAAA 7200
   TGATAATCAA GAAGTGCTGC CACAAAGAAT AACACATGAT GTAGGTATAA AACCTTTAAA 7260
   TCCAGATGAT TTTTGGAGAT GCACGTCTGG TCTTCCATCT TTAATGAAAA CTCCAAAAAT 7320
   AAGGTTAATG CCAGGGCCGG GATTATTAGC TATGCCAACG ACTGTTGATG GCTGTGTTAG 7380
   AACTCCGTCT TTAGTTATAA ATGATCTGAT TTATGCTTAT ACCTCAAATC TAATTACTCG 7440
   AGGTTGTCAG GATATAGGAA AATCATATCA AGTCTTACAG ATAGGGATAA TAACTGTAAA 7500
   CTCAGACTTG GTACCTGACT TAAATCCTAG GATCTCTCAT ACCTTTAACA TAAATGACAA 7560
   TAGGAAGTCA TGTTCTCTAG CACTCCTAAA TATAGATGTA TATCAACTGT GTTCAACTCC 7620
   CAAAGTTGAT GAAAGATCAG ATTATGCATC ATCAGGCATA GAAGATATTG TACTTGATAT 7680
   TGTCAATTAT GATGGTTCAA TCTCAACAAC AAGATTTAAG AATAATAACA TAAGCTTTGA 7740
   TCAACCATAT GCTGCACTAT ACCCATCTGT TGGACCAGGG ATATACTACA AAGGCAAAAT 7800
   AATATTTCTC GGGTATGGAG GTCTTGAACA TCCAATAAAT GAGAATGTAA TCTGCAACAC 7860
   AACTGGGTGC CCCGGGAAAA CACAGAGAGA CTGTAATCAA GCATCTCATA GTACTTGGTT 7920
   TTCAGATAGG AGGATGGTCA ACTCCATCAT TGTGGCTGAC AAAGGCTTAA ACTCAATTCC 7980
   AAAATTGAAA GTATGGACGA TATCTATGCG ACAAAATTAC TGGGGGTCAG AAGGAAGGTT 8040
   ACTTCTACTA GGTAACAAGA TCTATATATA TACAAGATCT ACAAGTTGGC ATAGCAAGTT 8100
   ACAATTAGGA ATAATTGATA TTACTGATTA CAGTGATATA AGGATAAAAT GGACATGGCA 8160
   TAATGTGCTA TCAAGACCAG GAAACAATGA ATGTCCATGG GGACATTCAT GTCCAGATGG 8220
   ATGTATAACA GGAGTATATA CTGATGCATA TCCACTCAAT CCCACAGGGA GCATTGTGTC 8280
   ATCTGTCATA TTAGACTCAC AAAAATCGAG AGTGAACCCA GTCATAACTT ACTCAACAGC 8340
   AACCGAAAGA GTAAACGAGC TGGCCATCCT AAACAGAACA CTCTCAGCTG GATATACAAC 8400
   AACAAGCTGC ATTACACACT ATAACAAAGG ATATTGTTTT CATATAGTAG AAATAAATCA 8460
   TAAAAGCTTA AACACATTTC AACCCATGTT GTTCAAAACA GAGATTCCAA AAAGCTGCAG 8520
   TTAATCATAA TTAACCATAA TATGCATCAA TCTATCTATA ATACAAGTAT ATGATAAGTA 8580
   ATCAGCAATC AGACAATAGA CAAAAGGGAA ATATAAAAAA CTTAGGAGCA AAGCGTGCTC 8640
   GGGAAATGGA CACTGAATCT AACAATGGCA CTGTATCTGA CATACTCTAT CCTGAGTGTC 8700
   ACCTTAACTC TCCTATCGTT AAAGGTAAAA TAGCACAATT ACACACTATT ATGAGTCTAC 8760
   CTCAGCCTTA TGATATGGAT GACGACTCAA TACTAGTTAT CACTAGACAG AAAATAAAAC 8820
   TTAATAAATT GGATAAAAGA CAACGATCTA TTAGAAGATT AAAATTAATA TTAACTGAAA 8880
   AAGTGAATGA CTTAGGAAAA TACACATTTA TCAGATATCC AGAAATGTCA AAAGAAATGT 8940
   TCAAATTATA TATACCTGGT ATTAACAGTA AAGTGACTGA ATTATTACTT AAAGCAGATA 9000
   GAACATATAG TCAAATGACT GATGGATTAA GAGATCTATG GATTAATGTG CTATCAAAAT 9060
   TAGCCTCAAA AAATGATGGA AGCAATTATG ATCTTAATGA AGAAATTAAT AATATATCGA 9120
   AAGTTCACAC AACCTATAAA TCAGATAAAT GGTATAATCC ATTCAAAACA TGGTTTACTA 9180
   TCAAGTATGA TATGAGAAGA TTACAAAAAG CTCGAAATGA GATCACTTTT AATGTTGGGA 9240
   AGGATTATAA CTTGTTAGAA GACCAGAAGA ATTTCTTATT GATACATCCA GAATTGGTTT 9300
   TGATATTAGA TAAACAAAAC TATAATGGTT ATCTAATTAC TCCTGAATTA GTATTGATGT 9360
   ATTGTGACGT AGTCGAAGGC CGATGGAATA TAAGTGCATG TGCTAAGTTA GATCCAAAAT 9420
   TACAATCTAT GTATCAGAAA GGTAATAACC TGTGGGAAGT GATAGATAAA TTGTTTCCAA 9480
   TTATGGGAGA AAAGACATTT GATGTGATAT CGTTATTAGA ACCACTTGCA TTATCCTTAA 9540
   TTCAAACTCA TGATCCTGTT AAACAACTAA GAGGAGCTTT TTTAAATCAT GTGTTATCCG 9600
   AGATGGAATT AATATTTGAA TCTAGAGAAT CGATTAAGGA ATTTCTGAGT GTAGATTACA 9660
   TTGATAAAAT TTTAGATATA TTTAATAAGT CTACAATAGA TGAAATAGCA GAGATTTTCT 9720
   CTTTTTTTAG AACATTTGGG CATCCTCCAT TAGAAGCTAG TATTGCAGCA GAAAAGGTTA 9780
   GAAAATATAT GTATATTGGA AAACAATTAA AATTTGACAC TATTAATAAA TGTCATGCTA 9840
   TCTTCTGTAC AATAATAATT AACGGATATA GAGAGAGGCA TGGTGGACAG TGGCCTCCTG 9900
   TGACATTACC TGATCATGCA CACGAATTCA TCATAAATGC TTACGGTTCA AACTCTGCGA 9960
   TATCATATGA AAATGCTGTT GATTATTACC AGAGCTTTAT AGGAATAAAA TTCAATAAAT 10020
   TCATAGAGCC TCAGTTAGAT GAGGATTTGA CAATTTATAT GAAAGATAAA GCATTATCTC 10080
   CAAAAAAATC AAATTGGGAC ACAGTTTATC CTGCATCTAA TTTACTGTAC CGTACTAACG 10140
   CATCCAACGA ATCACGAAGA TTAGTTGAAG TATTTATAGC AGATAGTAAA TTTGATCCTC 10200
   ATCAGATATT GGATTATGTA GAATCTGGGG ACTGGTTAGA TGATCCAGAA TTTAATATTT 10260
   CTTATAGTCT TAAAGAAAAA GAGATCAAAC AGGAAGGTAG ACTCTTTGCA AAAATGACAT 10320
   ACAAAATGAG AGCTACACAA GTTTTATCAG AGACCCTACT TGCAAATAAC ATAGGAAAAT 10380
   TCTTTCAAGA AAATGGGATG GTGAAGGGAG AGATTGAATT ACTTAAGAGA TTAACAACCA 10440
   TATCAATATC AGGAGTTCCA CGGTATAATG AAGTGTACAA TAATTCTAAA AGCCATACAG 10500
   ATGACCTTAA AACCTACAAT AAAATAAGTA ATCTTAATTT GTCTTCTAAT CAGAAATCAA 10560
   AGAAATTTGA ATTCAAGTCA ACGGATATCT ACAATGATGG ATACGAGACT GTGAGCTGTT 10620
   TCCTAACAAC AGATCTCAAA AAATACTGTC TTAATTGGAG ATATGAATCA ACAGCTCTAT 10680
   TTGGAGAAAC TTGCAACCAA ATATTTGGAT TAAATAAATT GTTTAATTGG TTACACCCTC 10740
   GTCTTGAAGG AAGTACAATC TATGTAGGTG ATCCTTACTG TCCTCCATCA GATAAAGAAC 10800
   ATATATCATT AGAGGATCAC CCTGATTCTG GTTTTTACGT TCATAACCCA AGAGGGGGTA 10860
   TAGAAGGATT TTGTCAAAAA TTATGGACAC TCATATCTAT AAGTGCAATA CATCTAGCAG 10920
   CTGTTAGAAT AGGCGTGAGG GTGACTGCAA TGGTTCAAGG AGACAATCAA GCTATAGCTG 10980
   TAACCACAAG AGTACCCAAC AATTATGACT ACAGAGTTAA GAAGGAGATA GTTTATAAAG 11040
   ATGTAGTGAG ATTTTTTGAT TCATTAAGAG AAGTGATGGA TGATCTAGGT CATGAACTTA 11100
   AATTAAATGA AACGATTATA AGTAGCAAGA TGTTCATATA TAGCAAAAGA ATCTATTATG 11160
   ATGGGAGAAT TCTTCCTCAA GCTCTAAAAG CATTATCTAG ATGTGTCTTC TGGTCAGAGA 11220
   CAGTAATAGA CGAAACAAGA TCAGCATCTT CAAATTTGGC AACATCATTT GCAAAAGCAA 11280
   TTGAGAATGG TTATTCACCT GTTCTAGGAT ATGCATGCTC AATTTTTAAG AATATTCAAC 11340
   AACTATATAT TGCCCTTGGG ATGAATATCA ATCCAACTAT AACACAGAAT ATCAGAGATC 11400
   AGTATTTTAG GAATCCAAAT TGGATGCAAT ATGCCTCTTT AATACCTGCT AGTGTTGGGG 11460
   GATTCAATCA CATGGCGATG TCAAGATGTT TTGTAAGGAA TATTGGTGAT CCATCAGTTG 11520
   CCGCATTGGC TGATATTAAA AGATTTATTA AGGCGAATCT ATTAGACCGA AGTGTTCTTT 11580
   ATAGGATTAT GAATCAAGAA CCAGGTGAGT CATCTTTTTT TGATTGGGCT TCAGATCCAT 11640
   ATTCATGCAA TTTACCACAA TCTCAAAATA TAACCACCAT GATAAAAAAT ATAACAGCAA 11700
   GGAATGTATT ACAAGATTCA CCAAATCCAT TATTATCTGG ATTATTCACA AATACAATGA 11760
   TAGAAGAAGA TGAAGAATTA GCTGAGTTCC TGATGGACAG GAAGGTAATT CTCCCTAGAG 11820
   TTGCACATGA TATTCTAGAT AATTCTCTCA CAGGAATTAG AAATGCCATA GCTGGAATGT 11880
   TAGATACGAC AAAATCACTA ATTCGGGTTG GCATAAATAG AGGAGGACTG ACATATAGTT 11940
   TGTTGAGGAA AATCAGTAAT TACGATCTAG TACAATATGA AACACTAAGT AGGACTTTGC 12000
   GACTAATTGT AAGTGATAAA ATCAAGTATG AAGATATGTG TTCGGTAGAC CTTGCCATAG 12060
   CATTGCGACA AAAGATGTGG ATTCATTTAT CAGGAGGAAG GATGATAAGT GGACTTGAAA 12120
   CGCCTGACCC ATTAGAATTA CTATCTGGGG TAGTAATAAC AGGATCAGAA CATTGTAAAA 12180
   TATGTTATTC TTCAGATGGC ACAAACCCAT ATACTTGGAT GTATTTACCC GGTAATATCA 12240
   AAATAGGATC AGCAGAAACA GGTATATCGT CATTAAGAGT TCCTTATTTT GGATCAGTCA 12300
   CTGATGAAAG ATCTGAAGCA CAATTAGGAT ATATCAAGAA TCTTAGTAAA CCTGCAAAAG 12360
   CCGCAATAAG AATAGCAATG ATATATACAT GGGCATTTGG TAATGATGAG ATATCTTGGA 12420
   TGGAAGCCTC ACAGATAGCA CAAACACGTG CAAATTTTAC ACTAGATAGT CTCAAAATTT 12480
   TAACACCGGT AGCTACATCA ACAAATTTAT CACACAGATT AAAGGATACT GCAACTCAGA 12540
   TGAAATTCTC CAGTACATCA TTGATCAGAG TCAGCAGATT CATAACAATG TCCAATGATA 12600
   ACATGTCTAT CAAAGAAGCT AATGAAACCA AAGATACTAA TCTTATTTAT CAACAAATAA 12660
   TGTTAACAGG ATTAAGTGTT TTCGAATATT TATTTAGATT AAAAGAAACC ACAGGACACA 12720
   ACCCTATAGT TATGCATCTG CACATAGAAG ATGAGTGTTG TATTAAAGAA AGTTTTAATG 12780
   ATGAACATAT TAATCCAGAG TCTACATTAG AATTAATTCG ATATCCTGAA AGTAATGAAT 12840
   TTATTTATGA TAAAGACCCA CTCAAAGATG TGGACTTATC AAAACTTATG GTTATTAAAG 12900
   ACCATTCTTA CACAATTGAT ATGAATTATT GGGATGATAC TGACATCATA CATGCAATTT 12960
   CAATATGTAC TGCAATTACA ATAGCAGATA CTATGTCACA ATTAGATCGA GATAATTTAA 13020
   AAGAGATAAT AGTTATTGCA AATGATGATG ATATTAATAG CTTAATCACT GAATTTTTGA 13080
   CTCTTGACAT ACTTGTATTT CTCAAGACAT TTGGTGGATT ATTAGTAAAT CAATTTGCAT 13140
   ACACTCTTTA TAGTCTAAAA ATAGAAGGTA GGGATCTCAT TTGGGATTAT ATAATGAGAA 13200
   CACTGAGAGA TACTTCCCAT TCAATATTAA AAGTATTATC TAATGCATTA TCTCATCCTA 13260
   AAGTATTCAA GAGGTTCTGG GATTGTGGAG TTTTAAACCC TATTTATGGG CCTAATATCG 13320
   CTAGTCAAGA CCAGATAAAA CTTGCCCTAT CTATATGTGA ATATTCACTA GATCTATTTA 13380
   TGAGAGAATG GTTGAATGGT GTATCACTTG AAATATACAT TTGTGACAGC GATATGGAAG 13440
   TTGCAAATGA TAGGAAACAA GCCTTTATTT CTAGACACCT TTCATTTGTT TGTTGTTTAG 13500
   CAGAAATTGC ATCTTTCGGA CCTAACCTGT TAAACTTAAC ATACTTGGAG AGACTTGATC 13560
   TATTGAAACA ATATCTTGAA TTAAATATTA AAGAAGACCC TACTCTTAAA TATGTACAAA 13620
   TATCTGGATT ATTAATTAAA TCGTTCCCAT CAACTGTAAC ATACGTAAGA AAGACTGCAA 13680
   TCAAATATCT AAGGATTCGC GGTATTAGTC CACCTGAGGT AATTGATGAT TGGGATCCGG 13740
   TAGAAGATGA AAATATGCTG GATAACATTG TCAAAACTAT AAATGATAAC TGTAATAAAG 13800
   ATAATAAAGG GAATAAAATT AACAATTTCT GGGGACTAGC ACTTAAGAAC TATCAAGTCC 13860
   TTAAAATCAG ATCTATAACA AGTGATTCTG ATGATAATGA TAGACTAGAT GCTAATACAA 13920
   GTGGTTTGAC ACTTCCTCAA GGAGGGAATT ATCTATCGCA TCAATTGAGA TTATTCGGAA 13980
   TCAACAGCAC TAGTTGTCTG AAAGCTCTTG AGTTATCACA AATTTTAATG AAGGAAGTCA 14040
   ATAAAGACAA GGACAGGCTC TTCCTGGGAG AAGGAGCAGG AGCTATGCTA GCATGTTATG 14100
   ATGCCACATT AGGACCTGCA GTTAATTATT ATAATTCAGG TTTGAATATA ACAGATGTAA 14160
   TTGGTCAACG AGAATTGAAA ATATTTCCTT CAGAGGTATC ATTAGTAGGT AAAAAATTAG 14220
   GAAATGTGAC ACAGATTCTT AACAGGGTAA AAGTACTGTT CAATGGGAAT CCTAATTCAA 14280
   CATGGATAGG AAATATGGAA TGTGAGAGCT TAATATGGAG TGAATTAAAT GATAAGTCCA 14340
   TTGGATTAGT ACATTGTGAT ATGGAAGGAG CTATCGGTAA ATCAGAAGAA ACTGTTCTAC 14400
   ATGAACATTA TAGTGTTATA AGAATTACAT ACTTGATTGG GGATGATGAT GTTGTTTTAG 14460
   TTTCCAAAAT TATACCTACA ATCACTCCGA ATTGGTCTAG AATACTTTAT CTATATAAAT 14520
   TATATTGGAA AGATGTAAGT ATAATATCAC TCAAAACTTC TAATCCTGCA TCAACAGAAT 14580
   TATATCTAAT TTCGAAAGAT GCATATTGTA CTATAATGGA ACCTAGTGAA ATTGTTTTAT 14640
   CAAAACTTAA AAGATTGTCA CTCTTGGAAG AAAATAATCT ATTAAAATGG ATCATTTTAT 14700
   CAAAGAAGAG GAATAATGAA TGGTTACATC ATGAAATCAA AGAAGGAGAA AGAGATTATG 14760
   GAATCATGAG ACCATATCAT ATGGCACTAC AAATCTTTGG ATTTCAAATC AATTTAAATC 14820
   ATCTGGCGAA AGAATTTTTA TCAACCCCAG ATCTGACTAA TATCAACAAT ATAATCCAAA 14880
   GTTTTCAGCG AACAATAAAG GATGTTTTAT TTGAATGGAT TAATATAACT CATGATGATA 14940
   AGAGACATAA ATTAGGCGGA AGATATAACA TATTCCCACT GAAAAATAAG GGAAAGTTAA 15000
   GACTGCTATC GAGAAGACTA GTATTAAGTT GGATTTCATT ATCATTATCG ACTCGATTAC 15060
   TTACAGGTCG CTTTCCTGAT GAAAAATTTG AACATAGAGC ACAGACTGGA TATGTATCAT 15120
   TAGCTGATAC TGATTTAGAA TCATTAAAGT TATTGTCGAA AAACATCATT AAGAATTACA 15180
   GAGAGTGTAT AGGATCAATA TCATATTGGT TTCTAACCAA AGAAGTTAAA ATACTTATGA 15240
   AATTGATCGG TGGTGCTAAA TTATTAGGAA TTCCCAGACA ATATAAAGAA CCCGAAGACC 15300
   AGTTATTAGA AAACTACAAT CAACATGATG AATTTGATAT CGATTAAAAC ATAAATACAA 15360
   TGAAGATATA TCCTAACCTT TATCTTTAAG CCTAGGAATA GACAAAAAGT AAGAAAAACA 15420
   TGTAATATAT ATATACCAAA CAGAGTTCTT CTCTTGTTTG GT 15462
(2) INFORMATION FOR SEQ ID NO:46:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:46:
      TTGTCTGGGA AT 12
(2) INFORMATION FOR SEQ ID NO:47:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:47:
      TTGCCTGGGA AT 12
(2) INFORMATION FOR SEQ ID NO:48:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:48:
      TTGTTTGGGA AT 12
(2) INFORMATION FOR SEQ ID NO:49:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:49:
      TTGTCTGGTA AT 12
(2) INFORMATION FOR SEQ ID NO:50:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:50:
      AACTTTAAAT TA 12
(2) INFORMATION FOR SEQ ID NO:51:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:51:
      AACTTAAAAT TA 12
(2) INFORMATION FOR SEQ ID NO:52:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:52:
      TTAAAGACAT TG 12
(2) INFORMATION FOR SEQ ID NO:53:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:53:
      TTTAAGACAT TG 12
(2) INFORMATION FOR SEQ ID NO:54:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:54:
      GCAGATGTCA AG 12
(2) INFORMATION FOR SEQ ID NO:55:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:55:
      GCAGATGCCA AG 12
(2) INFORMATION FOR SEQ ID NO:56:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:56:
      CGAATCTAAA GA 12
(2) INFORMATION FOR SEQ ID NO:57:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:57:
      CGAAGCTAAA GA 12
(2) INFORMATION FOR SEQ ID NO:58:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:58:
      GAAATATTGA TC 12
(2) INFORMATION FOR SEQ ID NO:59:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:59:
      GAAACATTGA TC 12
(2) INFORMATION FOR SEQ ID NO:60:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:60:
      TCTCTACCCA AC 12
(2) INFORMATION FOR SEQ ID NO:61:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:61:
      TCGTTAACCA AC 12
(2) INFORMATION FOR SEQ ID NO:62:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:62:
      AGTACAATAG GT 12
(2) INFORMATION FOR SEQ ID NO:63:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:63:
      AGTACTGTGG GT 12
(2) INFORMATION FOR SEQ ID NO:64:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:64:
      GCACTTGATC CA 12
(2) INFORMATION FOR SEQ ID NO:65:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:65:
      ACACTGGATC CA 12
(2) INFORMATION FOR SEQ ID NO:66:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:66:
      CCATCATTGT TGTTGACAA 19
(2) INFORMATION FOR SEQ ID NO:67:
   (i) SEQUENCE CHARACTERISTICS:
      (A) LENGTH: 19 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:67:
      CCATCATTGT GGCTGACAA 19
(2) INFORMATION FOR SEQ ID NO:68:
   (i) SEQUENCE CHARACTERISTICS:
      (A) LENGTH: 11 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:68: TTACATGGCC A 11
(2) INFORMATION FOR SEQ ID NO:69:
   (i) SEQUENCE CHARACTERISTICS:
      (A) LENGTH: 11 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:69:
      TCACATGGCG A 11
(2) INFORMATION FOR SEQ ID NO:70:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:70:
      TTTGGACTGG GC 12
(2) INFORMATION FOR SEQ ID NO:71: .
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:71:
      TTTTGATTGG GC 12
(2) INFORMATION FOR SEQ ID NO:72:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:72:
      GGTCCTAATA CT 12
(2) INFORMATION FOR SEQ ID NO:73:
   (i) SEQUENCE CHARACTERISTICS:
      (A) LENGTH: 12 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:73:
      GGGCCTAATA TC 12
(2) INFORMATION FOR SEQ ID NO:74:
   (i) SEQUENCE CHARACTERISTICS:
      (A) LENGTH: 38 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:74
      CCATAGAGAG TCCATGGAAA GCGACGCTAA AAACTATC 38

## Description

### Background of the invention

Human parainfluenza viruses (HPIV), HPIV1, HPIV2, and HPIV3 are significant causes of bronchiolitis, croup and pneumonia in infants and children. Karron et al., J. Infect. Dis. 172: 1445-50 (1995); Collins et al. "Parainfluenza Viruses", p. 1205-1243. In B. N. Fields et al., eds., Fields Virology, 3rd ed, vol. 1. Lippincott-Raven Publ., Philadelphia (1996); Murphy et al., Virus Res. 11:1-15 (1988). Infections by these viruses result in substantial morbidity in children less than 3 years of age, and are responsible for approximately 20% of hospitalizations among young infants and children for respiratory tract infections.

Despite considerable efforts to develop effective vaccine therapies against HPIV, no approved vaccine agents have yet been achieved for any HPIV strain, nor for ameliorating HPIV related illnesses. To date, only two live attenuated PIV vaccine candidates have received particular attention. One of these candidates is a bovine PIV (BPIV) strain that is antigenically related to HPIV3, and which has been shown to protect animals against HPIV3. BPIV3 is attenuated, genetically stable and immunogenic in human infants and children (Karron et al., J. Inf. Dis. 171: 1107-14 (1995a); Karron et al., J. Inf. Dis. 172:1445-1450, (1995b)). A second PIV3 vaccine candidate, JS *cp*45 is a cold-adapted mutant of the JS wildtype (wt) strain of HPIV3 (Karron et al., (1995b), supra; Belshe et al., J. Med. Virol. 10:235-42 (1982)). This live, attenuated, cold-passaged (*cp*) PIV3 vaccine candidate exhibits temperature-sensitive (*ts*), cold-adaptation (ca), and attenuation (att) phenotypes which are stable after viral replication *in vivo*. The *cp*45 virus is protective against human PIV3 challenge in experimental animals and is attenuated, genetically stable, and immunogenic in seronegative human infants and children (Hall et al., Virus Res. 22:173-184 (1992); Karron et al., (1995b), supra.

HPIV3 is a member of the Paramyxovirus genus of the Paramyxovirus family, order Mononegavirales. Its genome is a single strand of negative-sense RNA 15462 nucleotides (nt) in length (Galinski et al., Virology 165: 499-510, (1988); Stokes et al., Virus Res. 25:91-103 (1992)) and encodes at least eight proteins (Collins et al., supra, (1996); Galinski, supra, (1991); Spriggs and Collins, J. Gen. Virol. 67: 2705-2719, (1986)). Three of these proteins are associated with the RNA genome to form the nucleocapsid; namely the nucleocapsid protein N, phosphoprotein P, and large polymerase subunit L. Three additional proteins are associated with the envelope, namely the matrix protein M, taught to mediate viral attachment and release, the hemagglutinin-neuraminidase protein HN, and the fusion protein F. Two other proteins, HN and F, represent the neutralizing and protective antigens of PIVs (Collins et al. In Fields Virology, 3rd ed., 1:1205-43 (1996)). Significant sequence divergence in these two protective antigens among different PIVs is the basis for the type specificity of protective immunity against these pathogens (id.).

Another protein of PIV, the C protein, is encoded by an overlapping open reading frame (ORF) of the P protein mRNA (Spriggs and Collins, 1986), and the D protein is generated by RNA editing of the P cistron (Galinski et al. Virology 186:543-50 (1992)). The P mRNA also contains an internal ORF which has the potential to encode a cystein-rich domain called V. The V ORF is also found in other paramyxoviruses and typically is accessed by RNA editing, but this is not the case with PIV. Presently, it is not known whether the PIV V ORF is expressed.

The viral genome of PIV also contains extragenic leader and trailer regions, possessing promoters required for viral replication and transcription. Thus, the PIV genetic map is represented as 3' leader-N-P/C/D-M-F-HN-L-trailer. Transcription initiates at the 3' end and proceeds by a sequential stop-start mechanism that is guided by short conserved motifs found at the gene boundaries. The upstream end of each gene contains a gene-start (GS) signal, which directs initiation of its respective mRNA. The downstream terminus of each gene contains a gene-end (GE) motif which directs polyadenylation and termination.

Identification of attenuating mutations in *cp*45 and other PIV3 vaccine candidates is of interest for a variety of reasons (WO 97 20468 A). In particular, it will be useful to understand the genetic basis of attenuation and to characterize the molecular virology and pathogenesis of attenuated HPIV3 strains to provide clinically acceptable vaccines for use against these and other paramyxoviruses, especially HPIV1 and HPIV2 which together account for an additional 7% of pediatric hospital admissions. To achieve these and related goals, a method for producing virus with a wt phenotype from cDNA is needed to determine which mutation(s) in the *cp*45 virus specify the *ts*, *ca* and *a*tt phenotypes and which gene(s) of the BPIV3 specify the attenuation phenotype.

The complete nucleotide sequences of the prototype PIV3 strain, and of the JS wt HPIV3 and *cp*45 strains have been determined (Stokes et al., supra., (1992); Stokes et al., Virus Res. 30: 43-52 (1993)). From these studies, the *cp*45 strain was shown to possess at least seventeen nucleotide substitutions compared to the parental JS wt HPIV3 strain, eight of which are correlated with changes to viral proteins. However, it has not been previously shown which of these identified mutations specify desired, e.g., *ts*, *ca,* and *att*, phenotypes. Recently, growth of *cp*45 at nonpermissive temperatures was reported to be complemented by coexpression of a cDNA clone of the L gene of the 47885 wt PIV3 strain (Ray et al., J. Virol. 70:580-584 (1996)), suggesting that the L gene may contain one or more mutations which contribute to the *ts* phenotype of *cp*45. However, the results of this study are complicated by the fact that the 47885 strain is not isogenic with the JS parent of *cp*45 (for example, the two viruses are 4% different at the nucleotide level, and the L proteins differ at 41 amino acid positions (Stokes et al., supra, (1992); published erratum appears in Virus Res. 27:96 (1993); Virus Res. 25:91-103. Also, this method of complementation does not provide a clear measurement of the relative contribution of the L gene mutation(*s*) to the overall *ts* phenotype of *cp*45.

Rescue and analysis of attenuating mutations in PIV3 and other RNA viruses require effective methods to manipulate cDNAs for the particular viruses of interest. Despite previous advancements identifying cDNAs for PIV, manipulation of the genomic RNA of this and other negative-sense RNA viruses has proven difficult. One major obstacle in this regard is that the naked genomic RNA of these viruses is noninfectious.

Successful methods for direct genetic manipulation of non-segmented negative strand RNA viruses have only recently begun to be developed (for reviews, see Conzelmann, J. Gen. Virol. 77:381-89 (1996); Palese et al., Proc. Natl. Acad. Sci. U.S.A. 93:11354-58, (1996)) (see also EP 0 702 085 A and EP 0 440 219 A). Functional nucleocapsids have been successfully generated from the intracellular coexpression of separately transfected plasmids bearing the T7 RNA polymerase promoter and encoding either genomic or antigenomic RNA and the N, P, and L proteins. The intracellular cDNA expression is driven by T7 RNA polymerase which is produced by co-infecting with a vaccinia recombinant virus. This approach was first used to determine the transcription and replication requirements of cDNA-encoded minireplicons. Some success has been achieved in the application of these general methods to rescue infectious rabies virus, vesicular stomatitis virus (VSV), measles virus, and Sendai virus from cDNA-encoded antigenomic RNA in the presence of the nucleocapsid N, phosphoprotein P, and large polymerase subunit L (Garcin et al., EMBO J. 14:6087-6094 (1995); Lawson et al., Proc. Natl. Acad. Sci. U.S.A. 92:4477-81 (1995); Radecke et al., EMBO J. 14:5773-5784 (1995); Schnell et al., EMBO J. 13:4195-203 (1994); Whelan et al., Proc. Natl. Acad. Sci. U.S.A. 92:8388-92 (1995)). Respiratory synctial virus (RSV) has also been recovered from cDNA encoded antigenome but this required the transfection of an additional plasmid encoding the M2 ORF 1 transcription elongation factor (Collins et al., 1995).

Rescue of infectious PIV virus and other Mononegavirales members is complicated by virtue of their non-segmented negative-strand RNA genome. The genomic ribonucleoprotein complexes (RNPs) of segmented genome viruses, such as influenza, are generally small in size and loosely structured, and can be assembled in vitro from RNA and required viral proteins. However, PIV and other Mononegavirales members feature much larger and more tightly structured RNPs, which tend to be refractory to functional association in vitro. Furthermore, the coding potential of HPIV3 P mRNA is complicated by cotranscriptional "RNA editing" (Galinski et al., Virology 186: 543-50 (1992)). The resultant shifts in reading frame can access internal ORFs which are expressed as chimeras fused to the N-terminal part of P. In addition, HPIV3 appears to differ from most other paramyxoviruses which express a chimeric V protein, as noted above. The corresponding set of proteins from HPIV3 editing has not yet been identified, and the internal V ORF of HPIV3 is separated from the editing site by numerous translational stop codons (Galinski et al. (1992, supra). Yet another complicating factor is that editing by BPIV3 and HPIV3 produces a novel chimeric protein D, in which the upstream half of P is fused to a domain encoded by a second internal ORF (Pelet et al., EMBO J. 10: 443-448 (1991); Galinski et al., supra, (1992)). The D protein does not have a counterpart in other paramyxoviruses. In view of the foregoing, an urgent need exists in the art for tools and methods to engineer safe and effective vaccines to alleviate the serious health problems attributable to PIV, particularly illnesses among infants and children attributable to HPIV3. Quite surprisingly, the present invention satisfies these and other related needs.

### Summary Of The Invention

The present invention provides novel tools and methods for introducing defined, predetermined structural and phenotypic changes into infectious PIV. According to the invention, an isolated polynucleotide molecule according to claim 1 is provided which comprises an operably linked transcriptional promoter, a polynucleotide sequence encoding a PIV genome or antigenome, and a transcriptional terminator.

The PIV genome or antigenome is a recombinantly modified version of the human PIV3 sequence. In one embodiment, the polynucleotide sequence encodes a chimeric genome or antigenome comprising a human PIV3 sequence recombinantly joined with a bovine PIV sequence, such as a gene or gene segment from bovine PIV (BPIV). In additional examples, the polynucleotide of the invention encodes a chimera of sequences from human PIV3 and at least one other PIV of human or bovine origin.

In other embodiments, the invention provides an isolated infectious PIV particle comprising a recombinant PIV (rPIV) genome or antigenome, a N protein, a P protein and a L protein. The isolated infectious PIV particle can be a viral or subviral particle. As used herein, subviral particle refers to any infectious PIV particle which lacks a structural element, eg., a gene segment, gene, protein, or protein functional domain, which is present in a complete virus (eg., an assembled virion including a complete genome or antigenome, nucleocapsid and envelope). Thus, one example of a subviral particle of the invention is an infectious nucleocapsid containing a genome or antigenome, and the products of N, P, and L genes. Other subviral particles are produced by partial or complete deletions or substitutions of non-essential genes and/or their products (eg., F, HN, M, or C), among other non-essential structural elements.

The-isolated infectious PIV particle comprises chimeric sequences from two or more different PIV genomes incorporates polynucleotide sequences from HPIV3 and HPIV1, from HPIV3 and HPIV2 sequences, or is comprised of HPIV3 and BPIV sequences.

In related aspects of the invention, isolated, infectious PIV particles are provided which incorporate nucleotide sequences from HPIV3 joined to at least one sequence from a heterologous PIV, such as HPIV1, HPIV2 or BPIV. For example, entire genes of HPIV3 may be replaced by counterpart genes from other forms of PIV, such as the HN and/or F glycoprotein genes of PIV1 or PIV2. Genes or gene segments from one PIV can be added (i.e., without substitution) within a heterologous PIV background to create novel immunogenic properties within the resultant clone.

Other modifications can be produced by introducing into the HPIV3 genome or antigenome a nucleotide insertion, rearrangement, deletion or substitution selected to encode a desired phenotypic alteration, such as one that results in attenuation, temperature-sensitivity, cold-adaptation, small plaque size, host range restriction, improved growth in vitro. or a change in an immunogenic epitope of PIV. In one aspect of the invention, mutations occurring in biologically derived, attenuated PIV are identified and introduced individually or in combination into a full-length PIV clone. Typically these mutations are single amino acid changes displayed by biologically derived mutant viruses over a wild-type PIV, for example changes exhibited by PIV mutants having *ts*, *ca* or att phenotypes. These changes from biologically derived mutant PIV are incorporated into a recombinant PIV clone to specify desired characteristics in the resultant virus. Exemplary mutations include amino acid changes which specify an attenuated phenotype in the HPIV3 strain JS *cp*45. Among these exemplary mutations are mutations occurring within the PIV polymerase gene L specifying *ts*, *ca* or att phenotypes, for example amino acid substitutions occurring at Tyr₉₄₂, Leu₉₉₂, and/or Thr₁₅₅₈ of the JS wild type PIV strain. In more detailed aspects, attenuated PIV recombinants are described wherein Tyr₉₄₂ is replaced by His, Leu₉₉₂ is replaced by Phe, and/or Thr₁₅₅₈ is replaced by Ile.

Also provided within the invention are recombinant PIV having multiple, phenotype-specifying mutations introduced in selected combinations into the genome or antigenome of an infectious clone to yield desired characteristics including attenuation, temperature sensitivity, cold-adaptation, small plaque size, host range restriction, etc. For example, PIV clones are provided which incorporate at least two separate mutations adopted from a biologically derived PIV mutant, e.g., two *ts* mutations from HPIV3 JS *cp*45. Multiply attenuated viruses are thus obtained by selecting mutations from a "menu" of identified lesions and introducing these mutations in various combinations to calibrate a vaccine virus to selected levels of attenuation, immunogenicity and stability.

In additional embodiments, the invention provides for supplementation of one or more mutations adapted from biologically derived PIV, e.g., *ts*, *ca* or att mutations, with additional types of mutations involving the same or different genes. Target genes for mutation in this context include the nucleocapsid protein N, phosphoprotein P, large polymerase subunit L, matrix protein M, hemagglutinin-neuraminidase protein HN, fusion protein F and the C, D and V ORF products. In preferred aspects, attenuating mutations adopted from biologically derived PIV are incorporated within a chimeric PIV recombinant, e.g., a PIV recombinant having nucleotide sequences from both HPIV3 and HPIV1, or from both HPIV and BPIV viruses.

In other embodiments, the invention provides methods for producing an infectious PIV particle, e.g, a viral or subviral particle, from one or more isolated polynucleotide molecules encoding a PIV genome or antigenome (see also copending United States provisional patent application No. 60/047575, filed May 23 1997.

To produce an infectious PIV particle according to these methods, an expression vector comprising an isolated polynucleotide molecule encoding a PIV genome or antigenome is coexpressed in a cell or cell-free system with an expression vector comprising one or more isolated polynucleotide molecules encoding N, P, and L proteins of a PIV, whereby an infectious PIV particle is produced.

The PIV genome or antigenome and the N, P, and L proteins may be coexpressed by a single expression vector, or by separate expression vectors. In alternate embodiments, the N, P, and L proteins are each encoded on separate expression vectors.

Within the aforementioned methods, the polynucleotide molecule encoding the PIV genome or antigenome corresponds to a genomic or antigenomic sequence of human. PIV3. Alternatively, the PIV encoding polynucleotide may be a chimera of a human PIV3genomic or antigenomic sequence and the non-human PIV genomic or antigenomic sequence, BPIV. In additional methods for producing infectious PIV, the polynucleotide encoding the PIV genome or antigenome is a chimera of two or more human PIV genomes, i.e. a polynucleotide containing sequences from HPIV3 joined to sequences from one or more related forms of human PIV, such as human PIV1 or human PIV2. Individual genes of human PIV3 may be substituted by counterpart genes from heterologous PIV, for example the HN and F glycoprotein genes of PIV1 or PIV2, to yield a modified genome or antigenome encoding a chimeric PIV.

In yet additional methods for producing infectious PIV, the PIV genome or antigenome is modified to yield a chimera of a human PIV genomic or antigenomic sequence and at least one non-human PIV sequence, for example a polynucleotide containing sequences from both human and bovine PIV.

In other methods for producing infectious PIV, the PIV genome or antigenome is modified by a nucleotide insertion, rearrangement, deletion or substitution selected to encode a desirable phenotypic alteration, such as one that results in attenuation, temperature-sensitivity, cold-adaptation, small plaque size, host range restriction, or a change in an immunogenic epitope of PIV. Alternatively, the polynucleotide molecule encoding the PIV genome or antigenome can be modified to encode non-PIV molecules, e.g., a cytokine, a T-helper epitope, a restriction site marker, or a protein of a different microbial pathogen (e.g., virus, bacterium or fungus) capable of eliciting a protective immune response in the intended host. In one embodiment, the PIV genome or antigenome is modified to encode protein from a human RSV or from measles virus.

In other embodiments of the invention a cell or cell-free expression system (e.g., a cell-free lysate) is provided which incorporates an expression vector comprising an isolated polynucleotide molecule encoding a PIV genome or antigenome, and an expression vector comprising one or more isolated polynucleotide molecules encoding N, P, and L proteins of a PIV. Upon expression, the genome or antigenome and N, P, and L proteins combine to produce an infectious PIV particle, such as a viral or subviral particle. The isolated polynucleotide molecules encoding the PIV genome or antigenome and the one or more isolated polynucleotide molecules encoding N, P, and L proteins of PIV can be expressed by a single vector, or the genome and one or more of the N, P, and L proteins can be incorporated into two or more separate vectors.

### Brief Description of the Drawing

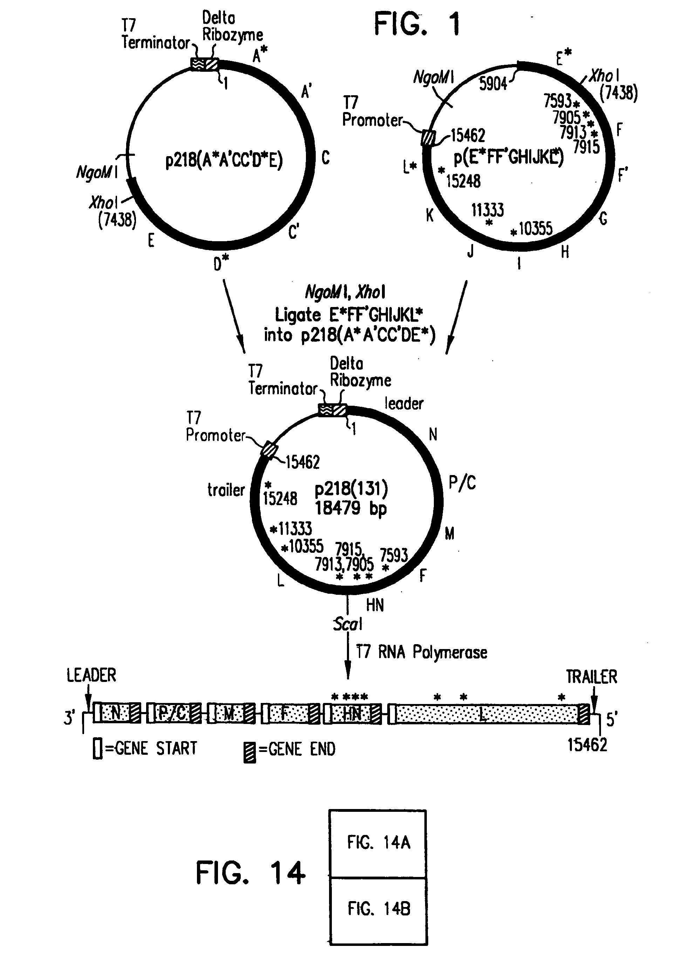

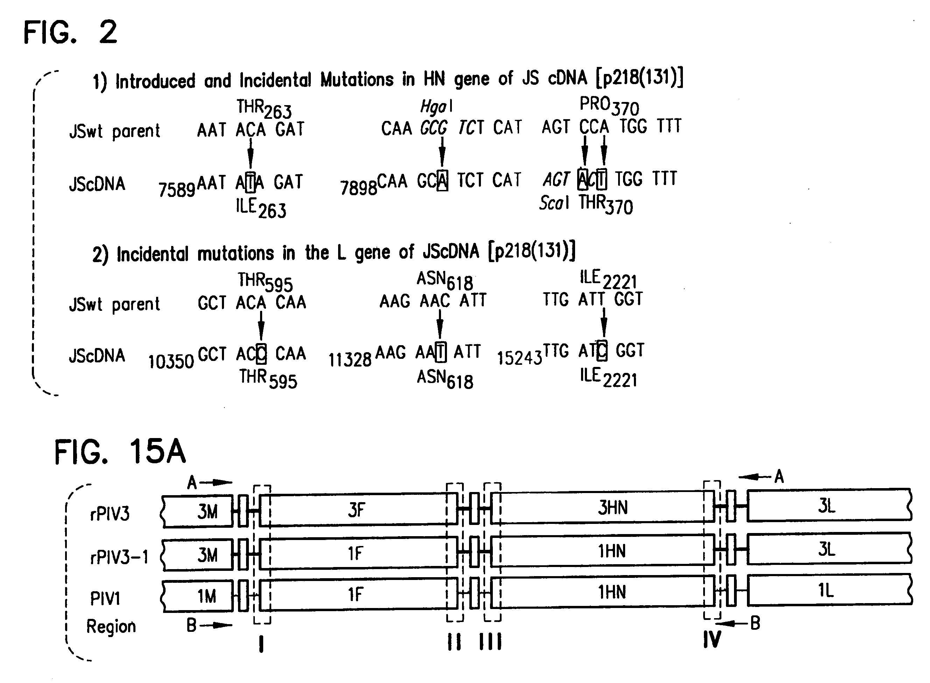

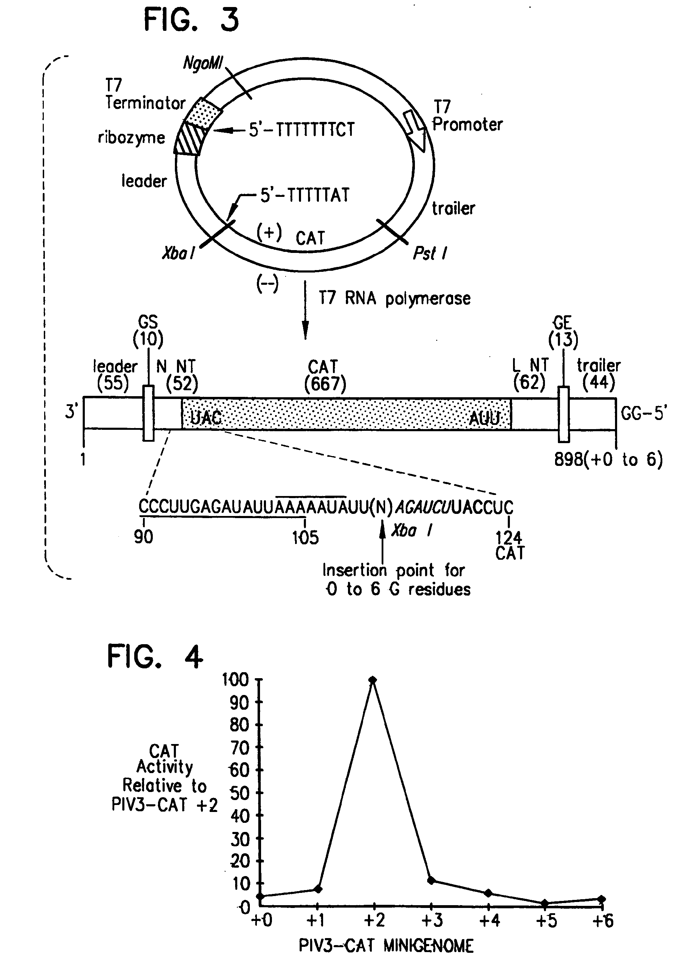

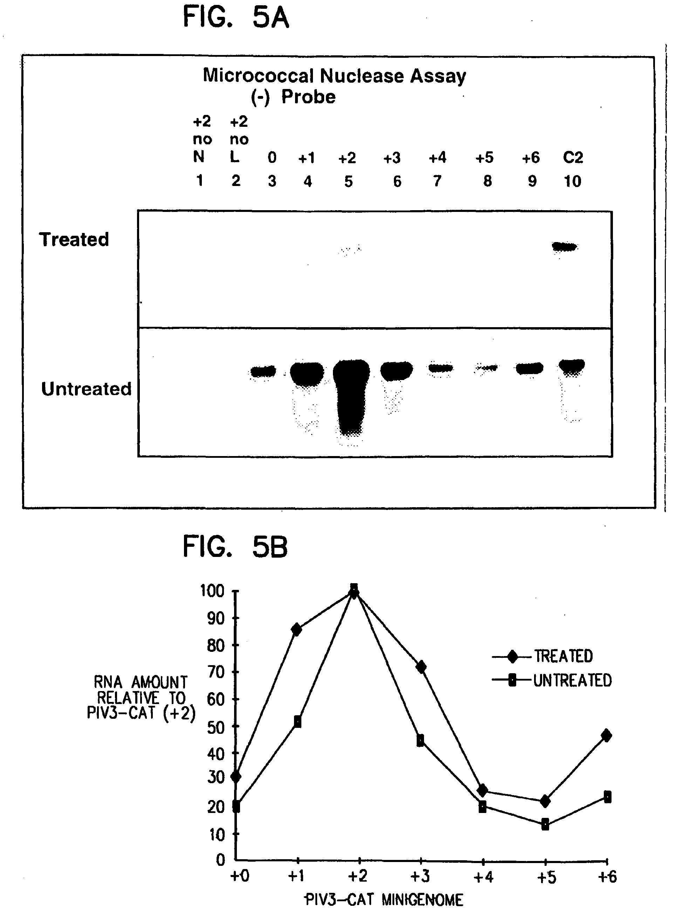

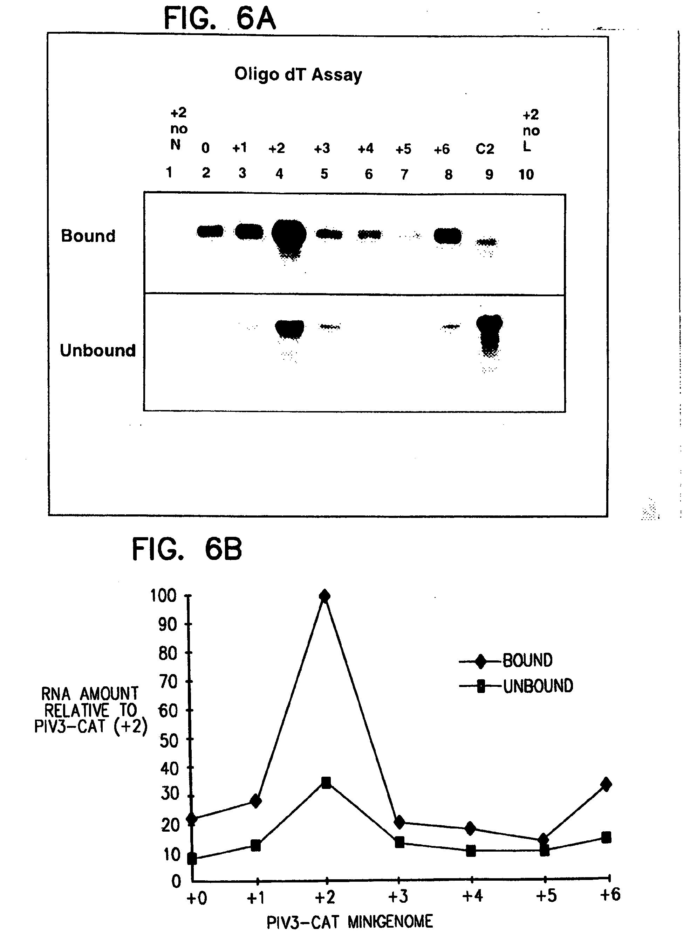

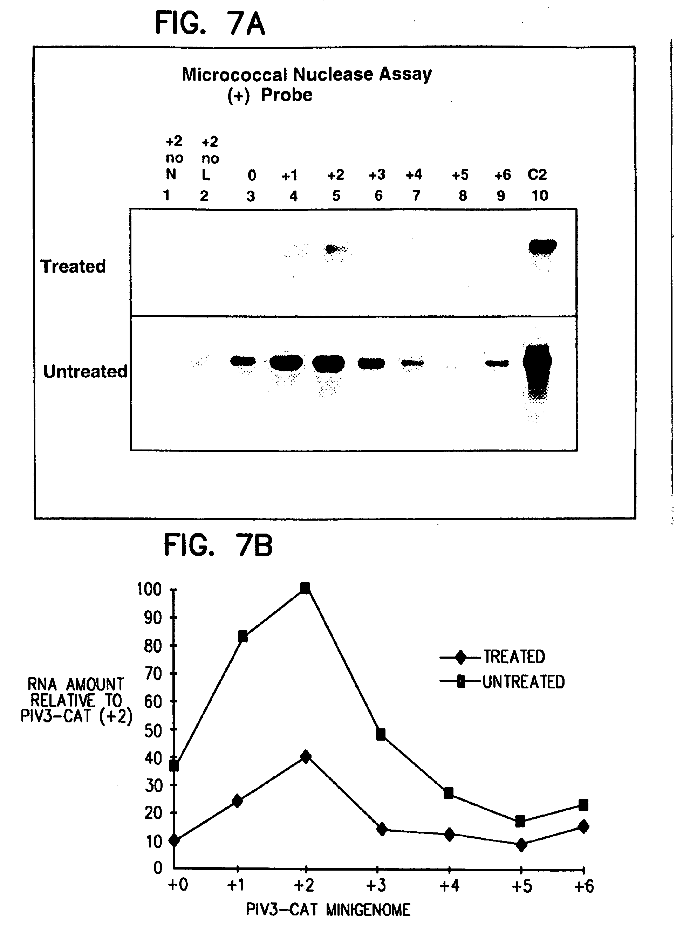

Figure 1 illustrates construction of the plasmid p218(131), encoding a complete copy of HPIV3 genomic RNA. The diagram of p218(A*A'CC'D*E) (top left) illustrates the location of the T7 terminator and delta ribozyme adjacent to nucleotide 1 of the HPIV3 genome. The top right diagram of p(E*FF'GHIJKL*) illustrates the locations of the 7 sequencing markers (asterisk), see Fig. 2 below, and the T7 promoter adjacent to nucleotide 15462 of HPIV3. The 15 overlapping clones used to construct the full length genomic cDNA are indicated by letters on the outside of the two plasmids. The unique restriction sites *NgoM*I and XhoI were used to clone p218(131) which encodes the complete negative-sense genomic RNA of HPIV3 illustrated at the bottom. The location of each sequencing marker in p218(131) differentiating recombinant JS from JS wt is indicated by an asterisk.
Reference Figure 2 depicts sequence markers in cDNA encoded genomic RNA of the HN (1) and L(2) genes of HPIV3. Sequences are negative sense, mutations are boxed.
   1) A non-coding nt substitution at 7903 removes a *Hga* I site from JS wt. Nucleotide substitutions in JS cDNA at 7913 and 7915 create a *Sca* I site and change amino acid 370 from proline to threonine, ablating the antibody epitope recognized by mAb 170/7 and 423/6. Also, the HN gene was found to contain an additional point mutation at nt 7593 which had not been recognized previously. This results in a threonine to isoleucine change at amino acid position 263 in the HN protein.
   2) The three incidental non-coding mutations in the L gene of JS cDNA that occurred during plasmid construction or assembly are boxed.
Reference Figure 3 includes a diagram (not to scale) of the PIV3-CAT cDNA (top, drawn as a circular plasmid) and the encoded minigenome RNA (bottom, drawn as a single strand of negative-sense RNA, 3' to 5').
   Plasmid: The T7 promoter is shown as a black arrow pointing in the direction of transcription. Positions are shown for the delta ribozyme, the T7 transcriptional terminator, the inserted vaccinia virus transcriptional terminators (T₇CT and T₅AT), restriction enzyme sites, and the coding sequence for the various domains of the encoded minigenome.
   Minigenome RNA: The 5' (right hand) end of the minigenome is defined by the promoter for T7 RNA polymerase, which contributes an extension of two nonviral G residues. These are not included in length calculations. The 3' end is defined by the ribozyme and is free of any heterologous nucleotides. PIV3-specific regions are shown as open boxes. The CAT ORF is shaded and the negative-sense complements of the translational initiation (UAC) and termination (AUU) codons are shown. Nucleotide positions are indicated according to the shortest, 898-nucleotide minigenome. Nucleotide lengths of specific regions are given in parentheses. The sequence of positions 90 to 124 (3' to 5', negative-sense) is shown at the bottom; PIV3-specific sequence is underlined, the complement of a vaccinia virus transcriptional terminator is overlined, the complement of the *Xba*I site is italicized, and the complement of the translational start codon for the CAT ORF is in bold. The site where 0 to 6 G residues were inserted, resulting in PIV3-CAT 0 to +6, is indicated. Abbreviations: GS, transcriptional gene-start motif; GE, transcriptional gene-end termination/polyadenylation motif; NT, nontranslated gene sequence.
Reference Figure 4 depicts expression of CAT enzyme by minigenomes PIV3-CAT 0 to +6. Cells were transfected with plasmids encoding the indicated PIV3-CAT minigenome and the N, P and L proteins, and were infected simultaneously with vaccinia virus recombinant vTF7-3 expressing T7 RNA polymerase. Lysates were prepared 48 h post-infection, and samples representing 3000 cells were analyzed for CAT activity. Activities are expressed relative to the +2 minigenome, which is a multiple of six, as 100. Samples in which the L plasmid was omitted did not have CAT activity detectable under these conditions.
Reference Figures 5A and 5B depict synthesis of mini-antigenomic RNA by PIV3-CAT minigenomes 0 to +6. Cells were transfected and infected as described for Fig. 4, except for controls in which N plasmid (lane 1) or L plasmid (lane 2) was omitted. The PIV3-CAT minigenome plasmid which was transfected in each case is indicated. Cell lysates were prepared at 48 h post-transfection: one aliquot was treated with micrococcal nuclease (treated) and the second was mock-treated (untreated), followed by processing for RNA purification. The RNAs were analyzed by Northern blot hybridization with a negative-sense CAT riboprobe. As a marker and control for nuclease treatment, the RSV-CAT C2 minigenome (Grosfeld et al., J. Virol. 69:5677-5686 (1995)) was complemented by the RSV N, P and L proteins and was processed in parallel (Lane 10). Autoradiograms of the hybridized blots are shown in Fig. 5A. Fig. 5B shows phosphorimager analysis in which the amount of hybridization relative to the +2 minigenome was calculated separately for the treated and untreated samples.
Reference Figures 6A and 6B depict synthesis of polyadenylated mRNA by PIV3-CAT minigenomes 0 to +6. Using aliquots of cells from the same experiment as shown in Figs. 5A and 5B, total intracellular RNA was harvested at 48 h post-transfection and fractionated by oligo(dT) chromatography into bound and unbound fractions, which were analyzed by Northern blot hybridization with a negative-sense CAT riboprobe. Fig. 6A shows autoradiograms of the hybridized blots. Fig. 6B shows the phosphorimager analysis in which the amount of hybridization was normalized relative to the bound RNA fraction of the +2 minigenome, this was calculated separately for the bound and unbound fractions.
Reference Figures 7A and 7B depict accumulation of intracellular minigenome in response to plasmid encoding PIV3-CAT minigenomes 0 to +6. This is a continuation of the experiment shown in Figs. 5A and 5B. Thus, cells which had been transfected with plasmids encoding a minigenome and the N, P and L proteins and infected with vTF7-3 were harvested 48 h post-transfection and cell lysates were prepared. One aliquot of lysate was treated with micrococcal nuclease (treated) and the other was mock-treated (untreated), followed by processing for RNA purification. The RNAs were analyzed by Northern blot hybridization with a positive-sense CAT riboprobe, whereas in Fig. 5A the riboprobe was negative-sense. Fig. 7A shows autoradiograms of the hybridized blots. Fig. 7B shows phosphorimager analysis in which the amount of hybridization relative to the +2 minigenome was calculated separately for the treated and untreated samples.

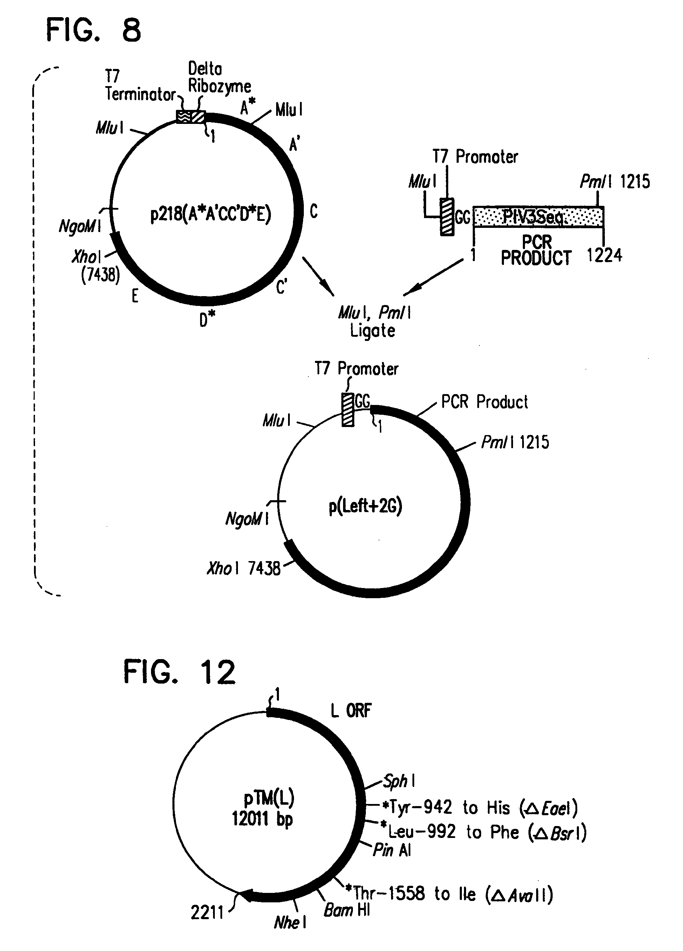

Figures 8-10 illustrate construction of p3/7(131)2G. p3/7(131)2G encodes a complete positive-sense HPIV3 antigenome and contains two G residues between the T7 promoter and nucleotide 1 of the antigenome. The construction involved modification separately of the left and right halves of cDNA 218(131) encoding the negative-sense genome. Using the same strategy, a second cDNA, p3/7(131) was constructed which is identical to p3/7(131)2G except that the two G residues were omitted.
Figure 8 depicts construction of p(Left+2G), performed by replacing the ribozyme and T7 transcription terminator of p218(A*A'CC'D*E) with a T7 promoter including two G residues. p218 (A*A'CC'D*E) was used as the template in a PCR that amplified the left-hand 1224 nucleotides of the HPIV3 genome (black rectangle labeled "PIV3seq.") using a left hand PCR primer that introduced the T7 promoter. This PCR fragment was cloned into the MluI-PmlI window of p218(A*A'CC'D*E), resulting in p(Left+2G).

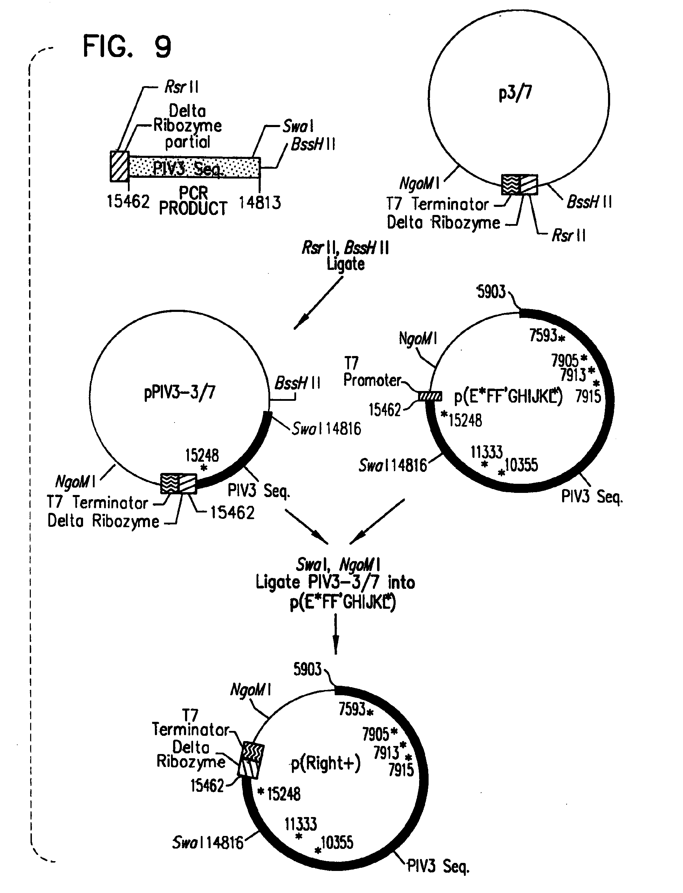

Figure 9 depicts construction of p(Right+), performed by replacing the T7 promoter of p(E*FF'GHIHKL*) with a ribozyme and T7 terminator placed adjacent to PIV3 nucleotide 15642 in the positive-sense (antigenome) strand. p(E*FF'GHIHKL*) was used as a template in a PCR that amplified the right-hand 649 nucleotides of the HPIV3 genome (black rectangle labeled "PIV3 seq.") using a mutagenic oligonucleotide that added part of the delta ribozyme (including a naturally-occurring RsrII site). This PCR product was cloned into the RsrII-BssHII window of p3/7 to yield pPIV3-3/7, thus reconstructing a complete ribozyme flanked by the T7 terminator. The SwaI-NgoMI fragment of pPIV3-3/7 was cloned into the SwaI-NgoMI windows of p(E*FF'GHIHKL*), resulting in p(Right+). The locations of the seven sequence markers are indicated with asterisks.

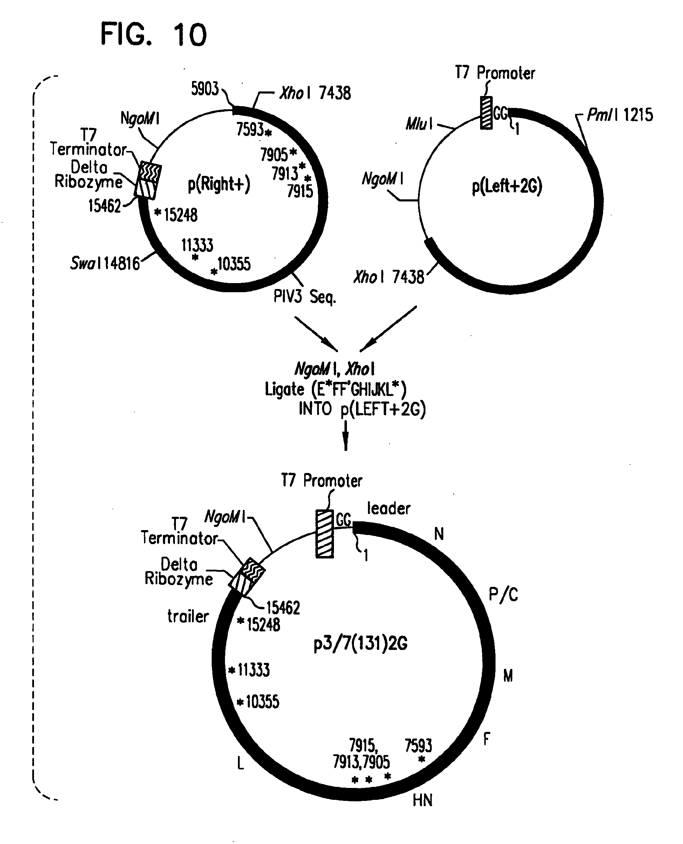

Figure 10 depicts construction of p3/7(131)2G. The NgoMI-XhoI fragment of p(Right+) was cloned into the NgoMI-XhoI window of p(Left+2G), resulting in p3/7(131)2G. The positions of HPIV3 genes are indicated (not to scale). The locations of sequence markers are indicated with asterisks.

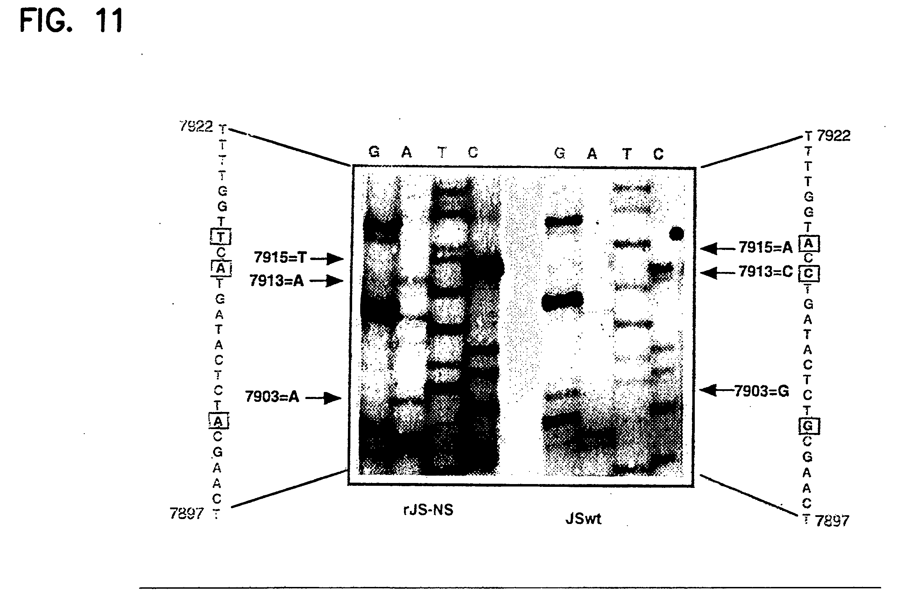

Figure 11 shows sequence confirmation of a negative sense recombinant PIV3. A 1379 bp fragment (nucleotides 7334-8713) spanning the mutations at 7903, 7913, and 7915 was generated by RT-PCR of RNA from infected cells and then analyzed by cycle-sequencing. The mutations differentiating recombinant PIV from JS wt are indicated by arrows. The complete sequence from nt 7897 to 7922 is shown in the margin next to each gel, with the three nucleotide differences indicated in bold.
Figure 12 provides a map of plasmid pTM(L)942/992/1558, which contains the PIV3 L cDNA with amino acid substitutions at positions 942, 992, and 1558 in the L protein sequence. The relative position of each of coding change is indicated, together with the aa difference and the naturally-occurring restriction site which was deliberately ablated as a marker. Restriction sites used for cloning (*Sph*I, *Pin*AI, *BamH*I and *Nhe*I) are indicated. The arrow shows the direction of the L protein coding sequence and is numbered according to amino acid position.

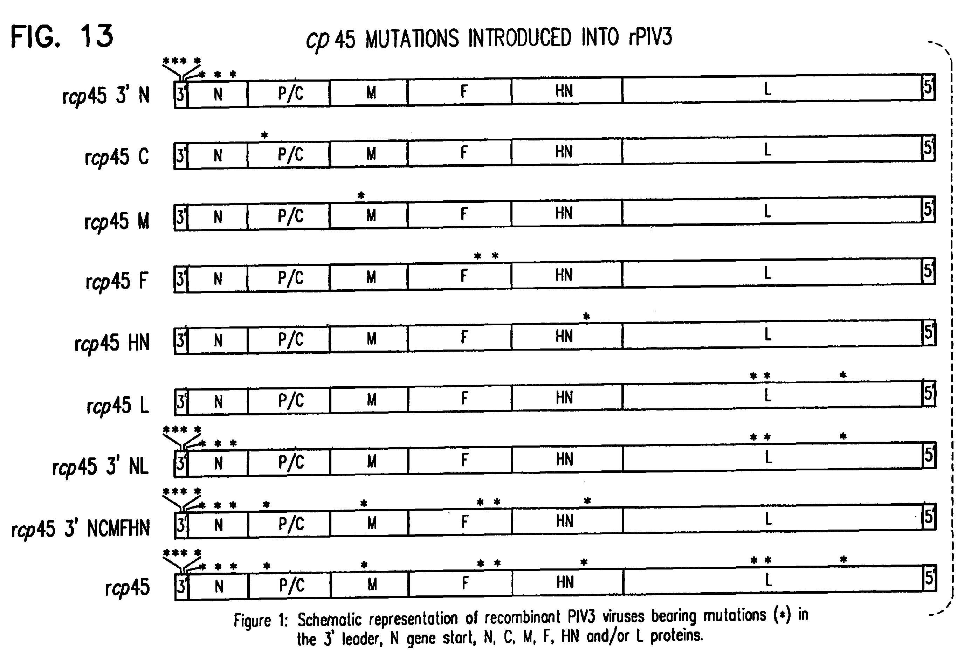

Figure 13 is a schematic representation of recombinant PIV3 viruses bearing representative mutations within the invention.

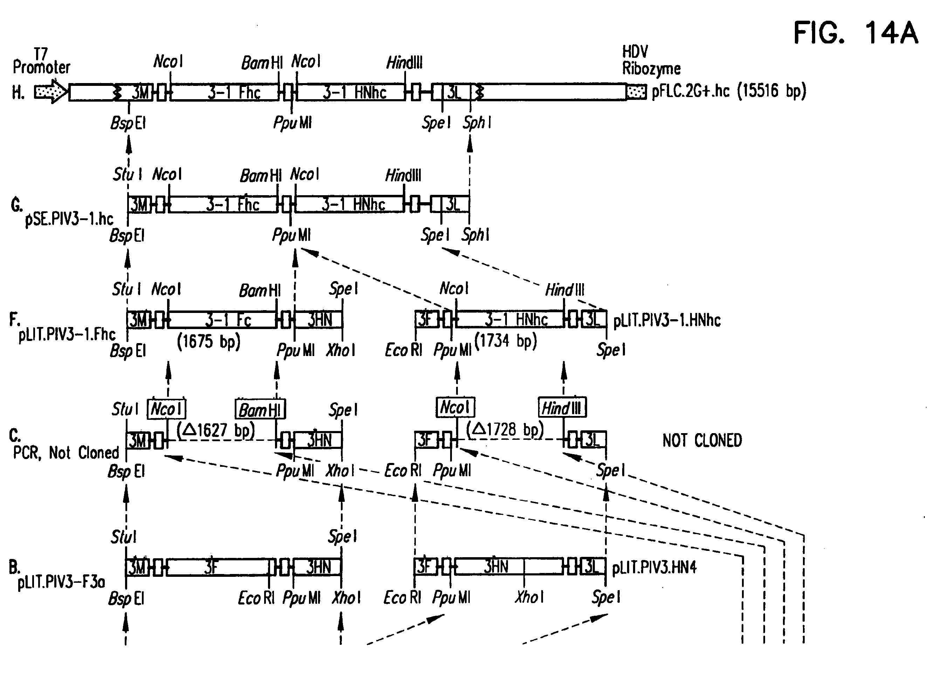

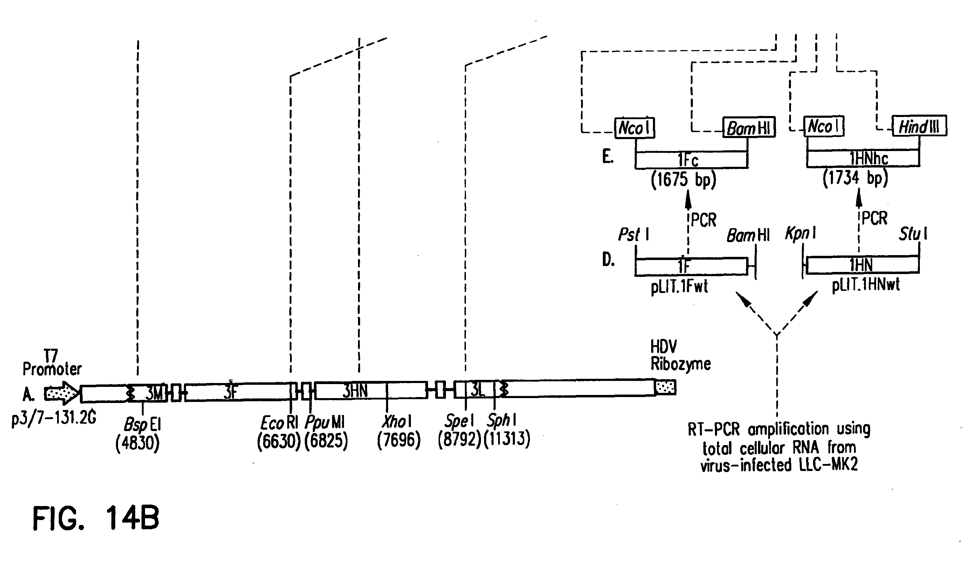

Figures 14A and 14B illustrate construction of cDNA encoding the chimeric PIV3-PIV1 antigenome in which the PIV3 HN and F ORFs were replaced by those of PIV1. First (starting from the bottom left) the PIV3 F and HN genes were subcloned (as *BspE*-I I-*Xho* I and *Eco*R I-*Spe* I fragments) from the full-length PIV3 cDNA clone p3/7 (131) 2G (panel A) to generate pLit.PIV3.F3a and pLit.PIV3.HN4 (panel B), respectively. PCR mutagenesis (Panel C) was performed to delete the complete coding regions of the PIV3 F and HN genes and to introduce new restriction sites (boxed). PIV1 F and HN cDNAs were prepared from infected-cell RNA by RT-PCR and cloned into pLITMUS28 (Panel D). These clones, pLit.1Fwt and pLit.1HNwt, were used as templates for modification of the PIV1 F and HN genes at their start and stop codons to introduce new restriction sites compatible with those introduced in the PIV3 deletions (Panel E) Chimeric F and HN genes were constructed by importing the PIV1 coding regions into the PIV3 deletions to generate pLit.PIV3-1.Fhc and pLit.PIV3-1.HNhc (Panel F) . The chimeric F and HN were assembled together to generate pSE.PIV3-1.hc (Panel G). The F and HN chimeric segment was introduced into full-length PT-V3 clone p3/7(131)2G by replacing its *BspE* I*-Sph* I fragment to generate pFLC.2G+.hc (Panel H). The small boxes between genes represent the gene end, intergenic, and gene start regions, and the lines represent the non-coding regions. Shaded portions are from PIVl, open boxes are from PIV3. The black arrows depict the T7 promoter, while black boxes depict the hepatitis delta virus (HDV) ribozyme.
Figure 15A is a diagram of the chimeric HN and F genes of the chimeric rPIV3-1 (middle) in parallel with that of rPIV3/JS (top; alternatively referred to herein as rJS and JS wt rPIV3) and PIV1/Wash64 (bottom). The four junction regions containing the sequence transitions from PIV3 to PIV1 are boxed and numbered I to IV. Each small box between genes represents the gene end, intergenic, and gene start region, and the lines represent the non-coding regions. RT-PCR primers, specific to PIV3 M and L genes (primer pair A at top) or specific to PIV1 M and HN genes (primer pair B at bottom) used in Figures 14A and 14B, are depicted as arrows.

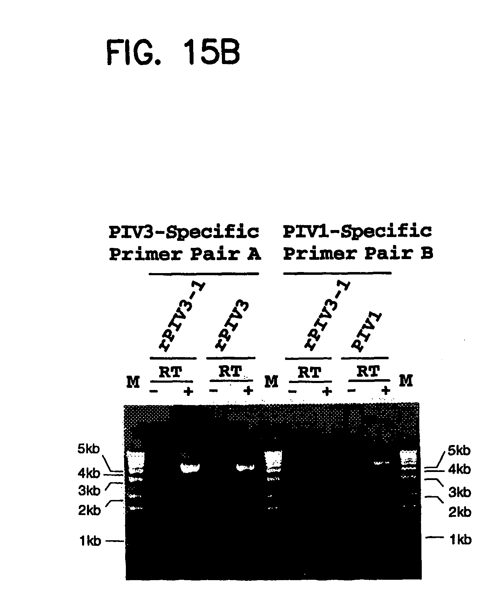

Figure 15B shows an evaluation of RT-PCR products generated using virion RNA (vRNA) as template and the PIV3- or PIV1-specific primer pairs described in Figures 14A and 14B. The reverse transcription step was omitted in a duplicate of each template, as indicated, to confirm that the PCR product was derived from RNA. For rPI1V3-1, the PIV3-specific primer pair A gave rise to the expected 4.6 kb product, whereas primer pair 3, specific to PIV-1 sequences not present in rPIV3-1, did not yield product. Positive controls using rPIV3/JS (labeled as rPIV3) or PIV1/Wash64 (labeled as PIV1) vRNA are shown in parallel.

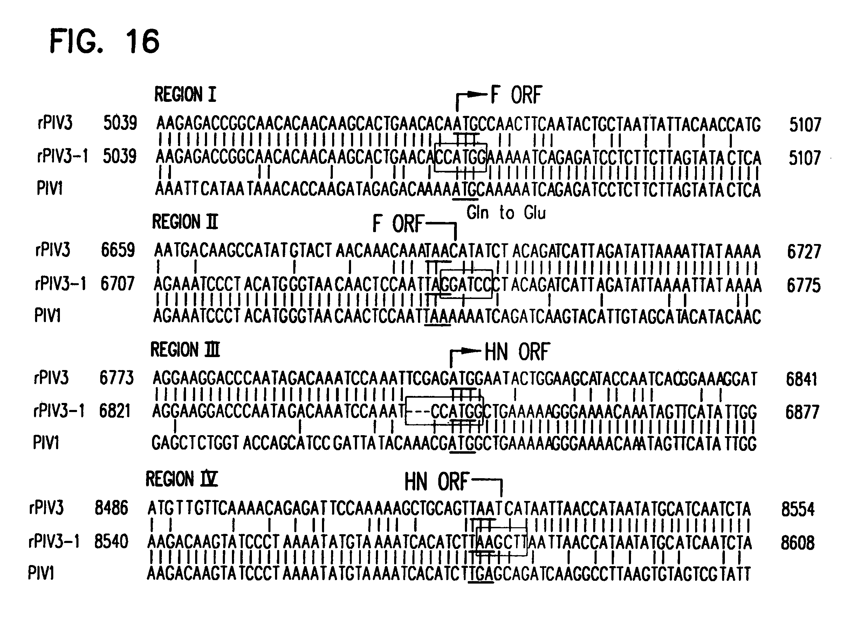

Figure 16 represents sequences of PIV3-PIV1 junctions in the RT-PCR products of rPIV3-1 shown in Figures 14A and 14B were determined. The sequence for each of the four junction regions (Regions I-IV) is presented and aligned with the corresponding regions of rPIV3/JS (top line) and PIV1/Wash64 (bottom line), which were sequenced in parallel from RT-PCR products. Vertical bars indicate sequence identity, and the boxed regions indicate introduced mutations and restriction sites. The Gln to Glu codon change in the chimeric F gene is indicated by shaded box. Start and stop codons are underlined.

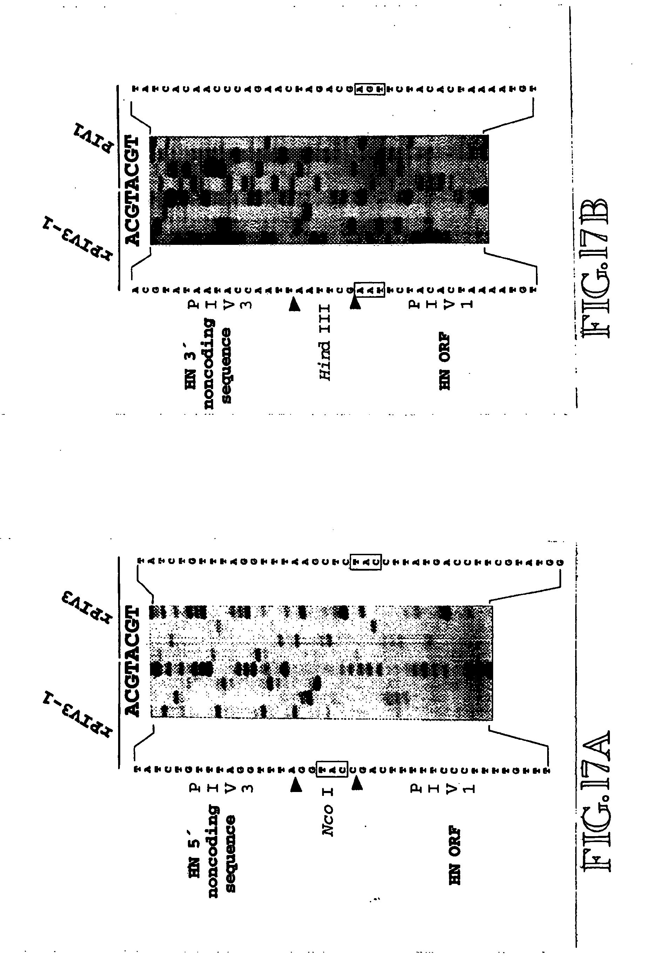

Figs. 17A-17B show sequencing gels for region III (left) and IV (right) of RT-FCR products of rPIV3-1 compared with rPIV3 (left) or PIV1 (right). Start and stop codons are marked by a box, restriction sites are marked by arrows.

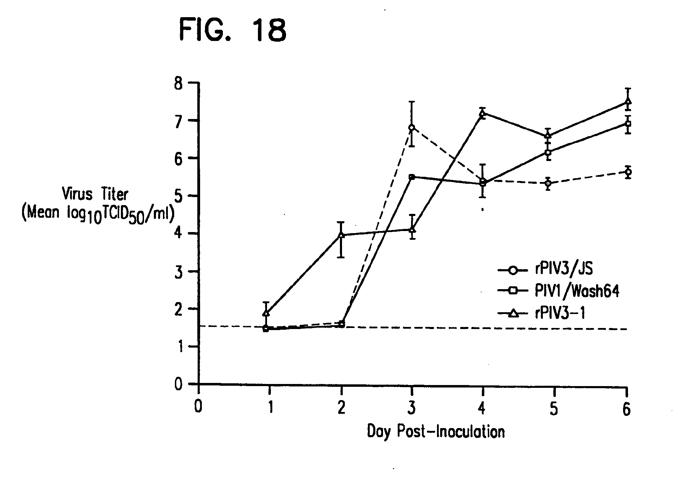

Figure 18 depicts multicycle growth of parental and chimeric PIVs in tissue culture. LLC-MK2 cell monolayers were inoculated with virus at an MOI of 0.01, and virus-infected cells were incubated at 32°C in the presence of trypsin. Tissue culture supernatants were harvested at 24 hour intervals, frozen, and analyzed in the same.TCID₅₀ assay using hemadsorption to identify virus-infected cultures. Each point represents the mean titer of three separate cultures, with S.E. Indicated. The dotted horizontal line indicates the lower limit of viral detection.

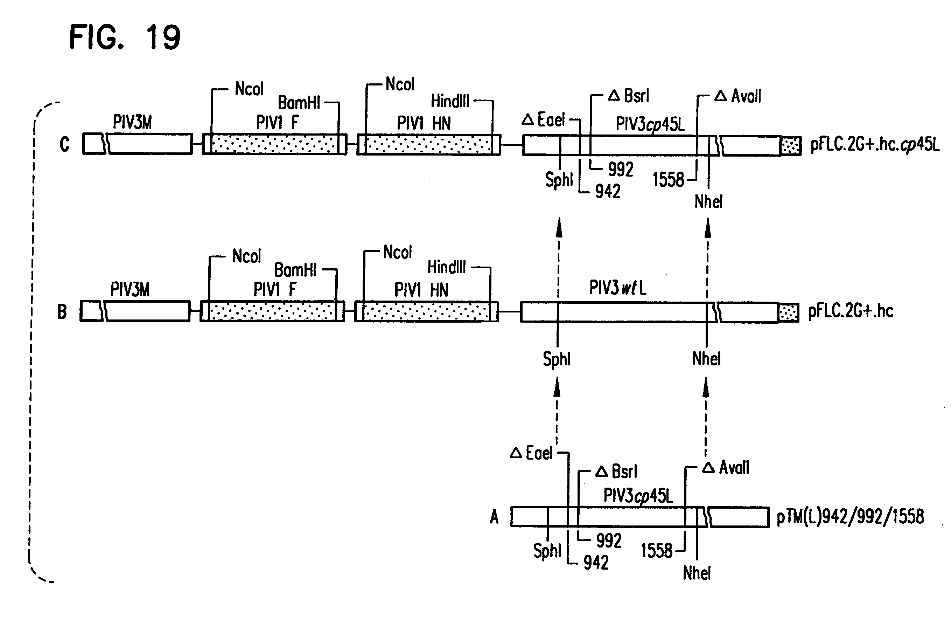

Figure 19 illustrates introduction of the three L gene mutations of *cp*45 into pFLC.2G+.hc, the antigenomic cDNA clone of the chimeric virus rPIV3-1. pTM(L)942/992/1558 (A) is a plasmid clone of the L gene that carries the three mutations found in *cp*45. The mutation at amino acid position 942 in the PIV3 L protein is a tyr (*wt*) to his (*cp*45) substitution and a nearby Eae I site was ablated to mark this site. Similarly, the 992 mutation is a leu to phe change with a Bsr I site ablated and the 1558 mutation is a thr to ile change with an Ava II site ablated. The 2.9 kb SphI-NheI fragment present in pTM(L)942/992/1558 was introduced into pFLC.2G+hc (B), the plasmid carrying the full length cDNA clone of rPIV3-1, to give pFLC+hc.*cp*45L (C). For the constructs in (B) and (C), the black boxes indicate the location of the hepatitis D virus ribozyme and the T7 teminator, the shaded regions are the PIV1 HN and F ORFs, and the open boxes represent sequences derived from PIV3. pFLC.2G+.hc.*cp*45L was used in the transfection to yield the attenuated chimeric recombinant virus designated rPIV3-1. *cp*45L.

### Description of the Specific Embodiments

The present invention relates to compositions and methods for producing and modifying infectious PIV from isolated polynucleotides molecules, preferably cDNA. Infectious PIV particles are produced by a recombinant coexpression system that permits introduction of defined changes into infectious PIV. These modifications are useful in a wide variety of applications, including the development of live attenuated vaccine strains bearing predetermined, defined attenuating mutations.

Infectious PIV of the invention are produced by intracellular or cell-free coexpression of one or more isolated polynucleotide molecules that encode a PIV genome or antigenome RNA, together with one or more polynucleotides encoding viral proteins necessary to generate a transcribing, replicating nucleocapsid. Among the viral proteins useful for coexpression to yield infectious PIV are the major nucleocapsid protein (N) protein, nucleocapsid phosphoprotein (P), large (L) polymerase protein, fusion protein (F), hemagglutinin-neuraminidase glycoprotein (HN), and matrix (M) protein. Also useful in this context are products of the C, D and V ORFs of PIV.

cDNAs encoding a PIV genome or antigenome are constructed for intracellular or in vitro coexpression with the necessary viral proteins to form infectious PIV. By "PIV antigenome" is meant an isolated positive-sense polynucleotide molecule which serves as a template for synthesis of progeny PIV genome. Preferably a cDNA is constructed which is a positive-sense version of the PIV genome corresponding to the replicative intermediate RNA, or antigenome, so as to minimize the possibility of hybridizing with positive-sense transcripts of complementing sequences encoding proteins necessary to generate a transcribing, replicating nucleocapsid.

In some embodiments of the invention the genome or antigenome of a recombinant PIV (rPIV) need only contain those genes or portions thereof necessary to render the viral or subviral particles encoded thereby infectious. Further, the genes or portions thereof may be provided by more than one polynucleotide molecule, i.e., a gene may be provided by complementation or the like from a separate nucleotide molecule. In other embodiments, the PIV genome or antigenome encodes all functions necessary for viral growth, replication, and infection without the participation of a helper virus or viral function provided by a plasmid or helper cell line.

By "recombinant PIV" is meant a PIV or PIV-like viral or subviral particle derived directly or indirectly from a recombinant expression system or propagated from virus or subviral particles produced therefrom. The recombinant expression system will employ a recombinant expression vector which comprises an operably linked transcriptional unit comprising an assembly of at least a genetic element or elements having a regulatory role in PIV gene expression, for example, a promoter, a structural or coding sequence which is transcribed into PIV RNA, and appropriate transcription initiation and termination sequences.

To produce infectious PIV from a cDNA-expressed PIV genome or antigenome, the genome or antigenome is coexpressed with those PIV N, P and L proteins necessary to (i) produce a nucleocapsid capable of RNA replication, and (ii) render progeny nucleocapsids competent for both RNA replication and transcription. Transcription by the genome nucleocapsid provides the other PIV proteins and initiates a productive infection. Alternatively, additional PIV proteins needed for a productive infection can be supplied by coexpression.

Synthesis of PIV antigenome or genome together with the above-mentioned viral proteins can also be achieved in vitro (cell-free), e.g., using a combined transcription-translation reaction, followed by transfection into cells. Alternatively, antigenome or genome RNA can be synthesized in vitro and transfected into cells expressing PIV proteins.

In certain embodiments of the invention, complementing sequences encoding proteins necessary to generate a transcribing, replicating PIV nucleocapsid are provided by one or more helper viruses. Such helper viruses can be wild type or mutant. Preferably, the helper virus can be distinguished phenotypically from the virus encoded by the PIV cDNA. For example, it is desirable to provide monoclonal antibodies which react immunologically with the helper virus but not the virus encoded by the PIV cDNA. Such antibodies can be neutralizing antibodies. In some embodiments, the antibodies can be used in affinity chromatography to separate the helper virus from the recombinant virus. To aid the procurement of such antibodies, mutations can be introduced into the PIV cDNA to provide antigenic diversity from the helper virus, such as in the HN or F glycoprotein genes.

In alternate embodiments of the invention, the N, P, L and other desired PIV proteins are encoded by one or more non-viral expression vectors, which can be the same or separate from that which encodes the genome or antigenome. Additional proteins may be included as desired, each encoded by its own vector or by a vector encoding one or more of the N, P, L and other desired PIV proteins, or the complete genome or antigenome. Expression of the genome or antigenome and proteins from transfected plasmids can be achieved, for example, by each cDNA being under the control of a promoter for T7 RNA polymerase, which in turn is supplied by infection, transfection or transduction with an expression system for the T7 RNA polymerase, e.g., a vaccinia virus MVA strain recombinant which expresses the T7 RNA polymerase (Wyatt et al., Virology, 210: 202-205 (1995). The viral proteins, and/or T7 RNA polymerase, can also be provided by transformed mammalian cells or by transfection of preformed mRNA or protein.

A PIV antigenome may be constructed for use in the present invention by, e.g., assembling cloned cDNA segments, representing in aggregate the complete antigenome, by polymerase chain reaction or the like (PCR; described in, e.g., U.S. Patent Nos. 4,683,195 and 4,683,202, and PCR Protocols: A Guide to Methods and Applications, Innis et al., eds., Academic Press, San Diego (1990), of reverse-transcribed copies of PIV mRNA or genome RNA. For example, a first construct is generated which comprises cDNAs containing the left hand end of the antigenome, spanning from an appropriate promoter (e.g., T7 RNA polymerase promoter) and assembled in an appropriate expression vector, such as a plasmid, cosmid, phage, or DNA virus vector. The vector may be modified by mutagenesis and/or insertion of synthetic polylinker containing unique restriction sites designed to facilitate assembly. For ease of preparation the N, P, L and other desired PIV proteins can be assembled in one or more separate vectors. The right hand end of the antigenome plasmid may contain additional sequences as desired, such as a flanking ribozyme and tandem T7 transcriptional terminators. The ribozyme can be hammerhead type (e.g., Grosfeld et al., (1995), supra), which would yield a 3' end containing a single nonviral nucleotide, or can be any of the other suitable ribozymes such as that of hepatitis delta virus (Perrotta et al., Nature 350:434-436 (1991)), which would yield a 3' end free of non-PIV nucleotides. The left- and righthand ends are then joined via a common restriction site.

A variety of nucleotide insertions, deletions and rearrangements can be made in the PIV genome or antigenome during or after construction of the cDNA. For example, specific desired nucleotide sequences can be synthesized and inserted at appropriate regions in the cDNA using convenient restriction enzyme sites. Alternatively, such techniques as site-specific mutagenesis, alanine scanning, PCR mutagenesis, or other such techniques well known in the art can be used to introduce mutations into the cDNA.

Alternative means to construct cDNA encoding the genome or antigenome include reverse transcription-PCR using improved PCR conditions (e.g., as described in Cheng et al., Proc. Natl. Acad. Sci. USA 91:5695-5699 (1994)), to reduce the number of subunit cDNA components to as few as one or two pieces. In other embodiments different promoters can be used (e.g., T3, SP6) or different ribozymes (e.g., that of hepatitis delta virus. Different DNA vectors (e.g., cosmids) can be used for propagation to better accommodate the larger size genome or antigenome.

Isolated polynucleotides (e.g., cDNA) encoding the genome or antigenome may be inserted into appropriate host cells by transfection, electroporation, mechanical insertion, transduction or the like, into cells which are capable of supporting a productive PIV infection, e.g., HEp-2, FRhL-DBS2, LLC-MK2, MRC-5, and Vero cells. Transfection of isolated polynucleotide sequences may be introduced into cultured cells by, for example, calcium phosphate-mediated transfection (Wigler et al., Cell 14: 725 (1978); Corsaro and Pearson, Somatic Cell Genetics 7: 603 (1981); Graham and Van der Eb, Virology 52: 456 (1973)), electroporation (Neumann et al., EMBOJ. 1: 841-845 (1982)), DEAE-dextran mediated transfection (Ausubel et al., (ed.) Current Protocols in Molecular Biology, John Wiley and Sons, Inc., NY (1987), cationic lipid-mediated transfection (Hawley-Nelson et al., Focus 15: 73-79 (1993)) or a commercially available transfection regent, e.g., LipofectACE^{®} (Life Technologies, Gaithersburg, MD) or the like As noted above, in some embodiments of the invention the N, P, L and other desired PIV proteins are encoded by one or more helper viruses which is phenotypically distinguishable from that which encodes the genome or antigenome. The N, P, L and other desired PIV proteins can also be encoded by one or more expression vectors which can be the same or separate from that which encodes the genome or antigenome, and various combinations thereof. Additional proteins may be included as desired, encoded by its own vector or by a vector encoding one or more of the N, P, L and other desired PIV proteins, or the complete genome or antigenome.

The compositions and methods of the invention permit analysis and manipulation of PIV molecular biology and pathogenic mechanisms using, e.g., defined mutations to alter the function or expression of selected PIV proteins. Using these methods and compositions, one can readily distinguish mutations responsible for desired phenotypic changes from silent incidental mutations, and select phenotype-specific mutations for incorporation into a recombinant PIV genome or antigenome for vaccine production.

Modifications of PIV provided within the invention are directed toward the production of improved vaccine viruses, e.g., to enhance viral attenuation and vaccine immunogenicity, to ablate epitopes associated with undesirable immunopathology, to accommodate antigenic drift, etc. To achieve these and other objectives, the compositions and methods of the invention allow for a wide variety of modifications to be introduced into a PIV genome or antigenome for incorporation into infectious, recombinant PIV. For example, foreign genes or gene segments encoding protective antigens or epitopes may be added within a PIV clone to generate PIV virus strains capable of inducing immunity to both PIV and another virus or agent from which the protective antigen was derived. Alternatively, foreign genes may be inserted, in whole or in part, encoding modulators of the immune system, such as cytokines, to enhance immunogenicity of a vaccine virus.

Other mutations which may be included within PIV clones of the invention include, for example, substitution of heterologous genes or gene segments (e.g., a gene segment encoding a cytoplasmic tail of a glycoprotein gene) with a counterpart gene or gene segment in a PIV clone. Alternatively, the relative order of genes within a PIV clone can be changed, the PIV genome promoter can be replaced with its antigenome counterpart, or selected gene(s) rendered non-functional (e.g., by functional ablation involving introduction of a stop codon to prevent expression of the gene). Other modifications in a PIV clone can be made to facilitate manipulations, such as the insertion of unique restriction sites in various non-coding or coding regions or elsewhere. In addition, nontranslated gene sequences can be removed to increase capacity for inserting foreign sequences.

Thus, by providing infectious clones of PIV the invention permits a wide range of alterations to be recombinantly produced within the PIV genome (or antigenome), yielding defined mutations which specify desired phenotypic changes. By "infectious clone" is meant cDNA or its product, synthetic or otherwise, RNA capable of being directly incorporated into infectious virions which can be transcribed into genomic or antigenomic RNA capable of serving as a template to produce the genome of infectious viral or subviral particles. As noted above, defined mutations can be introduced by a variety of conventional techniques (e.g., site-directed mutagenesis) into a cDNA copy of the genome or antigenome. The use of genomic or antigenomic cDNA subfragments to assemble a complete genome or antigenome cDNA as described herein has the advantage that each region can be manipulated separately, where small cDNA subjects provide for better ease of manipulation than large cDNA subjects, and then readily assembled into a complete cDNA. Thus, the complete antigenome or genome cDNA, or a selected subfragment thereof, can be used as a template for oligonucleotide-directed mutagenesis. This can be through the intermediate of a single-stranded phagemid form, such as using the MUTA-gen^{®} kit of Bio-Rad Laboratories (Richmond, CA), or a method using the double-stranded plasmid directly as a template such as the Chameleon^{®} mutagenesis kit of Strategene (La Jolla, CA), or by the polymerase chain reaction employing either an oligonucleotide primer or a template which contains the mutation(s) of interest. A mutated subfragment can then be assembled into the complete antigenome or genome cDNA. A variety of other mutagenesis techniques are known and can be routinely adapted for use in producing the mutations of interest in a PIV antigenome or genome cDNA of the invention.

Thus, in one illustrative embodiment mutations are introduced by using the MUTA-gene^{®} phagemid in vitro mutagenesis kit available from Bio-Rad Laboratories. In brief, cDNA encoding an PIV genome or antigenome is cloned into the plasmid pTZ18U, and used to transform CJ236 cells (Life Technologies). Phagemid preparations are prepared as recommended by the manufacturer. Oligonucleotides are designed for mutagenesis by introduction of an altered nucleotide at the desired position of the genome or antigenome. The plasmid containing the genetically altered genome or antigenome is then amplified.

Mutations can vary from single nucleotide changes to the introduction, deletion or replacement of large cDNA segments containing one or more genes or genome segments. Genome segments can correspond to structural and/or functional domains, e.g., cytoplasmic, transmembrane or ectodomains of proteins, active sites such as sites that mediate binding or other biochemical interactions with different proteins, epitopic sites, e.g., sites that stimulate antibody binding and/or humoral or cell mediated immune responses, etc. Useful genome segments in this regard range from about 15-35 nucleotides in the case of genome segments encoding small functional domains of proteins, e.g., epitopic sites, to about 50, 75, 100, 200-500, and 500-1,500 or more nucleotides.

The ability to introduce defined mutations into infectious PIV has many applications, including the manipulation of PIV pathogenic and immunogenic mechanisms. For example, the functions of PIV proteins, including the N, P, M, F, HN, and L proteins and C, D and V ORF products, can be manipulated by introducing mutations which ablate or reduce the level of protein expression, or which yield mutant protein. In one such exemplary modification, a sequence at the cleavage site of the F protein can be modified and the effects of this modification on growth in tissue culture and infection and pathogenesis of the resultant PIV can be routinely determined in experimental animals.

Various genome RNA structural features, such as promoters, intergenic regions, and transcription signals, can also be routinely manipulated within the methods and compositions of the invention. The effects of trans-acting proteins and cis-acting RNA sequences can be readily determined, for example, using a complete antigenome cDNA in parallel assays employing PIV minigenomes (Dimock, et al., J. Virol. 67: 2772-8 (1993), whose rescue-dependent status is useful in characterizing those mutants that may be too inhibitory to be recovered in replication-independent infectious virus.

The present invention further provides tools and methods to readily distinguish between silent incidental mutations and mutations responsible for phenotype differences, for example by introducing suspect mutations, separately and in various combinations, into the genome or antigenome of infectious wild-type (i.e., for one or more phenotypic character such as temperature sensitivity, replication in a selected host, etc.) PIV. This process permits identification of mutations responsible for desired vaccine phenotypes such as attenuation, temperature sensitivity, cold-adaptation, small plaque size, host range restriction, etc. Mutations identified by these methods can then be introduced in various combinations to modify a vaccine virus to an appropriate level of attenuation, etc., as desired. Moreover, the present invention provides the ability to combine mutations from different strains of virus into a single vaccine strain.

As noted above, mutations incorporated within recombinantly altered PIV clones may be selected based on their ability to alter expression and/or function of a selected PIV protein, yielding a desired phenotypic change, or for a variety of other purposes. Desired phenotypic changes include, e.g., changes in viral growth in culture, temperature sensitivity, plaque size, attenuation, and immunogenicity.

For example, a polynucleotide sequence encoding the genome or antigenome can be modified by a nucleotide insertion, rearrangement, deletion or substitution to specify attenuation, temperature-sensitivity, cold-adaptation, small plaque size, host range restriction, alteration in gene expression, or a change in an immunogenic epitope.

In one aspect of the invention, mutations occurring in biologically derived, attenuated PIV are identified and introduced individually or in combination into a full-length PIV clone, and the phenotypes of rescued recombinant viruses containing the introduced mutations are determined. In exemplary embodiments, amino acid changes displayed by biologically derived mutant viruses over a wild-type PIV, for example changes exhibited by PIV mutants having *ts*, *ca* or att phenotypes, are incorporated within recombinant PIV clones. These changes from biologically derived mutant PIV specify desired characteristics in the resultant clones, e.g., an attenuation phenotype specified by a mutation adopted from the HPIV3 mutant JS *cp*45. These changes are preferably introduced into recombinant virus using two or three nucleotide changes compared to a corresponding wild type or biologically derived mutant sequence, which has the effect of stabilizing the mutation against genetic reversion.

The present invention also provides recombinant PIV having multiple, phenotype-specifying mutations introduced in selected combinations into the genome or antigenome of an infectious clone. This process, coupled with evaluation of phenotype, provides mutant recombinant PIV having such desired characteristics as attenuation, temperature sensitivity, cold-adaptation, small plaque size, host range restriction, etc. Thus, exemplary PIV clones are disclosed herein which incorporate one or more, and preferably at least two attenuating mutations, e.g., *ts*, *ca* or att mutations adopted from a biologically derived PIV mutant, such as JS *cp*45. Target genes for adopting biologically derived mutations in a recombinant PIV in this context include the nucleocapsid protein N, phosphoprotein P, large polymerase subunit L, matrix protein M, hemagglutinin-neuraminidase protein HN, fusion protein F and the C, D and V ORF products. Also targeted are extragenic sequences, eg., sequences in the 3' leader or trailer regions of a PIV genome, and in cis-acting elements such as gene start and gene end sequences, eg., the N gene start signal. Exemplary mutations incorporated in recombinant PIV herein include one or more nucleotide substitutions specifying amino acid change(s) in the polymerase L gene, e.g., at Tyr_{942'} Leu₉₉₂, and/or Thr₁₅₅₈. For example, PIV recombinants are disclosed wherein Tyr₉₄₂ is replaced by His, Leu₉₉₂ is replaced by Phe, and/or Thr₁₅₅₈ is replaced by Ile. These mutations have been successfully incorporated in various exemplary PIV recombinants herein, including r942, r992, r1558, r942/992, r992/1558, r942/1558, or r942/992/1558 recombinants described in the Examples below. Other exemplary mutations adopted from a biologically derived PIV mutant include one or more mutations in the N protein, including specific mutations at a position corresponding to residues Val₉₆ or Ser₃₈₉ of JS *cp*45. In more detailed aspects, these mutations are represented as Val₉₆ to Ala or Ser₃₈₉ to Ala. Also disclosed in the Examples below are recombinant PIV which encode an amino acid substitution in the C protein, eg., a mutation at a position corresponding to Ile₉₆ of JS *cp*45, preferably represented by a substitution of Ile₉₆ to Thr. Further exemplary mutations adopted from biologically derived PIV mutants include one or more mutations in the F protein, including mutations adopted from JS *cp*45 at a position corresponding to residues Ile₄₂₀ or Ala₄₅₀ of JS *cp*45, preferably represented by acid substitutions Ile₄₂₀ to Val or Ala₄₅₀ to Thr. Other PIV recombinants within the invention adopt one or more amino acid substitutions in the HN protein, as exemplified hereinbelow by a recombinant PIV adopting a mutation at a position corresponding to residue Val₃₈₄ of JS *cp*45, preferably represented by the substitution Val₃₈₄ to Ala. Yet additional examples within this aspect of the invention include recombinant PIV which incorporate one or more mutations in an extragenic sequence, eg., a 3' leader sequence of recombinant PIV genome or antigenome. Exemplary mutations in this context include mutations in the 3' leader occurs at one or more positions corresponding to nucleotide 23, 24, and/or 28 of JS *cp*45, for example a T to C change at nucleotide 23, a C to T change at nucleotide 24, or a G to T change at nucleotide 28. Yet additional extragenic mutations include one or more mutations in a N gene start sequence, as exemplified hereinbelow by a mutation in the N gene start sequence at a position corresponding to nucleotide 62 of JS *cp*45, preferably represented by a A to T change. The above exemplary mutations adopted from biologically derived mutant PIV are evaluated and combined into recombinant PIV in the Examples below to result, individually and/or in combination, in novel, attenuated candidate vaccine strains, as exemplified by the recombinants designated herein as *rcp*45, *rcp*45 3'NCMFHN, *rcp*45 3'NL, *rcp*45 3'N, and *rcp*45 F. Other PIV recombinants within the invention will incorporate a plurality and up to a full complement of the mutations present in one or more of these exemplary recombinants, as well as mutations identified in other biologically derived mutant PIV strains identified and adopted in a recombinant PIV according to the teachings herein.

Mutations identified according to the methods disclosed herein are compiled into a "menu" and introduced in various combinations to calibrate a vaccine virus to a selected level of attenuation, immunogenicity and stability. In preferred embodiments, the invention provides for supplementation of one or more mutations adopted from biologically derived PIV, e.g., *ts*, *ca* or att mutations, with additional types of mutations involving the same or different genes. Target genes for mutation in this context also include the nucleocapsid protein N, phosphoprotein P, large polymerase subunit L, matrix protein M, hemagglutinin-neuraminidase protein HN, fusion protein F and the C, D and V ORF products. In one aspect, recombinant PIVs are provided wherein at least one attenuating mutation occurs in the PIV polymerase gene L and involves a nucleotide substitution specifying a *ts* or att phenotype adopted from a biologically derived mutant PIV strain, for example JS *cp*45. Exemplary HPIV3 recombinants disclosed herein include the r942, r992, r1558, r942/992, r992/1558, r942/1558, or r942/992/1558 recombinants described in the Examples below. These exemplary PIV clones incorporate one or more nucleotide substitutions resulting in an amino acid change in the polymerase gene, e.g., at Tyr₉₄₂, Leu₉₉₂, and/or Thr₁₅₅₈. For example, PIV recombinants are disclosed wherein Tyr₉₄₂ is replaced by His, Leu₉₉₂ is replaced by Phe, and/or Thr₁₅₅₈ is replaced by Ile. Preferably, two or three mutations are incorporated in a codon specifying an attenuating mutation adding increased stability against phenotypic reversion.

In additional aspects, a variety of other genetic alterations can be produced in a recombinant PIV genome or antigenome for incorporation into infectious recombinant PIV, alone or together with one or more attenuating mutations adopted from a biologically derived mutant PIV. Heterologous genes (e.g. from different PIV strains or non-PIV sources such as another virus, e.g., RSV or measles virus) may be inserted or substituted, in whole or in part, the order of genes changed, gene overlap removed, the PIV genome promoter replaced with its antigenome counterpart, and even entire, non-essential genes deleted. In one aspect, a selected PIV gene, for example the C, D, or V ORF, is functionally deleted to yield a recombinant PIV having novel phenotypic characteristics, for example enhanced growth *in vitro* and/or attenuation *in vivo*. An infectious PIV clone of the invention can also be engineered to enhance its immunogenicity and induce a level of protection greater than that provided by natural infection, or to ablate epitopes associated with undesirable immunopathologic reactions. Enhanced immunogenicity of the vaccines produced by the present invention addresses one of the greatest obstacles to controlling PIV, namely the incomplete nature of immunity induced by natural infection. In this context, additional gene(s) or gene segment(s) may be inserted into or proximate to the PIV genome or antigenome which may be placed under the control of a common or independent set of transcription signals. Genes of interest include those encoding cytokines (e.g., IL-2 through IL-15, especially IL-2, IL-6 and IL-12, etc.) and proteins rich in T helper cell epitopes. The additional protein can be expressed either as a separate protein or as a chimera engineered from a second copy of one of the PIV proteins, such as HN. This provides the ability to modify and improve the immune response against PIV both quantitatively and qualitatively.

Other mutations useful within the invention involve replacement of the 3' end of genome with its counterpart from antigenome, which is associated with changes in RNA replication and transcription. In addition, intergenic regions can be shortened or lengthened or changed in sequence content. In yet additional aspects, PIV useful in a vaccine formulation can be conveniently modified to accommodate antigenic drift in circulating virus. Typically the modification will be in the HN and/or F proteins. For example, a selected antigenic form of an entire HN or F gene, or the segment(s) encoding particular immunogenic regions thereof, is incorporated into a PIV genome or antigenome cDNA by replacement of a counterpart region in the infectious clone, or by adding one or more copies of the gene such that several antigenic forms are represented in the resultant clone. Progeny virus produced from the modified PIV cDNA are then used in vaccination protocols against emerging strains.

Other mutations for use in infectious PIV of the invention include mutations in cis-acting signals identified during mutational analysis of PIV minigenomes. For example, insertional and deletional analysis of leader and trailer and flanking sequences identify viral promoters and transcription signals and provide a series of mutations associated with varying degrees of reduction of RNA replication or transcription. Saturation mutagenesis (whereby each position in turn is modified to each of the nucleotide alternatives) of these cis-acting signals also identifies mutations which reduce or increase RNA replication or transcription. Any of these mutations can be inserted into the complete antigenome or genome as described herein.

Additional modifications in PIV clones can be made to facilitate manipulations, such as the insertion of unique restriction sites in various intergenic regions or elsewhere. Nontranslated gene sequences can also be removed to increase capacity for inserting foreign sequences.

Certain substitutions, insertions, deletions or rearrangements of genes or gene segments within recombinant PIV of the invention (e.g., substitutions of a gene segment encoding a selected protein or protein region, for instance a cytoplasmic tail, transmembrane domain or ectodomain, an epitopic site or region, a binding site or region, an active site or region containing an active site, etc.) are made in structural or functional relation to an existing, "counterpart" gene or gene segment from the same or different PIV or other source. Such modifications yield novel recombinants having desired phenotypic changes compared to wild-type or parental PIV or other viral strains. For example, recombinants of this type may express a chimeric protein having a cytoplasmic tail and/or transmembrane domain of one PIV fused to an ectodomain of another PIV. Other exemplary recombinants of this type express duplicate protein regions, such as duplicate immunogenic regions.

As used herein, "counterpart" genes, gene segments, proteins or protein regions, are typically from heterologous sources (e.g., from different PIV genes, or representing the same (i.e., homologous or allelic) gene or gene segment in different PIV types or strains). Typical counterparts selected in this context share gross structural features, e.g., each counterpart may encode a comparable protein or protein structural domain, such as a cytoplasmic domain, transmembrane domain, ectodomain, binding site or region, epitopic site or region, etc. Counterpart domains and their encoding gene segments embrace an assemblage of species having a range of size and sequence variations defined by a common biological activity among the domain or gene segment variants. For example, two selected protein domains encoded by counterpart gene segments within the invention share substantially the same qualitative activity, such as providing a membrane spanning function, a specific binding activity, an immunological recognition site, etc. More typically, a specific biological activity shared between counterparts, e.g., between selected protein segments or proteins, will be substantially similar quantitatively, i.e., they will not vary in respective quantitative activity levels by more than 30%, preferably by no more than 20%, more preferably by no more than 5-10%.

Counterpart genes and gene segments, as well as other polynucleotides disclosed herein for producing recombinant PIV within the invention, preferably share substantial sequence identity with a selected polynucleotide "reference sequence," e.g., with another selected counterpart sequence. As used herein, a "reference sequence" is a defined sequence used as a basis for sequence comparison, for example, a segment of a full-length cDNA or gene, or a complete cDNA or gene sequence. Generally, a reference sequence is at least 20 nucleotides in length, frequently at least 25 nucleotides in length, and often at least 50 nucleotides in length. Since two polynucleotides may each (1) comprise a sequence (i.e., a portion of the complete polynucleotide sequence) that is similar between the two polynucleotides, and (2) may further comprise a sequence that is divergent between the two polynucleotides, sequence comparisons between two (or more) polynucleotides are typically performed by comparing sequences of the two polynucleotides over a "comparison window" to identify and compare local regions of sequence similarity. A "comparison window", as used herein, refers to a conceptual segment of at least 20 contiguous nucleotide positions wherein a polynucleotide sequence may be compared to a reference sequence of at least 20 contiguous nucleotides and wherein the portion of the polynucleotide sequence in the comparison window may comprise additions or deletions (i.e., gaps) of 20 percent or less as compared to the reference sequence (which does not comprise additions or deletions) for optimal alignment of the two sequences. Optimal alignment of sequences for aligning a comparison window may be conducted by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Natl. Acad. Sci. USA 85:2444 (1988), by computerized implementations of these algorithms (GAP, BESTFIT, FASTA, and TFASTA in the Wisconsin Genetics Software Package Release 7.0, Genetics Computer Group, 575 Science Dr., Madison, WI), or by inspection, and the best alignment (i.e., resulting in the highest percentage of sequence similarity over the comparison window) generated by the various methods is selected. The term "sequence identity" means that two polynucleotide sequences are identical (i.e., on a nucleotide-by-nucleotide basis) over the window of comparison. The term "percentage of sequence identity" is calculated by comparing two optimally aligned sequences over the window of comparison, determining the number of positions at which the identical nucleic acid base (e.g., A, T, C, G, U, or I) occurs in both sequences to yield the number of matched positions, dividing the number of matched positions by the total number of positions in the window of comparison (i.e., the window size), and multiplying the result by 100 to yield the percentage of sequence identity. The terms "substantial identity" as used herein denotes a characteristic of a polynucleotide sequence, wherein the polynucleotide comprises a sequence that has at least 85 percent sequence identity, preferably at least 90 to 95 percent sequence identity, more usually at least 99 percent sequence identity as compared to a reference sequence over a comparison window of at least 20 nucleotide positions, frequently over a window of.at least 25-50 nucleotides, wherein the percentage of sequence identity is calculated by comparing the reference sequence to the polynucleotide sequence which may include deletions or additions which total 20 percent or less of the reference sequence over the window of comparison. The reference sequence may be a subset of a larger sequence.

In addition to these polynucleotide sequence relationships, proteins and protein regions encoded by recombinant PIV of the invention are also typically selected to have conservative relationships, i.e. to have substantial sequence identity or sequence similarity, with selected reference polypeptides. As applied to polypeptides, the term "sequence identity" means peptides share identical amino acids at corresponding positions. The term "sequence similarity" means peptides have identical or similar amino acids (i.e., conservative substitutions) at corresponding positions. The term "substantial sequence identity" means that two peptide sequences, when optimally aligned, such as by the programs GAP or BESTFIT using default gap weights, share at least 80 percent sequence identity, preferably at least 90 percent sequence identity, more preferably at least 95 percent sequence identity or more (e.g., 99 percent sequence identity). The term "substantial similarity" means that two peptide sequences share corresponding percentages of sequence similarity. Preferably, residue positions which are not identical differ by conservative amino acid substitutions. Conservative amino acid substitutions refer to the interchangeability of residues having similar side chains. For example, a group of amino acids having aliphatic side chains is glycine, alanine, valine, leucine, and isoleucine; a group of amino acids having aliphatic-hydroxyl side chains is serine and threonine; a group of amino acids having amide-containing side chains is asparagine and glutamine; a group of amino acids having aromatic side chains is phenylalanine, tyrosine, and tryptophan; a group of amino acids having basic side chains is lysine, arginine, and histidine; and a group of amino acids having sulfur-containing side chains is cysteine and methionine. Preferred conservative amino acids substitution groups are: valine-leucine-isoleucine, phenylalanine-tyrosine, lysine-arginine, alanine-valine, and asparagine-glutamine. Abbreviations for the twenty naturally occurring amino acids used herein follow conventional usage (Immunology - A Synthesis (2nd ed., E.S. Golub & D.R. Gren, eds., Sinauer Associates, Sunderland, MA, 1991). Stereoisomers (e.g., D-amino acids) of the twenty conventional amino acids, unnatural amino acids such as α,α-disubstituted amino acids, N-alkyl amino acids, lactic acid, and other unconventional amino acids may also be suitable components for polypeptides of the present invention. Examples of unconventional amino acids include: 4-hydroxyproline, γ-carboxyglutamate, ε-N,N,N-trimethyllysine, e-N-acetyllysine, O-phosphoserine, N-acetylserine, N-formylmethionine, 3-methylhistidine, 5-hydroxylysine, ω-N-methylarginine, and other similar amino acids and imino acids (e.g., 4-hydroxyproline). Moreover, amino acids may be modified by glycosylation, phosphorylation and the like.

The infectious PIV produced from cDNA-expressed genome or antigenome can be any of the PIV or PIV-like strains, e.g., human, bovine, murine, etc. To engender a protective immune response, the PIV strain may be one which is endogenous to the subject being immunized, such as human-PIV being used to immunize humans. The genome or antigenome can be modified, however, to express heterologous PIV genes or gene segments, or genes or gene segments from other heterologous sources, e.g., RSV or measles virus. Thus, infections PIV intended for administration to humans may be human PIV that has been modified to contain genes or gene segments from a bovine or murine PIV type such as for the purpose of attenuation. BPIV3 possesses host range mutations that restrict its replication in rhesus monkeys and humans (Karron et al., supra, 1995a; van Wyke Coelingh et al., 1988)). Gene (s), mutations and cis-acting regulatory sequences of BPIV3 that specify desired phenotypes, e.g., host range restriction, will be identified by their substitution for corresponding sequences in rPIV of the invention, and incorporated within further modified rPIV to develop yet additional useful vaccine agents. Similarly, mutations of JS *cp*45 which are known to impart non-ts host-range attenuating mutations for the rhesus monkey (Hall et al., supra, (1992)) will likewise be identified and incorporated into modified rPIV vaccine agents of the invention. Alternatively, a bovine PIV may be modified to contain genes that encode, e.g., proteins or immunogenic epitopes that elicit protection against human PIV infection. For example, human PIV glycoprotein genes can be substituted for counterpart bovine glycoprotein genes, such that the bovine PIV elicits a protective immune response in humans against human PIV strains.

Individual genes , gene segments, or single or multiple nucleotides of one PIV may be substituted by counterpart sequence(s) from a heterologous PIV or other source. For example, heterologous gene segments, such as one encoding a cytoplasmic tail, transmembrane domain or ectodomain, an epitopic site or region, a binding site or region, an active site or region containing an active site, etc., of a selected protein from one PIV is substituted for a counterpart gene segment in another PIV to yield novel recombinants, for example recombinants expressing a chimeric protein having a cytoplasmic tail and/or transmembrane domain of one PIV fused to an ectodomain of another PIV. Preferred genome segments in this regard range from about 15-35 nucleotides in the case of gene segments encoding small functional domains of proteins, e.g., epitopic sites, to about 50, 75, 100, 200-500, or 500-1,500 or more nucleotides for gene segments encoding larger domains or protein regions.

Selected domains of HN and/or F proteins of one PIV strain may be substituted into a heterologous PIV clone to produce a recombinant virus capable of stimulating a cross-protective immune response against both PIV strains in an immunized host. Modified PIV clones may comprise a chimera of a human PIV genomic or antigenomic sequence and at least one non-human PIV sequence, for example a polynucleotide containing sequences from both human and bovine PIV. The replacement of a human PIV coding sequence or non-coding sequence (e.g., a promoter, gene-end, gene-start, intergenic or other cis-acting element) with a counterpart bovine or murine PIV sequence yields recombinants having a variety of possible attenuating effects. For example, a host range effect will often arise from a heterologous PIV gene not functioning efficiently $in a human cell, from incompatibility of the heterologous sequence or protein with a biologically interactive human PIV sequence or protein (e.g., a sequence or protein that ordinarily cooperates with the substituted sequence or protein for viral transcription, translation, assembly, etc.), among other useful attenuating effects. In yet another aspect of the invention, insertion of foreign genes or gene segments, and in some cases of noncoding nucleotide sequences, into the PIV genome results in a desired increase in genome length causing yet additional, desired phenotypic effects. Increased genome length is expected to result in attenuation of the resultant PIV clone, dependent in part upon the length of the insert. In addition, the expression of certain proteins from a gene inserted into recombinant PIV will result in attenuation of the virus due to the action of the protein. This has been described for IL-2 expressed in vaccinia virus (see, e.g., Flexner et al., Nature 33:-259-62 (1987)) and also would be expected for gamma interferon.

Deletions, insertions, substitutions and other mutations involving changes of whole viral genes or gene segments in recombinant PIV of the invention yield highly stable vaccine candidates, which are particularly important in the case of immunosuppressed individuals. Many of these mutations will result in attenuation of resultant vaccine strains, whereas others will specify different types of desired phenotypic changes. For example, certain viral genes are known which encode proteins that specifically interfere with host immunity (see, e.g., Kato et al., EMBO. J. 16:578-87 (1997). Ablation of such genes in vaccine viruses is expected to reduce virulence and pathogenesis and/or improve immunogenicity.

In preferred aspects of the invention, the modified PIV clones represent a chimera of two or more human PIV genomes, for example a clone containing polynucleotide sequences from HPIV3 joined to sequences from one or more heterologous human PIV, such as HPIV1 and HPIV2. Thus, individual genes or gene segments of human PIV3 may be replaced or supplemented with counterpart genes or gene segments from HPIV 1 or HPIV2, or visa versa. In one example described hereinbelow, the invention provides a PIV clone, rPIV3-1, into which both the HN and F glycoprotein genes of HPIV1 are substituted for their counterpart genes in an HPIV3 background, yielding a chimeric virus having immunological characteristics representative of both parental strains.

In additional aspects of the invention, chimeric PIV or PIV clones having other alterations of genes or gene segments, as described above, are further modified by introducing one or more attenuating mutations adopted from a biologically derived mutant PIV, e.g., HPIV3 JS cp45 to achieve an attenuated, or further attenuated, chimeric mutant derivative. For example, one or more human PIV coding or non-coding polynucleotides may be substituted with a counterpart sequence from a heterologous human PIV, bovine PIV or murine PIV as described above, and this alteration may be combined with one or more mutations specifying, e.g., a ts, ca or att phenotype adopted from a biologically derived attenuated PIV mutant, to yield an attenuated or further attenuated (i.e., compared to either the chimeric clone or biologically derived parent virus) vaccine virus. Alternatively, functional deletion of a non-essential gene or gene segment, such as the C, D or V ORF, may be combined in a recombinant PIV with one or more mutations specifying a ts, ca or att phenotype from biologically derived PIV mutants to yield an attenuated vaccine strain. These combinatorial modifications yield recombinant PIV having desired phenotypic characteristics, e.g., increased yield of virus, enhanced attenuation, and/or genetic resistance to reversion from an attenuated phenotype, due to the combined effects of the different selected mutations.

In one combinatorial mutation design, a modified PIV is provided which comprises a chimera of a human PIV genomic or antigenomic sequence and at least one non-human PIV sequence, for example a polynucleotide containing sequences from both human and bovine PIV, and which also incorporates one or more mutations adopted from biologically derived PIV, e.g., one or more naturally occurring ts, ca or att mutations. Alternatively, the modified PIV can be a chimera of two or more human PIV genomes, for example a polynucleotide containing sequences from HPIV3 joined to sequences from one or more heterologous human PIVs, such as HPIV1 and HPIV2, which further incorporates one or more ts, ca att or other selected mutations from biologically derived PIV (e.g., a nucleotide substitution specifying a ts, ca or att phenotype adopted from a biologically derived mutant PIV strain such as JS cp45). In more detailed aspects, individual genes or gene segments of human PIV3 are replaced or supplemented with counterpart genes or gene segments from HPIV1 or HPIV2, or visa versa, in a clone that is attenuated or further attenuated by, e.g., a nucleotide change encoding an amino acid substitution conferring a ts mutation in the large polymerase L gene. For example, the invention provides PIV clones having the HN and/or F glycoprotein genes of HPIV1 substituted for their counterpart genes in an HPIV3 background, wherein the phenotype of the resultant chimeric clone is further modified by ts, ca or att mutation(s) encoded within one or more of the N, P, L, M, HN, F, C, D and V genes. Various combinations from a menu of possible mutations disclosed herein can be made to calibrate a vaccine virus to a selected level of attenuation, immunogenicity and stability, e.g., to achieve a satisfactorily attenuated and immunogenic, chimeric virus having immunological characteristics representative of multiple PIV strains. In one aspect, recombinant PIVs are provided wherein at least one attenuating mutation occurs in the PIV polymerase gene L (as exemplified by the recombinants r942, r992, r1558, r942/992, r942/1558, r992/1558, or r942/992/1558 described in the Examples below) incorporated in a chimeric PIV background. For example, useful chimeric PIV recombinants within this aspect of the invention will have one or more genes or gene segments of the HN and/or F glycoprotein genes from, e.g., HPIV1 substituted for their counterpart gene(s) in a heterologous background, e.g., in an HPIV3 clone, and will further incorporate one or more attenuating mutations, eg., nucleotide substitutions resulting in an amino acid change in the polymerase gene (such as change from Tyr to His at position 942, a change from Leu to Phe at position 992, and/or a change from Thr to Ile at position 1558) from a biologically derived PIV mutant. One such chimeric, attenuated recombinant exemplified hereinbelow is rPIV3-1.cp45L, a derivative of rPIV3-1 which incorporates all three L gene mutations specified above from JS cp45.

Yet additional mutations which can be incorporated in a chimeric PIV background for developing vaccine strains will be selected from biologically derived mutations in other genes, or will be created de novo in a recombinant genome by standard site directed mutagenesis or other purely recombinant mutagenic methods. Target genes for adopting biologically derived mutations or creating novel mutations in a recombinant PIV in this context include the nucleocapsid protein N, phosphoprotein P, large polymerase subunit L, matrix protein M, hemagglutinin-neuraminidase protein HN, fusion protein F and the C, D and V ORF products. Also targeted are extragenic sequences, eg., sequences in the 3' leader or trailer regions of a PIV genome. Exemplary mutations identified and incorporated in non-chimeric, recombinant PIV, described above will thus be readily incorporated within a chimeric PIV background, eg., as exemplified by rPIV3-1. These exemplary mutations include one or more mutations in the N protein, including specific mutations at a position corresponding to residues Val₉₆ or Ser₃₈₉ of JS cp45. In more detailed aspects, these mutations are represented as Val₉₆ to Ala or Ser₃₈₉ to Ala. Also desired for incorporation in chimeric PIV recombinants are mutations in the C protein, eg., a mutation at a position corresponding to Ile₉₆ of JS cp45, preferably represented by a substitution of Ile₉₆ to Thr, as described above. Further exemplary mutations for incorporation in a chimeric PIV background include one or more mutations in the F protein, for example adopted from JS cp45 at a position corresponding to residues Ile₄₂₀ or Ala₄₅₀, eg., substitutions Ile₄₂₀ to Val or Ala₄₅₀ to Thr. Yet additional chimeric PIV recombinants within the invention will adopt one or more amino acid substitutions in the HN protein, for example a mutation at a position corresponding to residue Val₃₈₄ of JS cp45, such as Val₃₈₄ to Ala. Yet additional chimeric recombinants will incorporate one or more mutations in an extragenic sequence, eg., a 3' leader sequence of the recombinant genome or antigenome. Exemplary mutations in this context include mutations in the 3' leader occurs at one or more positions corresponding to nucleotide 23, 24, 28, and/or 45 of JS cp45, for example a T to C change at nucleotide 23, a C to T change at nucleotide 24, a G to T change at nucleotide 28, or a T to A change at nucleotide 45. Yet additional extragenic mutations for incorporation within a chimeric PIV background include one or more mutations in a N gene start sequence, as exemplified herein by a mutation in the N gene start sequence at a position corresponding to nucleotide 62 of JS cp45, such as a A to T change. These exemplary mutations evaluated and combined into recombinant PIV in the Examples below will be readily incorporated within a chimeric PIV recombinant using the methods and tools provided herein, and will specify, individually and/or in combination, desired phenotypic changes to yield yet additional attenuated chimeric vaccine strains within the invention.

In additional combinatorial mutation designs, modified PIVs are provided which incorporate one or more of the foregoing ts, ca or att mutations adopted from biologically derived PIV or generated recombinantly in a PIV clone of the invention, in combination with another, distinct mutation disclosed herein (e.g., a deletion, addition, or rearrangement of a PIV N, P, L, M, HN, F, C, D or V gene or gene segment, or a gene or gene segment from another source such as RSV or measles virus). Also in this case, various combinations from a menu of mutations disclosed herein can be made to calibrate the vaccine virus to a selected level of attenuation, immunogenicity and stability. Thus, recombinant PIVs are provided which exhibit at least one attenuating mutation from a biologically derived PIV mutant, e.g., a mutation in the PIV polymerase gene L as found in JS *cp*45, or a recombinantly generated mutation, and which further incorporates one or more additional changes selected from, e.g., substitution or introduction of a heterologous gene or gene segment from a non-PIV source (e.g., an immunogenic RSV or measles gene or epitope, or a gene encoding a cytokine), a change in the order of viral genes to alter expression levels, removal of gene overlap, substitution of a PIV genome promoter with its antigenome counterpart, shortening, lengthening or removal of intergenic regions, e.g., to increase capacity for inserting foreign sequences, mutations in cis-acting signals to reduce or increase RNA replication or transcription, insertion of unique restriction sites, or deletion of even entire, non-essential genes, among other changes.

For vaccine use, virus produced according to the present invention can be used directly in vaccine formulations, or lyophilized, as desired, using lyophilization protocols well known to the artisan. Lyophilized virus will typically be maintained at about 4°C. When ready for use the lyophilized virus is reconstituted in a stabilizing solution, e.g., saline or comprising SPG, Mg⁺⁺ and HEPES, with or without adjuvant, as further described below.

PIV vaccines of the invention contain as an active ingredient an immunogenically effective amount of PIV produced as described herein. The modified virus may be introduced into a host with a physiologically acceptable carrier and/or adjuvant. Useful carriers are well known in the art, and include, e.g., water, buffered water, 0.4% saline, 0.3% glycine, hyaluronic acid and the like. The resulting aqueous solutions may be packaged for use as is, or lyophilized, the lyophilized preparation being combined with a sterile solution prior to administration, as mentioned above. The compositions may contain pharmaceutically acceptable auxiliary substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, triethanolamine oleate, and the like. Acceptable adjuvants include incomplete Freund's adjuvant, MPL^{™} (3-o-deacylated monophosphoryl lipid A; RIBI ImmunoChem Research, Inc., Hamilton, MT) and IL-12 (Genetics Institute, Cambridge MA), among many other suitable adjuvants well known in the art.

Upon immunization with a PIV composition as described herein, via aerosol, droplet, oral, topical or other route, the immune system of the host responds to the vaccine by producing antibodies specific for PIV virus proteins, e.g., F and HN glycoproteins. As a result of the vaccination with an immunogenically effective amount of PIV produced as described herein, the host becomes at least partially or completely immune to PIV infection, or resistant to developing moderate or severe PIV infection, particularly of the lower respiratory tract.

The host to which the vaccines are administered can be any mammal which is susceptible to infection by PIV or a closely related virus and which host is capable of generating a protective immune response to the antigens of the vaccinizing strain. Accordingly, the invention provides methods for creating vaccines for a variety of human and veterinary uses.

The vaccine compositions containing the PIV of the invention are administered to a host susceptible to or otherwise at risk for PIV infection to enhance the host's own immune response capabilities. Such an amount is defined to be a "immunogenically effective dose." In this use, the precise amount of PIV to be administered within an effective dose will depend on the host's state of health and weight, the mode of administration, the nature of the formulation, etc., but will generally range from about 10³ to about 10⁶ plaque forming units (PFU) or more of virus per host, more commonly from about 10⁴ to 10⁵ PFU virus per host. In any event, the vaccine formulations should provide a quantity of modified PIV of the invention sufficient to effectively protect the host patient against serious or life-threatening PIV infection.

The PIV produced in accordance with the present invention can be combined with viruses of other PIV serotypes or strains to achieve protection against multiple PIV serotypes or strains. Alternatively, protection against multiple PIV serotypes or strains can be achieved by combining protective epitopes of multiple serotypes or strains engineered into one virus, as described herein. Topically when different viruses are administered they will be in admixture and administered simultaneously, but they may also be administered separately. Immunization with one strain may protect against different strains of the same or different serotype.

In some instances it may be desirable to combine the PIV vaccines of the invention with vaccines which induce protective responses to other agents, particularly other childhood viruses. In another aspect of the invention the PIV can be employed as a vector for protective antigens of other pathogens, such as respiratory syncytial virus (RSV) or measles virus, by incorporating the sequences encoding those protective antigens into the PIV genome or antigenome which is used to produce infectious PIV, as described herein. The cloning of RSV cDNA and other disclosure relevant to the invention is described in copending United States patent applications Serial Nos. 08/534, 768, 60/021, 773, 08/720,132, 60/046,141, 60/047,634, and 08/892,403, and PCT patent application PCT/US97/12269.

Single or multiple administrations of the vaccine compositions of the invention can be carried out. In neonates and infants, multiple administration may be required to elicit sufficient levels of immunity. Administration should begin within the first month of life, and at intervals throughout childhood, such as at two months, six months, one year and two years, as necessary to maintain sufficient levels of protection against native (wild-type) PIV and/or other subject viral infections. Similarly, adults who are particularly susceptible to repeated or serious PIV infection, for example health care workers, day care workers, family members of young children, the elderly, and individuals with compromised cardiopulmonary function, may require multiple immunizations to establish and/or maintain protective immune responses.

Levels of induced immunity provided by the vaccines of the invention can be monitored by measuring amounts of neutralizing secretory and serum antibodies. Based on these measurements, vaccine dosages can be adjusted or vaccinations repeated as necessary to maintain desired levels of protection. Further, different vaccine viruses may be advantageous for different recipient groups. For example, an engineered PIV strain expressing an additional protein rich in T cell epitopes may be particularly advantageous for adults rather than for nfants.

The PIV of the invention may be employed as a vector for transient gene therapy of the respiratory tract. According to this embodiment the recombinant PIV genome or antigenome incorporates a sequence which is capable of encoding a gene product of interest. The gene product of interest is under control of the same or a different promoter from that which controls PIV expression. The infectious PIV produced by coexpressing the recombinant PIV genome or antigenome with the N, P, L and other desired PIV proteins, and containing a sequence encoding the gene product of interest, is administered to a patient. Administration is typically by aerosol, nebulizer, or other topical application to the respiratory tract of the patient being treated. Recombinant PIV is administered in an amount sufficient to result in the expression of therapeutic or prophylactic levels of the desired gene product. Representative gene products which may be administered within this method are preferably suitable for transient expression, including, for example, interleukin-2, interleukin-4, gamma-interferon, GM-CSF, G-CSF, erythropoietin, and other cytokines, glucocerebrosidase, phenylalanine hydroxylase, cystic fibrosis transmembrane conductance regulator (CFTR), hypoxanthine-guanine phosphoribosyl transferase, cytotoxins, tumor suppressor genes, antisense RNAs, and vaccine antigens.

The following example are provided by way of illustration, not limitation.

### EXAMPLE I

### Construction of Plasmid p218 (131) Encoding Negative sense PIV Genomic RNA

A full cDNA clone designated p218(131) (Fig. 1; SEQ ID NO: 1) (deposited under the terms of the Budapest Treaty with the American Type Culture Collection (ATCC) of 12301 Parklawn Drive, Rockville, Maryland 20852, U.S.A., and granted the designation 97991) was constructed to encode the complete 15462 nt genomic sequence of HPIV3 JS strain. A hepatitis delta ribozyme was placed abutting the 3' end of the genomic sequence such that self-cleavage would yield the 3' end of HPIV3 (Perrotta and Been, Nature 350: 434-436, (1991).

A T7 transcription terminator was placed following the delta ribozyme. The T7 promoter was placed adjacent to the 5' end of the genomic sequence such that the 5' terminal nucleotide of the HPIV3 genome was the first nucleotide synthesized. In this configuration, the cDNA encodes a complete negative-sense copy of PIV3 genomic RNA containing the correct genomic termini without any additional heterologous nucleotides.

The HPIV3 cDNA was assembled from 14 overlapping subclones (termed A*-L, which letters in parentheses designate individual plasmids and do not refer to specific viral genes) constructed by reverse transcription (RT) and polymerase chain reaction (PCR) of RNA isolated from virions purified by sucrose gradient centrifugation (Stokes et al., supra, 1992; Stokes et al., supra, 1993. The subclones spanned the following nucleotides of genomic RNA (numbered with the 3' end designated as position 1): 1-2058 (A*), 1874-3111 (A'), 3066-5140. (C), 4348-5276 (C'), 5072-6695 (D*), 5904-8532 (E), 7806-9898 (F), 9632-10740 (F'), 9760-10955 (G), 10862-11925 (H), 11835-12868 (I), 12426-13677 (J), 13630-14496 (K), and 14467-15462 (L). Each fragment was cloned into pBluescript KSII (Strategene, La Jolla, CA) using conventional cloning techniques and was sequenced completely.

Plasmid p(L) was then subjected to site-directed mutagenesis to introduce the T7 promoter via a single-stranded DNA intermediate according to the MUTA-GENE procedure (BioRad, Hercules, CA)). The T7 promoter was positioned so that transcription initiates at the precise 5' end of the HPIV3 genome using the negative-sense mutagenic primer: 5'-*AATACGACTCACTAT**A*ACCAAACAAGAGAAG*-3' (SEQ ID NO: 2; T7 sequences are italicized, HPIV3-specific sequences are underlined, and the 5'-end HPIV3 nucleotide, genome position 15462, is indicated by an asterisk). This modified p(L) was designated p(L*). Plasmid p(E) was modified to yield p(E*) by the same method using the negative-sense mutagenic oligonucleotide 5'-CCAAGTACTATGAGATGCTTGATT-3' (SEQ ID NO: 3) to insert three nucleotide substitutions (underlined) into the HN gene at HPIV3 position 7903, 7913, 7915 (Fig. 1). These substitutions removed an *Hga* I site, inserted a *Sca* I site, and modified amino acid 370 of the encoded HN protein such that the epitope recognized by monoclonal antibodies (mAb) 423/6 and 170/7 was ablated (van Wyke Coelingh et al., J. Virol. 61:1473-1477, (1987). The p (E*) to p(K) subclones were assembled in a step-wise fashion into the p (L*) plasmid to give p(E*FF'GHIJKL*) (Fig. 1A) . This plasmid includes HPIV3 nucleotides 5904-15462 with the T7 promoter adjacent to nucleotide 15462 at the 5' end of the genome. Subclones p(A*) to p(E) were assembled into a second, overlapping subclone, p(A*A'CC'D*E) which contained HPIV3 nucleotides 1-8532.

Both subclones p(E*FF'GHIJKL*) and p(A*A'CC'D*E) were sequenced completely. In addition to the introduced point mutations described above, the cDNA differed from the authentic JS HPIV3 sequence (Stokes et al., supra, 1992) by a single nucleotide substitution at positions 1615 which was within the N gene and caused a substitution at amino acid 506 in the encoded protein. Three other nucleotide substitutions were found at positions 10355, 11333, and 15248 in the L gene which did not change the encoded protein (Fig. 2). These three noncoding changes were retained as additional sequence markers to identify recombinant virus (designated rPIV) derived from cDNA, and the mutation in the N gene was corrected as described later.

Subclone p(A*A'CC'D*E) was then modified to insert the hepatitis delta virus ribozyme and T7 terminator adjacent to HPIV3 position 1. An HPIV3 minigenome in which the 3' end of the HPIV3 genome (GGT↓**GGG**) (underlined) was generated through self-cleavage of a flanking hepatitis delta virus antigenomic ribozyme (shown in part in bold-type) was previously constructed (Dimock and Collins, J. Virol. 67: 2772-2778, (1993); Perrotta and Been, supra, (1991). The ribozyme in turn was followed by a T7 transcription terminator. This minigenome cDNA was used as a template in a PCR reaction which modified the sequence adjacent to the ribozyme cleavage site to be a *Sma* I site (CCC↓GGG) and placed an ApaI site (GGGCC↓C) on the downstream side of the T7 terminator. The PCR product was cloned into pKSII which had been digested with *Bss*HII and made blunt-ended by filling in, yielding p218.

p218 was designed such that any sequence could be introduced into the opened Sma I site by blunt-end ligation and its RNA transcript would be cleaved at the delta ribozyme cut site (NNN↓GGG). The p(A*A'CC'D*E) subclone was digested with *Hga* I and *Sal* I (8533), which released the HPIV3 cDNA_{,} and was filled in with dNTPs and T4 DNA polymerase to give blunt termini. The *Hga* I site is 10 nucleotides upstream of HPIV3 position 1 and, when digested and filled in, leaves a blunt terminus beginning with HPIV3 position 1. The modified *Hga* I-*Sal* I fragment was gel purified and cloned into the *Sma* I site of p218. The mutation in the N gene (T at nt 1615) was corrected to the JS wt sequence (A at nt 1615) (see GenBank accession #ZI1575,) using Kunkel mutagenesis (Kunkel et al., Methods Enzymol. 154: 367-382, (1987)). This plasmid was designated b218(A*A'CC'D*E) (Fig. 1).

The X*ho I-Ngo* MI fragment of p(E*FF'GHIJKL*), which contained the T7 promoter and the HPIV3 cDNA from nucleotides 7438-15462, was cloned into the *Xho* I-*Ngo* MI window of p218(A*A'CC'D*E) (Fig. 1). This joined the two fragments of HPIV3 cDNA at HPIV3 nucleotide 7438, yielding a plasmid containing a full-length HPIV3 cDNA encoding a negative-sense genomic RNA with the above-mentioned three desired mutations in the HN gene and three incidental mutations in the L gene. The final construct, designated p218 (131) (Fig. 1; SEQ ID NO: 1), was then sequenced in its entirety by automated sequencing at the NCI Frederick Cancer Research and Development Center (Frederick, MD) using the Taq DYE Deoxy Terminator cycle sequencing kit (ABI, Foster City, CA). This identified an additional change in the HN gene, namely a change of C to T in the HN gene at position 7593 which changed HN amino acid 263 from threonine to isoleucine that is also indicated in Fig. 2.

### Reference EXAMPLE II

### Transcription and RNA Replication System for HPIV3

The present example is provided for reference only and describes compositions and methods for producing a reconstituted transcription and RNA replication system for human parainfluenza virus type 3 (HPIV3). This exemplary system was developed using components expressed intracellularly from transfected plasmids driven by a T7 RNA polymerase supplied by a vaccinia virus recombinant. The system is based on a negative-sense analog of HPIV3 genomic RNA in which the viral genes were deleted and replaced with a polynucleotide encoding bacterial chloramphenicol acetyl transferase (CAT). The N, P and L proteins are expressed from cotransfected plasmids so as to direct efficient transcription and RNA replication. Transcription according to this example yields subgenomic polyadenylated mRNA, which can be readily isolated, e.g., by oligo(dT) chromatography. RNA replication according to this example yields mini-antigenome and progeny minigenome, which are shown to be encapsidated based on resistance to digestion with micrococcal nuclease.

### A) Viruses and cells

A vaccinia virus recombinant, vTF7-3, that expresses bacteriophage T7 RNA polymerase, was provided as described by Fuerst et al. (Proc. Natl. Acad. Sci. U.S.A. 83: 8122-8126, 1986,). HEp-2 monolayer cultures were maintained at 37°C in 5% CO₂ with OptiMEM 1 (Life Technologies, Gaithersburg, MD) supplemented with 2% fetal bovine serum (FBS), 50 µg/ml gentamicin sulfate and 2mM glutamine.

### B) cDNAs

cDNAs corresponding to ORFs of the N, P, and L genes of the JS strain of HPIV3 (GenBank #Z11575; Stokes et al., 1992) were individually cloned into the *Nco* I-*Sal* I window of plasmid pTM-1, in which transcription is mediated by T7 RNA polymerase and translation by an internal ribosome entry site preceding the foreign ORF (Elroy-Stein et al., Proc. Natl. Acad. Sci. U.S.A. 86: 6126-6130 (1989). Each gene was first modified by polymerase chain reaction (PCR) to place an *Nco* I or *Nco* I-compatible site at the translational start site and a *Sal* I site on the downstream end.

The plasmid p(131), which is similar to p218 (131) except that it lacks the hepatitis delta virus ribozyme, was used as a template for each PCR. The primers used to amplify the N ORF were CCCTATAATTTCAA*CAT*GTTGAGCCTATTTG (SEQ ID NO: 4; forward primer relative to positive-sense) and GATTAAAATGTT*GGT*CGACTTAGTTGCTTCC (SEQ ID NO: 5; italics represent restriction enzyme sites, and the translational start site is in bold). The PCR product, a 1578 bp fragment flanked by an *Afl* III and *Sal* I site, was cloned into the *Nco* I- *Sal* I window of pTM-1 to yield pTM (N).

The primers used to amplify the PIV3 phosphoprotein (P) ORF were 5-'CCATAGAGAGT*CCATGG*AAAGCGATGCTAAAAACTATC-3' (SEQ ID NO:6; forward primer) and 5'-CGGTGTCGTTTCTTT*GTCGAC*TCATTGGCAATTGTTG-3' (SEQ ID NO:7; reverse primer). A full-length cDNA of JS strain of genomic RNA (p131) was used as template for the PCR. The resultant PCR product was an 1851 bp fragment flanked by an *Nco* I and *Sal* I restriction site (in italics). The PCR product was then cloned into the *Nco* I-*Sal* I window of pTM-1 to yield pTM(P).

A second PCR was performed to amplify the PIV3 phosphoprotein P ORF without the C ORF. p131 was again used as template cDNA. A different forward primer and the same reverse primer were used to amplify the PIV3 P ORF without C; 5'-CCATAGAGAGT*CC**ATG**G*AAAGCGACGCTAAAAACTATC-3' (SEQ ID NO: 74; forward primer) and 5'-CGGTGTCGTTTCTTTGTCGA*GTC*ATTGGCAATTGTTG-3' (SEQ ID NO:7; reverse primer). The resultant PCR product was an 1851 bp fragment flanked by an *Nco* I and *Sal* I restriction site (designated by italics). The underlined nucleotide in the forward primer represents a nucleotide substitution which is silent in the P ORF but changes the start codon of the C ORF to threonine. The next start codon for the C ORF is more than 400 nucleotides downstream. Thus, only the P protein would be produced. The PCR product was then cloned into the *Nco* I-*Sal* I window of pTM-1 to yield a second plasmid, pTM(P no C).

The L ORF of HPIV3 was cloned into pTM-1 in three parts: the ends were derived from PCR products and the main body was a restriction fragment from p218(131). The upstream end of the L ORF was amplified using the primers GCAAAGCGTGCCCGGGCC**ATG**GACACTGAATCTAACAATGGC (SEQ ID NO: 8) and GAAATTCCTTAATCGATTCTCTAGATTC (SEQ ID NO: 9). This yielded the 1,020-bp PCR product L1 in which positions 8625-9645 of the full-length genome were flanked by *Sma* I and *Nco* I sites on the upstream end and a *Cla* I site on the downstream end (all three sites are italicized). The downstream end of the L ORF was amplified using the primers CCCATCAACTGTAACA*TACGT*AAGAAAGAC (SEQ ID NO: 10) and GGTTAGGATAT*GTCGAC*ATTGTATTTATG (SEQ ID NO: 11). This yielded the 1,733-bp PCR product L2 in which positions 13, 645-15, 378 of the full-length genome were flanked by a SnaB I and *Sal* I site (italicized). Plasmid p (131) was digested with *Cla* I and *Pst* I to yield the 4,487-bp fragment L middle containing positions 9,630-14,120 of the full-Length genome. L1 and L middle were joined at the common *Cla* I site and cloned into the *Sma I-Pst* I window of pBluescript to yield p(L1+L middle). The L2 fragment was then cloned into the *Pst* I-*Sal* I window of p(L1+L middle) to yield the complete L ORF flanked by *Nco* I and *Sal* I. This was then cloned into the *Nco* I-*Sal* I window of pTM-1 to yield pTM (L). The sequences of PCR-generated regions of pTM(N) (SEQ ID NO: 20), pTM(P) (SEQ ID NO: 21), and pTM (L) (SEQ ID NO: 22) were confirmed by the dideoxynucleotide sequencing method.

To increase the efficiency of T7 transcription, certain modifications were made to a cDNA construct encoding a negative-sense PIV minigenome, called PIV3-CAT(-) (Dimock and Collins, J. Virol. 67: 2772-2778 (1993)). PIV3-CAT(-) includes the 3'-terminal 111 nucleotides and 5'-terminal 115 nucleotides of the HPIV3 genome fused to a negative-sense copy of the CAT ORF. This cDNA was designed to yield, upon linearization with *Hga*I and transcription with T7 RNA polymerase, a minigenome containing the exact correct ends of the HPIV3 genome. Two successive rounds of PCR, using mutagenic oligonucleotides (which added successive extensions to the cDNA end, were used to replace the *Hga*I site with the hepatitis delta ribozyme (Perotta and Been, Nature 350: 434-436 (1991)), such that self-cleavage generates the correct 3' HPIV3 genomic end. A T7 transcriptional termination signal was inserted immediately after the ribozyme (Fig. 3) to yield PIV3-CAT-delta.

PIV3-CAT-delta cDNA was modified by PCR mutagenesis to insert one, two or three G residues between the T7 promoter and the 5' end of the minigenome, using restriction sites flanking the trailer and T7 promoter. This modification yielded increased efficiency of T7 transcription. In preliminary experiments, the minigenome containing two G residues, called PIV3-CAT-GG, was the most active in the expression of CAT in the reconstituted transcription and replication system described below, and was used for all subsequent derivatives.

The PIV3-CAT-GG cDNA was further modified by overlapping PCR mutagenesis to introduce modifications simultaneously at two sites, as follows. First, the sequence T₇CT, which contains tandem transcriptional termination motifs for the vaccinia virus early-stage RNA polymerase (T₅NT) (Yuen and Moss, Proc. Natl. Acad. Sci. U.S.A. 84: 6417-6421 (1987), ), was inserted into the positive-sense strand cDNA strand between the delta ribozyme and T7 transcriptional terminator (Fig. 1). This motif, and a second motif described herein below, were added to prevent promiscuous transcription of the CAT gene by the vaccinia virus early RNA polymerase. Second, the minigenome cDNA insert was modified at the junction between the N nontranslated region and CAT gene to contain (i) the insertion of a second vaccinia virus termination motif (T₅AT) into the positive-sense cDNA strand at positions 103 to 109 relative to the encoded minigenome, and (ii) the insertion of 0 to 6 G residues (negative-sense) at minigenome position 112 (Fig. 3). The overlapping PCR mutagenesis involved three sets of reactions (PCR 1, 2 and 3) performed as follows. PCR 1 was a set of seven parallel reactions [PCR1 (0) to (+6)] which used the PIV3-CAT-GG cDNA as a template and the following two mutagenic oligonucleotides as primers: the forward primer was: ⁻¹¹¹GGGGTTATGCTACTGCAGGC**TTTTTTT**CTCCCTTAGCCATCCG⁻⁶² (SEQ ID :NO: 12) and the reverse primer was: ¹²⁴CTC*CATTCTAGA* (N) TT**ATAAAAA**TTATAGAGTTCCC^{90 (}SEQ ID NO:13). The bold sequence in the first oligonucleotide is the upstream tandem vaccinia terminator, and the bold sequence in the second oligonucleotide is the second terminator. This reaction amplified the ribozyme and adjacent leader region and inserted the mutations described above. PCR 2 was a single reaction that used the PIV3-CAT-GG cDNA as a template, and a forward primer that hybridized in plasmid sequence upstream of a unique *NgoM*I site (Fig. 3), and a reverse primer complementary to the forward primer of reaction one. Thus, the products of PCR 1 and 2 overlapped at this latter sequence. The products of PCR 1 (0) to (+6) and PCR 2 were gel purified. The products of PCR1 (0) to (+6) were each mixed separately with an aliquot of PCR 2 product and amplified in a third reaction (PCR3 (0) to (+6)) which also contained the forward primer of PCR 2 and the reverse primer of PCR 1. The products of PCR3 (0) to (+6) were digested with *NgoM*I, which cuts in plasmid-specific sequence, and XbaI, which cuts at the upstream end of the CAT gene (Fig. 3), and cloned into the *NgoM*I-*Xba*I window of PIV3-CAT-GG. This resulted in a panel of cDNAs encoding minigenomes which were named according to the number of inserted G residues: PIV3-CAT 0 to PIV3-CAT +6. The structures of all DNA regions derived from PCR were confirmed by dideoxynucleotide sequencing.

### C) Transfection

HEp-2 cells were grown to 90% confluence in 6 well plates. Each well of a six-well plate (1.5 X 10⁶ cells) was transfected with 0.4 µg pTM (P) , 0.4 µg pTM (N) , 0.05 µg pTM (L) , and 0.4 µg minigenome plasmid. The plasmids were added to 0.1 ml of OptiMEM (Life Technologies) and mixed with 0.1 ml of OptiMEM containing 12 µl of LipofectACE (Life Technologies). After an incubation period of approximately 15 minutes at room temperature, 0.8 ml of OptiMEM 1 containing 2% calf serum and 1.5 X 10⁷ pfu of vTF7-3 was added to each well. The plates were incubated at 37°C for 12 hours after which the media was replaced with fresh OptiMEM 1 containing 2% fetal bovine serum. The cells were then incubated at 37°C for a total of 48 hours and harvested for RNA analysis and CAT assay. Each minigenome was represented in triplicate (3 wells) which was scraped into the medium and pooled.

### D) CAT assay

An aliquot representing 3.33% (1.5 X 10⁵ cells) of each pooled sample of harvested cells described above was removed for CAT assay. The aliquot was centrifuged at 1,000 rpm for 5 minutes and the supernatant discarded. The cell suspension was washed with 1 ml of 40 mM Tris, pH 7.5, 1 mM EDTA, 150 mM NaCl and resuspended in 50 µl 0.25 M Tris, pH 7.5. Lysate was prepared by three cycles of freezing and thawing and clarified by centrifuging at 8,000 rpm for 5 minutes. 1 µl of lysate was assayed for the ability to acetylate D-threo-[dichloroacetyl 1-¹⁴C]chloramphenicol (Amersham) using a conventional assay (Gorman et al., Mol. Cell. Biol. 2: 1044-1051 (1982), ). Acetylation was visualized by thin-layer chromatography and quantified by phosphoimager analysis (Molecular Dynamics, Sunnyvale, CA).

### E) RNA analysis

The remaining cell harvest of each pooled sample was divided into three equal parts for isolation of encapsidated RNA, total RNA, and mRNA. The three aliquots were centrifuged at 1,000 rpm for five minutes and the supernatants discarded. Two aliquots of cell suspension were resuspended in 50 µl of RSB (10mM NaCl, 10mM Tris, pH 7.5, 1.5mM MgCl₂) containing 1% Triton X-100, 0.5% DOC. 50 µl of 10 mM Tris 7.5, 1 mM CaCl₂, and 20 µg (1mg/ml stock) of micrococcal nuclease was then added to one aliquot, and the other received the same mixture without micrococcal nuclease (Baker & Moyer, J. Virol. 62: 834-838 (1988)). The purpose of the micrococcal nuclease was to destroy nonencapsidated RNA, and the conditions used had been optimized in preliminary experiments. The mixtures were incubated at 30°C for 30 min and the RNA was isolated with Trizol (Life Technologies) according to the procedure of the supplier. The third aliquot of cell suspension was processed for RNA purification with Trizol and the purified RNA was separated by oligo(dT) cellulose chromatography into polyadenylated and nonpolyadenylated fractions (Grosfeld et al., J. Virol. 69 : 5677-5686 (1995)). RNA samples were run on 1.5% agarose gels containing 0.44 M formaldehyde, transferred to nitrocellulose (Chomczynski, Anal. Biochem. 201: 134-139 (1992)), hybridized with strand specific riboprobes, and quantified by phosphoimager analysis.

### Reference EXAMPLE III

### Construction and Expression of Modified PIV3 Minigenomes

In the present example, which is provided for reference only, a panel of cDNAs was constructed to encode PIV3 minigenomes which differed in length by single nucleotide increments. Transcription and RNA replication in this reconstituted system were the most efficient for the minigenome whose length was an even multiple of six. In this context, members of the *Paramyxovirus* and *Morbillivirus* genera typically abide by a "rule of six," i.e., genomes (or minigenomes) replicate efficiently only when their nucleotide length is a multiple of six (thought to be a requirement for precise spacing of nucleotide residues relative to encapsidating NP protein). However, the present findings illustrate that minigenomes whose lengths were one nucleotide greater than or less than an even multiple of six were surprisingly active, especially in RNA replication.

A panel of seven cDNAs was constructed to encode seven PIV3-CAT minigenomes, called PIV3-CAT 0 to +6, that differ in length by single-nucleotide increments (Fig. 3). Each minigenome is a short negative-sense analog of HPIV3 genomic RNA in which the viral genes had been deleted and replaced with a negative-sense copy of the CAT ORF. The CAT ORF is flanked by nontranslated segments of the N and L genes, the N GS and L GE transcription motifs, and the 3' leader and 5' trailer extragenic termini of genomic RNA. The 5' end of each PIV3-CAT minigenome is defined by the adjacent promoter for T7 RNA polymerase. This promoter was constructed to contribute an extension of two nonviral G residues to the 5' end of the encoded minigenome, as described above. The presence of additional G residues adjacent to the nontranscribed core of the T7 promoter improves its transcriptional efficiency, and preliminary work showed that presence of two G residues provided the highest levels of activity in the reconstituted system described below. These two G residues are not included in minigenome length calculations. The 3' minigenome end is created by self-cleavage by an abutting hepatitis delta virus ribozyme, which would generate the correct 3'-terminal nucleotide. The seven PIV3-CAT minigenomes differ by single-nucleotide increases in length due to the insertion of 0 to 6 G residues at the junction between the N nontranslated region and the CAT gene (Fig. 3), and range in length from 898 to 904 nucleotides. The PIV3-CAT +2 minigenome is the only one which is an even multiple of six (it should be noted that each minigenome contained two additional nonviral G residues at the 5' end contributed by the T7 promoter, but it is assumed that these would be lost during intracellular HPIV3-mediated RNA replication).

Each PIV3-CAT cDNA was transfected into HEp-2 cells that had been infected with vTF7-3, a vaccinia virus recombinant that expresses T7 RNA polymerase. Plasmids encoding the N, P and L proteins under the control of the T7 promoter were transfected in parallel. The P cDNA had been modified by site-directed mutagenesis to eliminate the translational start site of the C ORF, as described above. Cells were harvested at 48 h post-infection. An aliquot of the cell suspension was processed for CAT enzyme assay (Fig. 4). The remaining cells were divided into three equal aliquots and processed for RNA analysis as described below.

The minigenome cDNA was further modified to contain two tandem vaccinia virus early-gene transcription termination motifs (T₇NT) in the positive-sense plasmid strand upstream of the PIV3-CAT insert, and a third one (T₅AT) in the same strand immediately upstream of the CAT ORF (Fig. 3). These were designed to minimize promiscuous transcription of the CAT ORF by vaccinia virus polymerase (Grosfeld et al. (1995), supra). CAT expression was reproducibly detected when each of PIV3-CAT minigenomes was complemented by the N, P and L plasmids (Fig. 4), and detection of CAT was dependent on all three PIV3 proteins. However, expression was much higher for PIV3-CAT+2, which has a nucleotide length that is an even multiple of six. Preferred ratios and amounts of the minigenome and support plasmids were determined based on CAT enzyme expression.

### Reference EXAMPLE IV

### Synthesis of Positive-Sense RNAs by PIV Minigenomes

Transcription and replication of PIV minigenomes was confirmed in this example, which is provided for reference only, by detection of RNA products of both processes. As described in the foregoing Example, three equal aliquots of cell suspension were taken for RNA analysis. One aliquot was used for oligo(dT) analysis, as described below. The other two aliquots were lysed with detergent and incubated with micrococcal nuclease or mock-treated. RNA was then isolated, separated by electrophoresis on formaldehyde agarose gels, transferred to nitrocellulose, and analyzed by hybridization with negative-sense CAT riboprobe. RNA from micrococcal-treated and mock-treated lysates are shown in Fig. 5A upper and lower panels, respectively.

Analysis of RNA from mock-treated lysates showed that complementation of each minigenome with the N, P and L plasmids resulted in the synthesis of a band of RNA which was very similar in size to a marker consisting of RNA expressed by the 931-nucleotide RSV-CAT C2 minigenome (Grosfeld et al. (1995), supra). Phosphorimagery analysis is shown in Fig. 5B. Little or no RNA was detected when the N or L plasmids were omitted, confirming that these RNAs are products of the reconstituted PIV3 polymerase.

Each PIV3-CAT minigenome is expected to encode two positive-sense RNAs, namely the mini-antigenome and the subgenomic CAT mRNA. Each mini-antigenome is expected to be the exact complement of its minigenome, which was 898 to 904 nucleotides in length. The predicted subgenomic mRNA is defined by the GS and GE signals, and is expected to be 804 nucleotides in length and contain a polyA tail of 100 to 200 nucleotides.

Detection of a single gel band of positive-sense RNA in Fig. 5A (lower panel) suggested that the antigenome and mRNA were not resolved by gel electrophoresis. Accordingly, treatment with micrococcal nuclease was used to identify antigenome RNA, since the antigenome (and genome) but not mRNA would be encapsidated and resistant to digestion. The use of micrococcal nuclease for this purpose is well established (Baker & Moyer (1988), supra), and the conditions selected were verified with RSV minireplicons and shown to completely degrade mRNA contained in the HEp-2 cell lysates. Residual RNA was purified and analyzed by Northern blot analysis with negative-sense riboprobe (Fig. 5A, upper panel) and quantitated by phosphorimagery (Fig. 5B; note that in this analysis the micrococcal-treated and untreated RNA amounts were normalized separately). These investigations revealed the presence of a population of protected RNA corresponding to the positive-sense encapsidated mini-antigenome. Among several experiments, this protected RNA accounted for approximately 3 to 15% of the positive-sense RNA.

For both the total and the micrococcal-resistant RNA, accumulation was greatest in the case of the +2 minigenome, which is 900 nucleotides in length and thus a multiple of six. However, substantial amounts of RNA also accumulated in the case of the minigenomes which did not exhibit a length corresponding to a multiple of six nucleotides, in particular minigenomes +1 and +3 which were one nucleotide longer or shorter than the +2 minigenome. In fact, the amount of encapsidated antigenome produced by the +1 and +3 mingenomes was 85% and 72% that of the +2 minigenome (Fig. 5B). Even the least efficient minigenome, the +5 minigenome, was 20% as active as the +2 minigenome as determined by measurement of accumulated encapsidated RNA. In the case of measurements to detect total positive-sense RNA, the +1 and +3 minigenomes produced 52% and 45% as much total RNA as the +2 minigenome.

To confirm the presence of subgenomic mRNA, the final aliquot of harvested cell suspension was processed for RNA purification. The RNA was then subjected to oligo (dT) chromatography. RNAs which failed to bind, and those which bound and were eluted in low salt buffer, were analyzed by Northern blot hybridization (Fig. 6A) and phosphorimagery (Fig. 6B; note that in this case the bound and unbound are normalized together relative to the bound RNA of the +2 minigenome). These assays showed that approximately 64% of positive-sense RNA was polyadenylated, as expected for subgenomic mRNA. The accumulation of mRNA was greatest for the +2 minigenome. However, substantial amounts of mRNA also were observed for the other minigenomes. The amount of RNA synthesized by the +1 and +3 minigenomes was 30% and 20% respectively compared to that synthesized by the +2 minigenome, and was approximately 13% for the least active minigenomes.

### Reference EXAMPLE V

### Synthesis of Negative Sense RNA by PIV Minigenomes

This example is provided for reference only.

The various PIV3-CAT minigenomes described in the foregoing examples directed synthesis of mRNA and positive-sense encapsidated mini-antigenome, the latter representing the first step in RNA replication. The second step in RNA replication involves synthesis of encapsidated progeny minigenome from the mini-antigenome product. To evaluate this latter process, the samples of RNA from mock-treated and nuclease-treated lysates described in the preceding Example were analyzed by Northern blot hybridization with positive-sense CAT riboprobe (Fig. 7A) and quantitated by phosphorimagery (Fig. 7B).

Analysis of RNA from mock-treated lysates (Fig. 7A, lower panel) showed that considerable amounts of minigenome accumulated intracellularly in all samples, including negative controls in which the N or L support plasmid was omitted. The analyses described in Figs. 5A-B and 6A-B showed that the synthesis of positive-sense RNA was insignificant under these conditions. Therefore, the minigenome observed in the absence of N or L could not be the product of RNA replication mediated by the reconstituted HPIV3 polymerase, and instead must be the product of T7 transcription of transfected plasmid.

Minigenome produced by the reconstituted HPIV3 polymerase is expected to be encapsidated, whereas much of the minigenome produced by T7 RNA polymerase is expected to be unencapsidated. Therefore, RNA from the same micrococcal nuclease-treated samples described for Figs. 5A-B were used to prepare a second blot, which was hybridized with positive-sense CAT riboprobe (Fig. 7A, upper panel). This showed that all minigenome RNA accumulated in the absence of the N protein was degraded (Fig. 7A, upper panel, lane 1), as expected. Essentially all of the minigenome which accumulated in the absence of L was also sensitive to degradation (Fig. 7A, upper panel, lane 2). Plasmid-derived minigenome synthesized in the absence of L, and in the presence of N and P alone, did not appear to occur efficiently.

When the complete set of three support plasmids was present, significant amounts of micrococcal nuclease-resistant minigenome RNA accumulated for each of the minigenomes (Fig. 7A, upper panel). As was the case with the positive-sense RNAs, the greatest amount of progeny minigenome was observed with the +2 minigenome. The +1 and +3 minigenomes were next in abundance, with levels of genomic RNA that were 67% and 42% of that of the +2 minigenome.

The foregoing examples demonstrate that the HPIV3 N, P and L proteins were necessary and sufficient for efficient transcription and RNA replication. The very robust nature of transcription and RNA replication mediated by the reconstituted PIV3 polymerase confirmed the functionality of the encoded proteins. It is further expected that inclusion of additional viral proteins within the expression system will augment or modify these processes. Coexpression of PIV C, D and potentially V, within the compositions and methods of the invention will be useful, e.g., to augment and/or modify RNA replication. For this purpose, plasmids will be constructed and assayed according to the foregoing methods to achieve coexpression of one or more of these elements to determine their effects on PIV transcription and RNA replication, as well as on PIV phenotype in suitable infection models.

### EXAMPLE VI

### Construction of Infectious, Recombinant PIV from cDNA

The following examples describe production of infectious, recombinant PIV (rPIV) by intracellular co-expression of four plasmid-borne cDNAs. These cDNAs separately encode a complete HPIV3 genome and the HPIV3 nucleocapsid protein N, the phosphoprotein P, and the polymerase protein L.

### A) Viruses and cells

Modified vaccinia strain Ankara (MVA), the vaccinia virus recombinant that expresses bacteriophage T7 RNA polymerase, was provided according to Wyatt et al., Virol. 210:202-205, 1995.

HEp-2 monolayer cultures were maintained at 37° C in 5% CO₂ with OptiMEM 1 (Life Technologies) supplemented with 2% FBS, 50 µg/ml gentamicin sulfate and 2mM glutamine. The JS wt strain of HPIV3 and its attenuated ts derivative, JS *cp4*5, were propagated in LLC-MK2 cells as described by Hall et al., Virus Res. 22: 173-184, (1992).

### B) cDNAs

The full cDNA clone designated p218(131) (Fig. 1, SEQ ID NO: 1) encoding a complete genomic sequence of HPIV3 was constructed as described above. A second cDNA clone designated p3/7(131) (SEQ ID NO: 14) (deposited under the terms of the Budapest Treaty with the ATCC, and granted the designation 97990) was constructed to encode a complete antigenomic sequence of HPIV3. p3/7(131) differs from p218(131) in that the positions of the T7 promoter and the ribozyme/T7 terminator relative to the HPIV3 cDNA insert have been interchanged. Thus, the first nucleotide synthesized in p3/7 (131) is the 5' end of the antigenome, namely the positive-sense complement of genome position 1, and the 3' antigenome end defined by ribozyme cleavage is the complement of genome position 15462. A third clone designated p3/7(131)2G (SEQ ID NO: 15) (deposited under the terms of the Budapest Treaty with the ATCC, and granted the designation 97989) was also constructed identical to p3/7(131), except that two G residues were inserted between the 5' end of the antigenome and the T7 promoter.

For construction of p/37(131)2G and p3/7(131), the two plasmids p(A*A'CC'D*E) and p(E*FF'GHIJKL*) were modified and joined to encode the complete positive-sense antigenome of HPIV3. First, the T7 terminator and delta ribozyme abutting the 3' end of HPIV3 in p(A*A'CC'D*E) were replaced by a T7 promoter using PCR (see Fig. 8). The positive-sense primer 5'-GGCCCGTC*GACGCG***TAATACGACTCACTATA**GGACCAAACAAGAG-3' (SEQ ID NO: 18) placed the T7 promoter (bold) adjacent to the 3' leader of HPIV3 (HPIV3 sequence underlined) with 2 G residues inserted between these two elements to improve transcription. A unique *Mlu* I site (italicized) was placed upstream of the T7 promoter for cloning purposes (Fig. 8). The negative-sense primer 5' - ¹²²⁴CGGCAT*CACGTG*CTAC¹²⁰⁹- 3' (SEQ ID NO: 19) spanned nt 1209-1224 of the HPIV3 N gene and included a unique *Pml* I site (italicized) present in the natural HPIV3 sequence. Both the PCR product and the parent template p218 (A*A'CC'D*E) were digested with *Mlu* I and *Pml* I and the PCR product was then cloned into the *Mlu* I-*Pml* I window. A second PCR reaction was done using the same negative-sense primer and a positive-sense primer without 2G residues inserted between the T7 promoter and 3' end of HPIV3. These plasmids were designated p(Left + 2G) and p(Left +). The construction of p(Left + 2G) is illustrated in Fig. 8, the construction of p(Left +) followed the same strategy.

Plasmid p(E*FF'GHIJKL*) was modified by PCR to place the delta ribozyme and T7 terminator adjacent to the 5' end of HPIV3 (Fig. 9). The positive-sense primer: 5'-GGATTT*GCGCGC*¹⁴⁸¹³*AATTTAAAT*CATCTGG¹⁴⁸²⁸-3' (SEQ ID NO: 16) introduced a *BssH* II site (italicized) just upstream of a unique *Swa* I site (italicized, underlined) in the L gene of HPIV3 (HPIV3 specific sequence is underlined). The negative-sense primer: 5'-**CCCAGGT*CGGACCG*CGAGGAGGTGGAGATGCCATGCCAGCCC**¹⁵⁴³⁵ACCA AAACAAGAGAAGAACTCTGTTTGG¹⁵⁴³⁵- 3' (SEQ ID NO: 17) placed a portion of the delta ribozyme (bold) which included a naturally-occurring unique *Rsr* II site (italicized) adjacent to the 5' end of the HPIV3 genome (negative-sense underlined). This PCR product was digested with *BssH* II and *Rsr* II and cloned into the *BssH* II-*Rsr* II window of plasmid p3/7 (Fig. 9). Plasmid p3/7 is identical to p218 except that the delta ribozyme and T7 terminator are in the reverse orientation. The p3/7 plasmid containing the above-mentioned insertion was designated pPIV3-3/7 and contained the complete delta ribozyme and T7 terminator adjacent to 5' terminal 651 nucleotides of HPIV3. The *Swa*I and *NgoM*I fragment of plasmid pPIV3-3/7 was then isolated and cloned into the *Swa*I*-NgoM* I window of p(E*FF'GHIJKL*). The resulting plasmid, designated p(Right +) placed the complete delta ribozyme and T7 terminator adjacent to the 5' end of HPIV3 (see Fig. 9). The *Xho* I *-NgoM* I fragment of p(Right +) was cloned into the *Xho* I *-NgoM* I window of p(Left +) and p(Left + 2G) resulting in plasmids p3/7(131) (SEQ ID NO: 14) and p3/7(131 2G) (SEQ ID NO: 15), respectively (Fig. 10). These each encode the complete positive-sense analog of HPIV3 antigenomic RNA, with the latter cDNA containing two G residues adjacent to the T7 promoter for improved transcriptional efficiency.

### C) Transfection

HEp-2 cells were grown to 90% confluence in six well plates. Each well of a six-well plate (1.5 X 10⁶ cells) was transfected with the three previously-described support plasmids, 0.4 µg pTM (P), 0.4 µg pTM (N), 0.05 µg pTM (L) , together with 5 µg of full-length genomic or antigenomic HPIV3 cDNA. The plasmids were added to 0.2 ml of OptiMEM 1 (Life Technologies) containing 12µl of LipofectACE (Life Technologies). After an incubation period of approximately 15 minutes at room temperature, 0.8 ml of OptiMEM 1 containing 2% fetal bovine serum and 1.5 X 10⁷ pfu of MVA-T7 was added to each well. The cultures were incubated at 32°C for 12 hours, after which the media was replaced with fresh OptiMEM 1 containing 2% fetal bovine serum. The cultures were incubated at 32°C for three additional days then harvested and passaged (referred to as passage 1) onto fresh HEp-2 monolayers. These passage 1 cultures were incubated at 32°C for five days, and virus present in the culture was harvested, passaged once in LLC-MK2 cultures, and characterized by hemagglutination-inhibition (HAI) as described (van Wyke Coelingh et al., Virol. 143: 569-582, (1985)) to determine if it possessed the monoclonal antibody resistant mutation (MARM) that marked virus recovered from cDNA.

### D) Sequencing of Recombinant Virus

The presence of nucleotide sequence markers in the HN and L genes of recombinant PIV was determined by RT-PCR of RNA isolated from recovered virions. 1 ml of rPIV (1 X 10⁵pfu/ml, passage level 2) was precipitated with 200µl 25% polyethylene glycol by incubation on ice for one hour and centrifuging at 12,000g for 15 minutes. The RNA was purified with TRIzol reagent (Life Technologies) following the manufacturer's recommended procedure. RT-PCR was performed with the Superscript kit (Life Technologies) following the manufacturer's recommended protocol. Control reactions were identical except that reverse transcriptase was omitted from the reaction to confirm that the PCR products were derived solely from virus RNA and not from possible contaminating cDNA plasmids. Four primer pairs were used to generate PCR products from nt 7334-8715, 9364-10854, 10939-15392, and 13623-15392. The resultant PCR products were then sequenced using cycle dideoxynucleotide sequence analysis (New England Biolabs, Beverly, MA).

### EXAMPLE VII

### Recovery of Recombinant Virus From cDNA Encoding Negative-Sense Genomic RNA

Plasmid p218(131) and the three support plasmids pTM(N), pTM(P), and pTM (L) were transfected into HEp-2 cells with MVA expressing T7 RNA polymerase. A control group consisting of pTM(N), pTM(P), pTM(L), and MVA was cotransfected into HEp-2 cells without p218(131). On day four, the transfection was harvested, passaged onto fresh HEp-2 cell monolayers for five days, and passaged again for 5 days in LLC-MK2 cultures (passage 2). Virus present in the passage 2 harvest was further characterized by HAI. Cultures from the transfection group which received the three support plasmids without the full-length genomic clone p218(131) did not yield HPIV3. The rPIV recovered virus was confirmed to be HPIV3 since it reacted in the HAI assay with the mAbs 77/5, 101/1, and 454/11 which are specific for HPIV3 (Table 1). It was presumptively identified as being cDNA-derived because it failed to react with mAbs 170/7 and 423/6, consistent with the MARM mutation which had been introduced into the cDNA.

**TABLE 1**

| rJS-NS Contains the MARM Mutation Introduced into the p218(131) Full-Length Negative Sense cDNA | | | | | |
|---|---|---|---|---|---|
| | Hemagglutination-inhibiting | | Antibody Titer | (Reciprocal) of Indicated mAb | |
| Virus | 77/5¹ | 101/1¹ | 454/11¹ | 170/1² | 423/6² |
| JS wt³ | 800 | 6400 | 6400 | 25,600 | 25,600 |
| rJS-NS⁴ | 3200 | 25,600 | 6400 | ≤ 25 | ≤ 25 |

| | | | | | |
|---|---|---|---|---|---|
| ¹ mAb 77/5 recognizes antibody epitope IIB, mAbs 101/1 and 454/11 recognize antibody epitope IIIA of HN glycoprotein, all of which were not altered in p218(131). ² Mabs 170/7 and 423/6 which both recognize antibody epitope I of JSwt, fail to recognize rJS due to the MARM mutation at this site. ³ Biologically derived wild type HPIV3 JS. Recombinant JS virus derived from negative-sense genomic cDNA. | | | | | |

To confirm that rPIV was indeed recovered from cDNA, it was analyzed in parallel with wild-type JS strain HPIV3 by RT-PCR using four primer pairs flanking the seven inserted marker mutations. Each PCR product obtained was dependent upon the inclusion of RT, indicating that each was derived from RNA and not from contaminating cDNA. Cycle-sequencing of the four PCR products confirmed that the sequence of the rPIV contained each of the seven markers, sequencing data showing three of the markers is illustrated in Figure 11. The sequence differences between rPIV and JS wt in the HN gene, including nt 7903, 7913, and 7915 are readily apparent. Similar sequence analyses were carried out for the other four markers at nt positions 7593, 10355, 11333, and 15248, and the rPIV possessed each mutation.

These results demonstrate successful recovery of infectious rPIV from cDNA encoding a negative-sense genomic RNA. This differs from most published reports for recovery of nonsegmented negative strand RNA viruses, in which the cDNA used for virus recovery had been designed to encode positive-sense antigenomic RNA (Baron and Barrett, supra, 1997; Collins et al., supra, 1995; Conzelmann, supra, 1996; Garcin et al., supra, 1995; Lawson et al., supra, 1995; Radecke et al., supra, 1995; Whelan et al., supra, 1995). The recovery of infectious virus from a cDNA encoding genomic RNA had previously been reported only in the case of Sendai virus, and the efficiency of recovery was much lower than for cDNA encoding antigenomic RNA (Kato et al., 1996). In most other studies, the recovery of virus was achieved with antigenomic cDNA but not with genomic cDNA (Lawson et al., 1995; Whelan et al., 1995). A number of potential problems have been noted which may explain these refractory results, including possible annealment of cDNA-encoded genomic RNA with mRNA produced by the support plasmids; resulting in inactive hybrids (Conzelmann, supra, 1996; Lawson et al., supra, 1995). It has also been noted that T7 RNA polymerase appears to terminate preferentially at the gene junctions of genomic RNA, perhaps because the oligo U tract of the GE signal resembles the natural signal for transcription termination by T7 RNA polymerase (Whelan et al., supra, 1995).

### EXAMPLE VII

### Recovery of Recombinant Virus From cDNA Encoding Positive-Sense Antigenomic RNA

As described in more detail above, p3/7(131) and p3/7(131)2G were constructed to encode a positive-sense, antigenome that give rise to recombinant PIV. Plasmid p3/7(131)2G is identical to p3/7(131) but for the addition of two G residues between the T7 promoter and the first nucleotide of the antigenome. The addition of two G residues between the T7 promoter and the first HPIV3 nucleotide p3/7(131)2G is based on the preceding examples demonstrating that the presence of the two added G residues (as opposed to O, 1 or 3 added G residues) yielded substantially increased levels of minireplicon replication.

The two antigenome cDNAs (p3/7(131) and p3/7(131)2G] were transfected separately into cells together with the N, P and L support plasmids, and were infected simultaneously with the MVA-T7 recombinant virus using the same procedure described above for p218(131). Infectious virus from each antigenomic cDNA was recovered and was presumptively identified as being cDNA-derived by its inability to react with mAbs 423/6 and 170/7.

The efficiency of virus recovery was evaluated for each of the antigenome cDNAs versus the genome cDNA p218(131). Twelve transfection reactions using the negative-sense genome cDNA p218(131) (SEQ ID NO: 1) were conducted in parallel with twelve transfections using the positive-sense antigenome cDNA p3/7 (131) 2G (SEQ ID NO: 15) to compare efficiency of virus recovery from the two cDNAs. One ml of the transfection harvest from each well was titered on LLC-MK2 cells and the remaining 2mls were passaged (passage 1) onto fresh LLC-MK2 cells. At day five, passage 1 was harvested and titered as described above. Recombinant virus was recovered from 12/12 wells transfected with p3/7(131)2G but from only 4/12 wells transfected with p218 (131). The mean titer of virus present in culture of the passaged virus from the positive-sense antigenome was 10^{5.0}, nearly ten-fold higher than that from the negative-sense genome, which was 10^{4.1}. However, with one additional amplification the titers became equivalent.

**TABLE 2**

| Comparison of the Efficiency of Recovery of Recombinant Virus from cDNAs Encoding Genomic or Antigenomic RNA, the latter with or without 2G residues adds to the T7 promoter. | | | | |
|---|---|---|---|---|
| Experiment# | Transfected cDNA | Sense of Encoded RNA | Rescue Efficiency¹ | Mean Titer (log₁₀ pfu/ml) of Recovered Virus² |
| #1 | p3/7 (131) 2G³ | Antigenomic | 12/12 (100%) | 5.0 ± 0.27 |
| | p218(131) | Genomic | 4/12 (33%) | 4.1 ± 0.27 |
| #2 | p3/7 (131) 2G³ | Antigenomic | 6/6 (100%) | 6.6 ± 0.18 |
| | p3/7(131) | Antigenomic | 12/12 (100%) | 5.3 ± 0.12 |
| | p218(131) | Genomic | 5/5 (100%) | 4.9 ± 0.19 |

| | | | | |
|---|---|---|---|---|
| 1 Number of transfection cultures yielding rJS/number tested. 2 The mean Titer ± standard error was determined following one passage of transfection harvest for five days in LLC-MK2 cells. 3. Contains 2 G residues between the T7 promoter and the 5' end of the antigenome. | | | | |

The efficiency of recovery of recombinant virus from the three full-length plasmids encoding the genomic or antigenomic HPIV3 RNAs was next studied, (i) to determine whether genomic or antigenomic cDNA is more efficient at generating recombinant virus and (ii) to determine the effect of two extra 5' terminal G residues on the yield of recombinant viruses (Table 2.) Unfortunately, it was not possible to directly titer the transfection harvest by plaque titration because residual MVA-T7 interfered with plaque formation of rJS on LLC-MK2 monolayer cultures. Therefore, we first compared the efficiency of recovery of rJS from multiple independent transfections and, as seen in other experiments, found that rJS was more frequently recovered from cultures transfected with p3/7(131)2G than from cultures transfected with p218(131). Since each transfection harvest, including those with p218(131) yielded rJS in other experiments, it was not possible to use this method of analysis to compare the relative efficiency of generating recombinant virus. We therefore estimated the relative quantity of virus present in the transfection harvest by quantitating the amount of virus following one passage of the transfection harvest (Table 2). Since each plasmid was designed to yield an identical virus (rJS), differences in titers of the passaged virus were considered a reflection of differences in the titer of virus present in transfection harvest. In the above experiments, the construct with two 5' terminal G residues was the most efficient in generating rJS. This indicated that the two added residues indeed increased efficiency of recovery, but the mechanism for this small increment in efficiency was not defined. These findings indicate a small, but measurable, advantage of using p3/7(131)2G as substrate for future experiments designed to introduce mutations into the HPIV3 cDNA. The small advantage in recovery of recombinant virus from p3/7(131)2G might be especially needed to recover viruses that have attenuating mutations that restrict replication in vitro. Although VSV has been recovered from antigenomic cDNA which included three G residues at the 5'-terminal end, the efficiency of recovery of virus from this construct was not compared with recovery from the same construct lacking the extra residues (Whelan et al., Proc. Natl. Acad. Sci. USA 92:8388-8392 (1995),). In contrast to the experience with Sendai virus and measles virus, it is clear that extra-viral G residues at the 5'-terminal end of the antigenome are not deleterious to recovery of recombinant virus, and, in fact, appear to be advantageous [Garcin et al., supra, 1995; Radecke et al., supra, 1995; Kato et al., 1996). The genomic and antigenomic plasmids without two 5'-terminal guanine residues appeared equally efficient in generating recombinant virus. This is in contrast to previous reports which have found genomic cDNA to be less efficient than antigenomic cDNA in generating recombinant virus (Whelan, 1995; Kato et al., 1996).

The efficiency of recovery of virus from p3/7(131) (SEQ ID NO: 14) was compared to that of p3/7(131)2G (SEQ ID NO: 15 and p218(131) (SEQ ID NO: 1) in a similar experiment as above. Recovery from p3/7(131) was comparable to that of p217(131). Recovery was more consistent and efficient with both antigenome cDNAs containing the extra Gs (Table 2).

### EXAMPLE VII

### Efficiency of plaque formation of rPIV at permissive and restrictive temperatures

The level of temperature sensitivity of replication in vitro of control viruses and rPIV derived from both p218(131) and p3/7(131)2G was determined at 32°C, 37°C, 39°C, and 40°C in LLC-MK2 monolayer cultures as previously described (Hall et al., Virus Res. 22: 173-184, (1992)), with the following modifications. Viruses were inoculated onto LLC-MK2 monolayers on 24-well plates in serial 10-fold dilutions allowed to adsorb for one hour at room temperature. The cultures were then overlaid with 1ml of L-15 supplemented with 2mM glutamine and 0.8% methylcellulose and the plates were then incubated for 5 days at the indicated temperature. The methylcellulose overlay was removed and the monolayer fixed with 80% methanol at 4°C for 1h. The viral plaques present in the monolayer were scored using a mixture of two HPIV3-specific anti-HN murine mAbs 101/1 and 66/4 as ascites fluid used at a dilution of 1:500, using an immunoperoxidase method of staining specific for murine antibodies as previously described (Murphy et al., Vaccine 8 : 497-502 (1990)).

Recombinant virus derived from either positive or negative-sense cDNA were characterized by plaque assay at 32°C, 37°C, 39°C, and 40°C to determine if they were phenotypically similar to JS wt virus. Both positive and negative-sense rPIV were comparable to the JS wt virus in their level of replication at elevated temperatures of 39°C and 40°C (Table 3). This is in contrast to the *ts* mutant JS *cp*45 which exhibits a 30-fold reduction in titer at 37°C and fails to produce plaques at 39°C or 40°C.

**TABLE 3**

| The rJS Resembles its Biologically Derived Parent JS Wild-Type Virus in the Level of Replication at Restrictive Temperature (39°C - 40°C) | | | | |
|---|---|---|---|---|
| | | Virus Titer (log 10 pfu/ml) | | |
| Virus | 32°C | 37°C | 39°C | 40°C |
| rJS-PS¹ | 6.1 | 6.1 | 6.1 | 6.6 |
| rJS-NS² | 6.9 | 7.1 | 7.1 | 7.0 |
| JS*cp*45³ | 6.3 | 4.3 | <0.7 | <0.7 |
| JS wt | 6.5 | 6.8 | 6.6 | 6.7 |

| | | | | |
|---|---|---|---|---|
| ¹ Recombinant virus derived from the antigenomic-sense clone p3/7(131)2G ² Recombinant virus derived from the genomic-sense clone p218(131) ³ JS*cp*45 is a temperature sensitive mutant derived from JS wt. | | | | |

The sequence of JS *cp*45 has been fully determined (Stokes et al., supra, 1993) and mutations have been identified in the leader, N, P, M, F, HN, and L genes. However, it is unknown which mutation(s) are responsible for the *ca*, *att*, or *ts* phenotypes. Because exemplary rPIV of the invention demonstrate the *ts*⁺ phenotype like the JS wt parent, *cp*45 mutations among other mutations known or yet to be discovered for PIV can be introduced, alone or in combination, into the full-length cDNA to pinpoint the effects of individual mutations or combinations thereof, e.g., by evaluating replication of the recombinant virus incorporating the mutation(s) of interest at elevated temperatures. The mutation(s) thus identified can be incorporated into effective, attenuated vaccine agents as described in the Examples below. These and other mutations incorporated into recombinant PIV can be further optimized by, e.g., mutagenesis to create two or more nucleotide substitutions per codon to render a recombinant virus that is more genetically stable than a biologically derived mutant strain.

### EXAMPLE VIII

### Replication of rPIV in Hamsters

Thirty-six 16-week-old golden Syrian hamsters were divided into four groups of nine and inoculated intranasally with 0.1ml containing 10^{5.5} pfu of either rPIV recovered from negative-sense cDNA, rPIV recovered from positive-sense cDNA, JS *cp*45, or JS wt virus. On day 4, the hamsters were sacrificed and the lungs and nasal turbinates harvested. The lungs were homogenized in a 20% w/v L-15 suspension containing 2.5µg/ml amphotericin B (Quality Biologicals, Gaithersburg, MD), 200µg/ml pipericillin (Lederle Laboratories, Pearl River, NY), and 50µg/ml gentamicin (Quality Biologicals). The nasal turbinates similarly were homogenized in a 10% w/v L-15 suspension. After homogenization, the samples were aliquoted and rapidly frozen in a dry ice-ethanol bath. Virus present in the samples were titered at a later date in 96 well plates of LLC-MK2 cells at 32°C scoring CPE at five and seven days after inoculation. The mean log₁₀ TCID₅₀/gm was calculated for each group of nine hamsters.

Table 4 illustrates that rPIV recovered from negative-sense cDNA, rPIV recovered from positive-sense cDNA replicate to substantially the same level as the JS wt in the upper and lower respiratory tract of hamsters. This is in contrast to the JS *cp*45 virus, which is attenuated at each site.

**TABLE 4**

| The rJS Resembles its Biologically Derived Parent JS wt Virus in the Level of Replication in the Upper and Lower Respiratory Tract of Hamsters. | | |
|---|---|---|
| | Mean Virus Titer (log₁₀ TCID₅₀/g)⁵ | |
| Virus | Nasal Turbinates | Lungs |
| rJS-PS¹ | 6.6 ± 0.2 | 4.1 ± 0.3 |
| rJS-NS² | 6.4 ± 0.1 | 4.2 ± 0.2 |
| JS*cp*45³ | 4.2 ± 0.2 | ≤ 1.4 ± 0.0 |
| JS wt⁴ | 6.3 ± 0.2 | 4.6 ± 0.3 |

| | | |
|---|---|---|
| ¹ Recombinant virus recovered using p3/7(131)2G encoding the positive-sense HPIV3 antigenome. ² Recombinant virus recovered using p218(131) encoding the negative-sense HPIV3 genome. ³ Biologically derived *ts* mutant. ⁴ Biologically derived parent virus. ⁵ Mean titers ± standard errors for nine hamsters per group. | | |

Thus, exemplary rPIVs of the invention can retain the replicative capacity in hamsters exhibited by the biologically derived JS wt parent strain, whereby mutations such as those present in the JS *cp*45 candidate vaccine that restrict replication in hamsters and other hosts, including non-human primates and humans, can be identified and incorporated within modified rPIV strains of the invention, as described in the Examples below.

### EXAMPLE IX

### Identification of Amino Acid Substitutions in HPIV3 Specifying Attenuated Phenotypes, and Incorpration of Attenuating Mutations into Infections, Attenuated PIV Clones

The ability to generate infectious PIV from cDNA facilitates development of live-attenuated parainfluenza virus vaccines. More specifically, by using the methods and tools disclosed herein the genetic basis of attenuation of PIV candidate vaccines can be readily determined, and infectious PIV vaccines produced from cDNA can be designed to achieve a finely calibrated level of attenuation and immunogenicity.

In addition, the tools and methods of the invention provide for vaccine development for all three human parainfluenza viruses, HPIV1, HPIV2 and HPIV3 that are most important in human disease. For example, to produce and select effective HPIV3 vaccine agents within the invention, mutations associated with desired phenotypes of biologically deriving HPIV3 candidate vaccines or the attenuated BPIV3 virus, e.g. attenuating mutations, can be identified and incorporated into rPIV. Applying these methods, attenuating mutations from a large menu of candidate mutations are selected and combined to generate rPIV having a desired balance between attenuation and immunogenicity, and which retain the attenuation phenotype following replication in humans.

In the present example, the genetic bases of temperature-sensitive (*ts*) and *in vivo* attenuation (*att*) phenotypes of the PIV3 JS *cp*45 live-attenuated virus are described. Seven exemplary recombinant PIV3 viruses (three single-, three double-, and one triple-lesioned virus) were recovered from full-length antigenomic cDNA and analyzed for their *ts* and *att* phenotypes. These recombinants bore one or more amino acid substitution mutations present in the L gene of JS *cp*45 (alternatively referred to herein as *cp*45), adopted within a cDNA clone of the JS wt parent. These three exemplary, biologically derived mutations are all present in a representative strain of JS *cp*45 grown in Vero cells, designated JS *cp*45 Vero, deposited on August 21, 1997 under the terms of the Budapest Treaty with the American Type Culture Collection (ATCC) of 12301 Parklawn Drive, Rockville, Maryland 20852, U.S.A., and granted the accession number ATCC VR 2588.

Analyses of exemplary PIV recombinants, presented below, demonstrate that each of the three exemplary mutations in L (Tyr₉₄₂ to His, Leu₉₉₂ to Phe, and Thr₁₅₅₈ to Ile) contribute to the *ts* and *att* phenotypes of *cp*45 and are useful for generating of recombinant vaccine virus.

### Viruses and cells.

The PIV3 JS wt and *cp*45 viruses were grown in LLC-MK2 cells as described previously (Hall et al., Virus Res. 22:173-184 (1992)). The vTF7-3 recombinant vaccinia virus is described in Fuerst et al., Virology 225: 419-422 (1996) and the modified vaccinia virus Ankara (MVA) which expresses the T7 polymerase is described in Wyatt et al., Virology 210:202-205 (1995).

HEp-2 (ATCC CCL 23) and LLC-MK2 (ATCC CCL 7.1) cells were maintained in OptiMEM (Life Technologies) supplemented with 2% FBS and gentamicin sulfate (50ug/mL).

### Construction of point mutations in the L gene of PIV3.

pUC19 was modified to accept a fragment of the JS wt PIV3 L gene in order to introduce point mutations into the L gene by site-directed mutagenesis. First, a unique *Nhe* I restriction site was introduced into pUC19 by ligating a pair of complementary oligonucleotides (5' GATCGATGCTAGCCC 3' (SEQ ID NO: 23) and 5' GATCGGGCTAGCATC 3' (SEQ ID NO: 24)) containing an *Nhe* I restriction site into the *Hind* III site of pUC19 to create pUC19 (N). The *Sph* I (PIV3 nt 11317) to *Nhe* I (PIV3 nt 14087) fragment of pTM(L), which includes the positions where the three coding changes in *cp*45 occur and which can be directly introduced into the full-length PIV3 cDNA (see below), was cloned into the *Sph* I and *Nhe* I site of pUC19 (N) to create pUCL(N-S). Point mutations were introduced into pUCL(N-S) using mutagenic oligonucleotides with the Transformer mutagenesis kit (Clontech, Palo Alto, CA) for the purpose of (i) creating exemplary amino acid substitutions at L protein positions 942, 992, and 1558, individually and in combination, and (ii) ablating one specific naturally-occurring restriction enzyme site proximal to each codon substitution as a marker [See Table 5].

**TABLE 5: Nucleotide substitutions introduced into rPIV3 that encode cp45L protein gene amino acid substitutions and, as markers, ablate naturally-occurring restriction enzyme sites.**

| rPIV3 designation | Amino Acid Substitution (wt to *cp*45) | Sequence of wt^{a} | Sequence of Mutant | Restriction Enzyme site Ablated |
|---|---|---|---|---|
| r942 | Tyr-942 to His | 11468-T**TAC**A*TGGCCA*T (SEQ ID NO: 25) | 11468-TC**A**CATGGCGAT (SEQ. ID NO: 26) | *Eae* I |
| r992 | Leu-992 to Phe | 11618-T**TTT**GATTGGGC (SEQ ID NO: 27) | 11618-T**TTT**GATTGGGC (SEQ. ID NO: 28) | *Bsr* I |
| r1558 | Thr-1558 to Ile | 13307-T*GGTCC*TAAT**ACT**G (SEQ. ID NO: 29) | 13307-TGGGCCTAAT**A**TCG (SEQ. ID NO: 30) | *Ava* II |

| | | | | |
|---|---|---|---|---|
| a. The nucleotide sequence around each of the three mutated regions is shown. The first nucleotide in each provided sequence is numbered according to its position in the complete antigenomic RNA. The codon involved in each amino acid substitution is in bold. Naturally-occurring restriction enzyme sites present in the wt sequence, and which were ablated to mark the mutation, are in italics. The nucleotides that were mutated to produce an aa substitution or remove a restriction enzyme site are underlined. | | | | |

Mutations introduced in pUCL(N-S) derivatives were verified by dideoxynucleotide sequencing of plasmid DNA. The *Sph* I to *Bam*HI (nt 13733) fragment of pUCL(N-S) containing the *cp*45 individual L gene mutations was subcloned into the *Sph* I to *Bam*HI sites of pTM(L) to give pTM(L)-942, -992, -942/992, and -1558; the other double and triple mutations were assembled using the *Pin AI* and *Nhe I* sites (Fig. 12). The mutant pTM(L) plasmids were each tested at permissive temperature (32°C) for the ability to direct the expression of the chloramphenicol acetyl transferase marker gene in a minireplicon system comprising a plasmid-encoded minigenome RNA and the N, P and L proteins (Durbin et al., Virology 234:74-78 (1997)). The various mutant L plasmids supported marker gene expression to 75-106% the level of wt L, indicating that each engineered cDNA was free of significant spurious mutation (not shown). The *Sph* I to *Nhe* I fragments of each of the mutant pTM(L) plasmids were then subcloned into the *Sph* I to *Nhe* I window of the full-length PIV3 JS antigenomic cDNA p3/7(131)2G to create seven full-length PIV3 cDNA clones representing every possible combination of the three substitutions.

### Recovery of recombinant PIV3 (rPIV3) bearing one, two or three cp45 L protein substitutions.

Each full-length antigenomic cDNA bearing one or more *cp*45 L gene mutations, together with the three support plasmids pTM(N), pTM(P) and pTM(L), was transfected into HEp-2 cells on 6-well plates (Costar, Cambridge, MA) using LipofectACE (Life Technologies) and MVA-T7 as described above. After incubation at 32°C for 4 days, the transfection harvest was passaged onto HEp-2 cells on 6-well plates which were incubated at 32°C for 4 days. Each passage 1 supernatant was harvested and passed onto a T-25 flask of LLC-MK2 cells, which was incubated at 32°C for 5-6 days. The passage 2 supernatant was harvested and the presence of recombinant virus was initially confirmed by immunoperoxidase staining of virus plaques (Murphy et al., Vaccine 8:497-502 (1990)), with anti-HN monoclonal antibody (Mab) 77/5, which binds to both biologically derived and recombinant JS PIV3, and Mab 423/6, which does not bind to cDNA-derived virus because its epitope was ablated to serve as a marker. Virus present in passage 1 was subjected to two or three rounds of plaque purification on LLC-MK2 cells as described previously. Each biologically cloned recombinant virus was amplified twice in LLC-MK2 cells at 32°C to produce virus for further characterization. Virus was concentrated from clarified medium by polyethylene glycol precipitation, and viral RNA (vRNA) was extracted with Trizol Reagent (Life Technologies). Reverse transcription (RT) was performed on vRNA using the Superscript II kit with random hexamer primers (Life Technologies). The Advantage cDNA PCR kit (Clontech, Palo Alto, CA) and sense (5' nt 11190-GCATTATCTAGATGTGTCTTCTGGTCAGAG 3' nt-11219) (SEQ ID NO: 31) and antisense (5' nt 14140-CCTGAATTATAATAATTAACTGCAGGTCCT 3' nt-14111) (SEQ ID NO: 32) primers specific for the PIV3 L gene were used to amplify the region spanning the *Sph* I to *Nhe* I fragment. The PCR fragments were analyzed by digestion with each of the restriction enzymes whose recognition sites had been ablated during insertion of the three *cp*45 mutations in L (see Table 5).

### Efficiency of plaque formation (EOP) at permissive and restrictive temperatures of rPIV3 bearing one, two or three cp45 L protein amino acid substitutions.

The level of temperature sensitivity of plaque formation in vitro of control and recombinant viruses was determined at 32°C, 37°C, 38°C, 39°C, 40°C, and 41°C in LLC-MK2 monolayer cultures and plaques were enumerated by hemadsorption with guinea pig red blood cells following removal of the methylcellulose overlay. Alternatively, viral plaques present in the monolayer were identified by immunoperoxidase staining with a mixture of two PIV3-specific anti-HN murine mAbs 101/1 and 454/11 diluted 1:500, (Murphy et al., supra, (1990)).

### Hamster studies.

4 to 16 week-old golden Syrian hamsters in groups of six were inoculated intranasally with 0.1ml OptiMEM1 per animal containing 10^{5.5} pfu of rPIV3 JS wt, PIV3 *cp*45 virus, or one of the rPIV3 containing one or more *cp*45 L protein substitution(s). On day 4 post-infection, the hamsters were sacrificed, the lungs and nasal turbinates were harvested, and the virus was quantified as described above. The mean log₁₀ TCID₅₀/g was calculated for each group of six hamsters.

### RESULTS

### Introduction of the PIV3 cp45 L protein amino acid substitutions into wt JS rPIV3.

As noted above, the three amino acid substitutions present in the L protein of *cp*45 (Table 5) were introduced individually or in selected combinations into the antigenomic cDNA that encodes its wt parent, PIV3 JS strain. Each introduced mutation was engineered to be marked with a silent mutation that ablated a proximal, naturally-occurring restriction enzyme site to facilitate monitoring the mutation in recovered rPIV3 (Table 5, Fig. 12). The coding change at amino acid 1558 was designed to contain two nucleotide changes in r1558, compared to the one nt substitution in *cp*45, to reduce the chance of reversion at this site during *in vitro* or *in vivo* replication.

Seven rPIV3s bearing one, two or all three of the amino acid substitutions from *cp*45 were recovered in tissue culture by transfection of each antigenomic cDNA together with the pTM(N), pTM(P) and pTM(L) support plasmids and coinfection with the vaccinia virus MVA/T7 pol recombinant (Wyatt et al., supra, (1995)). Each rPIV3 possessed the Mab resistance marker that had been deliberately introduced into the HN gene by engineering the antigenomic cDNA. The rPIV3s were biologically cloned by two or three cycles of plaque to plaque passage to ensure that each virus preparation was genetically homogeneous. This precaution was taken because vaccinia virus can meditate recombination between the antigenomic cDNA and the support plasmids.

To confirm that each of the seven rPIV3 contained the engineered mutation(s) in the L gene, RNA was purified from precipitated virions and was copied into cDNA and amplified by RT-PCR. Control reactions showed that the RT step was required for generation of RT-PCR products, indicating that an RNA template rather than contaminating cDNA was required for the generation of the RT-PCR product. The RT-PCR products were subjected to digestion with the three restriction enzymes whose recognition sequences had been ablated as markers for the inserted coding changes. As expected, the RT-PCR product of JS wt rPIV3 was cleaved the appropriate number of times by each of the three enzymes, whereas r942/992/1558 lacked each of the three sites ablated during creation of the individual *cp*45 coding changes. Each of the other rPIV3s lacked the appropriate restriction site(s), indicating the presence of the introduced mutations.

### Efficiency of plaque formation at 32°C, 37°C, 38°C, 39°C, 40°C, and 41°C of rPIV3 bearing cp45 L mutations.

The seven rPIV3s bearing the various combinations of *cp*45 L protein amino acid substitutions were assayed for their ability to form plaques on LLC-MK2 monolayers at 32°C, 37°C, 38°C, 39°C, 40°C and 41°C. As shown in Table 6, each rPIV3 bearing a *cp*45 aa substitution was *ts*, whereas the JS wt rPIV3 parent was not restricted in plaque formation at any temperature tested. The shut-off temperature of plaque formation of r942, r992 and r1558 was 40°C. r942 manifested a 700-fold reduction of plaque formation at 40°C, indicating that its replication was marginally reduced at this restrictive temperature. However, the plaque size of r942 also was greatly reduced at 40°C, which also indicates that its replication was restricted at this temperature compared to the JS wt. r942 was completely restricted in replication at 41°C (data not shown). r992 and r1558 were greatly reduced (over a 1,000,000-fold reduction) in plaque formation at 40°C. These results indicate that each of the three *cp*45 L gene mutations individually specifies the *ts* phenotype, although that of the r942 mutation is somewhat less restrictive. The double mutant virus r942/1558 and the triple mutant r942/992/1558 had a shut-off temperature of 39°C, while that of r942/992 and *cp*45 was 38°C. The double mutant, r992/1558, was less *ts* than the r992 single mutant. r992/1558, like r942, was completely restricted in plaque formation at 41°C. The level of temperature sensitivity exhibited by double or triple results from a rPIVs delicate interplay of the three mutations that cannot be predicted from level of temperature-sensitivity exhibited by the single mutants. Also, since r942/992/1558 was slightly less *ts* than *cp*45, other mutations outside of the L gene also likely contribute to the *ts* phenotype of *cp*45, therefore representing additional mutations of interest within the invention.

**Table 6: The efficiency of plaque formation (EOP) at 32°C, 37°C, 38°C, 39°C and 40°C of rPIV3 bearing one, two or three cp45 L protein amino acid substitutions.**

| Virus^{a} | | | Virus titer (log₁₀ | | |
|---|---|---|---|---|---|
| | pfu/ml) | | | | |
| | 32°C | 37°C | 38°C | 39°C | 40°C |
| r942 | 6.8 | 6.8^{b} | 6.6^{b} | 6.5^{b} | 4.3^{b} |
| r992 | 6.9 | 6.8^{b} | 6.7^{b} | 6.1^{b} | <0.7 |
| r1558 | 6.6^{b} | 6.6^{b} | 6.4^{b} | 5.0^{b} | <0.7 |
| r942/992 | 6.7 | 6.5^{b} | 4.5^{b} | 3.0^{b} | <0.7 |
| r942/1558 | 5.2 | 5.0^{b} | 4.0^{b} | 1.0^{b} | <0.7 |
| r992/1558 | 6.7 | 6.7^{b} | 6.5^{b} | 5.9^{a} | 5.1^{b} |
| r942/992/1558 | 6.6 | 6.2^{b} | 6.2^{b} | 2.7^{b} | <0.7 |
| *cp*45^{b} | 6.6 | 4.8 | 4.5^{b} | <0.7 | <0.7 |
| rPIV3 JS | 8.3 | 8.4 | 8.5 | 8.5 | 8.3 |

| | | | | | |
|---|---|---|---|---|---|
| a. The *cp*45 virus is a biologically derived virus, and each of the other viruses tested is a recombinant. b. Plaques were of pinpoint size. c. Underlined numbers represent the shut-off temperature of plaque formation, which is defined as the lowest restrictive temperature at which a 100-fold reduction in titer is observed compared to the titer at 32°C. | | | | | |

**Growth in hamsters**. Groups of six Golden Syrian hamsters were inoculated intranasally with JS wt rPIV3, biologically-derived *cp*45, or with rPIV3 containing one or more *cp*45 L protein amino acid substitutions, and virus replication in the lungs and nasal turbinates was determined. In this experiment [Table 7], each of the rPIV3s bearing a single amino acid substitution was restricted in replication in the upper and lower respiratory tract [Table 7]. However, r942, the least *ts* virus, was only marginally suppressed in replication in the upper and lower respiratory tract in a second experiment. These data demonstrate that two of the three amino acid substitutions contribute to the *att* phenotype when present as single-lesioned recombinant viruses. However, the 942 mutation indeed contributes to attenuation (e.g., the r942/992 is more attenuated than r992 alone). Thus, each of the amino acid substitutions in L contribute to the *att* phenotype either acting alone or in concert with another L amino acid mutation. Each of the double mutants was attenuated indicating that loss of any of the three L gene substitutions following replication in vivo still leaves an attenuated virus. This is a partial explanation for the previously observed high level of stability of the *ts* phenotype of the *cp*45 following replication in vivo. The triple mutant r942/992/1558 was as restricted as *cp*45 for replication in the upper and lower respiratory tract indicating that the three amino acid substitutions in the L protein are the major contributors to the *att* phenotype of *cp*45.

**Table 7: The level of replication in the upper and lower respiratory tract of hamsters of rPIV3 bearing one, two or three cp45 L protein amino acid substitutions, compared to JS wt rPIV3 and cp45^{a}.**

| Virus | Mean virus titer (log₁₀ TCID₅₀/g ± S.E^{b}) | |
|---|---|---|
| | Nasal turbinates | Lungs |
| rPIV3 wt | 7.4 ± .16 | 5.1 ± .49 |
| r942 | 6.6 ± .17 | 3.0 ± .78 |
| r992 | 4.4 ± .16 | 3.1 ± .11 |
| r1558 | 3.8 ± .40 | 4.3 ± .34 |
| r942/992 | <1.5 ± 0 | <1.5 ± 0 |
| r942/1558 | 2.9 ± .23 | 1.8 ± .17 |
| r992/1558 | 5.7 ± .16 | 3.2 ± .57 |
| r942/992/15 58 | 3.9 ± .15 | <1.5 ± 0 |
| *cp*45 | 4.1 ± .27 | 1.6 ± .08 |

| | | |
|---|---|---|
| a. Groups of six hamsters each were intranasally administered 10^{5.5} pfu of virus per animal in an 0.1ml inoculum, and lungs and nasal turbinates were harvested four days later. b. Standard Error c. *cp*45 is a biologically derived virus and the others are recombinant. | | |

To summarize the above results, substitutions at L protein amino acid positions 992 and 1558 each specified a 1,000,000-fold reduction in plaque formation in cell culture at 40°C, while the substitution at position 942 specified a 700-fold reduction. Thus, each of the three mutations individually contributes to the *ts* phenotype. The triple recombinant which possesses all three L mutations is slightly less *ts* than *cp*45, suggesting that there are mutations outside of the L gene in *cp*45 that also might contribute to its *ts* phenotype. Two of the three individual mutations in L each contributed to restricted replication in the upper or lower respiratory tract of hamsters, which accounts for the observed stability of *ts* and *att* phenotypes of *cp*45 during replication *in vivo*. Importantly, the level of temperature sensitivity of recombinant vaccine strains in vitro was closely predictive of attenuation *in vivo*. The recombinant virus possessing all three mutations was as restricted in replication as the *cp*45 mutant in both the upper and lower respiratory tract of hamsters, indicating that the L gene of the *cp*45 virus is a major attenuating component of this candidate vaccine strain. While each mutation on its own specifies the *ts* phenotype, when placed together they are not simply additive but instead somehow influence each other. The effect of the three mutations together in the triple mutant seemed to ameliorate rather than enhance the level of temperature-sensitivity observed in the two double mutants which were evaluated. Interestingly, this should provide an unanticipated selective pressure to maintain at least some of the *cp*45 L mutations, since the loss by reversion of either the 992 or 1558 substitution would increase rather than decrease the level of temperature sensitivity. Considered together, these findings indicate that the high level of the stability of the *ts* and *att* phenotypes of *cp*45 virus results from the contribution of multiple *ts* mutations in L to the *att* phenotype. The identification of these three mutations as the major attenuating mutations of *cp*45 provides the basis for monitoring virus during all stages of manufacture and following replication in humans.

It is of further interest that the tyrosine to histidine mutation at position 942, arguably the most conservative substitution of the three mutations, was the least temperature sensitive. The L polymerase of PIV3 is a large polypeptide, 2233 aa in length, and is thought to be a multifunctional protein that encodes multiple domains including those required for complex formation with the P protein, RNA binding, RNA polyadenylation, RNA transcription and RNA replication (Collins et al., supra, (1996)). The amino acid substitutions in L at positions 942 and 992 are located near regions that are well-conserved among other members of the Paramyxovirus family (Blumberg et al., Virology 164:487-497 (1982); Galinski et al., Virology 165:499-510 (1988)). The mutation at position 1558 is in a region of the polymerase which appears to share less sequence identity with other L polymerases. Although the mechanism by which the *ts* phenotype is conferred by the triple amino acid substitution in L is not known, it is likely that multiple L protein domains and activities are affected, or that a common mechanism involving various activities of L is affected.

### EXAMPLE X

### Direct Identification, and Reconstitution into Recombinant Vaccine Viruses, of Mutations in a Biologically Derived, Live-Attenuated HPIV Type 3 Virus (cp45) Which Specify the Temperature-Sensitive, Cold-Adantation and Attenuation Phenotypes

The above Examples demonstrate that each of the three amino acid substitutions in the L polymerase protein of *cp*45 confer the temperature-sensitive (*ts*) and attenuation (*att*) phenotypes, but not the cold-adaptation (*ca*) phenotype (see also, Skiadopoulos et al., J. Virol 72 (3) : 1762-8, 1998). *cp*45 contains twelve additional mutations in other proteins (N, C, M, F and HN) or potential cis-acting sequences (the leader region and the transcription gene start {GS} signal of the N gene), and their contribution to these phenotypes has been heretofore undefined. The present Example further characterizes the genetic basis for the *ts*, *ca*, and *att* phenotypes of *cp*45 to provide yet additional information regarding basis for the observed high level of stability of these phenotypes following replication of *cp*45 in humans or non-human primates. In one aspect of this study, a recombinant *cp*45 (*rcp*45) virus containing all fifteen *cp*45-specific mutations was constructed, using a reverse genetics system, and was found to be essentially indistinguishable from the biologically-derived virus on the basis of plaque size, level of temperature-sensitivity, cold-adaptation, and level of replication in the upper and lower respiratory tract of hamsters. In addition, recombinant viruses containing: (1) the *cp*45-specific changes in the C, M, F or HN proteins, (2) the combined leader and N gene mutations, or (3) several combinations of the *cp*45 mutations were constructed. Analysis of these recombinant viruses showed that multiple *cp*45 mutations distributed throughout the genome contribute to the *ts*, *ca*, and *att* phenotypes. The mutations in C and F were not *ts* at 40°C but nonetheless conferred the *att* phenotype, and they, therefore, are non-*ts att* mutations. The HN mutation did not confer the *ca*, *ts* or *att* phenotypes. Viruses possessing the 3' leader and N mutations were *ts*, but exhibited only marginal attenuation in the lower respiratory tract of hamsters. Recombinants possessing several combinations of mutations exhibited a greater level of temperature sensitivity than *cp*45, but the increased level of temperature-sensitivity was not reflected in an increase in attenuation *in vivo*. These latter findings indicate that the multiple mutations identified in *cp*45 are interacting to affect replication *in vitro.* The presence of multiple *ts* and non-*ts* attenuating mutations in *cp*45 likely contributes to its high level of attenuation and phenotypic stability. Knowledge of the phenotypes associated with the various mutations of *cp*45 provided herein allows for accurate monitoring of biologically derived PIV viruses and ready manipulation of recombinant virus to achieve a large assemblage of useful vaccine recombinants within the invention.

### Viruses and cells.

The rPIV3s, PIV3 JS wt and *cp*45 viruses described in the present Example were grown in simian LLC-MK2 cells (ATCC CCL 7.1) as described above (see also, Durbin et al., Virology 235:323-332, 1997a; Hall et al., Virus Res. 22(3): 173-184, 1992; Skiadopoulos et al., J. Virol 72 (3) : 1762-8, 1998). The modified vaccinia virus Ankara was provided as described above. HEp-2 (ATCC CCL 23) and LLC-MK2 cells were maintained in OptiMEM I (Life Technologies, Gaithersburg, MD) supplemented with 2% FBS and gentamicin sulfate (50ug/mL), or in EMEM supplemented with 10% FBS, gentamicin sulfate (50ug/mL), and 2mM glutamine. L-132 cells (ATCC CCL 5) were grown in Earl's MEM (Life Technologies) supplemented with 10% FBS, 2mM glutamine, 20 mM Hepes, 1 mM non-essential amino acids, and 100 units streptomycin-neomycin/ml.

### Construction of point mutations in PIV3.

Subgenomic fragments of p3/7(131)2G, the antigenomic cDNA clone of PIV3 JS wt used above to recover infectious virus (see also, Durbin et al., Virology 235:323-332, 1997a; Skiadopoulos et al., J. Virol. 72 (3):1762-8, 1998), were cloned into pUC19 vectors modified to accept these fragments, using standard molecular cloning techniques. Point mutations corresponding to mutations identified in *cp*45, as well as mutations creating or ablating silent restriction enzyme recognition sequences (Table 8) were introduced using the Transformer Mutagenesis Kit (Clontech, Palo Alto, CA) as described previously.

**Table 8: PIV3 cp45 mutations introduced into rPIV3**

| | **seq id no** | **region affect ed** | **nt posit ion^{a}** | **sequence changes^{b}** | **restric tion marker^{c}** | **amino acid substitu tion^{d}** |
|---|---|---|---|---|---|---|
| 1 | 46 47 | **3' leader** | 20 | TTGTCTGGGAAT TTGCCTGGGAAT | none | non-coding |
| 2 | 46 48 | **3' leader** | 20 | TTGTCTGGGAAT TTGTTTGGGAAT | none | non-coding |
| 3 | 46 49 | **3' leader** | 20 | TTGTCTGGGAAT TTGTCTGGTAAT | none | non-coding |
| 4^{e} | 50 51 | **3' leader** | 40 | AAC*TTTAAA*TTA AACTTAAAATTA | *-Dra* I | non-coding |
| 5^{e} | 52 53 | **N gene** | 60 **start** | TTAAAGACATTG TTTAAGACATTG | none | non-coding |
| 6^{e} | 54 55 | **N** | 390 | GCAGAT**GT**CAAG GCAGAT**G**CCAAG | none | Val-96 to Ala |
| 7^{e} | 56 57 | **N** | 1271 | CGAATCTAAAGA CGAA**GCT**AAAGA | none | Ser-389 to Ala |
| 8 | 58 59 | **C** | 2076 | GAA***ATA**TT*GATC GAA**A**C**A**TTGATC | *-Ssp* I | Ile-96 to Thr |
| 9 | 60 61 | **M** | 4341 | TCTCTACCCAAC TC*GTTA**A**C*CAAC | *+Hpa* I | Pro-199 to Thr |
| 10^{f} | 62 63 | **F** | 6323 | AGTACA**ATA**GGT *AGTAC**T***GTG**GGT | *+Sca* I | Ile-420 to Val |
| 11 | 64 65 | **F** | 6419 | **GCA**CTTGATCCA **A**C**A**CT*GGATCC*A | *+Bam* HI | Ala-450 to Thr . |
| 12 | 66 67 | **HN** | 7944 | CCATCATTGTT**GTT**GACAA *CCA*TCATTG*TG****G*CT**GACAA | +*Bst* XI | Val-384 to Ala |
| 13 | 68 69 | **L** | 11468 | T**TA**CA*TGGCC*A T**CA**CATGGCGA | *-Eae* I | Tyr-942 to His |
| 14 | 70 71 | **L** | 11618 | **TTTG**G*ACTGGG*C **TTTT**GATTGGGC | -*Bsr* I | Leu-992 to Phe |
| 15^{r} | 72 73 | **L** | 13308 | *GGTCC*TAAT**A**CT GGGCCTAAT**A**TC | *-Ava* II | Thr-1558 to Ile |

| | | | | | | |
|---|---|---|---|---|---|---|
| **a.** Position of the first nucleotide in the PIV3 sequence shown. **b.** Wild type sequence is shown above the mutant sequence. Nucleotide changes are underlined. Codon substitutions are in bold font. **c.** Each relevant restriction endonuclease recognition sequence is in italics; (+) indicates addition of new restriction endonuclease recognition sequence; (-) indicates ablation of a naturally occurring restriction endonuclease recognition sequence. **d.** Mutations are indicated as the three letter amino acid assignment of wt, followed by the amino acid position, followed by the *cp*45 assignment. **e.** These mutations were identified by Joanne Tatem (unpublished observations), the others were from Stokes et al., Virus Res. 30 (1) : 43-52, 1993. **f.** Two nucleotides were changed in the indicated codon in order to reduce the chance of reversion to wild type sequence. | | | | | | |

After mutagenesis, restriction endonuclease fragments were sequenced completely and cloned back into the full-length clone, p3/7(131)2G. The 3' leader and N mutations were amplified by reverse transcription (RT)-PCR directly from PIV3 *cp*45 virion RNA and were cloned into pLeft+2G (see above), or a modified pUC19 vector for further manipulation. Combinations of mutations were constructed using standard molecular cloning techniques. The full-length plasmid clone containing the *cp*45 mutations, designated pFLC*cp*45, was completely sequenced to determine if extraneous mutations had been introduced during the cloning process, but none were found.

### Recovery of recombinant PIV3 (rPIV3).

Each full-length antigenomic cDNA bearing *cp*45 mutations, together with the three support plasmids pTM(N), pTM(P no C) and pTM(L), was transfected into HEp-2 cells on 6-well plates (Costar, Cambridge, MA) using LipofectACE (Life Technologies, Gaithersburg, MD) and MVA-T7 as described above. After incubation at 32°C for 4 days, the transfection harvest was passaged onto LLC-MK2 cells in T-25 flasks which were incubated at 32°C for four to eight days. This harvested virus was called passage 1 and was subjected to three rounds of plaque purification on LLC-MK2 cells as described above. Each biologically-cloned recombinant virus was amplified twice in LLC-MK2 cells at 32°C to produce virus for further characterization. Virus was concentrated from clarified medium by polyethylene glycol precipitation (see, Mbiguino and Menezes, J. Virol Methods 31 : 2-3, 1991, ), and viral RNA (vRNA) was extracted with Trizol Reagent (Life Technologies). Reverse transcription was performed on vRNA using the Superscript II Preamplification System (Life Technologies) with random hexamer primers. The Advantage cDNA PCR kit (Clontech) and sense and antisense primers specific for various portions of the PIV3 genome were used to amplify fragments for restriction endonuclease analysis. The PCR fragments were analyzed by digestion with each of the restriction enzymes whose recognition sites had been created or ablated during construction of the mutations (Table 8).

### Efficiency of plaque formation (EOP) of rPIV3 bearing cp45 mutations at permissive and restrictive temperatures.

The level of temperature-sensitivity of plaque formation *in vitro* of control and recombinant viruses was determined at 32°C, 35°C, 36°C, 37°C, 38°C, 39°C, 40°C, and 41°C in LLC-MK2 monolayer cultures as described above (see also, Hall et al., Virus Res. 22 (3) : 173-184, 1992. Plaques were enumerated by hemadsorption with guinea pig red blood cells following removal of the methylcellulose overlay, or alternatively, the viral plaques present in the monolayer were identified by immunoperoxidase staining with a mixture of two PIV3-specific anti-HN murine mAbs 101/1 and 454/11 diluted 1:250 (see, Murphy et al., Vaccine 8 (5) : 497-502, 1990; van Wyke Coelingh et al., Virology 143 (2) :569-582, 1985).

### Evaluation of rPIV3 mutant viruses for cold-adaptation phenotype.

Growth of mutant and wt rPIV3 viruses was determined at 32°C and 20°C on confluent L-132 cell monolayers prepared in 24-well tissue culture plates. Duplicate wells of each of two plates were inoculated with 0.2 ml of each mutant or wt rPIV3 virus at a multiplicity of infection of 0.01. After one hour adsorption at room temperature, the inoculum was aspirated and the monolayers were washed with 1 ml of PBS per well. The inoculated cultures were overlaid with 0.5 ml of Earl's MEM supplemented with 10% FBS, 2mM glutamine, 20 mM Hepes, 1 mM non-essential amino acids, and 100 units streptomycin-neomycin/ml. One plate was sealed in a waterproof pouch (Kapak) and then submerged in a 20°C bath for 13 days. The duplicate plate was placed at 32°C in a CO₂ incubator for 3 days. At the end of the incubation period, virus was harvested by freeze/thawing. The titer of virus recovered from each well was determined by plaque assay in LLC-MK2 cells at 32°C using hemadsorption with guinea pig red blood cells to visualize plaques. Two wt and two *cp*45 reference stocks were used as controls.

### Hamster studies.

5 week-old Golden Syrian hamsters in groups of five were inoculated intranasally with 0.1 ml OptiMEM I per animal containing 10^{6.0} pfu of JS wt rPIV3, PIV3 *cp*45 virus, or one of the mutant rPIV3s. On day 4 post-infection, the hamsters were sacrificed, the lungs and nasal turbinates were harvested, and the virus was quantified as described above. The mean log₁₀TCID₅₀/gram at 32°C was calculated for each group of hamsters.

### RESULTS

### Introduction of the PIV3 cp45 mutations into JS wt rPIV3.

The fifteen mutations in the 3' leader, the N GS signal, and the N, C, M, F, HN and L proteins of *cp*45 (Table 8) were introduced into the complete PIV3 antigenomic cDNA by site directed mutagenesis or by direct PCR amplification of a segment of *cp*45 cDNA bearing the desired mutations. The following antigenomic cDNAs were made (see Fig. 13): (i) *rcp*45 3'N, containing the four point mutations of the leader region, the point mutation in the N GS signal, and the two amino acid changes in the N protein; (ii) *rcp*45 C, containing the single amino acid change in the C protein; (iii) *rcp*45 M, containing the single amino acid change in M, (iv) *rcp*45 F, containing the two amino acid changes in F; (v) *rcp*45 HN, containing the single amino acid change in HN; (vi) *rcp*45 L, containing the three amino acid changes in L, as described above; (vii) *rcp*45 3'NL, containing the mutations from i. and vi. above; (viii) *rcp*45 3'NCMFHN, containing all of the mutations except for the three in L; and (ix) *rcp*45, containing all fifteen *cp*45 mutations listed in Table 8. In most cases, each *cp*45 change was engineered to be accompanied by one or more nearby silent changes which introduced or removed a restriction enzyme recognition site (Table 8). These served as markers to confirm the presence of the mutation in the cDNA and in recovered virus. Also, two of the amino acid coding changes (mutation numbers 10 and 15 in Table 8) were made using two nucleotide changes rather than the single change found in *cp*45, reducing the possibility of same-site reversion to wt. The *cp*45 cDNA, which contains all fifteen of the *cp*45 changes in Table 8, was assembled from the same mutagenized cDNA subclones as were used to introduce *cp*45 changes into the other antigenomic cDNAs; it was sequenced in its entirety, and was confirmed to contain only the intended mutations. This indicated that all of the regions which had been subjected to mutagenesis or PCR and had been manipulated by cloning possessed the desired sequences and lacked other unwanted mutations. Each full-length plasmid bearing one or more of the *cp*45 mutations was transfected into HEp-2 cells along with support plasmids and MVA-T7 to produce recombinant PIV3 as described above. Analysis of RT-PCR fragments encompassing the mutations indicated in Table 8 were amplified from virion RNA of the various recombinant viruses indicated in Figure 13 confirmed the presence of the introduced mutations, and other unintended mutations were not found.

### Plaque morphology.

Several of the recombinant viruses exhibited distinctive plaque morphologies when tested on LLC-MK2 cells. JS wt rPIV3 plaques averaged 1mm in size, and were indistinguishable in size from the biologically derived JS wt PIV3. Plaques of the *cp*45 and *rcp*45 viruses were larger than wt, averaging two- to three-fold larger in diameter than wt, and were indistinguishable from each other. This demonstrated the comparability of the biologically-derived and recombinant *cp*45 virus for this phenotype.

### Efficiency of plaque formation of rPIV3s bearing the cp45 mutations in LLC-MK2 cells at permissive and restrictive temperatures.

The rPIV3s were assayed for their ability to form plaques at permissive and restrictive temperatures ranging from 32°C to 41°C (Table 9). Analysis of the *ts* phenotypes of viruses bearing individual components of *cp*45 revealed that the *rcp*45 3'N and *rcp*45 M viruses had a shutoff temperature of 40°C, and the *rcp*45 C mutant had a shutoff of 41°C. The shutoff temperature of *rcp*45 F and *rcp*45 HN mutants was greater than 41°C. Consistent with the above results, the *rcp*45 L virus had a shutoff temperature of 39°C. A virus is considered to have a *ts* phenotype, for example, if its reduction of replication at 40°C (ie. titer at 32°C minus titer at 40°C) is approximately ≥ 100-fold that of wt virus at 40°C. Applying this definition, the present results indicated that at least two regions of *cp*45 (3'N, and L) contribute to the *ts* phenotype.

**Table 9: The efficiency of plaque formation (EOP) of recombinant and biologically-derived viruses at permissive and restrictive temperatures.**

| Virus | Mean Virus Titer^{a} (log₁₀ pfu/ml) | | | | | | | | |
|---|---|---|---|---|---|---|---|---|---|
| | 32°C | 35°C | 36°C | 37°C | 38°C | 39°C | 40°C | 41°C | *ts* phenotype c |
| ***rcp*45 3'N** | 7.1 | - | - | 7.0 | 6.4 | 5.4 | 4.2 | <3.7 | + |
| ***rcp*45 C** | 6.9 | - | --- | 7.0 | 6.7 | 6.6 | 5.9 | <3.7 | - |
| ***rcp*45 M** | 7.7 | - | - | 7.4 | 7.0 | 6.5 | 5.3 | <3.7 | - |
| ***rcp*45 F** | 7.5 | - | - | 7.2 | 6.0 | 6.6 | 5.9 | 5.7 | - |
| ***rcp*45 HN** | 6.4 | - | - | 6.5 | 6.2 | 6.4 | 4.7 | 4.4 | - |
| ***rcp*45 L** | 7.3 | - | - | 7.2 | 6.7 | 4.0 | <0.7 | <0.7 | + |
| ***rcp*45 3'NL** | 7.3 | 5.7 | <0.7 | <0.7 | <0.7 | <0.7 | <0.7 | - | + |
| ***rcp*45 3'NCMFHN** | 7.2 | 5.6 | <0.7 | 2.0 | 2.4 | <0.7 | <0.7 | - | + |
| ***rcp*45** | 8.5 | 7.5 | 7.1 | 6.4 | 6.0 | 2.0 | <0.7 | <0.7 | + |
| ***cp*45^{b}** | 8.3 | 8.0 | 7.4 | 7.0 | 6.2 | 2.3 | <0.7 | <0.7 | + |
| **rPIV3 wt** | 7.3 | 7.3 | 7.0 | 7.4 | 7.6 | 7.7 | 6.8 | 6.0 | - |

| | | | | | | | | | |
|---|---|---|---|---|---|---|---|---|---|
| a. Plaques were enumerated by immunoperoxidase staining after incubation for 6 days at the indicated temperature. Values are the mean of two experiments, values in bold are from a single experiment. Underlined values represent the lowest non-permissive temperature at which a 100-fold reduction of plaquing efficiency occurs compared to titer at 32°C, and this is defined as the shutoff temperature of plaque formation. b. *cp*45 is biologically-derived and the other viruses are recombinants. c. A virus is defined as bearing the *ts* phenotype if its reduction of replication at 40°C (ie. titer at 32°C minus titer at 40°C) is ≥ 100-fold that of wt virus at 40°C. | | | | | | | | | |

As shown in Table 9, *rcp*45, containing all of the *cp*45 mutations, had a shut-off temperature of 38°C, which was identical to that of the biologically-derived *cp*45. These results show that the *ts* phenotype of *cp*45 was successfully reproduced in *rcp*45. In addition, these results validate the sequence analysis of *cp*45 and the subsequent reconstruction of mutations into recombinant virus.

The rcp45 3'NCMFHN virus, which is identical to *rcp*45 except that it lacks the three L mutations, exhibited a shutoff temperature of 36°C. Since the L mutations are known to confer temperature-sensitivity individually and in combination, it is somewhat paradoxical that *rcp*45 3'NCMFHN was more, rather than less, *ts* than *cp*45. This implies that there is an interaction of mutations within *cp*45 whereby mutations compensate for each other to give a level of temperature- sensitivity which is less than the sum of the individual components.

Virus *rcp*45 3'NL was constructed to investigate whether the L mutations interact with the leader and/or N mutations, since all of these elements are believed to interact during RNA synthesis. This virus had a shutoff temperature 36°C, compared to 40°C and 39°C for *rcp*45 3'N and *rcp*45 L, respectively. These results suggest that there is an interaction between the 3'N and L mutations that results in augmentation of temperature-sensitivity. These results also provide another example in which a subset of *cp*45 mutations specifies a level of temperature sensitivity greater than that observed for *rcp*45 which contains the entire set of mutations

### ca phenotype of rPIV3s bearing cp45 mutations.

The rPIV3s were analyzed to determine which genetic elements of *cp*45 specified the *ca* phenotype (Table 10). It was demonstrated above that *rcp*45 L is *ts* and *att*, but not *ca*. This indicates that the genetic elements specifying the greater part of the *ca* phenotype are located outside L, and this was confirmed in the present study. Each of the rPIVs possessing the 3' leader and N mutations were *ca* except *rcp*45 3'NCMFHN, which exhibited an intermediate phenotype. This shows that the *ca* phenotype is specified mostly within the 3'N region. The finding that the level of *ca* of viruses containing the 3'N segment is less than that of *cp*45 or *rcp*45 indicates that other regions of *cp*45 contribute to the *ca* phenotype, even though this is not apparent from analysis of the other regions individually. The *rcp*45 3'NL virus is more *ca* than the *rcp*45 3'N virus, suggesting that the L mutations may make a modest contribution. The biologically-derived *cp*45 and *rcp*45 viruses exhibit comparable levels of *ca,* indicating that this phenotype, like the plaque size and *ts* phenotypes, was successfully reproduced in the recombinant *cp*45 virus provided herein. Therefore, the *ca* phenotype, like the *ts* phenotype, is a composite phenotype reflecting individual contributions to the overall phenotype as well as contributions from interacting genetic elements.

**Table 10 : Growth of wt and mutant PIV3s at 20°C compared to 32°C^{a} (cold adaptation phenotype).**

| **Virus** | **20°C** | **32°C** | ***ca* Phenotype** |
|---|---|---|---|
| ***cp*45^{b}** | 6.72 | 8.49 | + |
| ***rcp*45** | 6.57 | 8.35 | + |
| ***rcp*45 3'NL** | 5.02 | 7.23 | + |
| ***rcp*45 3'N** | 4.39 | 8.53 | + |
| ***rcp*45 3'NCMFHN** | 3.53 | 8.08 | +/- |
| ***rcp*45 L** | 3.18 | 7.98 | - |
| ***rcp*45 C** | 2.52 | 8.32 | - |
| ***rcp*45 F** | 2.47 | 8.10 | - |
| ***rcp*45 HN** | 2.11 | 8.01 | - |
| ***rcp*45 M** | 1.76 | 8.21 | - |
| **PIV3 WT** | 2.57 | 8.43 | - |

| | | | |
|---|---|---|---|
| **a.** Virus titer is expressed in log₁₀ PFU/mL. **b.** *cp*45 is biologically-derived and the other viruses are recombinant. The *ca* phenotype is defined as a greater than 10-fold increase in growth at 20°C relative to wt. | | | |

### Growth of the rcp45 mutant viruses in hamsters.

Groups of five Golden Syrian hamsters were inoculated intranasally with 10⁶ TCID₅₀ of recombinant or biologically-derived virus, and the level of virus replication in the lungs and nasal turbinates was determined four days later (Table 11). The fourth day post-inoculation has been shown previously to be the peak of virus replication in hamsters for both the wt and *cp*45 viruses (see, Crookshanks and Belshe, J. Med Virol 13 (3) : 243-9, 1984, ). The *rcp*45 virus was reduced approximately 40-fold in replication in the nasal turbinates and 1000-fold in the lungs, and thus was as attenuated as the biologically-derived *cp*45 virus. These results indicate that the attenuation phenotype of *cp*45 was successfully reproduced in its recombinant version. Since each phenotype of *cp*45 was fully reproduced in *rcp*45, the additional five mutations in *cp*45 that were not included in this Example likely made little contribution to the properties of *cp*45.

**Table 11: Level of replication in the upper and lower respiratory tract of hamsters^{a} of wt and mutant PIV3s ^{b}.**

| **Virus** | Mean virus titer (log₁₀ TCID₅₀/g ± S.E^{c}) in: | |
|---|---|---|
| | **Nasal turbinates** | **Lungs** |
| **JS wt rPIV3** | 6.9 ± 0.2 | 5.4 ± 0.5 |
| ***rcp*45 3'N** | 6.5 ± 0.2 | 3.9 ± 1.1 |
| ***rcp*45 C** | 4.8 ± 0.3 | 3.1 ± 0.7 |
| ***rcp*45 M^{d}** | 6.8 ± 0.2 | 6.7 ± 0.3 |
| ***rcp*45 F** | 4.6 ± 0.2 | 3.4 ± 0.6 |
| ***rcp*45 HN** | 6.3 ± 0.2 | 5.3 ± 1.0 |
| ***rcp*45L** | 4.2 ± 0.1 | 2.1 ± 0.3 |
| ***rcp*45 3'NL** | 4.7 ± 0.2 | 2.1 ± 0.3 |
| ***rcp*45 3'NCMFHN** | 5.8 ± 0.3 | 2.9 ± 0.9 |
| ***rcp*45** | 5.3 ± 0.1 | 2.4 ± 0.2 |
| ***cp*45** | 4.9 ± 0.4 | 1.9 ± 0.2 |

| | | |
|---|---|---|
| **a.** Groups of five hamsters each were intranasally administered 10^{6.0} TCID₅₀ of virus per animal in an 0.1ml inoculum, and lungs and nasal turbinates were harvested four days later. **b.** *cp*45 is a biologically-derived virus, the other viruses are recombinant. **c.** TCID₅₀, 50% tissue infectious dose ± Standard Error. **d.** The virus pool used in this study was found to contain a mixed plaque phenotype. The attenuation phenotype of this mutant will be reassessed using additional virus preparations. | | |

As demonstrated above, the mutations in the L gene of cp45 specify the majority of the attenuation phenotype of this virus. In the present Example, the contribution of the *cp*45 mutations outside of L as a group was examined. The *rcp*45 3'NCMFHN mutant was only slightly reduced in replication in the nasal turbinates, but was more than 100-fold reduced in replication in the lungs, which shows that additional attenuating mutations were present outside of the L protein. Importantly, if each of the three mutations in the L gene of *rcp*45 reverted to wild type sequence, the resulting virus, *rcp*45 3'NCMFHN, would still retain the attenuation phenotype. The *rcp*45 M and *rcp*45 HN mutant viruses were not defective for replication in the respiratory tract of hamsters, and the *rcp*45 3'N virus showed only a marginal decrease in replication in the lower respiratory tract. This suggests that the mutations present in the 3' leader, in the N gene start site and the N , M and HN proteins are not attenuating in and of themselves. However, these mutations could contribute to the overall attenuation of *cp*45 in the context of the other *cp*45 mutations. Also, individual mutations within the 3'N region may have effects which are not apparent when the set of mutations is analyzed together, which can be readily determined according to the present disclosure.

Replication of the *rcp*45 C and *rcp*45 F mutant viruses was approximately 100-fold reduced in both the nasal turbinates and the lungs, demonstrating that the mutations present in the C and F proteins of *cp*45 confer the attenuation, phenotype in hamsters, although the level of attenuation is not as great as that conferred by the *cp*45 L mutations. As described above, the *rcp*45 F and *rcp*45 C mutant viruses did not possess the *ts* phenotype, and therefore, the mutations that occur in the C and F proteins are considered to be non-*ts* attenuating mutations.

### EXAMPLE XI

### Recovery of Recombinant, Chimeric PIV3 in which the Hemagglutinin and Fusion Glycoproteins have been Substituted with Corresponding Glycoproteins From PIV1

Within the present example, a chimeric rPIV virus is generated and selected which incorporates one or more heterologous genes or large polynucleotide sequences from one PIV into a different rPIV background. Within this aspect of the invention, individual genes or gene segments of one PIV are substituted by counterpart sequence(s) from a heterologous PIV or other source. In one embodiment described in the present Example, tools and methods are provided for substituting, e.g., the HN and/or F protective antigens of HPIV1 or HPIV2 into a recombinant HPIV3 to yield a chimeric recombinant suitable for developing live-attenuated vaccines.

### Viruses and cells.

The PIV1 strain used in this study, PIV1/Washington/20993/1964 (PIV1/Wash64), was isolated in Washington DC in 1964 and was confirmed to be a virulent wild type virus in clinical studies in adult human volunteers (Murphy et al. Infect. Immun. 12:62-8 (1975)). It was propagated in LLC-MK2 cells (ATCC CCL 7.1) in Opti-MEM I (Life Technologies) with 50 µg/ml gentamicin sulfate, 2 mM glutamine and 0.75 µg/ml trypsin (Catalog No. 3741, Worthington Biochemical Corp., Freehold, NJ). The Greer strain of human PIV2 (Catalog No. V-322-001-020, NIAID Repository, Rockville, MD) used in the hemagglutination-inhibition assay (HAI) was propagated in the same way. The JS strain of human PIV3 virus and its recombinant derivative from cDNA (rPIV3/JS) with wild type phenotype were propagated as described above. The modified vaccinia Ankara (MVA) recombinant that expresses the bacteriophage T7 RNA polymerase is described in Wyatt et al., Virology 210:202-205 (1995).

HEp-2 cells were obtained from ATCC (ATCC CCL 23) and maintained in Opti-MEM I (Life Technologies) with 2% fetal bovine serum (FBS), 50 µg/ml gentamicin sulfate and 2 mM glutamine.

### Construction of a cDNA encoding a complete chimeric PIV3-PIV1 antigenome.

A cDNA encoding a full-length PIV3 antigenomic RNA in which the PIV3 HN and F ORFs were replaced by their PIV1 counterparts was constructed as shown in Figure 14. cDNA clones of the HN and F genes of PIV1/Wash64 were prepared from RNA extracted from LLC-MK2 cells which had been infected with PIV1/Wash64 wild type virus. cDNA was generated using the SuperScript Preamplification System using random hexamer primers (Life Technologies). PIV1 F and HN cDNAs were amplified with Vent DNA polymerase (New England Biolabs, Beverly, MA) using gene specific primer pairs based on consensus sequences present in GenBank. All primers described below are annotated so that PIV specific sequences are underlined, restriction sites are in italics, nt altered from wild type sequences are in lowercase, and start and stop codons are in bold. The positive sense PIV1 F primer, hybridizing to nt 69-97 upstream of the start codon, was 5' -GGGAAAGAAtCCAGAGAGAAGAACGG-3' (SEQ ID NO: 33). The negative- sense PIV1 F primer, hybridizing to nt 36-61 downstream of the F stop codon, was 5'-GGTGAAGT*GG*AT*cc*ATTTGATTG-3' (SEQ ID NO: 34). It carries a BamH I site. The positive-sense primer for PIV1 HN was 5'-CAACCTGTAA*GG*t*Ac**C*ACATCCG-3' (SEQ ID NO: 35). It hybridizes to nt 13-36 upstream the HN start codon and carries a *Kpn* I site. The negative-sense PIV1 HN primer was 5'-GATATGGTGTT*aGGc**CTT*GATCTGTTC-3' (SEQ ID NO: 36). It hybridizes to the last two nt of the stop codon and 25 nt further downstream and carries a *Stu* I site. The PIV1 F cDNA was cloned as a *Bam*H I and blunt-end fragment into *Bam*H I-EcoR V digested pLITMUS28 (New England Biolabs), while the PIV1 HN cDNA was cloned as a *Kpn* I-*Stu* I fragment into the same vector. The nt sequences of the resulting plasmids, designated as pLit.1HNwt and pLit.1Fwt (GenBanK Accession number: AF016280, AF016279), were determined using the Circumvent Sequencing Kit (New England Biolabs). These two clones were modified using mutagenic PCR primers to delete their non-coding regions and to introduce new restriction sites flanking their start and stop codons for the purpose of constructing the PIV3-1 chimeric HN and F genes. The sequences of positive-sense and negative-sense PIV1 F mutagenic primers were 5' -Cg*cc**ATG**g*AAAAATCAGAGATCCTCTTCT-3' (SEQ ID NO: 37) and 5'-Ct*ggatc**c**t***A**ATTGGAGTTGTTACCCATGTA-3' (SEQ ID NO: 38), respectively, the introduced restriction sites are Nco I and BamH I. The sequences of positive-sense and negative sense PIV1 HN mutagenic primers were 5'-AAC*c**A**TGG*CTGAAAAAGGGAAAA-3' (SEQ ID NO: 39) and 5'-GGTG*AaGC**T**t***AA**GATGTGATTTTACATATTTTA-3' (SEQ ID NO: 40), respectively, the introduced restriction sites are *Nco* I and *Hind* III.

The second step involved the modification of the PIV3 HN and F genes to serve as acceptors of the PIV1 coding regions generated above. The PIV3 F and HN genes were subcloned in several steps from the full-length clone of PIV3/JS, p3/7(131)2G to generate pLit.PIV3.HN.4 and pLit.PIV3.F.3a (Fig. 14). The PIV3 F or HN coding regions were deleted by PCR mutagenesis and replaced by restriction sites appropriate to accept the PIV1 HN and F cDNA described above. The sequences of the PIV3 F positive sense and negative sense mutagenic primers were 5'-AAATA*ggatcc*CTACAGATCATTAGATATTAAAAT-3' (SEQ ID NO: 41) and 5' -Cg*cCATg**G*TGTTCAGTGCTTGTTG-3' (SEQ ID NO: 42), respectively, the introduced restriction sites were *BamH*I and *Nco*I. The sequences of PIV3 HN positive sense and negative sense mutagenic primers were 5' -CCACAA*gCt**T*AATTAACCATAATATGCATCA-3' (SEQ ID NO: 43) and 5' -TT*CCATgg*ATTTGGATTTGTCTATTGGGT-3' (SEQ ID NO: 44), respectively, the introduced restriction sites were *Hind* III and *Nco* I. The stop codon for the chimeric HN would be regenerated upon ligation with mutagenized PIV1 HN cassette. The PIV1 HN or F cDNAs described above were imported in as a *Nco* I-*Hind* III fragment for HN or as a *Nco* I-*BamH* I fragment for F, which generated pLit.PIV3-1.HNhc and pLit.PIV3-1.Fhc. These two cDNAs were then joined into pSE.PIV3-1.hc, which was subsequently sequenced in its entirety. Then the *Bsp*E I*-Sph* I fragment was inserted into p3/7(131)2G to generate pFLC.2G+.hc-( Fig. 14). The nucleotide sequence of this *Bsp*E I*-Sph* I fragment, containing the chimeric F and HN genes, is in GenBank (Accession No. AF016281). Importantly, the cDNA engineering was designed so that the final rPIV3-1 antigenome conformed to the rule of six (Durbin et al. Virology 234:74-78 (1997), ). The plasmid pFLC.2G+.hc encoding the rPIV3-1 chimera was deposited on 9/17/97 under the terms of the Budapest Treaty with the American Type Culture Collection (ATCC) of 12301 Parklawn Drive, Rockville, Maryland 20852, U.S.A.

**Transfection**. HEp-2 cell monolayers were grown to confluence in six-well plates (Costar Corp, Cambridge, MA) and transfections were performed as described above. Cell monolayers were infected with MVA-T7 at a multiplicity of infection (MOI) of 3 in 0.8 ml of serum-free Opti-MEM I containing 50 *µ*g/ml gentamicin, and 2 mM glutamine and then transfected with the three support plasmids, 0.4 *µ*g pTM(N), 0.4 *µ*g pTM(P), 0.05 *µ*g pTM(L), and 5 *µ*g the PIV3-1 antigenome cDNA. The plasmids were mixed in 0.2 ml of Opti-MEM I containing 12 *µ*l LipofectACE (Life Technologies) and added to each well. The PIV3/JS cDNA plasmid, p3/7(131)2G, which encodes wild type PIV3/JS antigenomic RNA, was transfected in parallel as a positive control. After incubation at 32°C for 12 hours, the transfection medium was replaced with 1.5 ml of fresh Opti-MEM I supplemented with 50 *µ*g/ml gentamicin, and cultures were incubated at 32°C for two additional days. Trypsin was added to a final concentration of 0.75 *µ*g/ml on day 3 post transfection. Cell culture supernatants were harvested on day 4 and passaged (referred to as passage 1) onto fresh LLC-MK2 cell monolayers. After overnight adsorption, the medium was replaced with fresh Opti-MEM I with 0.75 *µg*/ml trypsin. Passage 1 cultures were incubated at 32°C for 4 days, and the amplified virus was harvested and further passaged on LLC-MK2 cells (referred to as passage 2) for another 4 days at 32°C in the presence of 0.75 *µ*g/ml trypsin. The recovered virus was characterized by reaction with rabbit PIV1 antiserum, guinea pig PIV2 antiserum, and two PIV3 HN monoclonal antibodies in a hemagglutination-inhibition (HAI) assay as described previously (van Wyke Coelingh et al., supra, (1985)) with the exception that chicken red blood cells (RBCS) were used for viruses possessing the PIV1 HN glycoproteins, whereas guinea pig RBCs were used for PIV3. The HAI was performed to determine if rPIV3 possessed the monoclonal antibody resistant mutation (MARM) that marked virus recovered from cDNA or if rPIV3-1 possesses the PIV1 HN.

**Nucleotide sequence analysis.** The chimeric cDNA construct pSE.PIV3-1.hc was sequenced with the Circumvent Sequencing kit (New England Biolabs) before the final assembly into full-length clone pFLC.2G+.hc. For analysis of RNA, the appropriate PIVs were amplified in T75 flasks of LLC-MK2 cells. Virus was harvested on day 5 post-infection and concentrated by polyethylene glycol precipitation (Mbiguino et al., J. Virol. Methods 31:161-70 (1991)). Viral RNA was extracted from the viral pellets and used in reverse transcription (RT) with the Superscript Preamplification System (Life Technologies). RT-PCR was performed using the Advantage cDNA synthesis kit (Clontech) and PIV1 or PIV3 specific primer pairs. RT-PCR products were gel purified by electrophoresis onto, and elusion from, strips of NA45 DEAE nitrocellulose membrane (Schleicher & Schnuell, Keene, NH), and were sequenced.

**Replication of PIVs in LLC-MK2 cells**. Plaque enumeration on LLC-MK2 monolayers was performed as previously described except that trypsin was added in the case of the PIV1 and rPIV3-1 viruses (Hall et al. Virus Res. 22:173-84 (1992)). Briefly, serially-diluted virus was inoculated onto LLC-MK2 monolayers in six-well plates; virus was adsorbed for 1 hour at 32°C; the cultures were overlaid with L-15 medium (Life Technologies) containing 0.8% agarose with or without added trypsin and incubated at 32°C for 6 days; and plaques were identified by hemadsorption (HAD) with guinea pig red blood cells following the removal of the agarose overlay.

Growth of the PIV viruses in tissue culture was evaluated by infecting confluent LLC-MK2 monolayers on twelve-well plates with virus at a MOI of 0.01. After adsorption at 32°C for 1 hour, the inoculum was replaced with 1.5 ml/well Opti-MEM I supplemented with gentamicin (50 *µ*g/ml) and trypsin (0.75 *µ*g/ml), and further incubated at 32°C for 6 days. At 24 hour intervals, 0.3 ml medium was removed from each well and 0.3 ml fresh medium with trypsin was added back. The titer of virus was determined at 32°C by hemadsorption assay on LLC-MK2 cell monolayers using fluid overlay as previously described (Hall et al. Virus Res. 22:173-84 (1992)), and the titers were expressed as log₁₀TCID₅₀/ml.

**Replication of PIVs in the respiratory tract of hamsters**. Golden Syrian hamsters in groups of 12 were each inoculated intranasally with 0.1 ml of L-15 medium containing 10⁵ pfu of rPIV3/JS, rPIV3-1, or PIV1/Wash64. On days 4 and 5 post-inoculation, six hamsters from each group were sacrificed, and their lungs and nasal turbinates harvested, and homogenized, and virus present in the samples was titered on LLC-MK2 cell monolayers at 32°C. The titers were expressed as mean log₁₀ TCID₅₀/g for each group of six hamsters.

### RESULTS

### Construction of a cDNA clone encoding a full-length, chimeric PIV3-1 antigenomic RNA yielded recovery of the Chimeric virus rPIV3-1.

As noted above, the final construct of the PIV3 and PIV1 chimeric cDNA, in which the ORPs of the JS wild type PIV3 HN and F glycoproteins genes were replaced by those of PIVI/Wash64 coding sequences (Figures 14A and 14B) encodes a PIV3-1 chimeric antigenomic RNA of 15, 516 nt, which conforms to the rule of six (Durbin et al., Virology 234:74-78 (1997)). The pFLC.2G+.hc cDNA encoding the chimeric PIV3-1 antigenome was transfected onto HEp-2 cells together with the N, P and L support plasmids. The p3/7(131)2G cDNA encoding the JS wt PIV3 antigenome was transfected in parallel to generate a rPIV3 control parental virus. Virus was recovered from each transfection following the second amplification on LLC-MK2 cells, and studies were initiated to confirm that each recombinant virus was derived from cDNA.

Recombinant viruses rPIV3-1 and rPIVs3 were first characterized for the presence of the PIV1 or PIV3 HN glycoprotein by HAI assay with serotype-specific anti-HN monoclonal or polyclonal antibodies. As shown in Table 12, rPIV3 reacted with only one of the two PIV3 mAbs as expected, whereas its biologically derived parent PIV3/JS reacted with both. This confirmed that rPIV3 contained the introduced MARM mutation that marks this virus as being derived from cDNA. The rPIV3-1 virus reacted with antibodies specific to the PIV1 HN glycoprotein, but not to ones specific to HN of PIV3 or PIV2, showing that the virus contained the PIV1 HN gene as expected.

**Table 12. rPIV3-1 possesses the HN glycoprotein of PIV1**

| Hemagglutination-inhibition titer^{a} (reciprocal) of indicated monoclonal antibody or polyclonal antiserum | | | | |
|---|---|---|---|---|
| Virus | | | | |
| | PIV1^{b} antiserum | PIV2^{b} antiserum | α-PIV3 mAb 423/6^{c} | α-PIV3 mAb 77/5^{c} |
| PIV1/Wash64 | 256 | 32^{d} | ≤50 | ≤50 |
| rPIV3-1 | 64 | ≤2 | ≤50 | ≤50 |
| rPIV3/JS | 4 | ≤2 | ≤50 | 3,200 |
| PIV3/JS | 8 | ≤2 | 12,800 | 6,400 |
| PIV2/Greer | 8 | 512 | ≤50 | ≤50 |

| | | | | |
|---|---|---|---|---|
| ^{a}chick red blood cells (RBC) were used in HAI assay for PIV1, PIV2, and rPIV3-1 and guinea pig RBCs were used for PIV3/JS and rPIV3/JS. ^{b}PIV1 rabbit antiserum was purchased from Denka Seiken Co. Ltd., Japan (Catalog No. 410-701), and PIV2 guinea pig antiserum was obtained from NIAID repository, Rockville, MD (Catalog-No. V-322-50-558). ^{c}Biologically derived PIV3/JS contains epitopes recognized by both mAb 423/6 and 77/5, whereas rPIV3/JS was engineered to lack reactivity with mAb 423/6. ^{d}The PIV2 antiserum had some reactivity with PIV1 virus, and therefore is not completely type specific. | | | | |

It was next confirmed that the rPIV3-1 virus contained the engineered, chimeric PIV3-1 HN and F genes. As designed, the genetic structure of rPIV3-1 was unique in four junction regions when compared with either of its parents, PIVl/Wash64 or rPIV3/JS (boxed in Figure 15A). These regions are the transition points at which the sequence switches from the PIV3 non-coding region to the PIV1 coding region and then from the PIV1 coding region back to the PIV3 non-coding region. Using the primer pair A specific to PIV3 M and L genes, or primer pair B specific to the PIV1 M and the very 3'-end of HN gene, RT-PCR products were generated for virion-derived RNAs from rPIV3-1, rPIV3/JS, and PIV1/Wash64. Control reactions showed that the RT step was required for generation of RT-PCR products, indicating that an RNA template, rather than contaminating DNA, was required to produce the RT-PCR product. An early indication that rPIV3-1 was indeed a chimeric virus came from the finding that only the PIV3-specific primer pair A generated the expected 4.6 kb cDNA product that spans the F and HN genes (Fig. 15B). Thus, while rPIV3-1 virus contains only HPIV1-specific HN glycoprotein (See Table 12), the non-coding regions are specific to PIV3. Conversely, the PIV1 specific primer pair B amplified an appropriately sized product from PIV1 control but not from rPIV3-1. Restriction digestion analysis also demonstrated that rPIV3-1 RT-PCR product had unique restriction patterns different from that of rPIV3/JS and PIV1/Wash64 and appropriate for its predicted sequence.

The nt sequence of the 4.6 kb RT-PCR product of rPIV3-1 was determined in its four regions (Fig. 15 A) and compared with that of rPIV3/JS and PIV1/Wash64 (Fig. 16). The rPIV3-1 sequence was completely in agreement with the cDNA from which it was derived. Examination of the sequence alignment of the Region I-IV for the three RT-PCR products illustrates that rPIV3-1 contains the PIV1 F and HN glycoprotein ORFs with altered start and stop codons and flanked by the 5' and 3' non-coding regions of PIV3. Examples of sequencing ladders spanning the Region III and IV cf rPIV3-1 (Figures 17A and 17B), compared in parallel with rPIV3/JS or PTV1/Wash64, were evaluated, and this analysis conformed that rPIV3-1 is a recombinant chimeric virus whose structure is completely in agreement with the cDNA from which it was generated.

### Trypsin-dependence and cytopathicity of rPIV3-1 in vitro.

PIV1, like Sendai virus but contrary to PIV3, requires trypsin for cleavage of its F glycoprotein in order to undergo multicycle replication on continuous lines of tissue culture cells (Frank et al. J. Clin. Microbiol. 10:32-6 (1979)). In addition, PIV1 is a ncn-cytopathic virus whereas PIV3 readily produces extensive CPE (Collins et al. In fields Virology, 3rd ed., 1:1205-43 (1996)). rPIV3-1, rPIV3 and PIV1/Wash64 were compared on the basis of these properties. rPIV3-1, like PIV1/Wash64, had a higher HA titer using chicken, rather than guinea pig (Table 13), RBCs. rPIV3-1, like its PIV1/Wash64 parent, required trypsin for efficient replication in cultures with fluid overlay as well as for efficient plaque formation. rPIV3-1 produced plaques at 32°C, 37°C or 40°C with similar efficiency. It is therefore evident that rPIV3-1 possesses the F glycoprotein of the PIV1 parent virus, and it is not temperature sensitive. On the other hand, rPIV3-1 produced CPE, as indicated by the cell rounding and detaching in the virus infected monolayers, almost to the same extent as its PIV3 parent suggesting that this biological property is a function of its PIV3 genes, which lie outside of the HN and F coding regions. Thus, rPIV3-1 possesses biological properties from both parents which is consistent with the findings above demonstrating that it is a chimeric virus. This exemplary recombinant, chimeric virus within the invention was deposited on May 21, 1998 under the terms of the Budapest Treaty with the American Type Culture Collection (ATCC) of 12301 Parklawn Drive, Rockville, Maryland 20852, U.S.A.

**Table 13. Comparison of the HA titer and the infectivity and cytopathicity of parental and chimeric PIVs^{a}**

| Virus | HA titer using indicated RBC | | Infectious titer^{b} (Log₁₀TCID₅₀/ml) using CPE or HAD as endpoint | | | | PFU/ml^{c} (Log₁₀) | |
|---|---|---|---|---|---|---|---|---|
| | Chicken | Guinea Pig | CPE | | HAD | | Trypsin | |
| | | | Trypsin | | Trypsin | | | |
| | | | - | + | - | + | - | + |
| PIV1/Wash64 | 16 | 8 | ≤2.5 | ≤2.5 | 4.8^{d} | 6.3 | ≤0.7^{e} | 5.8 |
| rPIV3-1 | 64 | 16 | ≤2.5 | 5.8 | 5.5^{d} | 7.8 | ≤0.7^{e} | 7.1 |
| rPIV3/JS | 0 | 8 | 4.5 | 7.3 | 5.0^{d} | 7.5 | 5.0 | 6.2 |

| | | | | | | | | |
|---|---|---|---|---|---|---|---|---|
| ^{a}Virus stocks were grown in LLC-MK2 cells which were infected at an MOI of 0.01 and incubated for 6 days in the presence (PIV1/Wash64, rPIV3-1) or absence (rPIV3/JS) of 0.75 *µ*g/ml trypsin. The resulting virus stocks were assayed by the tests below in the presence or absence of trypsin as indicated. ^{b}The TCID₅₀ assay was read at 6 days by direct visualization of CPE or by hemadsorption (HAD). ^{c}Plaques were visualized by HAD after six days of incubation. ^{d}the HAD of PIV3-infected monolayers was grossly apparent whereas that of PIV1 and rPIV3-1 was observable only under the microscope in which single cells with RBC adsorbed were observed. ^{e}The lowest level of virus detectable was 10^{0.7}/ml. | | | | | | | | |

### Comparison of the level of replication of rPIV3-1 and its parental viruses in LLC-MK2 cells and hamsters.

The multicycle replication of rPIV3, rPIV3-1, and PIV1 Wash/64 viruses was evaluated following inoculation of LLC-MK2 tissue culture cells at a MOI of 0.01 (Fig. 18). It can be seen that the kinetics and magnitude of replication of the three viruses are very similar. This indicates that the substitution of the HN and F genes of PIV1 for those of PIV3 did not attenuate the virus for replication *in vitro*. It was next determined whether rPIV3-1 was attenuated *in vivo*, specifically for replication in the upper and lower respiratory tracts of hamsters (TABLE 14). The observed level of replication of rPIV3-1 was similar to, if not slightly higher than, either parent in the upper and lower respiratory tract of hamsters.

**Table 14. Level of replication of parental and chimeric PIVs in the upper and lower respiratory tract of hamsters^{a}**

| Virus | | Virus titer (mean in log₁₀ indicated tissue TCID₅₀/gram±S.E.) | | |
|---|---|---|---|---|
| | Nasal Turbinates | | Lungs | |
| | Day 4 | Day 5 | Day 4 | Day 5 |
| PIV1/Wash64 | 5.2±.24 | 5.2±.12 | 5.0±.31 | 5.0±.38 |
| rPIV3-1 | 4.9±.23 | 6.2±.17 | 5.8±.15 | 6.0±.09 |
| rPIV3/JS | 4.5±.09 | 5.0±.18 | 5.1±.26 | 5.0±.32 |

| | | | | |
|---|---|---|---|---|
| ^{a}Hamsters were infected intranasally with 10^{5.0} TCID₅₀ per animal of the indicated virus, and lungs and nasal turbinates were removed on day 4 or 5 after infection. The titers are means of six animals per day and are expressed as mean log₁₀TCID₅₀/gram ± standard error. | | | | |

In summary, the present Example demonstrates successful recovery of a rPIV3-1 chimeric virus in which the ORFs of the PIV1 HN and F glycoproteins were substituted for those of rPIV3. This chimeric virus replicated like its wild type PIV1 and PIV3 parental viruses *in vitro* and *in vivo*, demonstrating that the substitution of the glycoproteins ORFs did not result in attenuation of rPIV3-1. This successful recovery of a recombinant PIV3 which bears the HN and F glycoproteins of PIV1 is surprising because the two viruses, representing distinct serotypes, are not closely related. In particular, it is remarkable that the chimeric recombinant grows as well as the two parents. Notably, chimeric recombinant viruses possessing a substitution in the glycoprotein gene have also been recovered for vesicular stomatitis virus (VSV) (Lawson et al. Proc. Natl. Acad. Sci. USA 92:4477-81 (1995)). Specifically, the VSV G glycoproteins gene of Indiana serotype was replaced by that from New Jersey serotype which share only 50% amino acid sequence identity. In contrast to rPIV3-1, the chimeric recombinant VSV_{I/NJ} replicates to only 10% the level of recombinant VSV_{I} or biologically derived, VSV.

In the present Example, the HN and F glycoproteins have 43 and 47% sequence identity, respectively, between PIV1 and PIV3. The transfer of the two glycoproteins together would, of course, obviate glycoprotein-to-glycoprotein incompatibility (Tanabayashi, K. and Compans, R.W. J. Virol. 70:6112-18 (1996)). On the other hand, it is generally thought that the glycoproteins interact with the M protein (which is 63% identical between PIV1 and PIV3) through their cytoplasmic (CT) or transmembrane (TM) domains, and that this interaction is important in virion morphogenesis and structure. In this regard, the degree of sequence identity between the HN and F proteins of the two serotypes in the TM and CT domains is low indeed: 30% and 22%, respectively for the TM domain, and 9 and 11% respectively for the CT domain. In light of this low level of sequence relatedness, we have also pursued a parallel strategy of constructing chimeric glycoproteins in which the PIV1 ectodomain of each glycoprotein was fused to the PIV3 TM and CT domains. Regarding possible interaction with the M protein or other internal proteins, it might be that a conserved structure, such as a constellation of charged amino acids, is important rather than a conserved sequence. Alternatively, it might be that interaction of the TM and CT domains of the glycoproteins with internal proteins is not as critical as has been previously thought. It will be possible to examine these factors more closely using the methods and tools provided herein. For example, these factors will be further elucidated by work in progress employing the methods described above to construct a PIV3 virus bearing HN and F of PIV2.

It was expected that rPIV3-1 would require trypsin for efficient replication in tissue culture since this is a property conferred by the PIV1 F glycoprotein, and this was found to be the case. However, it was interesting to observe that rPIV3-1 caused CPE that more closely resembled that of PIV3 parent virus, indicating that a PIV3 gene(s) other than HN or F specifies this phenotype. These roles will also be further elucidated using the methods and tools provided herein to exchange additional gene(s) between the non-cytopathic PIV1 and the cytopathic PIV3.

### EXAMPLE XII

### Recovery of Live-Attenuated Chimeric Recombinant PIV Encoding the Internal Proteins of PIV Type 3 and the Surface Glycoproteins of PIV Type 1

In the present Example, a derivative of rPIV3-1 carrying the three temperature-sensitive and attenuating amino acid coding changes found in the L gene of the live-attenuated cp45 PIV3 candidate vaccine virus, termed rPIV3-1.cp45L, is shown to exhibit a temperature sensitive phenotype with a shut-off temperature of 38°C, similar to that of the recombinant rPIV3cp45L which possesses the same three mutations. rPIV3-1.*cp*45L is attenuated in the respiratory tract of hamsters to the same extent as rPIV3cp45L. Infection of hamsters with rPIV3-1.cp45L generates a moderate level of hemagglutination-inhibiting antibodies against wt PIV1 and induces complete resistance to challenge with wild type PIV1. This demonstrates that attenuated chimeric PIV according to the invention are capable of inducing a highly effective immune response against PIV1. This disclosure also confirms the above described data demonstrating that the surface glycoproteins of parainfluenza viruses are sufficient to induce a high level of resistance to homologous virus challenge. Unexpectedly, infection with recombinant chimeric virus rPIV3 1.*cp*45L or rPIV3 1, each bearing the surface glycoprotein genes of PIV1 and the internal genes of PIV3, also induces a moderate level of resistance to replication of PIV3 challenge virus. This indicates that the internal genes of PIV3 can independently induce protective immunity against PIV3 in rodents. Thus, a reverse genetics system for PIV3 as disclosed herein successfully produces live attenuated PIV1 vaccine candidates that are attenuated and protective in accepted model subjects.

### Viruses and cells.

The wt PIV1 strain used in this study is PIV1/Washington/20993/1964 (PIV1/Wash64) (see, eg., Murphy et al., Infect. Immun. 12:62, 1975). Chimeric rPIV3-1, recovered from chimeric PIV3 cDNA in which the PIV3 F and HN ORFs were replaced with those of PIV1/Wash64, as described above and in Tao ec al., J. Virol. 72 : 2955, 1998. These viruses were propagated in LLC-MK2 cells (ATCC CCL 7.1) in Opti-MEM I (Life Technologies, Gaithersburg, MD) with 50 *µ*g/ml gentamicin sulfate, and 0.75 *µ*g/ml trypsin (Catalog No. 3741, Worthington Biochemical Corp., Freehold, NJ). Trypsin is included because the F glycoprotein of PIV1, but not that of PIV3, is dependent on exogenous trypsin for cleavage when grown in cell culture under these conditions. The wt JS strain of human PIV3 virus and its recombinant derivative from cDNA (rPIV3/JS) were propagated as described above and in Durbin et al., virology 235:323, 1997. The propagation of cp45, an attenuated derivative of wt PIV3/JS (see above; and Karron et al., J. Infect . Dis. 171:1107, 1995), and rPIV3cp45L, a recombinant PIV3 carrying the three ts mutations found, in the L gene of cp45, were propagated as described above and in Skiadopoulos et al., J Virol 72:1762, 1998. The modified vaccinia Ankara (MVA) recombinant that expresses the bacteriophage T7 RNA polymerase is described in Virology 210: 202, 1995.

HEp-2 cells, which are used in transfection, were obtained from ATCC (ATCC CCL 23) and maintained in Opti-MEM I with 2% fetal bovine serum (FBS), 50 *µ*g/ml gentamicin sulfate.

### Introduction of L. mutations into rPIV3-1 antigenomic cDNA.

The three L mutations of cp45 present in the pTM(L)942/992/1558 plasmid, described above (see also, Skiadopoulos et al., J.Virol 72:1762, 1998, were introduced into chimeric cDNA pFLC.2G+.hc (described above; see also, Tao et al., J Virol 72 : 2955, 1998), as a 2.8 kb SphI-NheI fragment (nt 11313 to 14092 in PIV3 antigenomic cDNA) to generate the full-length pFLC.2G+.hc.cp45L bearing the PIV1 F and HN ORFs and the three cp45 L gene mutations (Fig. 19). The specific mutations present in pTM(L)942/992/1558 are indicated in the legend to Figure 19.

**Transfection**. HEp-2 cell monolayers in six-well plates were grown to confluence and transfections were performed as described above (see also, Tao et al., J virol 72:2955, 1998). Trypsin was added to a final concentration of 0.75 *µ*g/ml on day 3 post transfection prior to harvesting on day 4. Cell culture supernatants were clarified and passaged (referred to as passage 1) onto fresh LLC-MK2 cell monolayers. After overnight adsorption, the medium was replaced with fresh Opti-MEM I with 0.75 *µ*g/ml trypsin. Passage 1 cultures were incubated at 32°C for 4 days, and the virus present in the supernatant was harvested and passaged again under the same conditions (referred to as passage 2). Virus present in the passage 2 harvest was tested for the presence of the PIV1 HN protein by hemagglutination-inhibition (HAI) assay as described above (see also Tao et al., J Virol 72:2955, 1998).

### Replication of PIVs in LLC-MK2 at various temperatures.

Plaque enumeration on LLC-MK2 monolayers was performed as described above, with 0.75 *µ*g/ml trypsin added to the agarose overlay in the case of PIV1, rPIV3-1, and rPIV3-1.*cp*45L (see also, Tao et al., J Virol 72:2955, 1998). After incubation at various temperatures for 6 days, the agarose overlay was removed and plaques were identified by hemadsorption (HAD) with guinea pig erythrocytes (RBCs).

### Replication of PIVs in the respiratory tract of hamsters.

Groups of five hamsters were inoculated intranasally with 0.1 ml of L15 medium containing 10⁶ plaque forming units (PFU) of rPIV3/JS, rPIV3cp45L, cp45, PIV1/Wash64, rPIV3-1, or rPIV3-1.*cp*45L. Hamsters were sacrificed on day 4 post-infection, and their lungs and nasal turbinates were harvested and homogenized. Virus present in the tissue samples was titered on LLC-MK2 cell monolayers at 32°C as described above and in Tao et al., J Virol 72:2955, 1998. The titers are expressed as reciprocal mean log₁₀TCID₅₀/gram of tissue for each group.

### Immunization and challenge studies in hamsters.

Groups of ten hamsters were immunized intranasally with 10⁶ PFU of virus per animal, as described above. Serum was collected for HAI assay prior to infection and on day 33. The level of HAI antibodies present in the sera of each group of 10 hamsters was determined using PIV1/Wash64 and PIV3/JS as antigens, and the HAI titers determined are presented as mean log₂ (see also, Tao et al., J Virol 72:2955, 1998).

Thirty-five days post-immunization, five hamsters from each group were challenged intranasally with 10⁶ PFU of either PIV1/Wash64 or rPIV3/JS. Nasal turbinates and lungs of these challenged hamsters were harvested four days post challenge. Virus titers in tissue samples were determined on LLC-MK2 monolayers as described above and in Tao et al., J Virol 72:2955, 1998, and the titers are presented as mean log₁₀TCID₅₀/gram of tissue.

### RESULTS

### Recovery and characterization of the recombinant chimeric virus rPIV3-1.cp45L.

As noted above, the cDNA clone pFLC.2G+.hc, a full-length antigenomic cDNA of PIV3 in which the ORFs encoding the F and HN glycoproteins have been replaced by those of PIV1, was modified by introduction of three amino acid coding changes (designated 942, 992 and 1558, according to amino acid position in the L protein) identified in the L gene of cp45 and shown to be independent ts and attenuating mutations (Fig. 19; see also, Skiadopoulos et al., J Virol 72:1762, 1998). Each coding change was marked by the co-introduction of contiguous translationally silent nt substitutions that ablate a naturally-occurring restriction site (Fig. 19; Table 8). The final full-length plasmid construct, pFLC.2G+.hc.cp45L (Fig. 19), encodes a PIV3-1 chimeric antigenomic RNA of 15516 nt in length and conforms to the rule of six (see, Durbin et al., Virology 234:74, 1997). The authenticity of pFLC.2G+.hc.cp45L was confirmed by digestion with appropriate restriction enzymes.

The pFLC.2G+.hc.cp45L cDNA was transfected into HEp-2 cells together with the PIV3 N, P and L support plasmids and infected with MVA-T7 as described above and in Tao et al., J Virol 72:2955, 1998). Virus recovered after two passages on LLC-MK2 cells, termed rPIV3-1-cp45L, was biologically cloned by plaque-to-plaque-to-plaque passage, and amplified virus was analyzed to confirm that it possessed the PIV1 glycoproteins and the three introduced mutations in L. First, the presence of the PIV1 HN protein in rPIV3-1.cp45L was confirmed by reactivity with PIV1 specific antibodies in HAI assay as described above and in Tao et al., J Virol 72:2955, 1998). The presence of the chimeric PIV3-1 HN and F genes as well as the introduced L gene mutations in rPIV3-1.*cp*45L genomic RNA was confirmed by restriction enzyme digestion or nucleotide sequence analysis of RT-PCR products generated from virion RNA as described above and in Tao et al., J Virol 72:2955, 1998. These data confirmed that rPIV3-1.cp45L is a recombinant chimeric virus bearing the three codon substitutions of the L gene of cp45.

### rPIV3-1.cp45L is temperature sensitive.

The three L gene mutations of cp45 were shown above to confer the ts phenotype when introduced into wt PIV3 (see also, Skiadopoulos et al., J Virol 72:1762, 1998). To evaluate whether their presence in the chimeric virus would have the same effect, the efficiency of plaque formation of rPIV3-1.*cp*45L was determined at various temperatures. As shown in Table 15, the three L mutations indeed conferred the ts phenotype to the chimeric virus. The level of temperature sensitivity specified by the cp45 L mutations in the recombinant viruses rPIV3cp45L and rPIV3-1. *cp*45L was equivalent (Table 15), indicating that the effect of the mutations is independent of the PIV3 or PIV1 HN and F glycoproteins. The level of temperature sensitivity of rPIV3cp45L and rPIV3-1.cp45L was comparable to that of the biologically derived cp45 virus, despite the fact that the latter virus possesses mutations outside of L (see, Stokes et al., Virus Res 30:43, 1993.

**Table 15. The recombinant chimeric rPIV3-1.cp45L candidate vaccine virus is temperature sensitive**

| **Virus ^{a}** | **Virus titer ^{b} at indicated temperatures (log₁₀PFU/ml)** | | | | | |
|---|---|---|---|---|---|---|
| | 32°C | 36°C | 37°C | 38°C | 39°C | 40°C |
| rPIV3/JS | 7.4 | 7.2 | 7.2 | 7.2 | 7.2 | 7.2 |
| PIV1/Wash64 ^{c} | 7.5 | 7.6 | 7.5 | 7.5 | 7.4 | 7.2 |
| rPIV3-1 | 7.5 | 7.5 | 7.5 | 7.2 | 6.0 | 6.1 |
| PIV3cp45 ^{c} | 7.4 | 6.9 | 6.8 | 4.7 ^{d} | <0.7 | <0.7 |
| rPIV3cp45L | 7.9 | 7.4 | 7.7 | 5.3 | 1.2 | <0.7 |
| rPIV3-1.cp45L | 8.1 | 8.0 | 8.2 | 6.1 | <0.7 | <0.7 |

| | | | | | | |
|---|---|---|---|---|---|---|
| ^{a} Virus nomenclature: rPIV3/JS, recombinant wt PIV3 strain JS; PIV1/Wash64, biologically-derived wt PIV1; rPIV3-1, recombinant chimeric PIV3 in which the F and HN ORFs have been replaced with those of PIV1/Wash64; PIV3cp45, biologically-derived cp45 candidate vaccine virus; rPIV3cp45L, recombinant PIV3 containing the three L gene mutations of cp45; rPIV3-1.cp45L, recombinant chimeric rPIV3-1 containing the three L gene mutations of cp45. ^{b} Virus titers were determined using LLC-MK2 monolayers in 12-well plates. Titers are the average of two assays. ^{c} Biologically-derived viruses. All others are recombinant viruses. ^{d} The shut-off temperature, i.e. the lowest restrictive temperature at which a two log₁₀ reduction in virus titer is observed, of each ts virus is indicated in bold. | | | | | | |

### Level of replication of rPIV3-1.cp45L in hamsters.

The three L gene mutations of cp45 were shown above to confer attenuation of virus replication in the upper and lower respiratory tract of hamsters when introduced into wt PIV3 (see also, Skiadopoulos et al., J Virol 72:1762, 1998). Their effect on the chimeric virus was evaluated by intranasal infection of hamsters, as shown in Table 16. These findings indicate that rPIV3-1.cp45L indeed was attenuated at both sites and, furthermore, that its level of attenuation was comparable to that of rPIV3cp45L. Thus, the ability of the cp45 L mutations to confer attenuation, like temperature sensitivity, is independent of the antigenic specificity of the surface glycoproteins.

**Table 16. The recombinant chimeric rPIV3-1.cp45L candidate vaccine virus is attenuated in the respiratory tract of hamsters a**

| Virus | Virus titer in indicated tissue (log₁₀TCID₅₀/g±S.E) ^{b} | |
|---|---|---|
| | Nasal turbinates | Lungs |
| rPIV3-1.cp45L | 4.6±0.3 | 1.9±0.4 |
| rPIV3-1 | 6.0±0.3 | 6.3±0.4 |
| rPIV3cp45L | 3.0±0.3 | <1.2 |
| rPIV3/JS | 5.7±0.3 | 5.0±0.3 |

| | | |
|---|---|---|
| ^{a} Groups of five hamsters were infected intranasally with indicated viruses at a dosage of 10⁶ PFU per hamster. On day 4 post infection, the tissue samples were harvested and assayed for virus. ^{b} Virus titers are given as Log₁₀TCID₅₀ per gram of tissue. | | |

### Infection with rPIV3-1 or rPIV3-1.cp45L, containing the internal proteins of PIV3 and the glycoproteins of PIV1, confers resistance to PIV1 challenge in hamsters.

The chimeric rPIV3-1 virus and its attenuated rPIV3-1.cp45L derivative were evaluated for immunogenicity and protective efficacy in hamsters. As shown in Table 17, infection with either virus induced HAI antibodies against PIV1, but not PIV3, confirming that these chimeric viruses possess the PIV1 HN glycoprotein and are highly immunogenic. The level of HAI antibodies induced by rPIV3-1.cp45L was two-fold less than that by rPIV3-1, which indicates that its attenuation resulted in a modest decrease in immunogenicity. Similarly, rPIV3 and rPIV3cp45L induced HAI antibodies against PIV3, but not PIV1, and the level induced by the attenuated virus was approximately two-fold lower. Despite the restricted replication in hamsters of the recombinant viruses bearing the cp45 L mutations, infection with either rPIV3cp45L or rPIV3-1.cp45L induced complete resistance to replication of challenge virus bearing homologous glycoproteins.

**Table 17. The recombinant chimeric rPIV3-1.cp45L candidate vaccine virus induces complete resistance to PIV1 and partial resistance to PIV3 upon challenge in hamsters ^{a}**

| Virus used for immunization | Origin of glycoproteins | Post-immunization HAI titer (log₂Reciprocal±S.E.) | | Virus Titer in Indicated Tissue ^{c} (log₁₀TCID₅₀/Gram±S.E.) | | | |
|---|---|---|---|---|---|---|---|
| | | | | Replication of PIV1 challenge virus | | Replication of PIV3 challenge virus | |
| | | α-PIV1 | α-PIV3 | Nasal Turbinates | Lungs | Nasal Turbinates | Lungs |
| Control ^{b} | - | ≤1 | ≤1 | 5.0±0.3 | 4.6±0.5 | 5.5±0.4 | 5.0±0.7 |
| rPIV3-1.cp45L | PIV1 | 6.9±0.5 | ≤1 | ≤1.2 | ≤1.2 | 1.9±0.6 | 1.7±0.5 |
| rPIV3-1 | PIV1 | 7.9±0.4 | ≤1 | ≤1.2 | ≤1.2 | 2.9±0.3 | 2.6±0.4 |
| rPIV3cp45L | PIV3 | ≤1 | 9.3±0.2 | 4.6±0.2 | 2.8±0.7 | ≤1.2 | ≤1.2 |
| rPIV3/JS | PIV3 | ≤1 | 10.3±0.3 | 4.6±0.4 | 2.4±0.5 | ≤1.2 | ≤1.2 |
| PIV3p45^{d} | PIV3 | ≤1 | 8.9±0.4 | 4.9±0.2 | 2.5±0.8 | ≤1.2 | ≤1.2 |

| | | | | | | | |
|---|---|---|---|---|---|---|---|
| ^{a} Groups of 10 hamsters were immunized intranasally with 10⁶ PFU of indicated viruses. Post-immunization sera were collected on days 33, two days prior to challenge (see ^{c}). ^{b} Hamsters in control group were not inoculated. ^{c} Five weeks after immunization, five hamsters from each group were challenged intranasally with **10⁶** PFU of indicated virus. Tissue samples were harvested 4 days post challenge. Viruses present in tissue samples were titered on LLC-MK2 monolayers and the data are presented as log₁₀TCID₅₀/gram of tissue ± standard error. ^{d} Biologically-derived virus. | | | | | | | |

### Infection with rPIV3-1.cp45L also confers resistance to PIV3.

Information on the role of the non-HN or F glycoproteins of PIVs (i.e., the internal proteins) in resistance is limited. The disclosure and use of rPIV3-1 and rPIV3-1.*cp*45L herein provides an opportunity to examine the role that internal proteins play in resistance to challenge with PIV3, since the only genes shared by immunizing and challenge viruses are the internal protein genes. PIV3 challenge virus replication was significantly restricted in both the upper and lower respiratory tracts by prior infection of hamsters with rPIV3-1 or rPIV3-1.cp45L (Table 17). Thus, these data indicate that the internal proteins of PIV3, like the HN and F proteins, are capable of inducing partial resistance to replication of the challenge PIV3.

Among the findings demonstrated by the immunogenicity and efficacy studies above a particularly unexpected finding was that infection with wt or attenuated rPIV3 induced a 100-fold reduction in the replication of PIV1 challenge virus in the lungs. Thus, infection with one serotype of PIV provided significant protection against a heterologous serotype. This was unexpected in part because previous studies indicated that infection of animals with one type of human PIV did not induce significant heterologous protection against a PIV belonging to a different human serotype, conforming to a general belief that immunity to human PIV infections was largely type-specific (see, eg., Cook et al., Amer. Jour. Hyg. 77:150, 1963; Ray et al., J. Infect. Dis. 162:746, 1990,).

The present Example demonstrates successful exploitation of novel methods and reagents developed for generating PIV3 vaccines to further provide rapid, rational development of live attenuated candidate vaccines for PIV1. A cDNA encoding infectious PIV3 was modified by substitution of the ORFs encoding the PIV1 HN and F protective antigens for their PIV3 counterparts. Subsequently, attenuating mutations, exemplified by three attenuating mutations present in the L gene of the cp45 PIV3, were incorporated within this modified chimeric PIV3-PIV1 cDNA. From this cDNA, a recombinant virus was recovered bearing the HN and F genes of PIV1, the internal proteins of PIV3, and the PIV3 cp45 L gene mutations. This recombinant, rPIV3-1.cp45L, was temperature sensitive, highly attenuated in hamsters, and highly efficacious against PIV1 challenge in hamsters. The level of temperature sensitivity, attenuation, and immunogenicity exhibited by rPIV3-1.cp45L was comparable to that of cp45 PIV3, indicating that the phenotypes specified by the set of cp45 L gene mutations are independent of the HN and F surface glycoproteins. These findings, which represent the first live attenuated PIV1 vaccine candidate generated by reverse genetics, provide a generally successful scheme for developing vaccines against PIV1.

Little information is known concerning the role that internal proteins of parainfluenza viruses play in resistance to reinfection with homologous virus. Infection with vaccinia recombinants expressing N, epitopes within N, or M reportedly induce resistance to replication of challenge virus, but the magnitude of the resistance reported is less than that induced by vaccinia recombinants bearing HN or F glycoproteins (see, eg., Kast et al., Proc._Natl. Acad. Sci. USA 88 : 2283, 1991; Sakaguchi et al., J. Gen. Virol. 74:479, 1993; Thomson et al., J. Immunol. 157:822, 1996). These studies suggested that the internal proteins were making only minor contributions to resistance to reinfection. Therefore, the present disclosure presents unexpected results by showing that prior infection of hamsters with rPIV3-1.*cp*45L or rPIV3-1 induced about 250-to 4000-fold reduction of replication of PIV3 in both the nasal turbinates and lungs. These two chimeric recombinant viruses differ from the PIV3 challenge virus in that they possess the HN and F glycoproteins of PIV1 rather than PIV3, but they share all other genes with the challenge virus. The HN and F glycoproteins of PIV1 share 47% and 43% sequence identity with those of PIV3, respectively. Although it is likely that the shared internal proteins are mediating the observed resistance, it is also possible that shared protein sequences between PIV1 and PIV3 F and HN glycoproteins are contributing to the observed immunity. For example, there are 5 stretches in HN and 2 stretches in F extending at least 9 amino acid residues in length that are shared between PIV1 and PIV3 and have the potential to act as protective CTL epitopes. It is reasonable to consider that the shared internal proteins are contributing to the restriction of replication of wt PIV3 challenge virus, since this level of cross-immunity has not been seen in previous studies (see, eg., Cook et al., Amer. Jour. Hyg. 77:150, 1963; Ray et al., J. Infect. Dis. 162:746, 1990).

The finding that the internal PIV3 proteins of the rPIV3-1 and rPIV3-1.*cp*45L chimeras conferred resistance to PIV3 challenge demonstrates that attenuated derivatives of PIV3 can be used as vectors for PIV1 and PIV2 protective antigens. Following the teachings of the invention, immunization with one PIV3-based live-attenuated vaccine virus can restrict the replication of other PIV3-based vaccine viruses administered subsequently, thereby decreasing the immunogenicity of the second virus. Since PIV3, like RSV, induces significant illness in early infancy, a combined RSV-PIV3 vaccine for use in the very young 2- to 4-week old infant is therefore an important aspect of the invention (see, eg., Collins et al., Fields Virology 3rd ed. Philadelphia: Lippincott-Raven Publishers, 1205 (1), 1996; Reed et al., J. Infect. Dis. 175:807, 1997). According to this aspect of the invention, immunization with a PIV1-PIV2 vaccine will be preferably initiated at about 6 months of age, since most PIV1 and PIV2 disease occurs after the age of six months. In the possible circumstance that immunization with rPIV3 cp45 significantly inhibits replication of a chimeric recombinant PIV3-1 vaccine virus with which it shares internal protein genes, successful immunization with a recombinant PIV3-1 vaccine may be compromised. In this event, a trivalent PIV vaccine will be administered simultaneously rather than sequentially, thereby preventing the above noted inhibition.

The disclosure herein that infection with a vaccine or wt PIV3 would induce a 100-fold reduction of pulmonary virus replication of the heterologous wt PIV1 was clearly unexpected, in part because the human PIV viruses are serologically distinct by neutralization assay, and previous studies in hamsters found that prior infection with one type of PIV failed to induce resistance to challenge with a high dose of a different PIV type (see eg., Cook et al., Amer. Jour. Hyg. 77:150, 1963; Ray et al., J. Infect. Dis. 162:746, 1990; Cook et al., Amer. Jour. Hyg. 69:250, 1959). Furthermore, there is little epidemiological data documenting that prior infection with one PIV significantly modifies subsequent infection with a heterotypic PIV.

In summary, the present Example shows that rPIV3 was successfully converted into a vaccine for PIV1 by substituting the ORFs encoding the F and HN glycoproteins and introducing known attenuating mutations into the PIV3 internal genes. Thus, the extensive methods and reagents provided herein can be applied directly and predictably to attenuating the PIV3 backbone of the rPIV3-1 chimeric virus, as well as for generating live-attenuated PIV2 vaccine viruses.

The foregoing disclosure makes it possible to exploit the reagents and methods provided herein to develop a broad assemblage of PIV and related vaccines. In this context, recovery of live, immunogenic chimeras between PIV3 and PIV2 exemplifies powerful new tools for developing a range of recombinant PIV viruses for vaccine use. In conjunction with this work, identification and characterization of the genetic basis for attenuation of naturally occurring PIV mutants, e.g., cp45 and BPIV3 vaccine candidates, following the teachings of the present disclosure also enables development of a large host of recombinant vaccine viruses and subviral particles. In particular, desired mutations present in biologically derived mutant viruses will be readily identified and selected by their introduction, singly and in combination, into a wild type, partially attenuated, or chimeric PIV background, as shown in the Examples above. These findings will expand the menu of exemplary, attenuating mutations within the invention which can introduced into PIV clones to calibrate the level of attenuation and immunogenicity in vaccine recombinants. Biologically deprived mutations can also be introduced within PIV clones having different types of mutations, e.g., mutations involving alterations, deletions, or substitutions of a gene or gene segment. Within this aspect of the invention, recombinant PIV are provided which have a selected gene deletion, addition, or substitution, such as rPIV having a deletion of the C, D or V ORF(s). Such alternatively mutated clones can be further modified according to the present disclosure by introducing one or more mutations specifying a *ts*, *ca* or *att* phenotype adopted from a biologically derived mutant PIV, as exemplified by the PIV recombinants r942, r992, r1558, r942/992, r992/1558, or r942/1558, and r942/992/1558. In additional aspects of the invention, biologically derived mutations will be combined with *de novo* attenuating mutations not found in nature, as exemplified by attenuating gene deletions, e.g., of the C, D and/or V ORFs. Other types of mutations disclosed herein conferring desired phenotypic characteristics will also be combined with biologically derived, attenuating mutations, similar to the range of combinatorial mutations disclosed for recombinant RSV vaccine strains in United States Patent Application Serial No. 08/892, 403, filed July 15, 1997. Comparable mutations can be readily introduced, e.g., into a chimeric virus, to achieve a desired levels of attenuation and immunogenicity in a chimeric vaccine strain. In this manner, a large menu of mutations are provided within the invention that are useful to engineer a wide assemblage of live attenuated rPIV vaccines having a desired balance of attenuation and immunogenicity, along with other desired phenotypic characteristics.

Although the foregoing invention has been described In some detail by way of illustration and example for purposes of clarity of understanding, it will be obvious that certain changes and modifications may be practiced within the scope of the appended claims.

## Claims

1. An isolated polynucleotide molecule comprising an operably linked transcriptional promoter, a polynucleotide sequence encoding a PIV genome or antigenome and a transcriptional terminator, wherein said polynucleotide sequence encoding said PIV genome or antigenome is selected from the group consisting of: SEQ ID NO:1, SEQ ID NO:14, and SEQ ID NO:15, or the complementary sequence thereof, and is modified by introduction of a heterologous PIV sequence selected from a HPIV1 sequence, a HPIV2 sequence, or a BPIV sequence to form a chimeric PIV genome or antigenome.

2. The isolated polynucleotide molecule of claim 1, wherein the polynucleotide sequence encoding the genome or antigenome incorporates a BPIV gene or gene segment.

3. The isolated polynucleotide molecule of claim 1, which incorporates a heterologous sequence from a respiratory syncytial virus or measles virus.

4. The isolated polynucleotide of claim 1 in which the polynucleotide encoding the chimeric PIV genome or antigenome is further modified by a nucleotide insertion, rearrangement, deletion or substitution specifying a phenotypic alteration selected from attenuation, temperature-sensitivity, cold-adaptation, small plaque size, host range restriction, or a change in an immunogenic epitope of PIV.

5. The isolated polynucleotide of any one of claims 1 to 4, wherein the PIV genome of antigenome encodes an infectious PIV particle.

6. The isolated polynucleotide molecule of claim 4 or 5, wherein said polynucleotide sequence encoding said PIV genome or antigenome incorporates one or more mutations of JS cp45.

7. The isolated polynucleotide molecule of claim 4 or 5, wherein said polynucleotide sequence encoding said PIV genome or antigenome incorporates a mutation stabilized by multiple nucleotide substitutions in a codon specifying the mutation.

8. The isolated polynucleotide molecule of claim 4 or 5, wherein said chimeric genome or antigenome incorporates multiple mutations each specifying a phenotype selected from attenuation, temperature-sensitivity, cold-adaptation, small plaque size, or host range restriction.

9. The isolated polynucleotide molecule of claim 4 or 5, wherein a mutation specifying a phenotypic alteration selected from attenuation, temperature-sensitivity, cold-adaptation, small plaque size, host range restriction, or a change in an immunogenic epitope of PIV is incorporated in a chimeric PIV background comprising a genome or antigenome having one or more of the PIV3 HN or F glycoprotein gene or genome segment substituted by one or more counterpart PIV1 or PIV2 HN and F glycoprotein gene or genome segment.

10. The isolated polynucleotide molecule of claim 4 or 5, which incorporates a cis-acting regulatory sequence of HPIV1, HPIV2, or BPIV.

11. The isolated polynucleotide molecule of any one of claims 4 to 10 which incorporates a heterologous sequence from a respiratory syncytial virus or measles virus.

12. The isolated polynucleotide molecule of any one of the preceding claims, which incorporates a polynucleotide sequence encoding a non-PIV molecule selected from a cytokine, a T-helper epitope, a restriction site marker, or a protein of a microbial pathogen capable of eliciting a protective immune response in a mammalian host.

13. A cell or cell-free composition including an expression vector which comprises an isolated polynucleotide molecule encoding a PIV genome or antigenome and an expression vector which comprises one or more isolated polynucleotide molecules that encode N, P and L proteins of PIV, wherein the polynucleotide molecule encoding said PIV genome or antigenome is selected from the group consisting of: SEQ ID NO:1, SEQ ID NO:14, and SEQ ID NO:15, or the complementary sequence thereof, and is modified by introduction of a heterologous PIV sequence selected from a HPIV1 sequence, a HPIV2 sequence, or a BPIV sequence to form a chimeric PIV genome or antigenome, whereby expression of said PIV genome or antigenome and N, P, and L proteins yields an infectious PIV particle.

14. The cell or cell-free composition of claim 13, wherein the infectious PIV particle is a virus.

15. The cell or cell-free composition of claim 13, wherein the infectious PIV particle is a subviral particle.

16. A method for producing an infectious PIV particle from one or more isolated polynucleotide molecules encoding said PIV, comprising: coexpressing in a cell or cell-free system an expression vector which comprises a polynucleotide molecule encoding a PIV genome or antigenome and an expression vector which comprises one or more polynucleotide molecules encoding N, P and L proteins, thereby producing an infectious PIV particle wherein the polynucleotide molecule encoding said PIV genome or antigenome is selected from the group consisting of: SEQ ID NO:1, SEQ ID NO:14, and SEQ ID NO:15, or the complementary sequence thereof, and is modified by introduction of a heterologous PIV sequence selected from a HPIV1 sequence, a HPIV2 sequence, or a BPIV sequence to form a chimeric PIV genome or antigenome.

17. The method of claim 16, wherein the polynucleotide molecule that encodes the PIV genome or antigenome is cDNA.

18. The method of claim 16, wherein the infectious PIV particle is a virus.

19. The method of claim 16, wherein the infectious PIV particle is a subviral particle.

20. The method of any one of claims 16 to 19, wherein the polynucleotide molecule encoding the chimeric PIV genome or antigenome is a chimera of human and bovine PIV sequences.

21. The method of any one of claims 16 to 20, wherein the polynucleotide molecule encoding the PIV genome or antigenome further incorporates an attenuating mutation from a biologically derived PIV strain.

22. The method of claim 21, wherein the polynucleotide molecule encoding the PIV genome or antigenome incorporates at least one mutation of JS cp45.

23. The method of claim 21 or 22, wherein said polynucleotide molecule encoding the PIV genome or antigenome incorporates a mutation that is stabilized by multiple nucleotide substitutions in a codon which specifies the mutation.

24. The method of any one of claims 16 to 23, wherein a heterologous sequence from HPIV1 or HPIV2 encoding a gene or gene segment of an HN or F glycoprotein is substituted for a corresponding gene or gene segment of HPIV3.

25. The method of any one of claims 16 to 24, wherein said chimeric genome or antigenome incorporates multiple mutations each specifying a phenotype selected from attenuation, temperature-sensitivity, cold-adaptation, small plaque size, or host range restriction.

26. The method of any one of claims 16 to 25, wherein said polynucleotide molecule encoding said PIV genome or antigenome incorporates a heterologous sequence from a respiratory syncytial virus or measles virus.

27. The method of any one of claims 16 to 26, wherein the polynucleotide molecule encoding the PIV genome or antigenome is modified to encode a non-PIV molecule selected from a cytokine, a T-helper epitope, a restriction site marker, or a protein of a microbial pathogen capable of eliciting a protective immune response in a mammalian host.

28. An infectious PIV particle which comprises a recombinant PIV genome or antigenome, a N protein, a P protein, and a L protein, wherein the recombinant PIV genome or antigenome is selected from the group consisting of: SEQ ID NO: 1, SEQ ID NO: 14, and SEQ ID NO: 15, or the complementary sequence thereof, and is modified by introduction of a heterologous PIV sequence selected from a HPIV1 sequence, a HPIV2 sequence, or a BPIV sequence to form a chimeric PIV genome or antigenome.

29. The infectious PIV particle of claim 28, which is a subviral particle.

30. The infectious PIV particle of claim 28, which is a virus.

31. The infectious PIV particle of any one of claims 28 to 30, wherein the recombinant PIV genome or antigenome incorporates a heterologous sequence from RSV or measles virus.

32. The infectious PIV particle of any one of claims 28 to 31, wherein the recombinant PIV genome or antigenome further comprises a nucleotide insertion, rearrangement, deletion or substitution specifying a phenotypic alteration selected from attenuation, temperature-sensitivity, cold-adaptation, small plaque size, host range restriction, or a change in an immunogenic epitope of PIV.

33. The infectious PIV particle of any one of claims 28 to 32, wherein the recombinant PIV genome or antigenome incorporates at least one mutation of JS cp45.

34. The infectious PIV particle of any one of claims 28 to 33, wherein a mutation specifying a phenotypic alteration selected from attenuation, temperature-sensitivity, cold-adaptation, small plaque size, host range restriction, or a change in an immunogenic epitope of PIV is incorporated in a chimeric PIV background comprising a genome or antigenome having one or more PIV3 HN or F glycoprotein genes or gene segments substituted by one or more counterpart PIV1 or PIV2 HN and F glycoprotein genes or gene segments.

35. The infectious PIV particle of any one of claims 28 to 34, wherein the recombinant PIV genome or antigenome is modified to encode a non-PIV molecule selected from a cytokine, a T-helper epitope, a restriction site marker, or a protein of a microbial pathogen capable of eliciting a protective immune response in a mammalian host.

36. The infectious PIV particle of any one of claims 28 to 35, in which the chimeric PIV genome or antigenome further comprises a polynucleotide encoding an RSV antigen or epitope which elicits protective immunity to RSV in an immunized host.

37. The infectious PIV particle of any one of claims 28 to 35 in which the chimeric PIV genome or antigenome further comprises a polynucleotide encoding a measles virus antigen or epitope.

38. An immunogenic composition comprising an immunogenically effective amount of an infectious PIV particle of any one of claims 28 to 37 in a pharmaceutically acceptable carrier.

## Patentansprüche

1. Isoliertes Polynucleotidmolekül, das einen operabel gebundenen Transkriptionspromotor, eine für ein PIV-Genom oder -Antigenom kodierende Polynucleotidsequenz und einen Transkriptionsterminator umfasst, worin die für das PIV-Genom oder -Antigenom kodierende Polynucleotidsequenz aus der aus Seq.-ID Nr. 1, Seq.-ID Nr. 14 und Seq.-ID Nr. 15 oder der komplementären Sequenz davon bestehenden Gruppe ausgewählt ist und durch die Inkorporation einer aus einer HPIV1-Sequenz, einer HPIV2-Sequenz oder einer BPIV-Sequenz ausgewählten heterologen PIV-Sequenz zur Bildung eines chimären PIV-Genoms oder -Antigenoms modifiziert ist.

2. Isoliertes Polynucleotidmolekül nach Anspruch 1, worin die für das Genom oder Antigenom kodierende Polynucleotidsequenz ein BPIV-Gen oder -Gensegment inkorporiert.

3. Isoliertes Polynucleotidmolekül nach Anspruch 1, das eine heterologe Sequenz eines respiratorischen Synzytial- oder eines Masernvirus inkorporiert.

4. Isoliertes Polynucleotidmolekül nach Anspruch 1, worin das für das chimäre PIV-Genom oder -Antigenom kodierende Polynucleotid weiter durch eine Insertion, Neuanordnung, Deletion oder Substitution eines Nucleotids modifiziert ist, wodurch eine Veränderung des Phänotyps festgelegt wird, der aus Attenuation, Temperaturempfindlichkeit, Kälteanpassung, Größe der kleinen Plaques, Einschränkung des Wirtsspektrums oder Änderung eines immunogenen Epitops von PIV ausgewählt ist.

5. Isoliertes Polynucleotidmolekül nach einem der Ansprüche 1 bis 4, worin das PIV-Genom oder -Antigenom für ein infektiöses PIV-Partikel kodiert.

6. Isoliertes Polynucleotidmolekül nach Anspruch 4 oder 5, worin die für das PIV-Genom oder -Antigenom kodierende Polynucleotidsequenz eine oder mehrere JS-cp45-Mutationen inkorporiert.

7. Isoliertes Polynucleotidmolekül nach Anspruch 4 oder 5, worin die für das PIV-Genom oder -Antigenom kodierende Polynucleotidsequenz eine Mutation inkorporiert, die durch mehrere Nucleotidsubstitutionen in einem Codon, das die Mutation festlegt, stabilisiert ist.

8. Isoliertes Polynucleotidmolekül nach Anspruch 4 oder 5, worin das chimäre Genom oder Antigenom mehrere Mutationen inkorporiert, die jeweils einen Phänotyp festlegen, der aus Attenuation, Temperaturempfindlichkeit, Kälteanpassung, Größe der kleinen Plaques oder Einschränkung des Wirtsspektrums ausgewählt ist.

9. Isoliertes Polynucleotidmolekül nach Anspruch 4 oder 5, worin eine Mutation, die eine Veränderung des Phänotyps festlegt, der aus Attenuation, Temperaturempfindlichkeit, Kälteanpassung, Größe der kleinen Plaques oder Einschränkung des Wirtsspektrums oder einer Veränderung eines immunogenen Epitops von PIV ausgewählt ist, in einen chimären PIV-Hintergrund inkorporiert ist, der ein Genom oder Antigenom umfasst, das ein oder mehrere der PIV3-HN- oder -F-Glykoproteingen- oder -genomsegmente aufweist, die durch ein oder mehrere entsprechende PIV1- oder PIV2-HN- und -F-Glykoproteingen- oder -genomsegmente substituiert sind.

10. Isoliertes Polynucleotidmolekül nach Anspruch 4 oder 5, das eine cis-wirkende Regulationssequenz von HPIV1, HPIV2 oder BPIV inkorporiert.

11. Isoliertes Polynucleotidmolekül nach einem der Ansprüche 4 bis 10, das eine heterologe Sequenz eines respiratorischen Synzytial-Virus oder eines Masernvirus inkorporiert.

12. Isoliertes Polynucleotidmolekül nach einem der vorangegangenen Ansprüche, das eine Polynucleotidsequenz inkorporiert, die für ein Nicht-PIV-Molekül kodiert, das aus einem Zytokin, einem T-Helfer-Epitop, einem Restriktionsstellenmarker oder einem Protein eines mikrobiellen Pathogens ausgewählt ist, das in der Lage ist, eine schützende Immunreaktion in einem Säugetierwirt hervorzurufen.

13. Zell- oder zellfreie Zusammensetzung, die einen Expressionsvektor, der ein isoliertes Polynucleotidmolekül umfasst, das für ein PIV-Genom oder -Antigenom kodiert, und einen Expressionsvektor umfasst, der ein oder mehrere isolierte Polynucleotidmoleküle umfasst, die für N-, P- und L-Proteine von PIV kodieren, worin das für das PIV-Genom oder -Antigenom kodierende Polynucleotidmolekül aus der aus Seq.-ID Nr. 1, Seq.-ID Nr. 14 und Seq.-ID Nr. 15 oder der komplementären Sequenz davon bestehenden Gruppe ausgewählt ist und durch die Inkorporation einer aus einer HPIV1-Sequenz, einer HPIV2-Sequenz oder einer BPIV-Sequenz ausgewählten heterologen PIV-Sequenz zur Bildung eines chimären PIV-Genoms oder -Antigenoms modifiziert ist, wobei die Expression des PIV-Genoms oder -Antigenoms und der N-, P- und L-Proteine ein infektiöses PIV-Partikel liefert.

14. Zell- oder zellfreie Zusammensetzung nach Anspruch 13, worin das infektiöse PIV-Partikel ein Virus ist.

15. Zell- oder zellfreie Zusammensetzung nach Anspruch 13, worin das infektiöse PIV-Partikel ein subvirales Partikel ist.

16. Verfahren zur Herstellung eines infektiösen PIV-Partikels aus einem oder mehreren isolierten Polynucleotidmolekülen, die für das PIV kodieren, wobei das Verfahren Folgendes umfasst: das gemeinsame Exprimieren eines Expressionsvektors, der ein Polynucleotidmolekül umfasst, das für ein PIV-Genom oder -Antigenom kodiert, und eines Expressionsvektors, der ein oder mehrere Polynucleotidmoleküle umfasst, die für N-, P- und L-Proteine von PIV kodieren, in einer Zelle oder einem zellfreien System, wodurch ein infektiöses PIV-Partikel erzeugt wird, in dem das für das PIV-Genom oder -Antigenom kodierende Polynucleotidmolekül aus der aus Seq.-ID Nr. 1, Seq.-ID Nr. 14 und Seq.-ID Nr. 15 oder der komplementären Sequenz davon bestehenden Gruppe ausgewählt ist und durch die Inkorporation einer aus einer HPIV1-Sequenz, einer HPIV2-Sequenz oder einer BPIV-Sequenz ausgewählten heterologen PIV-Sequenz zur Bildung eines chimären PIV-Genoms oder -Antigenoms modifiziert ist.

17. Verfahren nach Anspruch 16, worin das Polynucleotidmolekül, das für das PIV-Genom oder -Antigenom kodiert, cDNA ist.

18. Verfahren nach Anspruch 16, worin das infektiöse PIV-Partikel ein Virus ist.

19. Verfahren nach Anspruch 16, worin das infektiöse PIV-Partikel ein subvirales Partikel ist.

20. Verfahren nach einem der Ansprüche 16 bis 19, worin das für das chimäre PIV-Genom oder -Antigenom kodierende Polynucleotidmolekül eine Chimäre menschlicher und boviner PIV-Sequenzen ist.

21. Verfahren nach einem der Ansprüche 16 bis 20, worin das für das PIV-Genom oder -Antigenom kodierende Polynucleotidmolekül weiters eine attenuierende Mutation von einem biologisch abgeleiteten PIV-Stamm inkorporiert.

22. Verfahren nach Anspruch 21, worin das für das PIV-Genom oder -Antigenom kodierende Polynucleotidmolekül zumindest eine Mutation von JS cp45 inkorporiert.

23. Verfahren nach Anspruch 21 oder 22, worin das für das PIV-Genom oder -Antigenom kodierende Polynucleotidmolekül eine Mutation inkorporiert, die durch mehrere Nucleotidsubstitutionen in einem Codon, das die Mutation festlegt, stabilisiert ist.

24. Verfahren nach einem der Ansprüche 16 bis 23, worin eine heterologe Sequenz von HPIV1 oder HPIV2, die für ein Gen oder Gensegment eines HN- oder F-Glykoproteins kodiert, für ein entsprechendes Gen oder Gensegment von HPIV3 substituiert ist.

25. Verfahren nach einem der Ansprüche 16 bis 24, worin das chimäre Genom oder Antigenom mehrere Mutationen inkorporiert, die jeweils einen Phänotyp festlegen, der aus Attenuation, Temperaturempfindlichkeit, Kälteanpassung, Größe der kleinen Plaques oder Einschränkung des Wirtsspektrums ausgewählt ist.

26. Verfahren nach einem der Ansprüche 16 bis 25, worin das für das PIV-Genom oder -Antigenom kodierende Polynucleotidmolekül eine heterologe Sequenz eines respiratorischen Synzytial-Virus oder Masernvirus inkorporiert.

27. Verfahren nach einem der Ansprüche 16 bis 26, worin das für das PIV-Genom oder -Antigenom kodierende Polynucleotidmolekül modifiziert ist, um für ein Nicht-PIV-Molekül zu kodieren, das aus einem Zytokin, einem T-Helfer-Epitop, einem Restriktionsstellenmarker oder einem Protein eines mikrobiellen Pathogens ausgewählt ist, das in der Lage ist, eine schützende Immunreaktion in einem Säugetierwirt hervorzurufen.

28. Infektiöses PIV-Partikel, das ein rekombinantes PIV-Genom oder -Antigenom, ein N-Protein, ein P-Protein und ein L-Protein umfasst, worin das rekombinante PIV-Genom oder -Antigenom aus der aus Seq.-ID Nr. 1, Seq.-ID Nr. 14 und Seq.-ID Nr. 15 oder der komplementären Sequenz davon bestehenden Gruppe ausgewählt ist und durch das Einbringen einer heterologen PIV-Sequenz, die aus einer HPIV1-Sequenz, einer HPIV2-Sequenz oder einer BPIV-Sequenz ausgewählt ist, zur Bildung eines chimären PIV-Genoms oder -Antigenoms modifiziert ist.

29. Infektiöses PIV-Partikel nach Anspruch 28, das ein subvirales Partikel ist.

30. Infektiöses PIV-Partikel nach Anspruch 28, das ein Virus ist.

31. Infektiöses PIV-Partikel nach einem der Ansprüche 28 bis 30, worin das rekombinante PIV-Genom oder -Antigenom eine heterologe Sequenz von RSV oder Masernvirus inkorporiert.

32. Infektiöses PIV-Partikel nach einem der Ansprüche 28 bis 31, worin das rekombinante PIV-Genom oder -Antigenom weiters eine Insertion, Neuanordnung, Deletion oder Substitution eines Nucleotids umfasst, die eine Änderung des Phänotyps festlegt, der aus Attenuation, Temperaturempfindlichkeit, Kälteanpassung, Größe der kleinen Plaques, einer Einschränkung des Wirtsspektrums oder einer Veränderung eines immunogenen Epitops von PIV ausgewählt ist.

33. Infektiöses PIV-Partikel nach einem der Ansprüche 28 bis 32, worin das rekombinante PIV-Genom oder -Antigenom zumindest eine Mutation von JS cp45 inkorporiert.

34. Infektiöses PIV-Partikel nach einem der Ansprüche 28 bis 33, worin eine Mutation, die eine Veränderung des Phänotyps festlegt, der aus Attenuation, Temperaturempfindlichkeit, Kälteanpassung, Größe der kleinen Plaques, einer Einschränkung des Wirtsspektrums oder einer Veränderung eines immunogenen Epitops von PIV ausgewählt ist, in einen chimären PIV-Hintergrund inkorporiert ist, der ein Genom oder Antigenom mit einem oder mehreren PIV3-HN- oder -F-Glykoproteingenen oder -gensegmenten aufweist, die durch ein oder mehrere entsprechende PIV1- oder PIV2-HN- und -F-Glykoproteingene oder -gensegmente substituiert sind.

35. Infektiöses PIV-Partikel nach einem der Ansprüche 28 bis 34, worin das rekombinante PIV-Genom oder -Antigenom modifiziert ist, um für ein Nicht-PIV-Molekül zu kodieren, das aus einem Zytokin, einem T-Helfer-Epitop, einem Restriktionsstellenmarker oder einem Protein eines mikrobiellen Pathogens ausgewählt ist, das in der Lage ist, eine schützende Immunreaktion in einem Säugetierwirt hervorzurufen.

36. Infektiöses PIV-Partikel nach einem der Ansprüche 28 bis 35, worin das chimäre PIV-Genom oder -Antigenom weiters ein Polynucleotid umfasst, das für ein RSV-Antigen oder -Epitop kodiert, das eine schützende Immunreaktion auf RSV in einem immunisierten Wirt hervorruft.

37. Infektiöses PIV-Partikel nach einem der Ansprüche 28 bis 35, worin das chimäre PIV-Genom oder -Antigenom weiters ein Polynucleotid umfasst, das für ein Masernvirusantigen oder -epitop kodiert.

38. Immunogene Zusammensetzung, die eine immunogen wirksame Menge eines infektiösen PIV-Partikels nach einem der Ansprüche 28 bis 37 in einem pharmazeutisch annehmbaren Träger umfasst.

## Revendications

1. Molécule polynucléotidique isolée comprenant un promoteur de la transcription en liaison fonctionnelle, une séquence polynucléotidique codant pour un génome ou un anti-génome de PIV et un terminateur de la transcription, où ladite séquence polynucléotidique codant pour ledit génome ou anti-génome de PIV est sélectionnée dans le groupe consistant en: SEQ ID NO: 1, SEQ ID NO: 14, et SEQ ID NO: 15, ou la séquence complémentaire de celles-ci, et est modifiée par l'introduction d'une séquence de PIV hétérologue sélectionnée parmi une séquence du HPIV1, une séquence du HPIV2, ou une séquence du BPIV, afin de former un génome ou anti-génome de PIV chimère.

2. Molécule polynucléotidique isolée selon la revendication 1, dans laquelle la séquence polynucléotidique codant pour le génome ou l'anti-génome incorpore un gène ou un segment de gène du BPIV.

3. Molécule polynucléotidique isolée selon la revendication 1, qui incorpore une séquence hétérologue issue d'un virus respiratoire syncytial ou d'un virus de la rougeole.

4. Polynucléotide isolé selon la revendication 1, où le polynucléotide codant pour le génome ou l'anti-génome de PIV chimère est en outre modifié par une insertion, un réarrangement, une délétion ou une substitution de nucléotide spécifiant une altération phénotypique sélectionnée parmi une atténuation, une sensibilité à la température, une adaptation au froid, une petite taille de plage, une restriction de gamme d'hôtes, ou une modification dans un épitope immunogène du PIV.

5. Polynucléotide isolé selon l'une quelconque des revendications 1 à 4, dans lequel le génome ou l'anti-génome de PIV code pour une particule de PIV infectieuse.

6. Molécule polynucléotidique isolée selon la revendication 4 ou 5, dans laquelle ladite séquence polynucléotidique codant pour ledit génome ou anti-génome de PIV incorpore une ou plusieurs mutations de JS cp45.

7. Molécule polynucléotidique isolée selon la revendication 4 ou 5, dans laquelle ladite séquence polynucléotidique codant pour ledit génome ou anti-génome de PIV incorpore une mutation stabilisée par de multiples substitutions de nucléotides dans un codon spécifiant la mutation.

8. Molécule polynucléotidique isolée selon la revendication 4 ou 5, dans laquelle ledit génome ou anti-génome chimère incorpore de multiples mutations spécifiant chacune un phénotype sélectionné parmi une atténuation, une sensibilité à la température, une adaptation au froid, une petite taille de plage, ou une restriction de gamme d'hôtes.

9. Molécule polynucléotidique isolée selon la revendication 4 ou 5, dans laquelle une mutation spécifiant une altération phénotypique sélectionnée parmi une atténuation, une sensibilité à la température, une adaptation au froid, une petite taille de plage, une restriction de gamme d'hôtes, ou une modification dans un épitope immunogène du PIV, est incorporée dans un contexte de PIV chimère comprenant un génome ou un anti-génome dans lequel un ou plusieurs gènes ou segments génomiques d'une glycoprotéine HN ou F du PIV3 sont substitués par une ou plusieurs contreparties des gènes ou segments génomiques d'une glycoprotéine HN et F du PIV1 ou PIV2.

10. Molécule polynucléotidique isolée selon la revendication 4 ou 5, qui incorpore une séquence régulatrice agissant en cis du HPIV1, HPIV2, ou BPIV.

11. Molécule polynucléotidique isolée selon l'une quelconque des revendications 4 à 10, qui incorpore une séquence hétérologue issue d'un virus respiratoire syncytial ou d'un virus de la rougeole.

12. Molécule polynucléotidique isolée selon l'une quelconque des revendications précédentes, qui incorpore une séquence polynucléotidique codant pour une molécule non-PIV sélectionnée parmi une cytokine, un épitope de lymphocyte T auxiliaire, un marqueur de site de restriction, ou une protéine d'un agent pathogène microbien capable de provoquer une réponse immunitaire protectrice chez un hôte mammalien.

13. Composition cellulaire ou acellulaire comprenant un vecteur d'expression qui comprend une molécule polynucléotidique isolée codant pour un génome ou un anti-génome de PIV et un vecteur d'expression qui comprend une ou plusieurs molécules polynucléotidiques isolées qui codent pour les protéines N, P et L du PIV, où la molécule polynucléotidique codant pour ledit génome ou anti-génome de PIV est sélectionnée dans le groupe consistant en: SEQ ID NO: 1, SEQ ID NO: 14, et SEQ ID NO: 15, ou la séquence complémentaire de celles-ci, et est modifiée par l'introduction d'une séquence de PIV hétérologue sélectionnée parmi une séquence du HPIV1, une séquence du HPIV2, ou une séquence du BPIV, afin de former un génome ou un anti-génome de PIV chimère, moyennant quoi l'expression dudit génome ou anti-génome de PIV et des protéines N, P, et L produit une particule de PIV infectieuse.

14. Composition cellulaire ou acellulaire selon la revendication 13, dans laquelle la particule de PIV infectieuse est un virus.

15. Composition cellulaire ou acellulaire selon la revendication 13, où la particule de PIV infectieuse est une particule sous-virale.

16. Procédé pour produire une particule de PIV infectieuse à partir d'une ou de plusieurs molécules polynucléotidiques isolées codant pour ledit PIV, consistant à: co-exprimer dans un système cellulaire ou acellulaire un vecteur d'expression qui comprend une molécule polynucléotidique codant pour un génome ou un anti-génome de PIV et un vecteur d'expression qui comprend une ou plusieurs molécules polynucléotidiques codant pour des protéines N, P et L, ce qui produit ainsi une particule de PIV infectieuse, où la molécule polynucléotidique codant pour ledit génome ou anti-génome de PIV est sélectionnée dans le groupe consistant en: SEQ ID NO: 1, SEQ ID NO: 14, et SEQ ID NO: 15, ou la séquence complémentaire de celles-ci, et est modifiée par l'introduction d'une séquence de PIV hétérologue sélectionnée parmi une séquence du HPIV1, une séquence du HPIV2, ou une séquence du BPIV, afin de former un génome ou un anti-génome de PIV chimère.

17. Procédé selon la revendication 16, dans lequel la molécule polynucléotidique qui code pour le génome ou l'anti-génome de PIV est de l'ADNc.

18. Procédé selon la revendication 16, dans lequel la particule de PIV infectieuse est un virus.

19. Procédé selon la revendication 16, dans lequel la particule de PIV infectieuse est une particule sous-virale.

20. Procédé selon l'une quelconque des revendications 16 à 19, dans lequel la molécule polynucléotidique codant pour le génome ou l'anti-génome de PIV chimère est une chimère de séquences du PIV humain et bovin.

21. Procédé selon l'une quelconque des revendications 16 à 20, dans lequel la molécule polynucléotidique codant pour le génome ou l'anti-génome de PIV incorpore en outre une mutation d'atténuation issue d'une souche du PIV dérivée de façon biologique.

22. Procédé selon la revendication 21, dans lequel la molécule polynucléotidique codant pour le génome ou l'anti-génome de PIV incorpore au moins une mutation de JS cp45.

23. Procédé selon la revendication 21 ou 22, dans lequel ladite molécule polynucléotidique codant pour le génome ou l'anti-génome de PIV incorpore une mutation qui est stabilisée par de multiples substitutions de nucléotides dans un codon qui spécifie la mutation.

24. Procédé selon l'une quelconque des revendications 16 à 23, dans lequel une séquence hétérologue du HPIV1 ou du HPIV2 codant pour un gène ou segment de gène d'une glycoprotéine HN ou F est substituée à un gène ou segment de gène correspondant du HPIV3.

25. Procédé selon l'une quelconque des revendications 16 à 24, dans lequel ledit génome ou anti-génome chimère incorpore de multiples mutations spécifiant chacune un phénotype sélectionné parmi une atténuation, une sensibilité à la température, une adaptation au froid, une petite taille de plage, ou une restriction de gamme d'hôtes.

26. Procédé selon l'une quelconque des revendications 16 à 25, dans lequel ladite molécule polynucléotidique codant pour ledit génome ou anti-génome de PIV incorpore une séquence hétérologue issue d'un virus syncytial respiratoire ou d'un virus de la rougeole.

27. Procédé selon l'une quelconque des revendications 16 à 26, dans lequel ladite molécule polynucléotidique codant pour le génome ou l'anti-génome de PIV est modifiée pour coder pour une molécule non-PIV sélectionnée parmi une cytokine, un épitope de lymphocyte T auxiliaire, un marqueur de site de restriction, ou une protéine d'un agent pathogène microbien capable de provoquer une réponse immunitaire protectrice chez un hôte mammalien.

28. Particule de PIV infectieuse qui comprend un génome ou un anti-génome de PIV recombinant, une protéine N, une protéine P, et une protéine L, où le génome ou anti-génome de PIV recombinant est sélectionné dans le groupe consistant en: SEQ ID NO: 1, SEQ ID NO: 14, et SEQ ID NO: 15, ou la séquence complémentaire de celles-ci, et est modifié par l'introduction d'une séquence de PIV hétérologue sélectionnée parmi une séquence du HPIV1, une séquence du HPIV2, ou une séquence du BPIV, afin de former un génome ou un anti-génome de PIV chimère.

29. Particule de PIV infectieuse selon la revendication 28, qui est une particule sous-virale.

30. Particule de PIV infectieuse selon la revendication 28, qui est un virus.

31. Particule de PIV infectieuse selon l'une quelconque des revendications 28 à 30, dans laquelle le génome ou l'anti-génome de PIV recombinant incorpore une séquence hétérologue issue du RSV ou du virus de la rougeole.

32. Particule de PIV infectieuse selon l'une quelconque des revendications 28 à 31, dans laquelle le génome ou l'anti-génome de PIV recombinant comprend en outre une insertion, un réarrangement, une délétion ou une substitution de nucléotide spécifiant une altération phénotypique sélectionnée parmi une atténuation, une sensibilité à la température, une adaptation au froid, une petite taille de plage, une restriction de gamme d'hôtes, ou une modification dans un épitope immunogène du PIV.

33. Particule de PIV infectieuse selon l'une quelconque des revendications 28 à 32, dans laquelle le génome ou l'anti-génome de PIV recombinant incorpore au moins une mutation de JS cp45.

34. Particule de PIV infectieuse selon l'une quelconque des revendications 28 à 33, dans laquelle une mutation spécifiant une altération phénotypique sélectionnée parmi une atténuation, une sensibilité à la température, une adaptation au froid, une petite taille de plage, une restriction de gamme d'hôtes, ou une modification dans un épitope immunogène du PIV, est incorporée dans un contexte de PIV chimère comprenant un génome ou un anti-génome dans lequel un ou plusieurs gènes ou segments de gènes d'une glycoprotéine HN ou F du PIV3 sont substitués par une ou plusieurs contreparties de gènes ou de segments de gènes d'une glycoprotéine HN et F du PIV1 ou PIV2.

35. Particule de PIV infectieuse selon l'une quelconque des revendications 28 à 34, dans laquelle un génome ou un anti-génome de PIV recombinant est modifié pour coder pour une molécule non-PIV sélectionnée parmi une cytokine, un épitope de lymphocyte T auxiliaire, un marqueur de site de restriction, ou une protéine d'un agent pathogène microbien capable de provoquer une réponse immunitaire protectrice chez un hôte mammalien.

36. Particule de PIV infectieuse selon l'une quelconque des revendications 28 à 35, dans laquelle le génome ou l'anti-génome de PIV chimère comprend en outre un polynucléotide codant pour un antigène ou un épitope du RSV qui provoque une immunité protectrice contre le RSV chez un hôte immunisé.

37. Particule de PIV infectieuse selon l'une quelconque des revendications 28 à 35, dans laquelle le génome ou l'anti-génome de PIV chimère comprend en outre un polynucléotide codant pour un antigène ou un épitope du virus de la rougeole.

38. Composition immunogène comprenant une quantité immunologiquement efficace d'une particule de PIV infectieuse selon l'une quelconque des revendications 28 à 37, dans un véhicule pharmaceutiquement acceptable.
